(19) Europäisches Patentamt
European Patent Office
Office européen des brevets

(11) EP 3 494 112 B1

(12) **EUROPÄISCHE PATENTSCHRIFT**

(45) Veröffentlichungstag und Bekanntmachung des Hinweises auf die Patenterteilung:
**20.04.2022 Patentblatt 2022/16**

(21) Anmeldenummer: **17758054.5**

(22) Anmeldetag: **01.08.2017**

(51) Internationale Patentklassifikation (IPC):
***C07D 403/04*** *(2006.01)* ***H01L 51/50*** *(2006.01)*

(52) Gemeinsame Patentklassifikation (CPC):
**C07D 403/04; H01L 51/0053; H01L 51/0067; H01L 51/0072;** H01L 51/5012; Y02E 10/549

(86) Internationale Anmeldenummer:
**PCT/EP2017/069434**

(87) Internationale Veröffentlichungsnummer:
**WO 2018/024725 (08.02.2018 Gazette 2018/06)**

(54) **ORGANISCHE MOLEKÜLE ZUR VERWENDUNG IN ORGANISCHEN OPTOELEKTRONISCHEN VORRICHTUNGEN**

ORGANIC MOLECULES FOR USE IN ORGANIC OPTOELECTRONIC DEVICES

MOLÉCULES ORGANIQUES SYMÉTRIQUES DESTINÉES À ÊTRE UTILISÉES DANS DES DISPOSITIFS ORGANIQUES OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.08.2016 EP 16182886**
**20.09.2016 EP 16189677**
**20.12.2016 EP 16205432**

(43) Veröffentlichungstag der Anmeldung:
**12.06.2019 Patentblatt 2019/24**

(73) Patentinhaber: **cynora GmbH**
**76646 Bruchsal (DE)**

(72) Erfinder:
- **SEIFERMANN, Stefan**
  **77815 Bühl (DE)**
- **DANZ, Michael**
  **76344 Eggenstein-Leopoldshafen (DE)**
- **LIAPTSIS, Georgios**
  **68161 Mannheim (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2016/042070 WO-A1-2016/046034**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 494 112 B1

## Beschreibung

Die Erfindung betrifft rein organische Moleküle und deren Verwendung in organischen lichtemittierenden Dioden (OLEDs) und in anderen organischen optoelektronischen Vorrichtungen.

## Hintergrund

Organische optoelektronische Vorrichtungen zeichnen sich dadurch aus, dass entweder elektrische Energie in Photonen umgewandelt wird (Organische Lichtemittierende Dioden, OLED oder Lichtemittierende Elektrochemische Zellen, LEEC) oder der umgekehrte Prozess abläuft (Organische Photovoltaik, OPV). Dabei ist es wichtig, dass diese Prozesse möglichst effizient ablaufen. Für den Bereich von OLEDs müssen daher idealerweise Materialien mit möglichst hoher photolumineszenter Quantenausbeute verwendet werden. Begrenzte Effizienzen von OLED-Materialien können durch Verwendung effizienter Materialien, die thermisch aktivierte verzögerte Fluoreszenz (TADF) zeigen, verbessert werden, da im Gegensatz zu rein fluoreszenten Materialien statt 25 % der in einer OLED gebildeten Exzitonen bis zu 100 % der Exzitonen genutzt werden können. Hierbei können auch die entstandenen Triplett-Excitonen in Singulett-Excitonen überführt werden, aus welchem Zustand dann Photonen emittiert werden können. Voraussetzung für eine solche thermische Rückbesetzung ist dabei ein geringer energetischer Abstand zwischen dem niedrigsten angeregten Singulett-(S1) und Triplett-Niveau (T1). Dies kann beispielweise durch Verwendung von Kupfer-(I)-Komplexen (siehe hierzu z. B.: H. Yersin, U. Monkowius, T. Fischer, T. Hofbeck, WO 2010/149748 A1) aber auch durch rein organische Materialien (siehe hierzu z. B.: Q. Zhang et al., J. Am. Chem. Soc. 2012, 134, 14706, WO 2013161437 A1) erreicht werden. WO 2016/042070 hat organische Moleküle, geeignet zur Verwendung in OLEDs, zum Gegenstand, welche aus einem Carbazolteil und einem Phthalimidteil bestehen, welcher (hetero)aromatisch substituierte Phenyl-Gruppen aufweist.

Es gibt weiterhin einen großen Bedarf an neuen Materialien, insbesondere an tiefblauen TADF-OLEDs. Bestehende blaue TADF-Materialien zeigen oft hohe Exzitonenlebensdauern, welche schlecht für effiziente und langlebige OLEDs sind. Neben den erwähnten Eigenschaften der Materialien ist für eine Kommerzialisierung ebenso die Zugänglichkeit relevant. Dies schließt die Verfügbarkeit von Synthesebausteinen, als auch den Aufwand für die eigentliche Synthese des funktionellen Materials, insbesondere dessen Aufreinigung mit ein.

## Beschreibung

Der vorliegenden Erfindung lag die Aufgabe zu Grunde, Moleküle bereitzustellen, die sich zur Verwendung als Emittermaterialien in OLEDs eignen.

Überraschenderweise wurden organische Moleküle gefunden, welche hohe Quantenausbeuten und geringe Exzitonenlebensdauern aufweisen.

Die Erfindung betrifft in einem ersten Aspekt organische Moleküle aufweisend

- eine erste chemische Einheit aufweisend eine oder bestehend aus einer Struktur der Formel A1

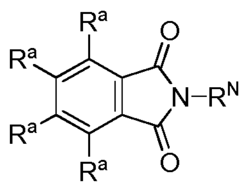

**Formel A1**

und

- mindestens eine zweite chemische Einheit aufweisend eine oder bestehend aus einer Struktur der Formel D1

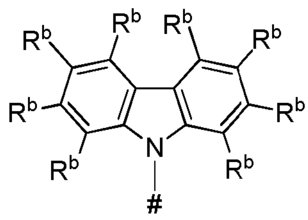

**Formel D1**

mit

# = Anknüpfungspunkt der zweiten chemischen Einheit an die erste chemische Einheit;

$R^N$ ist eine Struktur der Formel N1:

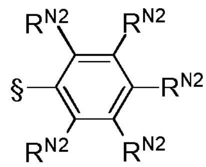

**Formel N1**

mit

§ = Anknüpfungspunkt der chemischen Einheit $R^N$ gemäß Formel N1 an die erste chemische Einheit;

$R^{N2}$ ist bei jedem Auftreten gleich oder verschieden H, Phenyl oder Naphtyl;

$R^a$ ist bei jedem Auftreten gleich oder verschieden ausgewählt aus der Gruppe bestehend aus einem Anknüpfungspunkt der ersten chemischen Einheit an die zweite chemische Einheit und H;

$R^b$ ist bei jedem Auftreten unabhängig voneinander ausgewählt aus der Gruppe bestehend aus H, Deuterium, $CF_3$, $C(=O)R^1$, CN, unsubstituiertes oder mit einem oder mehreren $R^2$ substituiertes Carbazolyl, $Si(R^4)_3$, einer unsubstituierten oder mit einem oder mehreren $R^2$ substituierten Alkylgruppe, einer unsubstituierten oder mit einem oder mehreren $R^2$ substituierten Arylgruppe und einer unsubstituierten oder mit einem oder mehreren $R^2$ substituierten Heteroarylgruppe;

$R^1$ ist bei jedem Auftreten unabhängig voneinander eine unsubstituierte oder mit einem oder mehreren $R^3$ substituierte Arylgruppe;

$R^2$ ist bei jedem Auftreten unabhängig voneinander ausgewählt aus der Gruppe bestehend aus $CF_3$, $C(=O)R^1$, CN, einer unsubstituierten oder mit einem oder mehreren $R^3$ substituierten Alkylgruppe, einer unsubstituierten oder mit einem oder mehreren $R^3$ substituierten Arylgruppe und einer unsubstituierten oder mit einem oder mehreren $R^3$ substituierten Heteroarylgruppe;

$R^3$ ist bei jedem Auftreten unabhängig voneinander ausgewählt aus der Gruppe bestehend aus einer unsubstituierten Alkylgruppe, einer unsubstituierten Arylgruppe und einer unsubstituierten Heteroarylgruppe;

$R^4$ ist bei jedem Auftreten unabhängig voneinander ausgewählt aus der Gruppe bestehend aus, einer unsubstituierten oder mit einem oder mehreren $R^3$ substituierten Arylgruppe und einer unsubstituierten oder mit einem oder mehreren $R^3$ substituierten Heteroarylgruppe;

dabei können zwei oder mehrere der Substituenten $R^1$, $R^2$ und $R^3$ miteinander ein monocyclisches oder polycyclisches, aliphatisches oder aromatisches und/oder benzoanelliertes Ringsystem bilden;

wobei mindestens ein und maximal drei $R^{N2}$ Phenyl oder Naphtyl ist.

In einer Ausführungsform weist das organische Molekül eine Struktur der Formel A2 auf oder besteht aus einer Struktur der Formel A2.

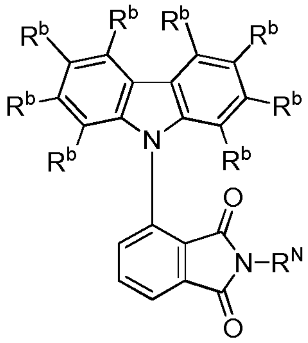

**Formel A2**

wobei die oben angegebenen Definitionen gelten.

In einer Ausführungsform weist das organische Molekül eine Struktur der Formel A3 auf oder besteht aus einer Struktur der Formel A3.

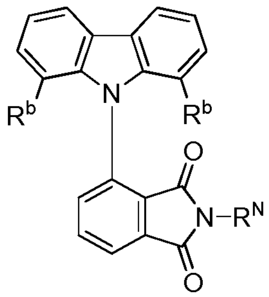

**Formel A3**

wobei die oben angegebenen Definitionen gelten.

In einer Ausführungsform weist das organische Molekül eine Struktur der Formel A4 auf oder besteht aus einer Struktur der Formel A4.

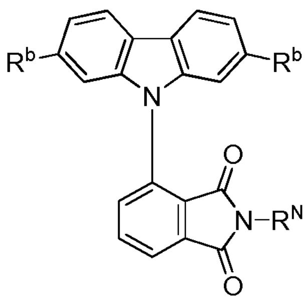

**Formel A4**

wobei die oben angegebenen Definitionen gelten.

In einer Ausführungsform weist das organische Molekül eine Struktur der Formel A5 auf oder besteht aus einer Struktur der Formel A5.

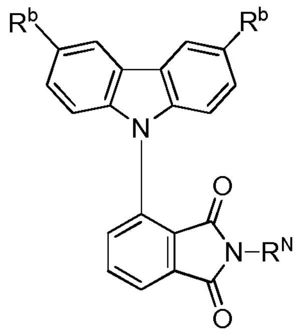

**Formel A5**

wobei die oben angegebenen Definitionen gelten.

In einer Ausführungsform weist das organische Molekül eine Struktur der Formel A3, A4 oder A5 auf mit $R^b$ bei jedem Auftreten unabhängig voneinander ausgewählt aus der Gruppe bestehend aus Deuterium, $CF_3$, $C(=O)R^1$, CN, unsubstituiertes oder mit einem oder mehreren $R^2$ substituiertes Carbazolyl, $Si(R^4)_3$, einer unsubstituierten oder mit einem oder mehreren $R^2$ substituierten Alkylgruppe, einer unsubstituierten oder mit einem oder mehreren $R^2$ substituierten Arylgruppe und einer unsubstituierten oder mit einem oder mehreren $R^2$ substituierten Heteroarylgruppe.

In einer Ausführungsform des organischen Moleküls ist $R^N$ bei jedem Auftreten gleich oder verschieden ausgewählt aus einer der Strukturen O1 bis O10:

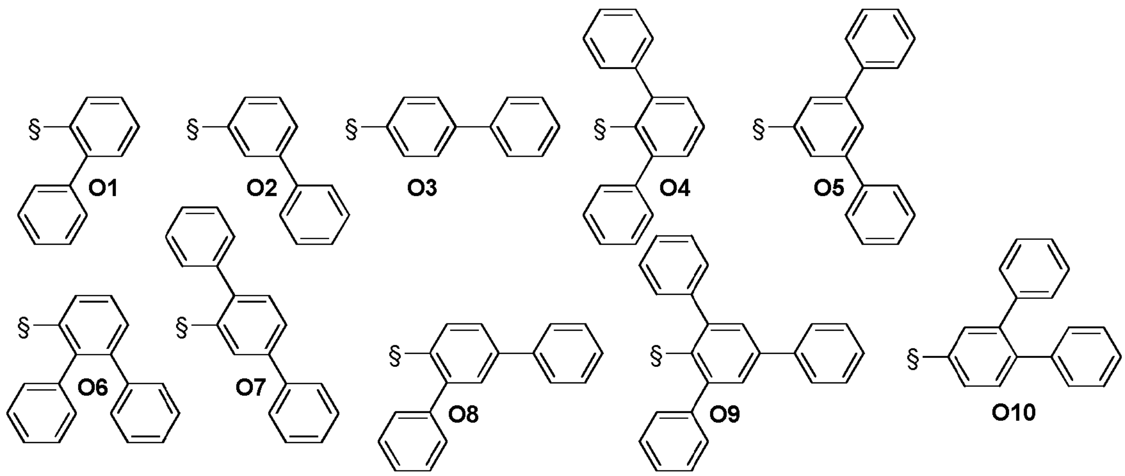

O1 O2 O3 O4 O5 O6 O7 O8 O9 O10

wobei die oben angegebenen Definitionen gelten.

In einer Ausführungsform des organischen Moleküls ist $R^N$ bei jedem Auftreten gleich oder verschieden ausgewählt aus einer der Strukturen P1 bis P3:

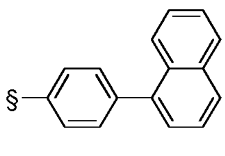 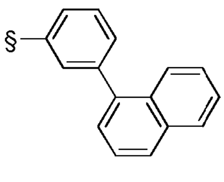 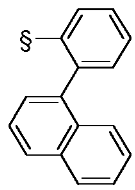

P1 P2 P3

wobei die oben angegebenen Definitionen gelten.

In einer Ausführungsform des organischen Moleküls ist $R^b$ bei jedem Auftreten gleich oder verschieden ausgewählt aus der Gruppe bestehend aus H, Deuterium, Methyl, iso-Propyl, tertiär-Butyl, $CF_3$, CN, einer unsubstituierten oder mit einem oder mehreren $R^6$ substituierten Phenylgruppe, einer unsubstituierten oder mit einem oder mehreren $R^6$ substituierten Pyridingruppe, einer unsubstituierten oder mit einem oder mehreren $R^6$ substituierten Pyrimidingruppe,

einer unsubstituierten oder mit einem oder mehreren $R^6$ substituierten Pyrazingruppe und einer unsubstituierten oder mit einem oder mehreren $R^6$ substituierten Triazingruppe.

$R^6$ ist bei jedem Auftreten gleich oder verschieden ausgewählt aus der Gruppe bestehend aus Deuterium, Methyl, iso-Propyl, tertiär-Butyl, $CF_3$, CN und Phenyl.

Das organische Molekül der Erfindung kann auch eine Struktur der Formeln A6, A7 oder A8 aufweisen bzw. aus einer Struktur der Formeln A6, A7 oder A8 bestehen (wobei die darin vorkommenden Variablen oben definiert sind).

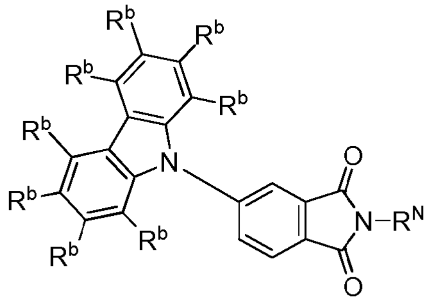

**Formel A6**

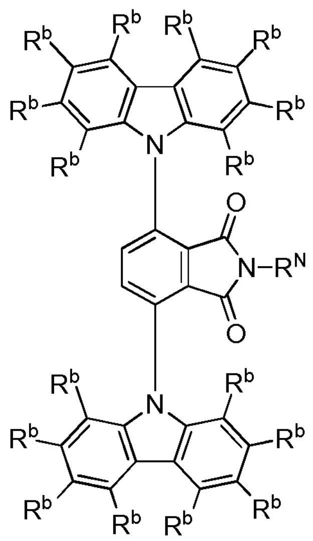

**Formel A7**

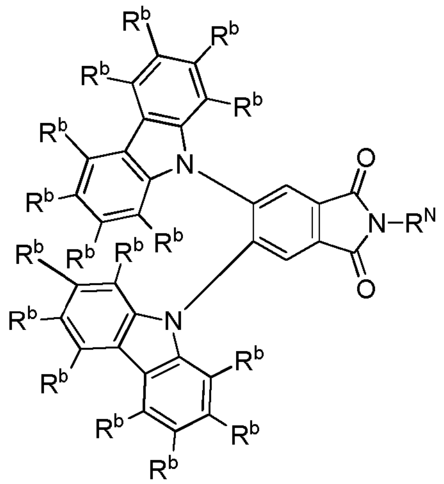

**Formel A8**

Im Sinne dieser Erfindung enthält eine Arylgruppe 6 bis 60 aromatische Ringatome; eine Heteroarylgruppe enthält 5 bis 60 aromatische Ringatome, von denen mindestens eines ein Heteroatom darstellt. Die Heteroatome sind insbesondere N, O und/oder S. Werden in der Beschreibung bestimmter Ausführungsformen der Erfindung andere, von der genannten Definition abweichende Definitionen angegeben, beispielsweise bezüglich der Zahl der aromatischen Ringatome oder der enthaltenen Heteroatome, so gelten diese.

Unter einer Arylgruppe bzw. Heteroarylgruppe wird ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (anellierter) aromatischer bzw. heteroaromatischer Polycyclus besteht im Sinne der vorliegenden Erfindung aus zwei oder mehr miteinander kondensierten einfachen aromatischen bzw. heteroaromatischen Cyclen.

Unter einer Aryl- oder Heteroarylgruppe, die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen der genannten Gruppen.

Unter einer cyclischen Alkyl-, Alkoxy- oder Thioalkoxygruppe wird hier eine monocyclische, eine bicyclische oder eine polycyclische Gruppe verstanden.

Im Rahmen der vorliegenden Erfindung werden unter einer Alkylgruppe beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Die erfindungsgemäßen organischen Moleküle weisen hohe Photolumineszenz Quantenausbeuten und geringe Exzitonenlebensdauern auf und stellen daher vorteilhafte Emittermaterialien für blaue, himmelblaue oder grüne OLEDs dar.

Eine Ausführungsform der Erfindung betrifft organische Moleküle, welche ein Emissionsmaximum bei zwischen 400 und 500 nm, insbesondere zwischen 420 und 481 nm, bevorzugter zwischen 430 und 470 nm, noch bevorzugter zwischen 440 und 460 nm aufweisen.

Insbesondere zeigen die erfindungsgemäßen organische Moleküle einen $\Delta E(S_1-T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ und/oder eine Emissionslebensdauer von höchsten 50 $\mu$s, insbesondere von höchstens 20 $\mu$s, oder von höchstens 10 $\mu$s, bevorzugt höchstens 5 $\mu$s und/oder eine Hauptemissionsbande mit einer Halbwertsbreite kleiner als 120 nm, insbesondere kleiner als 100 nm, kleiner als 80 nm, oder kleiner als 60 nm und/oder eine Photolumineszenz-Quantenausbeute (PLQY) von größer 50 %, von größer 55 %, von größer 60 %, von größer 65 %, von größer 70 %, oder von größer 80 %.

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas-und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und

(b) mindestens ein, d. h. ein oder mehrere Emitter- und/oder Hostmaterialien, die von dem erfindungsgemäßen organischen Molekül verschiedenen ist bzw. sind und

(c) optional eine oder mehreren Farbstoffen und/ oder einem oder mehreren organischen Lösungsmitteln.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett ($T_1$)- und Singulett ($S_1$)- Energieniveaus auf, die energetisch höher liegen als die Triplett ($T_1$)- und Singulett ($S_1$)-Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das jeweilige des elektronendominanten Hostmaterials. Das HOMO des lochdominanten Hostmaterials liegt energetisch unter dem HOMO des erfindungsgemäßen organischen Moleküls, während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.

In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.

Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt einen weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)
2. Kathode
3. Elektroneninjektionsschicht (electron injection layer, EIL)
4. Elektronenleitschicht (electron transport layer, ETL)
5. Lochblockierschicht (hole blocking layer, HBL)
6. Emissionsschicht bzw. Emitterschicht (emitting layer, EML)
7. Elektronenblockierschicht (electron blocking layer, EBL)

8. Lochtransportschicht (hole transport layer, HTL)

9. Lochinjektionsschicht (hole injection layer, HIL)

10. Anode

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

In einer Ausführungsform wird bei der invertierten OLED die Anodenschicht des typischen Aufbaus, z.B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen.

Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm. Des Weiteren können kleine Moleküle verwendet werden (z. B. Kupfer-Phthalocyanin (CuPc z. B. 10 nm dick)) oder Metalloxide wie beispielhaft $MoO_3$, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N,N*-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10 nm bis100 nm.

Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10 nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan) oder DPEPO (Bis[2-((oxo)diphenylphosphino)phenyl]ether). Für im Grünen oder im Roten emittierendes Emittermaterial oder eine Mischung aufweisend mindestens zwei Emittermaterialien eignen sich die gängigen Matrixmaterialien wie CBP. Für im Blauen emittierendes Emittermaterial oder eine Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), Nbphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, BPyTP2 (2,7-Di(2,2'-bipyridin-5-yl)triphenyl) ), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Eine Ausführungsform der Erfindung betrifft organische optoelektronische Vorrichtungen, welche eine externe Quanteneffizienz (EQE) bei 1000 cd/$m^2$ von größer 5 %, insbesondere von größer 8 %, insbesondere von größer 10 %, oder von größer 13 %, oder von größer 16 % und insbesondere von größer 20 % und/oder ein Emissionsmaximum bei einer Wellenlänge zwischen 420 nm und 500 nm, insbesondere zwischen 430 nm und 490 nm, oder zwischen 440 nm und 480 nm und insbesondere zwischen 450 nm und 470 nm und/oder einen LT80 Wert bei 500 cd/$m^2$ von größer 30 h, insbesondere von größer 70 h, oder von größer 100 h, oder von größer 150 h und insbesondere von größer 200 h aufweisen.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht weist in einer Ausführungsform ausschließlich ein erfindungsgemäßes organisches Molekül in 100 % Konzentration auf, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierende Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein oder mehrere Hostmaterialen aufweist, deren Triplett (T1)- und Singulett (S1)-Energieniveaus energetisch höher liegen als die Triplett (T1)- und Singulett (S1)-Energieniveaus des organischen Moleküls, wobei die Anode und die Kathode auf das Substrat aufgebracht ist, und die löcher- und elektroneninjizierende Schicht

zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

**Beispiele**

**Allgemeine Vorschriften**

AAV1:

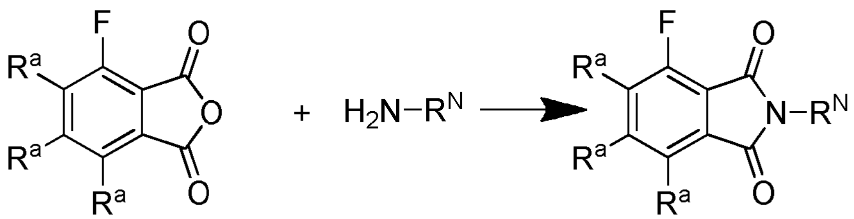

**E1** **A1** **E2**

In einem Rundkolben mit Rückflusskühler wird **E1** (1 Äquivalent) in Eisessig suspendiert. Nach Zugabe von **A1** (1,1 Äquivalente) wird 3 Stunden bei 100 °C gerührt. Nach abkühlen wird die Reaktionslösung am Rotationsverdampfer soweit möglich eingeengt. Der Rückstand wird in $CH_2Cl_2$ aufgenommen und zweimal mit ges. $Na_2CO_3$ gewaschen. Die vereinigten organischen Phasen werden über $MgSO_4$ getrocknet. Das Lösungsmittel wird am Rotationsverdampfer entfernt. Nach Trocknen am Hochvakuum wird **E2** als Produkt erhalten, welches in der Regel ohne weitere Aufreinigung eingesetzt werden kann. Bei Bedarf kann das Produkt **E2** durch Umkristallisation weiter aufgereinigt werden.

**Beispielhafte A1 und E2 Kombinationen**

A1 | E2

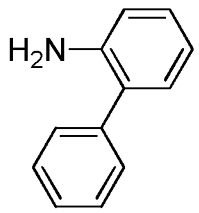
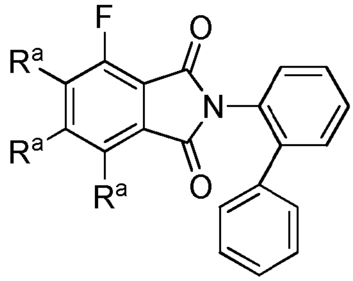
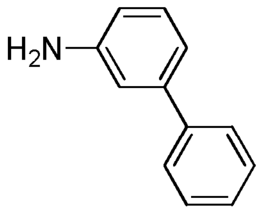
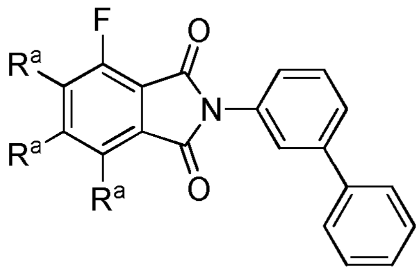
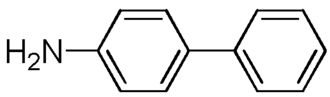
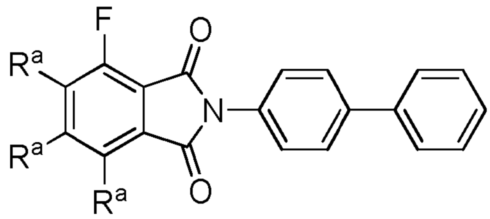
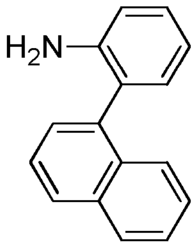
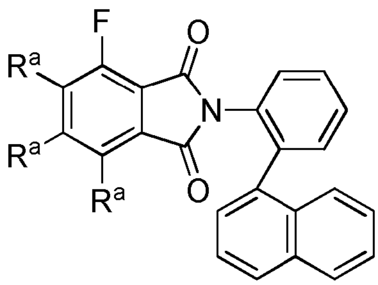
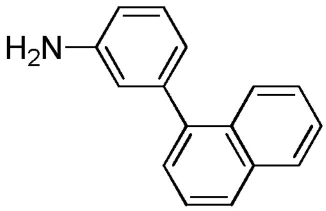
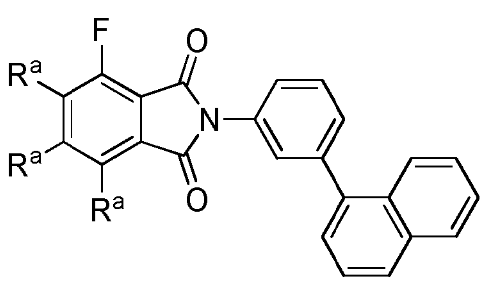
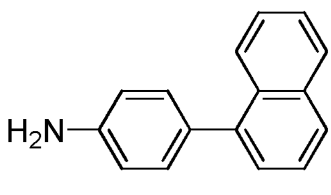
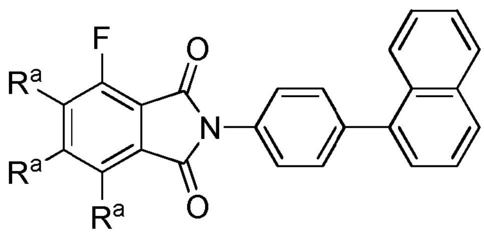

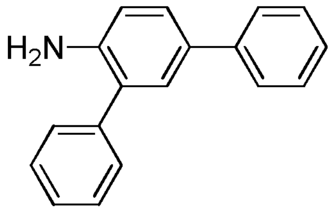

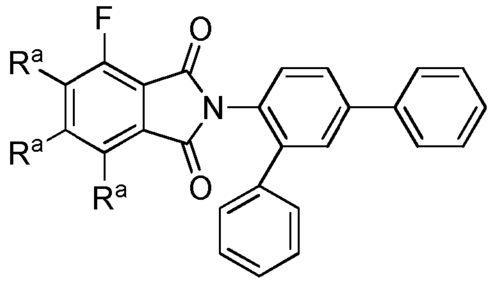

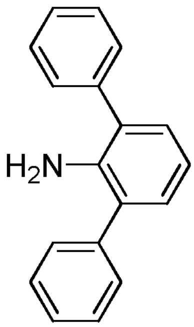

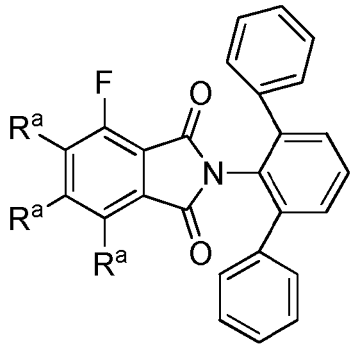

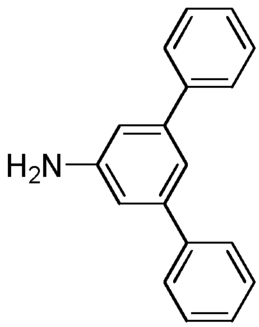

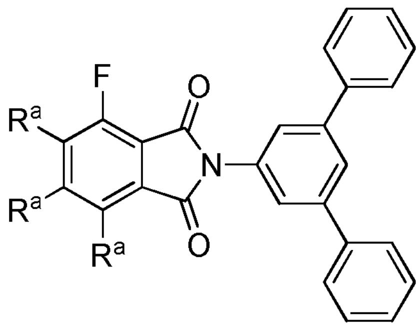

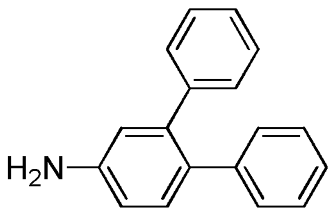

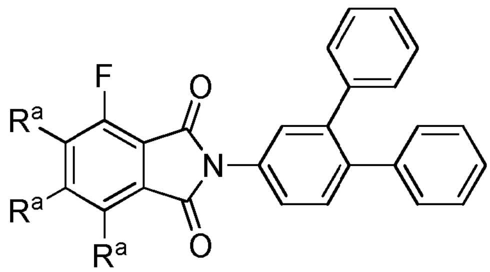

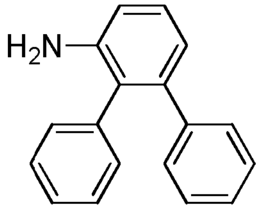

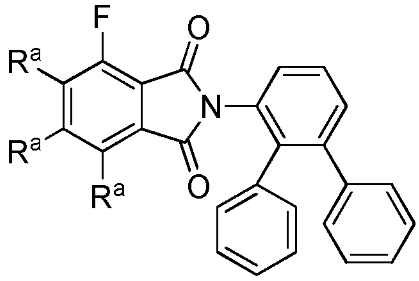

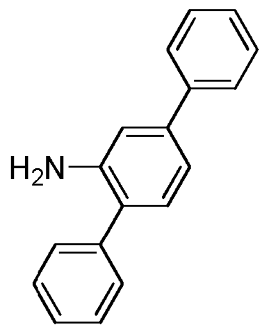

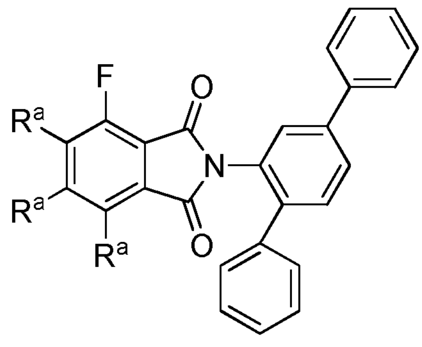

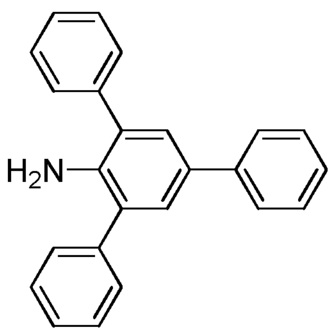

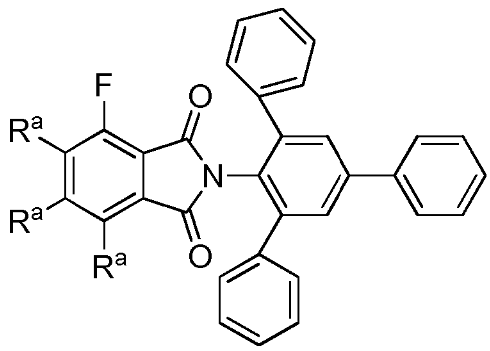

**AAV2:**

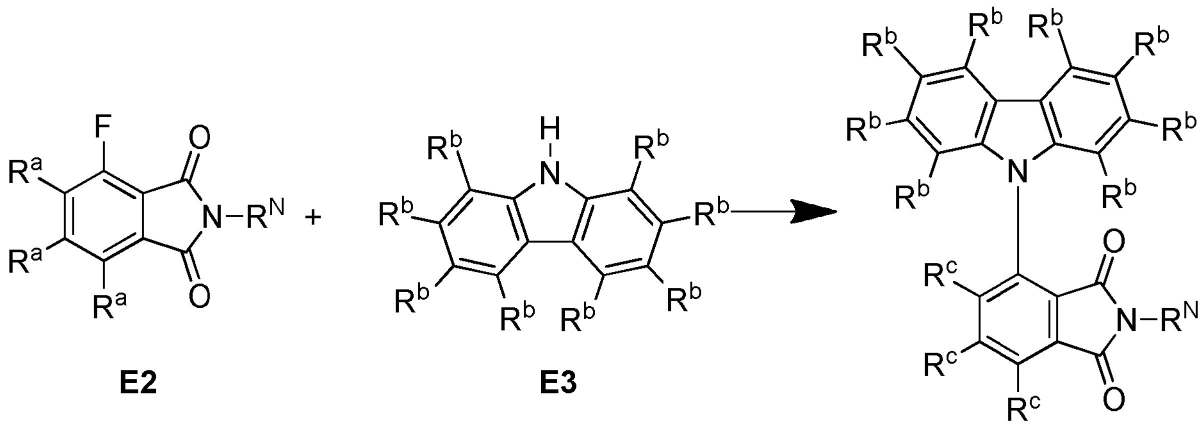

E2 E3

In einem Rundkolben werden Phthalimid **E2** (1 Äquivalent), ein Carbazol-Derivat **E3** (1 Äquivalent) und $K_3PO_4$ (2 Äquivalente) vorgelegt und für 5 min evakuiert. Unter Inertgasatmosphäre wird DMSO (trocken) zugegeben und die Reaktionslösung für 16 h bei 100 °C gerührt. Nach abkühlen wird die Reaktionslösung auf Wasser geschüttet und mit $CH_2Cl_2$ extrahiert. Nach erneuter Extraktion mit $CH_2Cl_2$ werden die vereinigten organischen Phasen 2x mit Wasser und mit gesättigter NaCl-Lösung gewaschen. Anschließend wird über $MgSO_4$ getrocknet und das Lösemittel am Rotationsverdampfer entfernt. Das jeweilige Produkt kann durch Umkristallisation gereinigt werden.

**AAV3:**

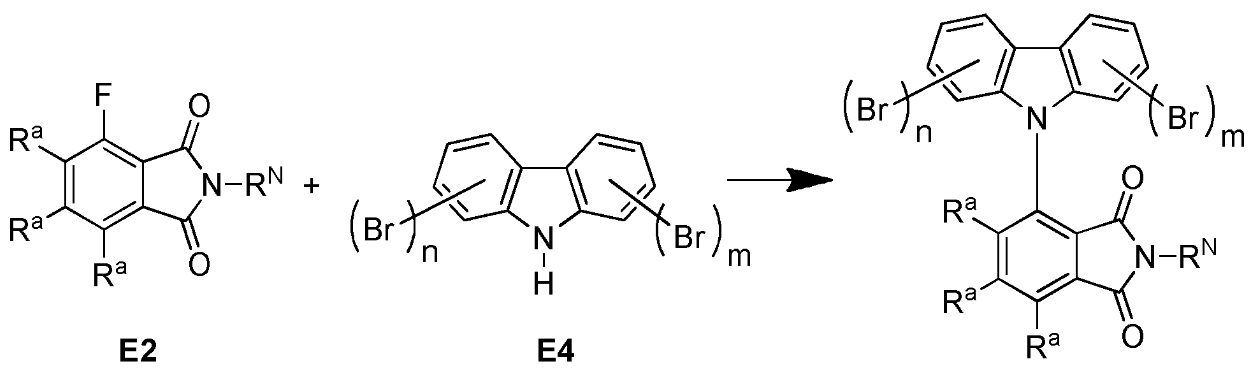

E2 E4 E5

n = 1 bis 4, m = 0 bis 4

In einem Rundkolben werden **E2** (1,2 Äquivalente), ein Brom-substituiertes Carbazol **E4** (1 Äquivalent) und $K_3PO_4$ (2 Äquivalente) vorgelegt und für 5 min evakuiert. Unter Inertgasatmosphäre wird DMSO (trocken) zugegeben und die Reaktionslösung für 16 h bei 100 °C gerührt. Nach abkühlen wird die Reaktionslösung auf Wasser geschüttet und mit $CH_2Cl_2$ extrahiert. Nach erneuter Extraktion mit $CH_2Cl_2$ werden die vereinigten organischen Phasen 2x mit Wasser und mit gesättigter NaCl-Lösung gewaschen. Anschließend wird über $MgSO_4$ getrocknet und das Lösemittel am Rotationsverdampfer entfernt. Das jeweilige Produkt kann durch Umkristallisation gereinigt werden.

Erfindungsgemäß kann statt Brom-substituiertem Carbazol auch Chlor- oder Iodsubstituiertes Carbazol verwendet werden.

**Beispielhafte E4 und E5 Kombinationen**

| E4 | E5 |
|---|---|
| 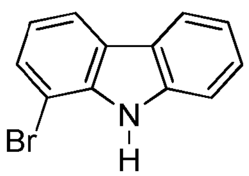 | 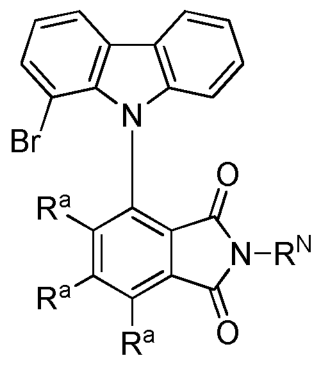 |
| 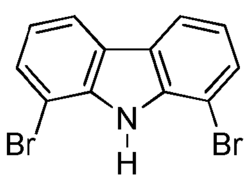 | 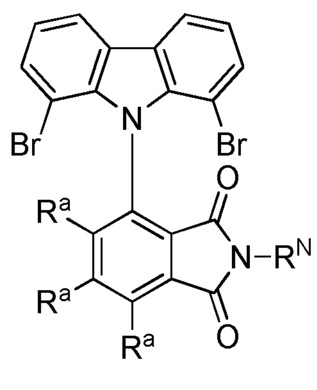 |

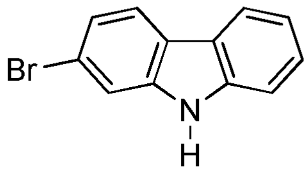

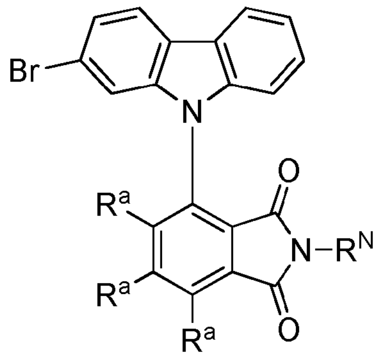

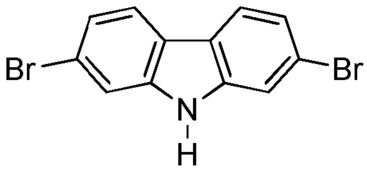

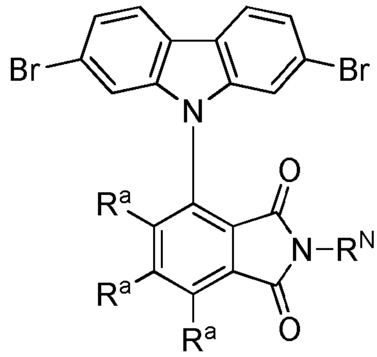

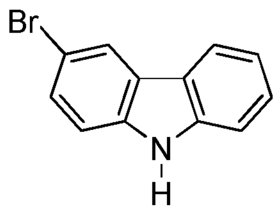

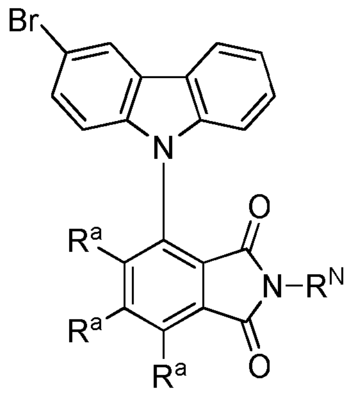

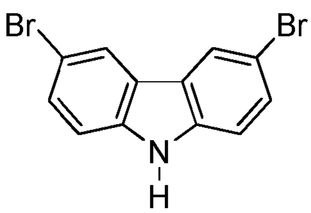

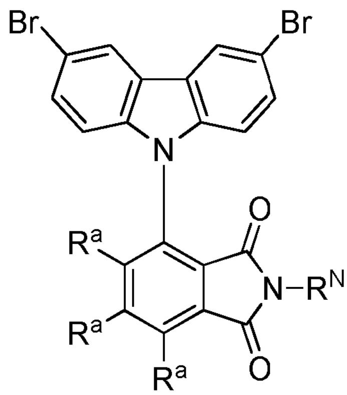

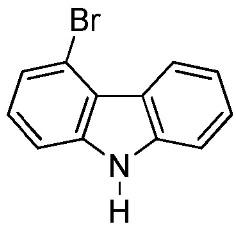

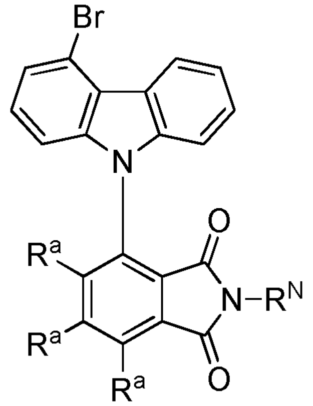

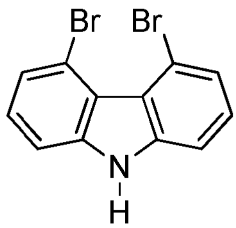

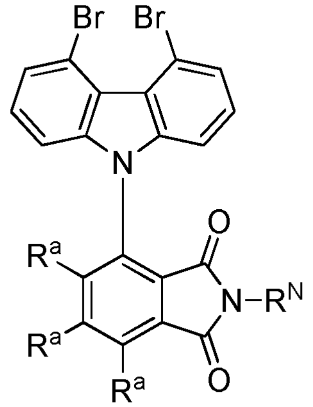

**AAV4:**

**Stufe 1**

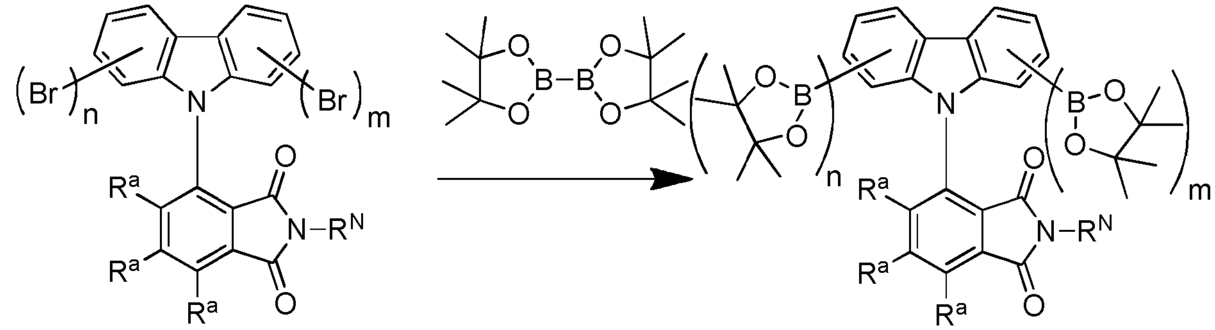

**E5** **E6**

**E5** (1,00 Äquivalente), Bis(pinacolato)diboron (1,5 (n+m) Äquivalente), Tris(dibenzylideneacetone)dipalladium (0,01 Äquivalente), 2-(Dicyclohexylphosphino)-2',4',6'-triisopropylbiphenyl (0,04 Äquivalente) und tribasisches Kaliumphosphat (3n + 3m Äquivalente) werden unter Stickstoff in Dioxan für 12 h bis 24 h bei 110 °C gerührt. Das erhaltene Rohprodukt kann durch Umkristallisation gereinigt werden.

**Stufe 2:**

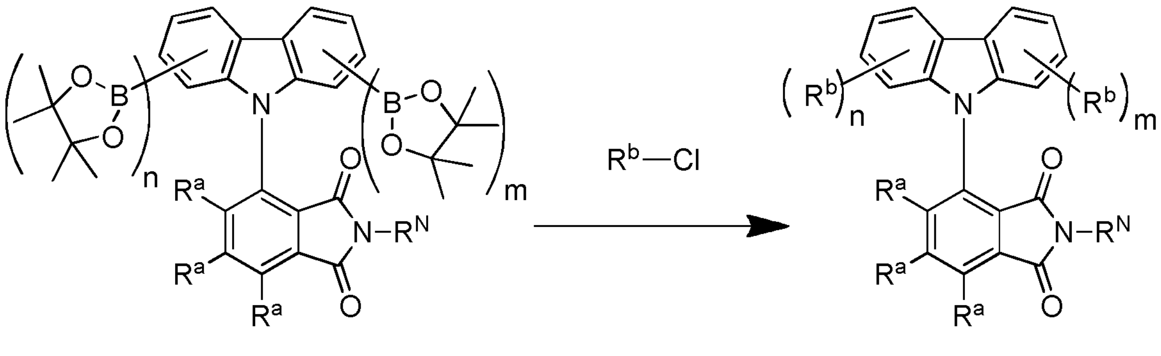


E6

**E6** (1,00 Äquivalente), $R^b$-Cl (1,3n + 1,3m Äquivalente), Tris(dibenzylideneacetone)dipalladium (0,01 Äquivalente), 2-(Dicyclohexylphosphino)-2',4',6'-triisopropylbiphenyl (0,04 Äquivalente) und tribasisches Kaliumphosphat (2,5n + 2,5m Äquivalente) werden unter Stickstoff in einem Toluol/ Wasser (10:1) Gemisch für 12 h bis 24 h bei 100 °C gerührt. Das erhaltene Rohprodukt wird durch Flash-Chromatographie oder durch Umkristallisation gereinigt.

Erfindungsgemäß kann statt $R^b$-Cl auch $R^b$-Br oder $R^b$-I verwendet werden.

**AAV5:**

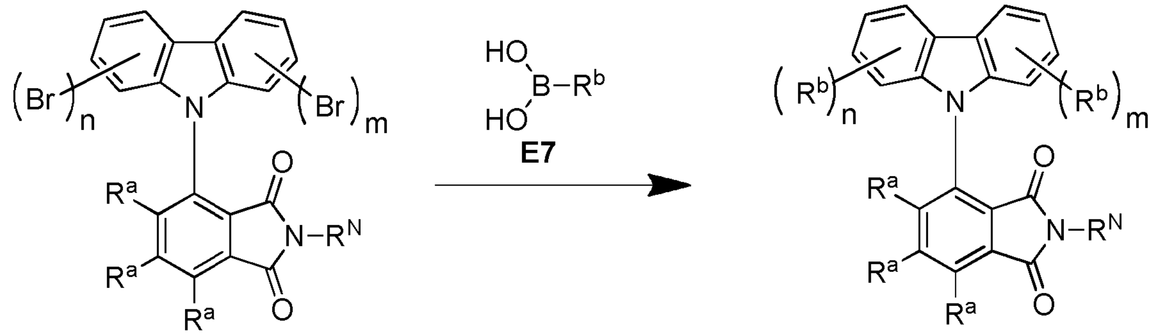


E5

**E5** (1,00 Äquivalente), die entsprechende Boronsäure des Restes $R^b$ E7 (1,3n + 1,3m Äquivalente), Tris(dibenzylideneacetone)dipalladium (0,01 Äquivalente), 2-(Dicyclohexylphosphino)-2',4',6'-triisopropylbiphenyl (0,04 Äquivalente) und tribasisches Kaliumphosphat (3n + 3m Äquivalente) werden unter Stickstoff in Dioxan/Wasser (10:1) für 12 h bis 24 h bei 110 °C gerührt. Das erhaltene Rohprodukt wird durch Flash-Chromatographie oder durch Umkristallisation gereinigt.

Erfindungsgemäß kann statt einer Boronsäure auch ein entsprechender Boronsäureester verwendet werden.

Die erfindungsgemäßen Moleküle können jeweils gemäß der AAV2 oder einer Kombination von AAV3 und AAV4 oder AAV3 und AAV5 erhalten werden. Die Produkte der Synthesewege unterscheiden sich jeweils lediglich durch die erhaltenen Ausbeuten bzw. Reinheit vor der Aufreinigung. Nach entsprechender Aufreinigung sind die Produkte von äquivalenter Qualität.

**Berechnungen nach der Dichtefunktionaltheorie**

Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D., J.Chem.Phys. 98 (1993) 5648-5652; Lee, C; Yang, W; Parr, R.G. Phys. Rev. B 37 (1988) 785-789) zum Einsatz kam. Anregungsenergien wurden bei der mit BP86 optimierten Struktur mit der Time-Dependent DFT-Methode (TD-DFT) unter Verwendung des B3LYP-Funktionals (Becke, A.D., J.Chem.Phys. 98 (1993) 5648-5652; Lee, C; Yang, W; Parr, R.G. Phys. Rev. B 37 (1988) 785-789; Vosko, S. H.; Wilk, L.; Nusair, M. Can. J. Phys. 58 (1980) 1200-1211; Stephens, P. J.; Devlin, F. J.; Chabalowski,

C. F.; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) und ein m4 -Grid zur numerischen Integration verwendet. Alle DFT-Rechnungen wurden mit dem Turbomole-Programmpaket (Version 6.5) (TURBOMOLE V6.4 2012, eine Entwicklung der Universität Karlsruhe und Forschungszentrum Karlsruhe GmbH, 1989-2007, TURBOMOLE GmbH, seit 2007; http://www.turbomole.com) durchgeführt.

Die in den Figuren dargestellten Grenzorbitale wurden mit dem B3LYP-Funktional für die optimierten Grundzustandsgeometrien berechnet.

**Photophysikalische Messungen**

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

***Probenvorbereitung: Lösungen***

1-2 mg der Probe wurden in 100 ml des jeweiligen Lösemittels gelöst, Konzentration $10^{-5}$ mol/L. Die Küvette wurde luftdicht verschlossen und 10 min. entgast.

***Probenvorbereitung, Film: Spin-Coating*** (Gerät: Spin150, SPS euro.)

Die Probenkonzentration entsprach 10mg/ml, angesetzt in Toluol oder Chlorbenzol. Programm: 1) 3 s bei 400 U/min; 2) 20 Sek. bei 1000 U/min bei 1000 Upm/ s. 3) 10 s bei 4000 U/min bei 1000 Upm/ s. Die Filme wurden nach dem Beschichten für 1 min bei 70 °C an Luft auf einer Präzisionsheizplatte von LHG getrocknet.

***Absorptionsspektroskopie***

Lösungen: UV-VIS-Spektren wurden auf einem Gerät der Firma Thermo Scientific, Modell Evolution 201 aufgenommen. (Siehe Probenvorbereitung: Lösungen)

Film: UV-VIS-Spektren wurden auf einem Gerät der Firma Thermo Scientific, Modell Evolution 201 aufgenommen. (Siehe Probenvorbereitung, Film: Spin-Coating)

***Photolumineszenzspektroskopie und TCSPC***

Steady-state Emissionsspektroskopie wurde mit einem Fluoreszenzspektrometer der Firma Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer TCSPC-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen: NanoLED 370 (Wellenlänge: 371 nm, Pulsdauer: 1,1 ns), NanoLED 290 (Wellenlänge: 294 nm, Pulsdauer: <1 ns), SpectraLED 310 (Wellenlänge: 314 nm), SpectraLED 355 (Wellenlänge: 355 nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$: Durch den Fit vorhergesagte Größe und $o_i$: Gemessenen Größe.

***Quanteneffizienzbestimmung***

Die Messung der Photolumineszenzquantenausbeute (PLQY) erfolgte mittels eines *Absolute PL Quantum*

*Yield Measurement C9920-03G*-Systems der Firma *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0

Für G9920-OXG (PMA-12). Das Emissionsmaximum wird in nm, die Quantenausbeute Φ in % und die CIE-Farbkoordinaten als x,y-Werte angegeben.

Die PLQY wurde für Polymerfilme, Lösungen und Pulverproben nach folgendem Protokoll bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorptionsmaximum des organischen Moleküls bestimmt und mit diesem angeregt.
3) Durchführung der Probenmessung: Es wurde von entgasten Lösungen und Filmen unter Stickstoff-Atmosphäre die absolute Quantenausbeute bestimmt. Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

## Herstellung und Charakterisierung von organischen Elektrolumineszenzvorrichtungen aus der Gasphase

Mit den erfindungsgemäßen organischen Molekülen können OLED-Devices mittels Vakuum-Sublimationstechnik erstellt werden. Enthält eine Schicht mehrere Komponenten, so ist das Verhältnis dieser in Massenprozent angegeben.

Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, und dem Strom aufgenommen. Aus dem zeitlichen Verlauf der Elektrolumineszenzspektren kann die Lebensdauer der OLEDs bestimmt werden. Der angegebene LT50-Wert entspricht hierbei der Zeit, bei der die Leuchtdichte auf 50 % des Startwertes abgefallen ist. Analog entspricht der LT70-Wert der Zeit, bei der die Leuchtdichte auf 70 % des Startwertes abgefallen ist.

## Beispiel 1

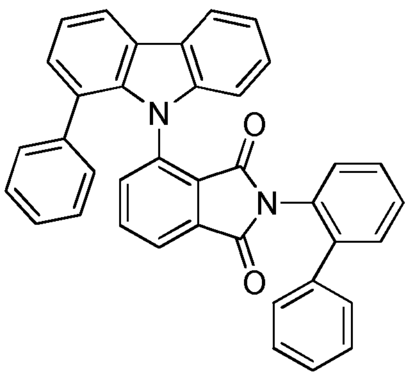

Figur 1 zeigt das Emissionsspektrum von Beispiel 1 (10 % in PMMA). Das Emissionsmaximum liegt bei 487 nm. Die Photolumineszenzquantenausbeute (PLQY) beträgt 61 % und die Halbwertsbreite (FWHM) 97 nm (0,49 eV). Die Emissionslebensdauer beträgt 3,8 μs.

## Beispiel 2

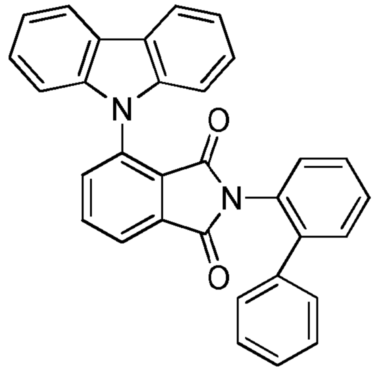

Figur 2 zeigt das Emissionsspektrum von Beispiel 2 (10 % in PMMA). Das Emissionsmaximum liegt bei 487 nm. Die Photolumineszenzquantenausbeute (PLQY) beträgt 57 % und die Halbwertsbreite (FWHM) 95 nm (0,49 eV). Die Emissionslebensdauer beträgt 7,2 µs.

## Beispiel 3

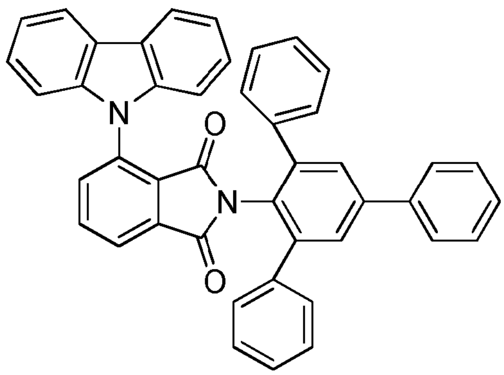

Figur 3 zeigt das Emissionsspektrum von Beispiel 3 (10 % in PMMA). Das Emissionsmaximum liegt bei 490 nm. Die Photolumineszenzquantenausbeute (PLQY) beträgt 59 % und die Halbwertsbreite (FWHM) 96 nm (0,48 eV). Die Emissionslebensdauer beträgt 6,4 µs.

## Beispiel 4

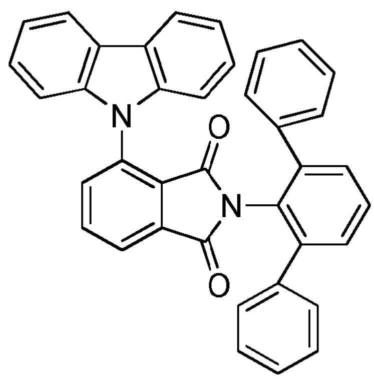

Figur 4 zeigt das Emissionsspektrum von Beispiel **4** (10 % in PMMA). Das Emissionsmaximum liegt bei 494 nm. Die Photolumineszenzquantenausbeute (PLQY) beträgt 61 % und die Halbwertsbreite (FWHM) 95 nm (0,47 eV).

Die Emissionslebensdauer beträgt 5,9 µs.

## Beispiel 5

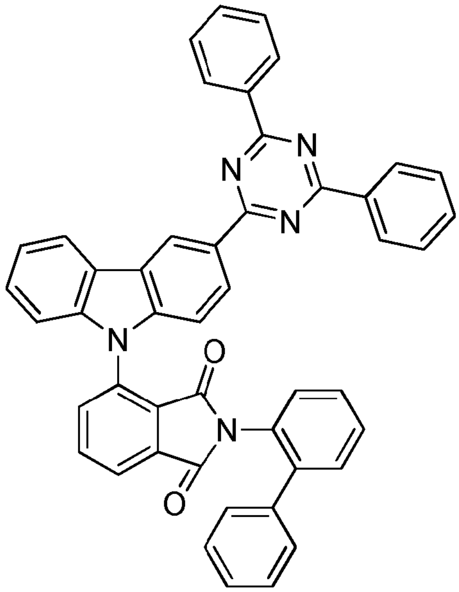

Beispiel 5 wurde nach AAV 4 ausgehend von 3-(3-Bromcarbazolyl)-*N*-(*o*-biphenyl)phthalimid in einer Ausbeute von 66% hergestellt.

$^1$H NMR (500 MHz, Chloroform-d): δ (ppm) = 9.54 (dd, *J* = 12.2, 1.6 Hz), 8.87-8.85 (m), 8.83-8.81 (m), 8.73 (dd, J = 8.7, 1.7 Hz), 8.33-8.31 (m), 8.05 (td, J = 7.5, 1.0 Hz), 7.98 (td, J = 7.7, 3.1 Hz, 1H), 7.92 (dd, J = 7.9, 1.1 Hz), 7.91 (dd, *J* = 7.9, 1.1 Hz), 7.65-7.27 (m), 6.60-6.57 (m). Das Produkt besteht aus zwei Rotameren, deren NMR-Signale sich überlagern.

Figur 5 zeigt das Emissionsspektrum von Beispiel **5** (10 % in PMMA). Das Emissionsmaximum liegt bei 481 nm. Die Photolumineszenzquantenausbeute (PLQY) beträgt 68 % und die Halbwertsbreite (FWHM) 92 nm (0,47 eV). Die Emissionslebensdauer beträgt 5,7 µs.

## Beispiel 6

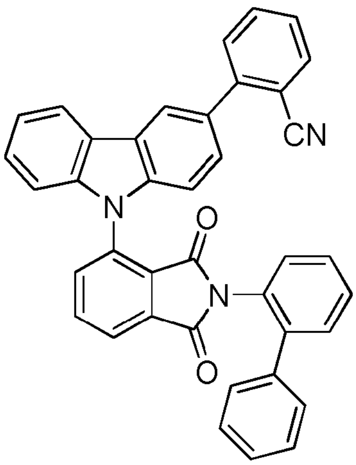

Beispiel 6 wurde nach AAV 4 ausgehend von 3-(3-Bromcarbazolyl)-*N*-(*o*-biphenyl)phthalimid in einer Ausbeute von 42% hergestellt.

Das Emissionsspektrum von Beispiel **6** (10 % in PMMA) wurde aufgenommen. Das Emissionsmaximum liegt bei 489 nm. Die Photolumineszenzquantenausbeute (PLQY) beträgt 62 % und die Halbwertsbreite (FWHM) 97 nm (0,48 eV). Die Emissionslebensdauer beträgt 5,9 µs.

## Beispiel 7

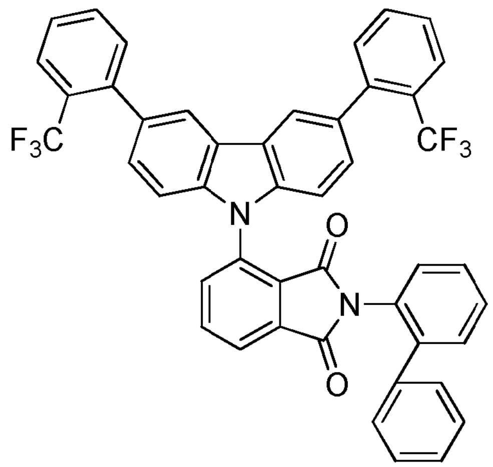

Beispiel 7 wurde nach AAV 5 aus 3-(3,6-Dibromcarbazolyl)- *N*-(*o*-biphenyl)phthalimid und 2-Trifluormethylphenylboronsäure in einer Ausbeute von 80% hergestellt.

Das Emissionsspektrum von Beispiel **7** (10 % in PMMA) wurde gemessen. Das Emissionsmaximum liegt bei 483 nm. Die Photolumineszenzquantenausbeute (PLQY) beträgt 68 % und die Halbwertsbreite (FWHM) 93 nm (0,47 eV).

## Beispiel 8

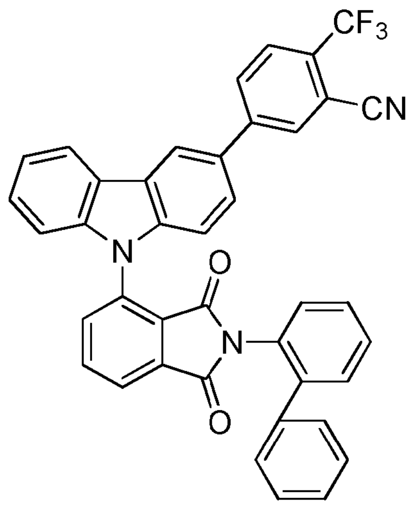

Beispiel 8 wurde nach AAV 4 durch die Überführung von 3-(3-Bromcarbazolyl)-*N*-(*o*-biphenyl)phthalimid in den entsprechenden Bis(boronsäurepinacolester) und die anschließende Umsetzung mit 3-Brom-6-trifluormethyl-benzonitril in einer Ausbeute von 64% hergestellt

Das Emissionsspektrum von Beispiel **8** (10 % in PMMA) wurde gemessen. Das Emissionsmaximum liegt bei 491 nm. Die Photolumineszenzquantenausbeute (PLQY) beträgt 65 % und die Halbwertsbreite (FWHM) 96 nm (0,48 eV.

## Beispiel 9

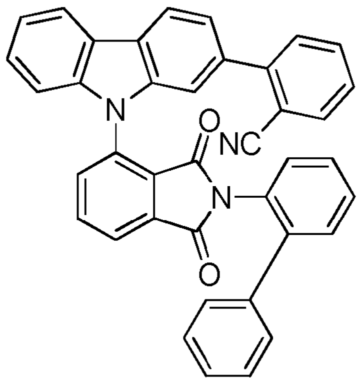

Beispiel 9 wurde nach AAV 4 durch die Überführung von 3-(2-Bromcarbazolyl)-*N*-(*o*-biphenyl)phthalimid in den entsprechenden Bis(boronsäurepinacolester) und die anschließende Umsetzung mit 2-Brombenzonitril in einer Ausbeute von 97 % hergestellt. Das Emissionsspektrum von Beispiel **9** (10 % in PMMA) wurde aufgenommen. Das Emissionsmaximum liegt bei 476 nm. Die Photolumineszenzquantenausbeute (PLQY) beträgt 61 % und die Halbwertsbreite (FWHM) 91 nm (0,48 eV).

## Beispiel 10

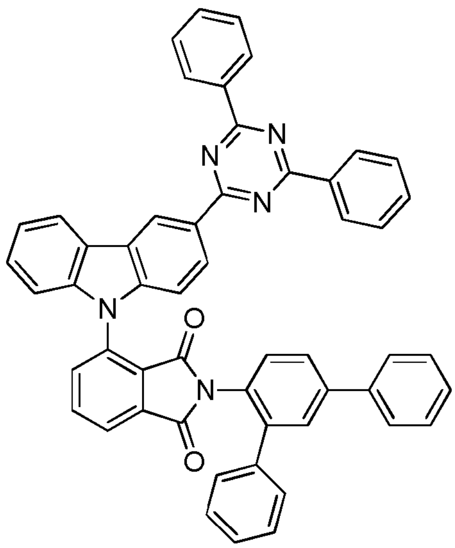

Beispiel 10 wurde nach AAV 4 durch die Überführung von 3-(2-Bromcarbazolyl)-*N*-(*o*-(meta-terphenyl))phthalimid in den entsprechenden Bis(boronsäurepinacolester) und die anschließende Umsetzung mit Chlordiphenyltriazin in einer Ausbeute von 44% hergestellt.

Das Emissionsspektrum von Beispiel **10** (10 % in PMMA) wurde aufgenommen. Das Emissionsmaximum liegt bei 486 nm. Die Photolumineszenzquantenausbeute (PLQY) beträgt 65 % und die Halbwertsbreite (FWHM) 93 nm (0,47 eV). Die Emissionslebensdauer beträgt 5,6 μs.

## Beispiel 11

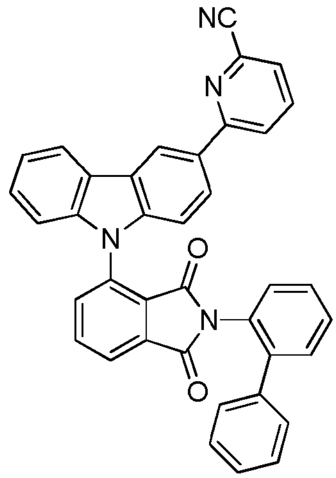

Beispiel 11 wurde nach AAV 4 durch die Überführung von 3-(2-Bromcarbazolyl)-*N*-(*o*-biphenyl)phthalimid in den entsprechenden Bis(boronsäurepinacolester) und die anschließende Umsetzung mit 2-Brom-6-cyanopyridin in einer Ausbeute von 13% hergestellt. Das Emissionsspektrum von Beispiel **11** (10% in PMMA) wurde aufgenommen. Das Emissionsmaximum liegt bei 489 nm. Die Photolumineszenzquantenausbeute (PLQY) beträgt 61 % und die Halbwertsbreite (FWHM) 97 nm (0,48 eV).

## Beispiel 12

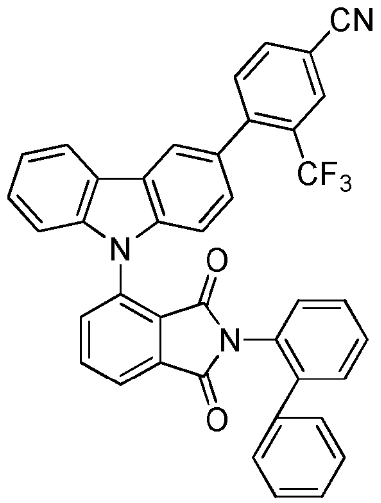

Beispiel 12 wurde nach AAV 4 durch die Überführung von 3-(3-Bromcarbazolyl)-*N*-(*o*-biphenyl)phthalimid in den entsprechenden Bis(boronsäurepinacolester) und die anschließende Umsetzung mit 4-Brom-3-trifluormethyl-benzonitril in einer Ausbeute von 94% hergestellt.

Das Emissionsspektrum von Beispiel **12** (10 % in PMMA) wurde gemessen. Das Emissionsmaximum liegt bei 479 nm. Die Photolumineszenzquantenausbeute (PLQY) beträgt 63 % und die Halbwertsbreite (FWHM) 92 nm (0,46 eV).

## Beispiel 13

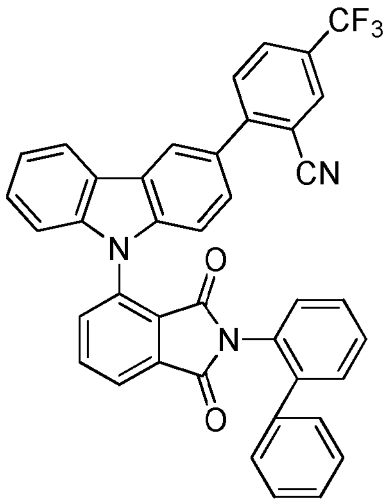

Beispiel 13 wurde nach AAV 4 durch die Überführung von 3-(3-Bromcarbazolyl)-*N*-(*o*-biphenyl)phthalimid in den entsprechenden Bis(boronsäurepinacolester) und die anschließende Umsetzung mit 2-Brom-5-trifluormethyl-benzonitril in einer Ausbeute von 36% hergestellt.

Das Emissionsspektrum von Beispiel **13** (10 % in PMMA) wurde gemessen. Das Emissionsmaximum liegt bei 475 nm. Die Photolumineszenzquantenausbeute (PLQY) beträgt 67 % und die Halbwertsbreite (FWHM) 91 nm (0,48 eV).

## Beispiel 14

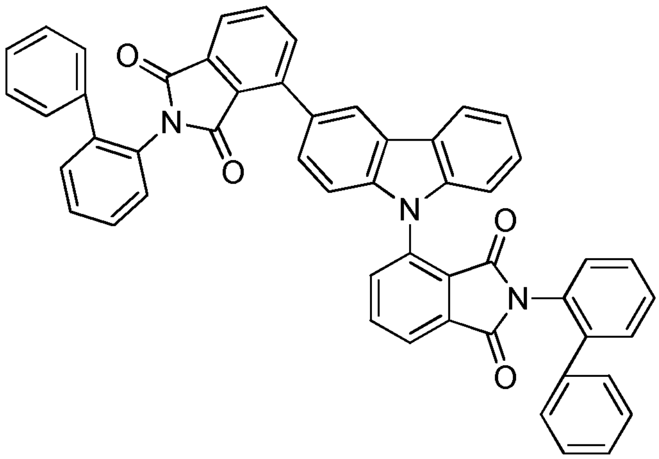

Beispiel 14 wurde nach AAV 4 durch die Überführung von 3-(3-Bromcarbazolyl)- *N-(o*-biphenyl)phthalimid in den entsprechenden Boronsäurepinacolester und die anschließende Umsetzung mit *N*-(*o*-biphenyl)phthalimid-3-chlorphthalimid in einer Ausbeute von 45% hergestellt.

Das Emissionsspektrum von Beispiel **14** (10 % in PMMA) wurde gemessen. Das Emissionsmaximum liegt bei 490 nm. Die Photolumineszenzquantenausbeute (PLQY) beträgt 61 % und die Halbwertsbreite (FWHM) 96 nm (0,48 eV).

**Beispiel 15**

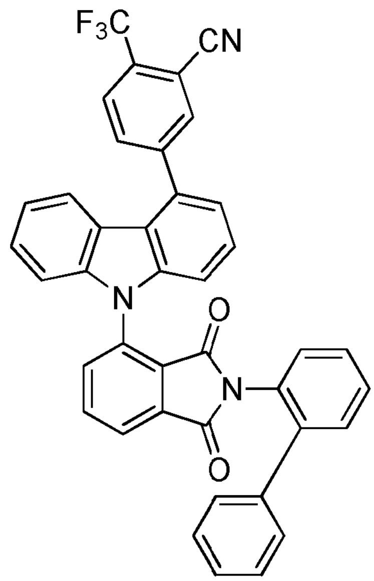

Beispiel 15 wurde nach AAV 4 durch die Überführung von 3-(4-Bromcarbazolyl)-*N*-(*o*-biphenyl)phthalimid in den entsprechenden Bis(boronsäurepinacolester) und die anschließende Umsetzung mit 3-Brom-6-trifluormethyl-benzonitril in einer Ausbeute von 40% hergestellt.

Das Emissionsspektrum von Beispiel **15** (10 % in PMMA) wurde gemessen. Das Emissionsmaximum liegt bei 474 nm. Die Photolumineszenzquantenausbeute (PLQY) beträgt 61 % und die Halbwertsbreite (FWHM) 93 nm (0,48 eV).

Beispiel 16

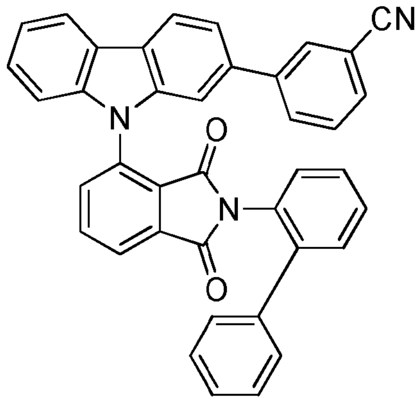

Beispiel 16 wurde nach AAV 5 aus 3-(2-Bromcarbazolyl)-*N*-(*o*-biphenyl)phthalimid und 3-Cyanophenylboronsäure in einer Ausbeute von 31% hergestellt.

Das Emissionsspektrum von Beispiel 16 (10 % in PMMA) wurde gemessen. Das Emissionsmaximum liegt bei 474 nm. Die Photolumineszenzquantenausbeute (PLQY) beträgt 54 % und die Halbwertsbreite (FWHM) 93 nm (0,48 eV).

**Beispiel D1**

Beispiel **5** wurde in einem OLED-Bauteil ("Bauteil D1") mit folgendem Aufbau getestet (Anteil des erfindungsgemäßen Moleküls an der Emissionsschicht ist in Massenprozent angegeben):

| Schicht | Dicke | D1 |
|---|---|---|
| **7** | 100 nm | Al |
| **6** | 2 nm | Liq |
| **5** | 40 nm | NBPhen |
| **4** | 20 nm | **5** (30%):mCBP |
| **3** | 10 nm | TCTA |
| **2** | 80 nm | NPB |
| **1** | 130 nm | ITO |
| **Substrat** | | Glas |

| | Maximalwerte | bei 1000 cd/m$^2$ | LT80 bei 500 cd/m$^2$ |
|---|---|---|---|
| Externe Quanteneffizienz (EQE): | 8,9 $\pm$ 0,03 % | 8,7 $\pm$ 0,02 % | 72 h |

Das Emissionsmaximum liegt bei 486 nm, CIEx wurde mit 0,24 und die CIEy: 0,39 bei 4,5 V bestimmt.

**Weitere Beispielmoleküle:**

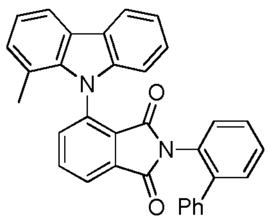
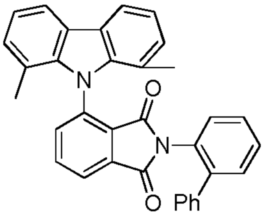
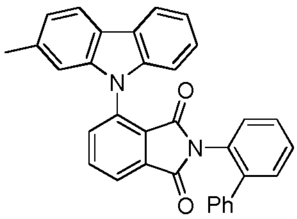
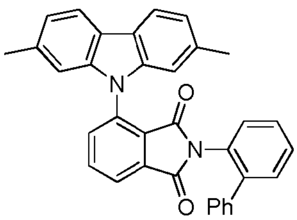

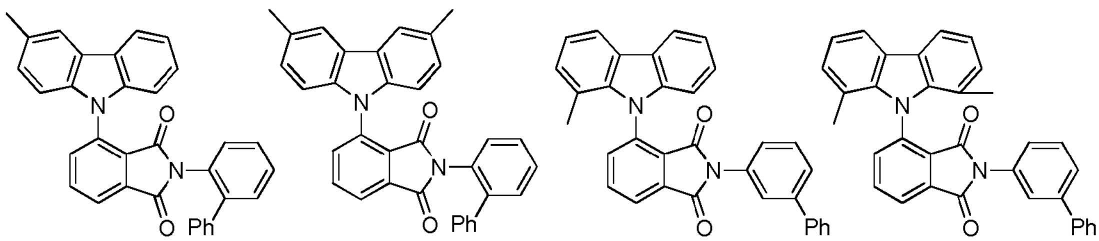

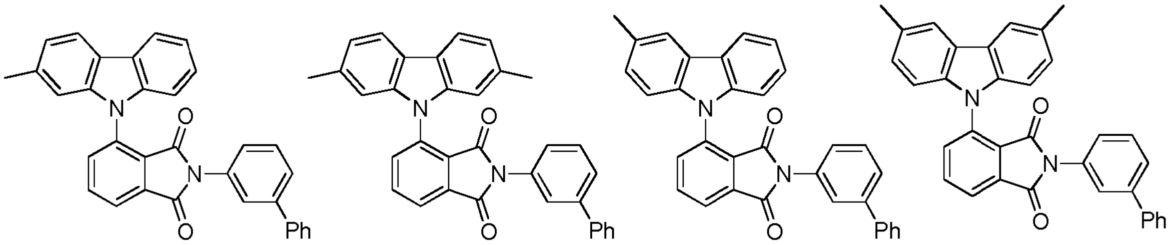

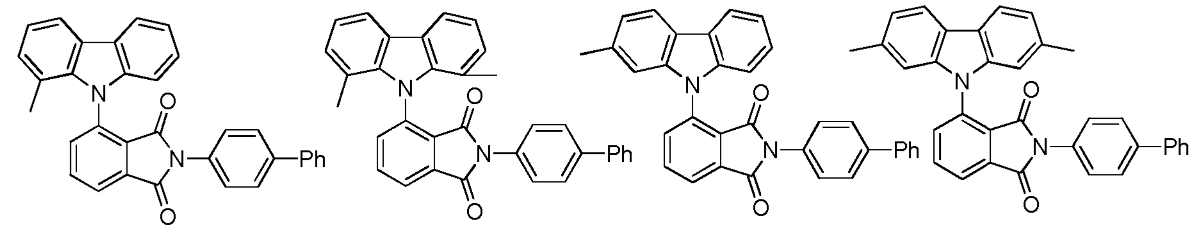

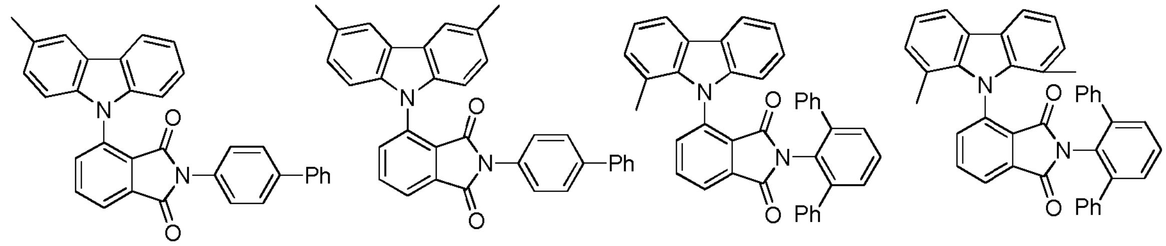

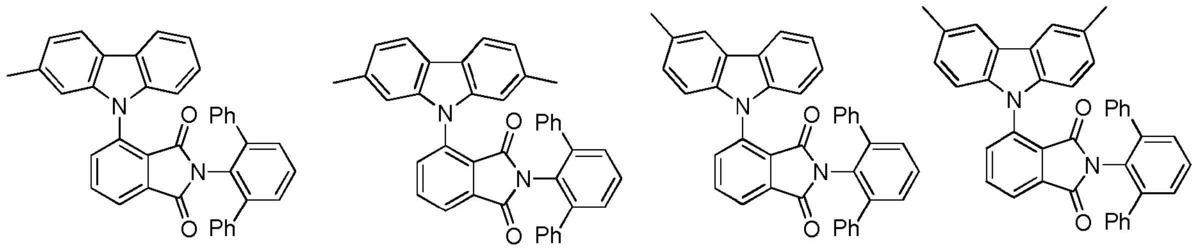

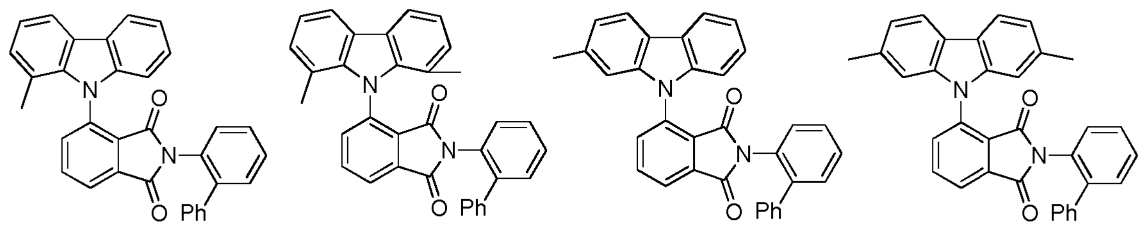

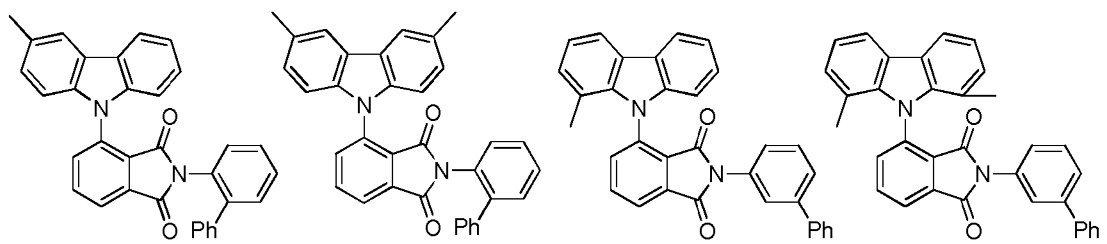

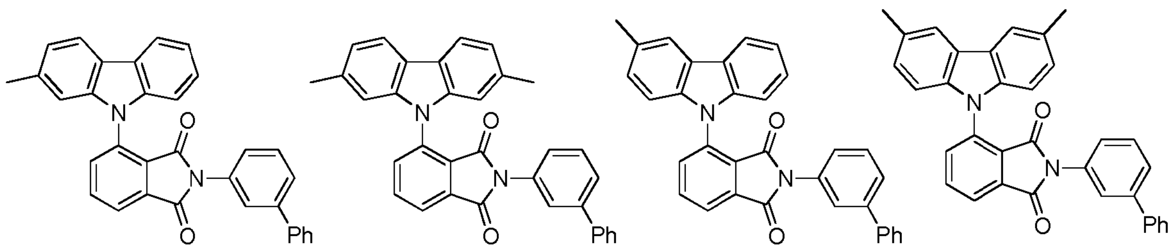

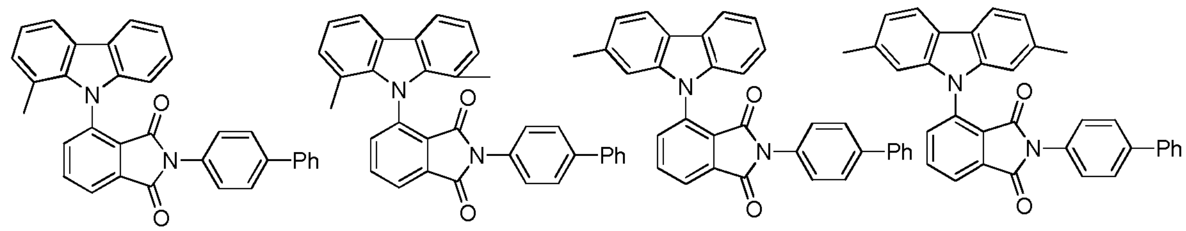

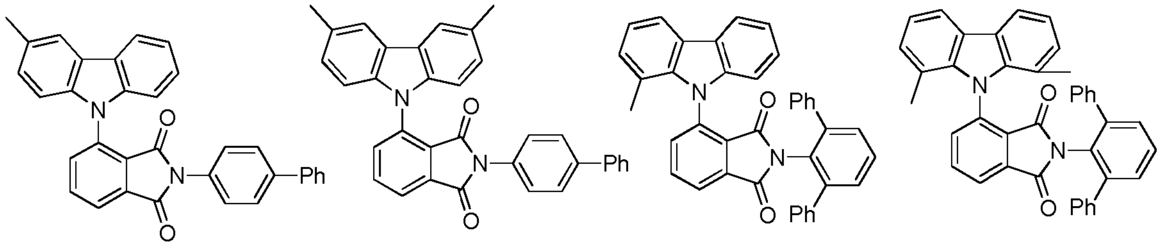

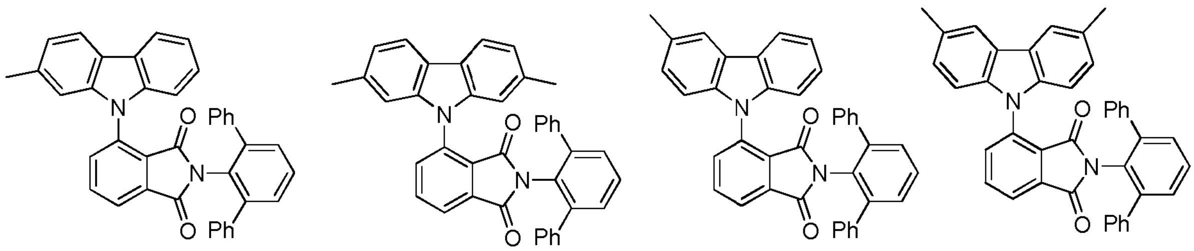

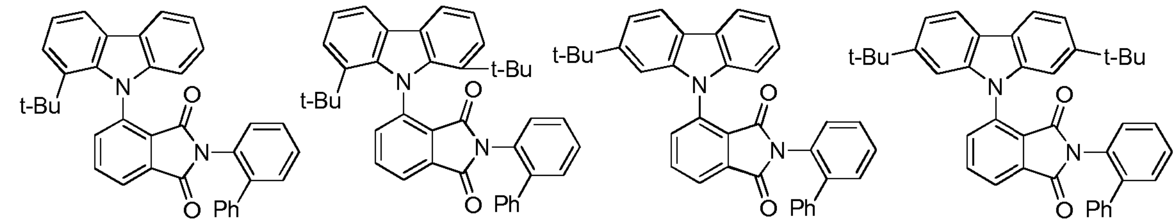

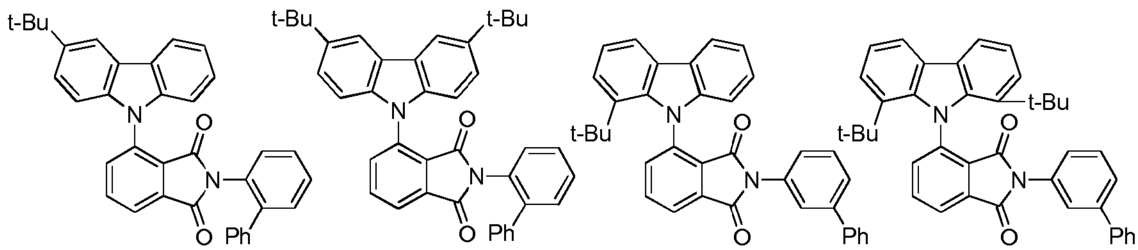

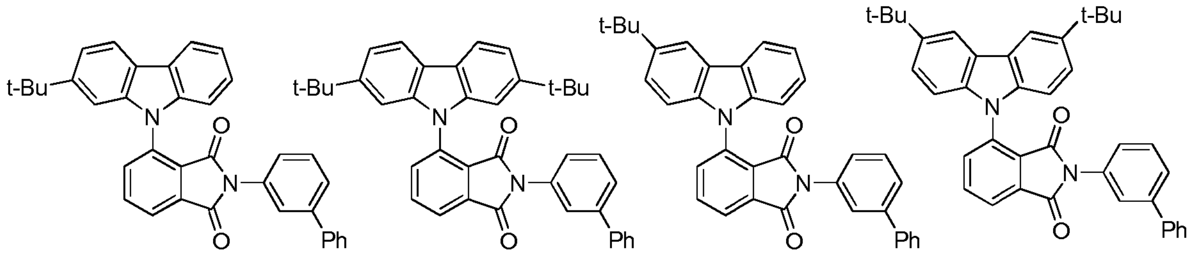

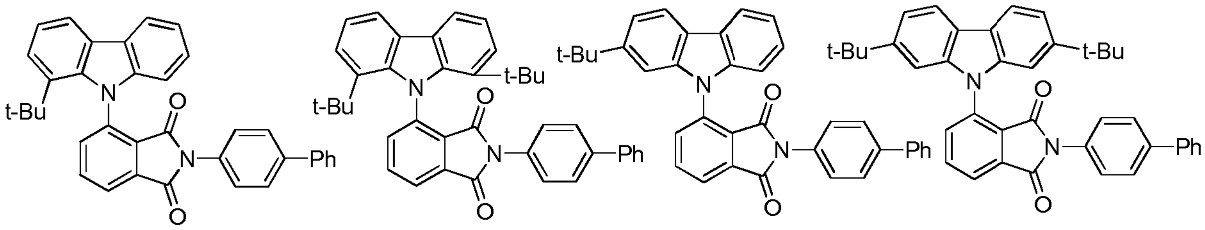

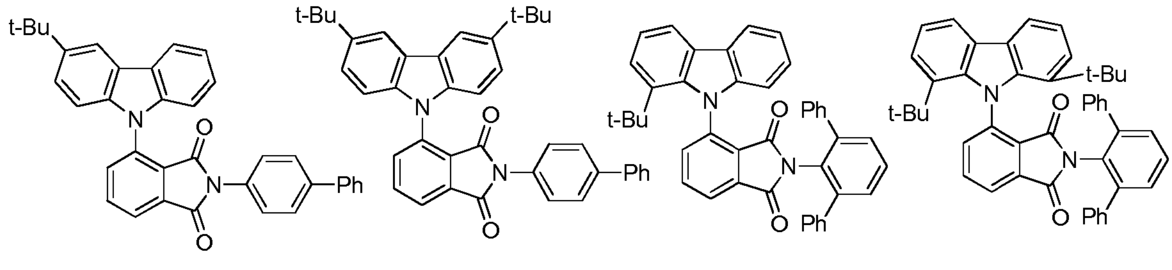

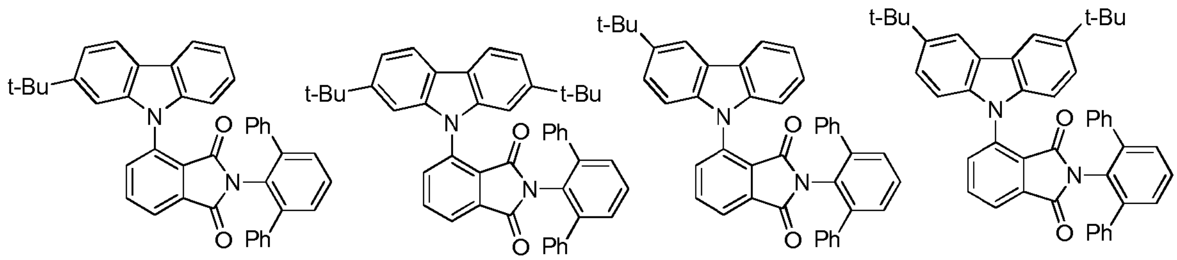

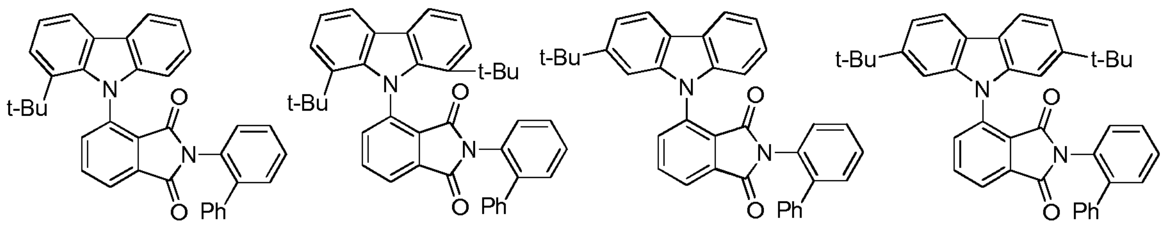

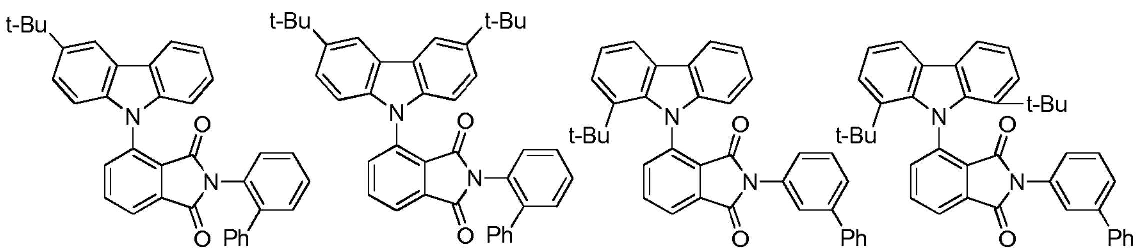

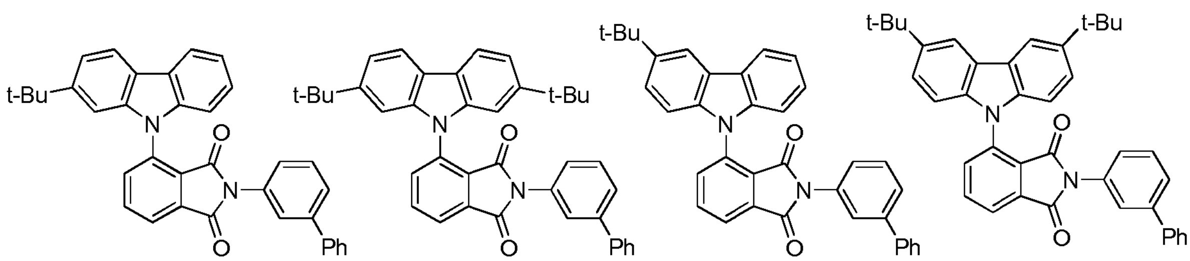

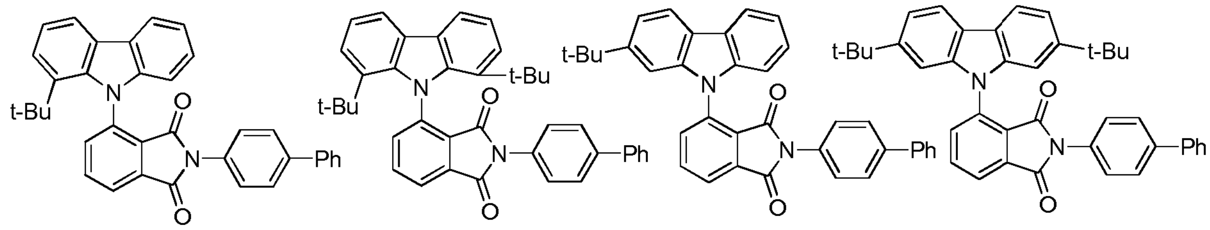

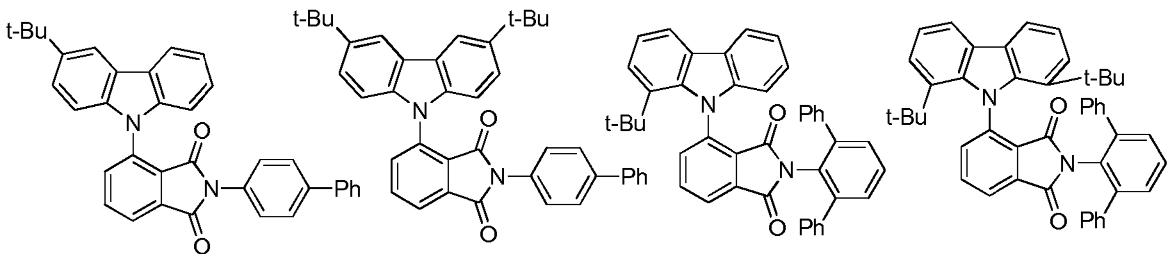

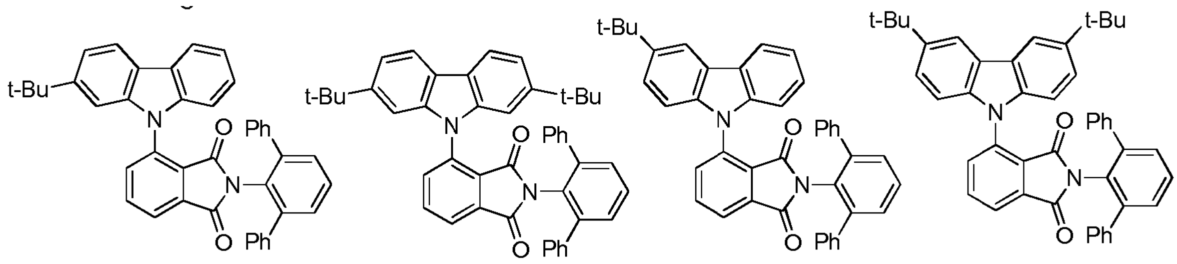

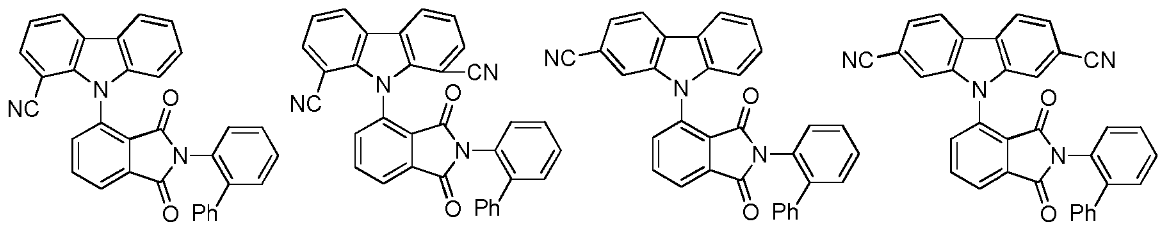

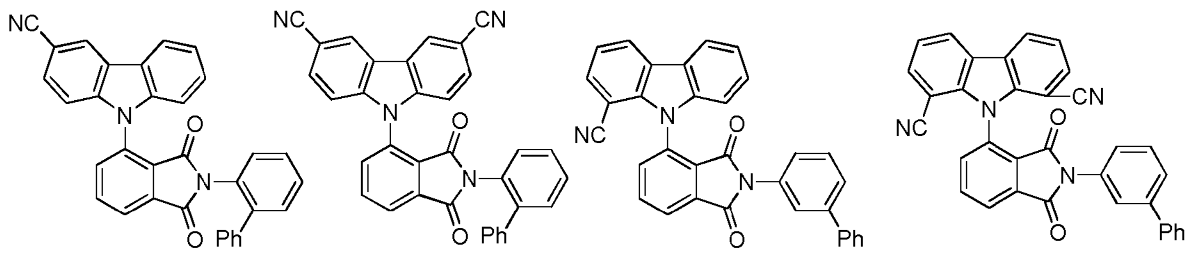

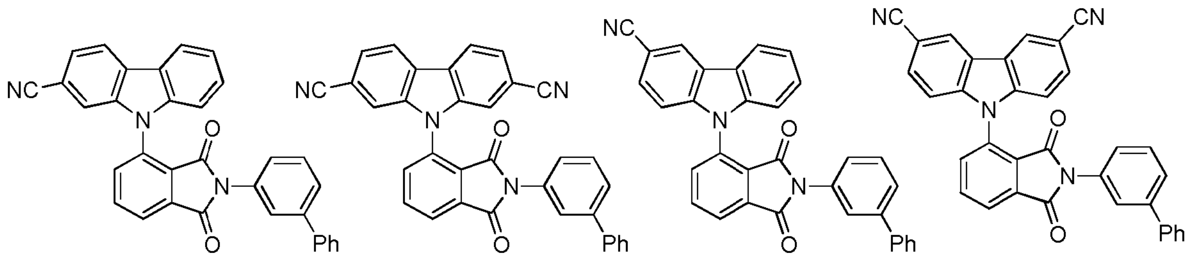

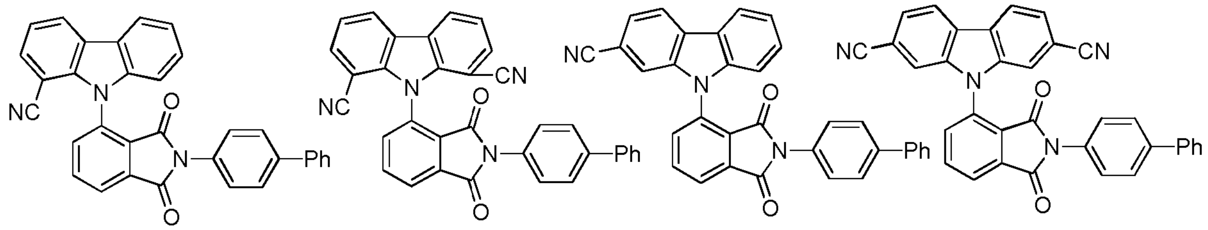

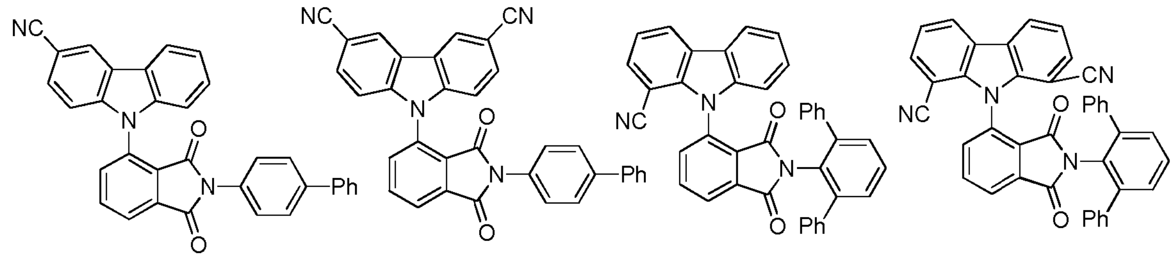

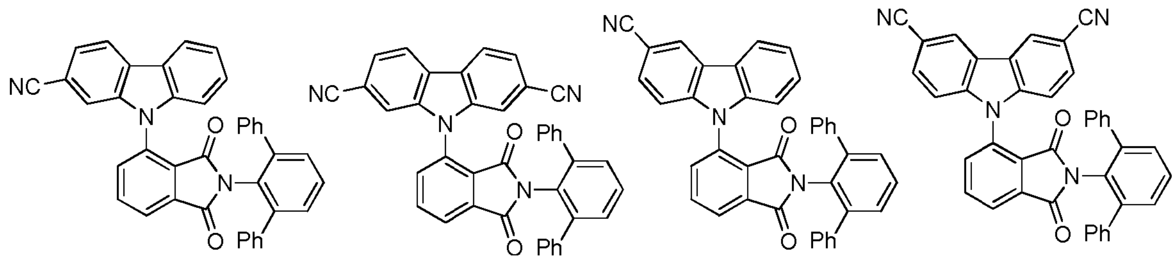

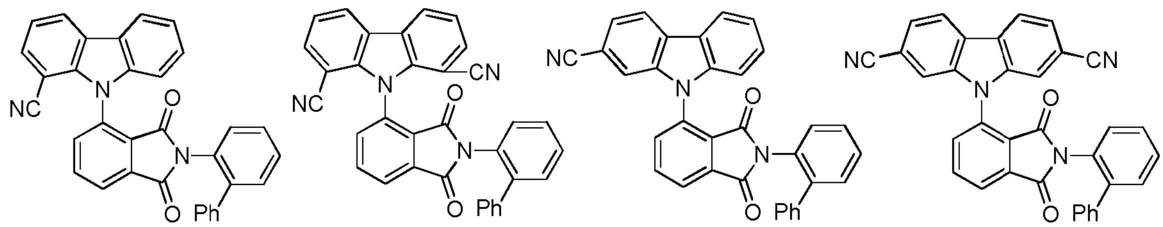

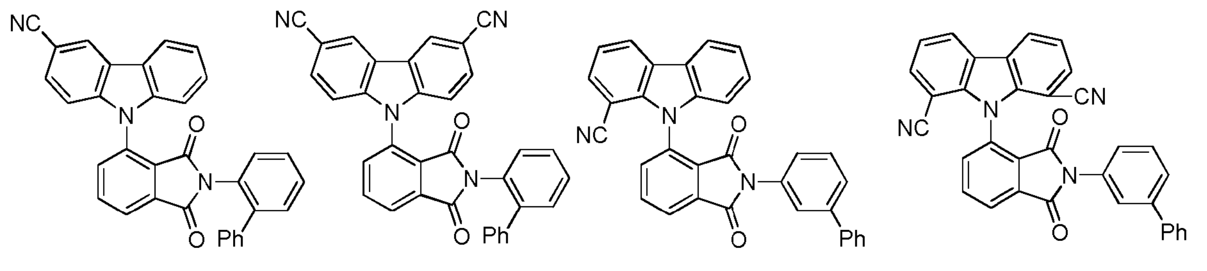

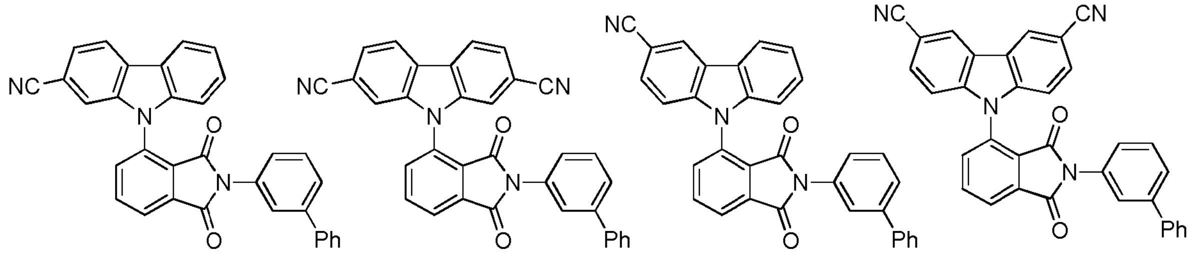

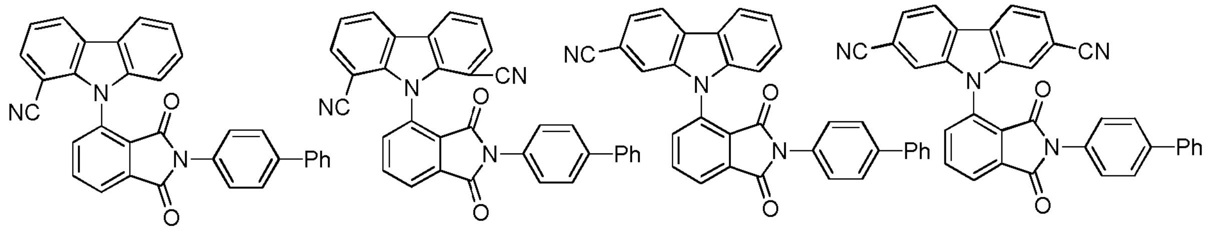

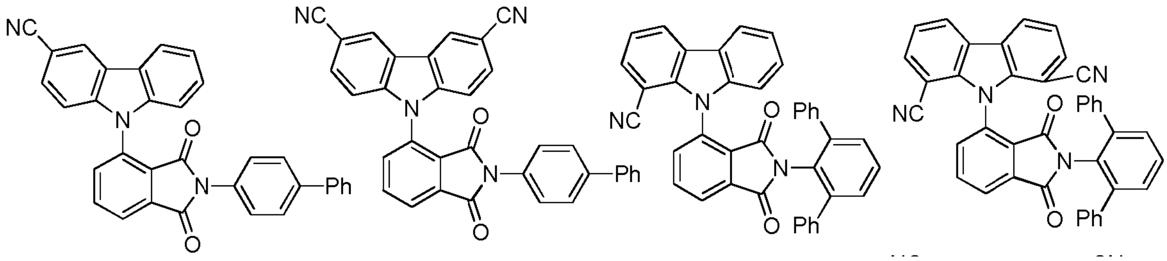

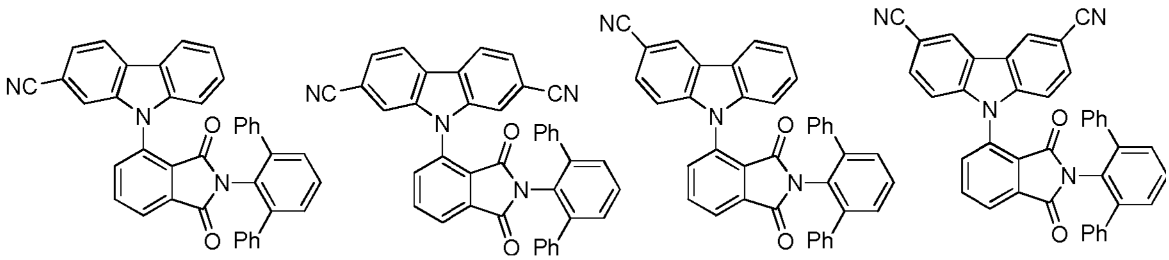

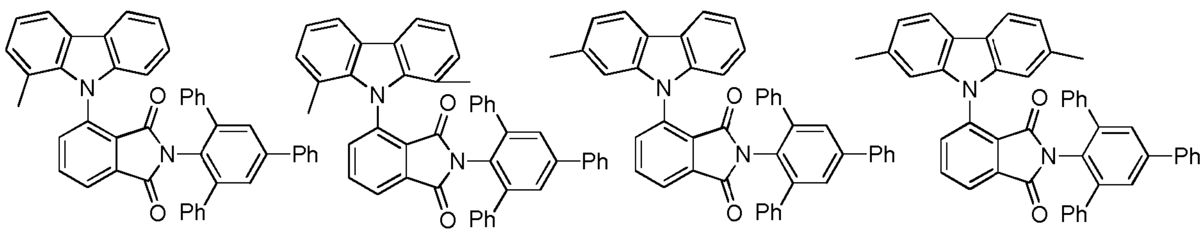

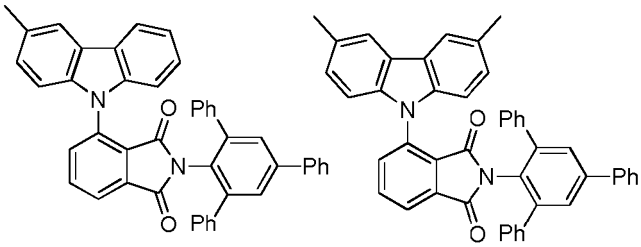

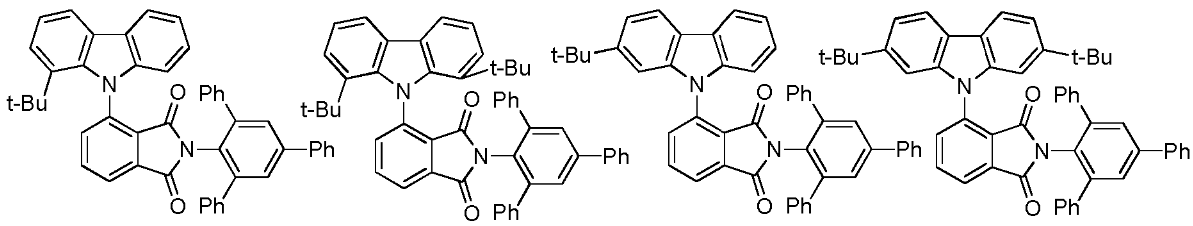

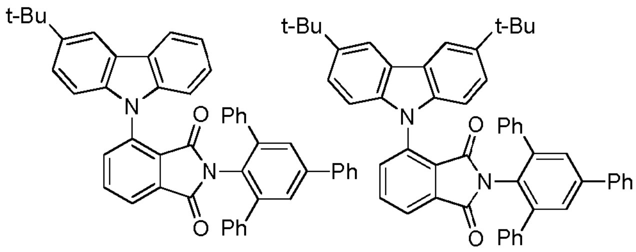

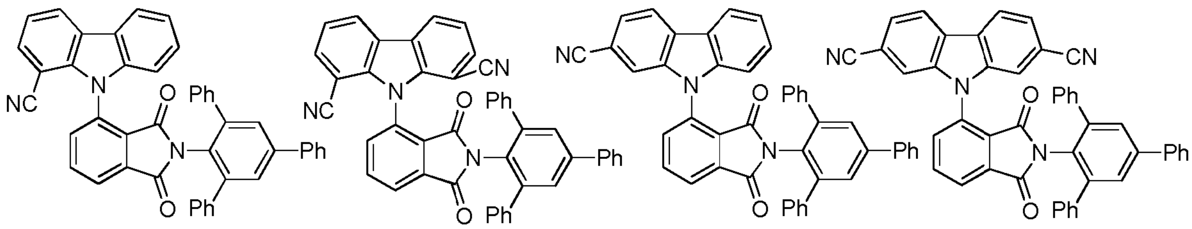

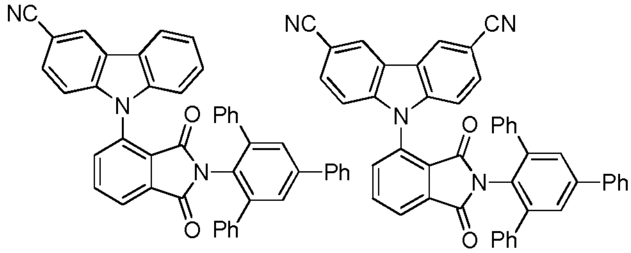

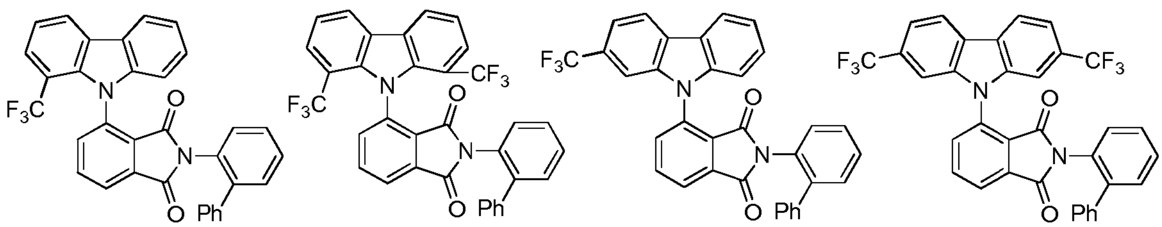

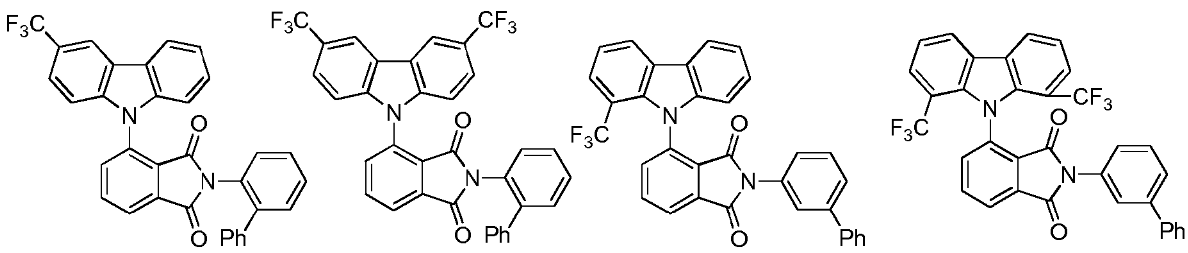

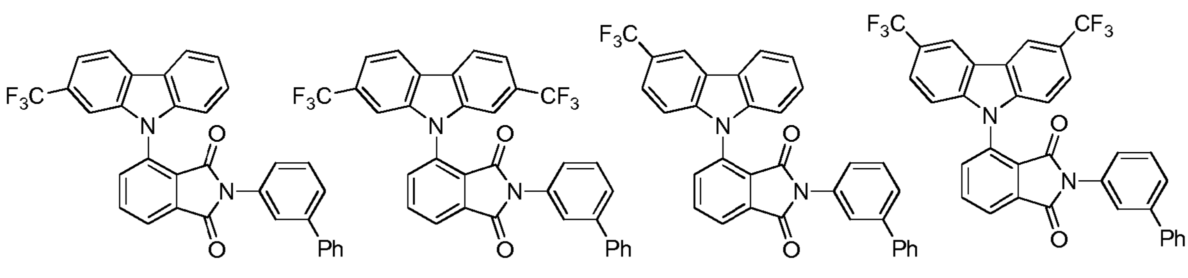

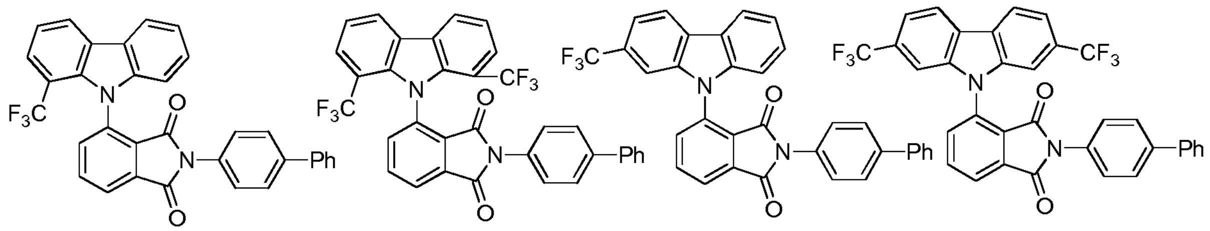

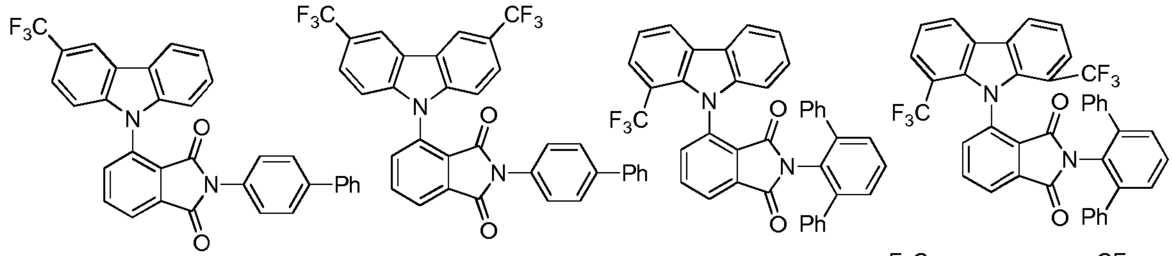

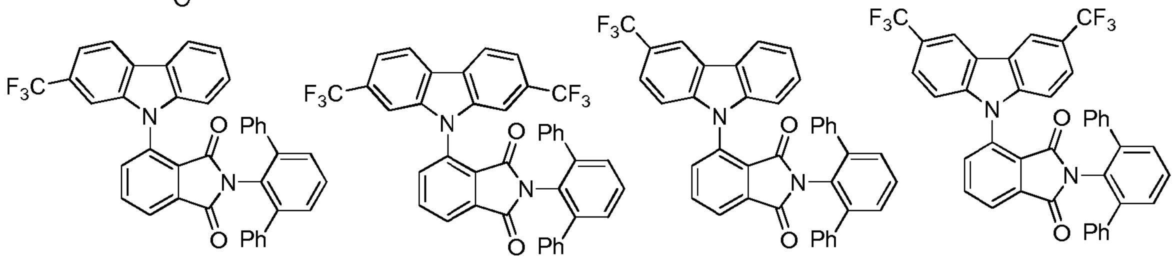

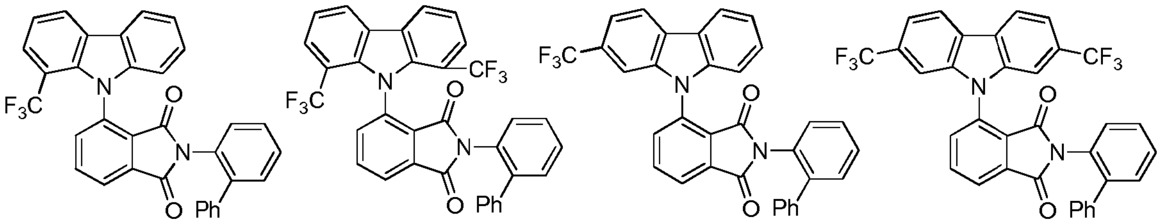

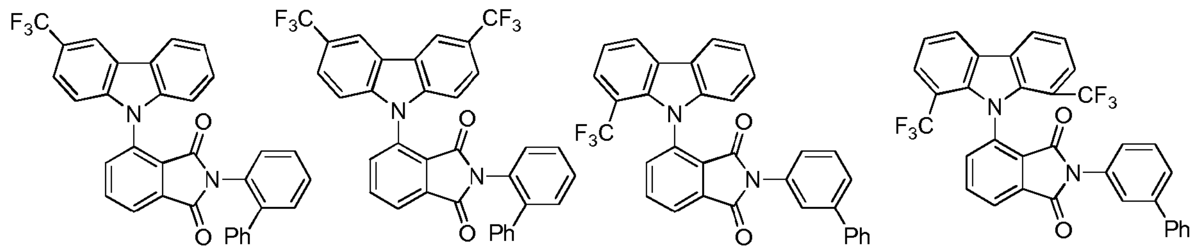

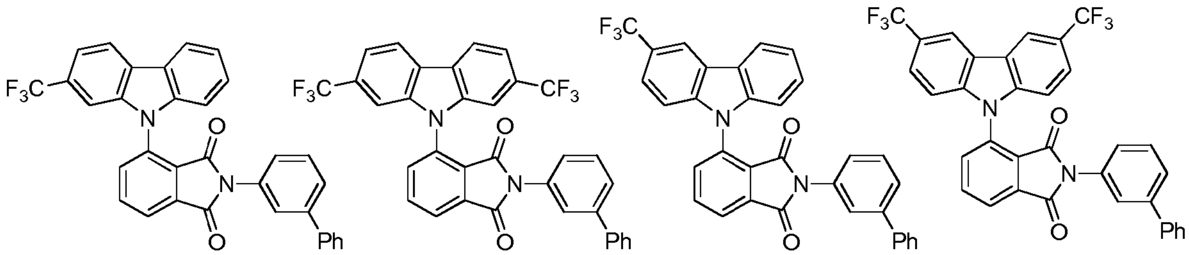

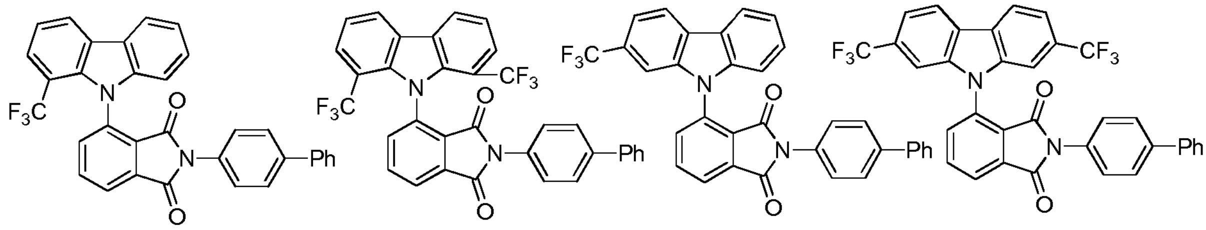

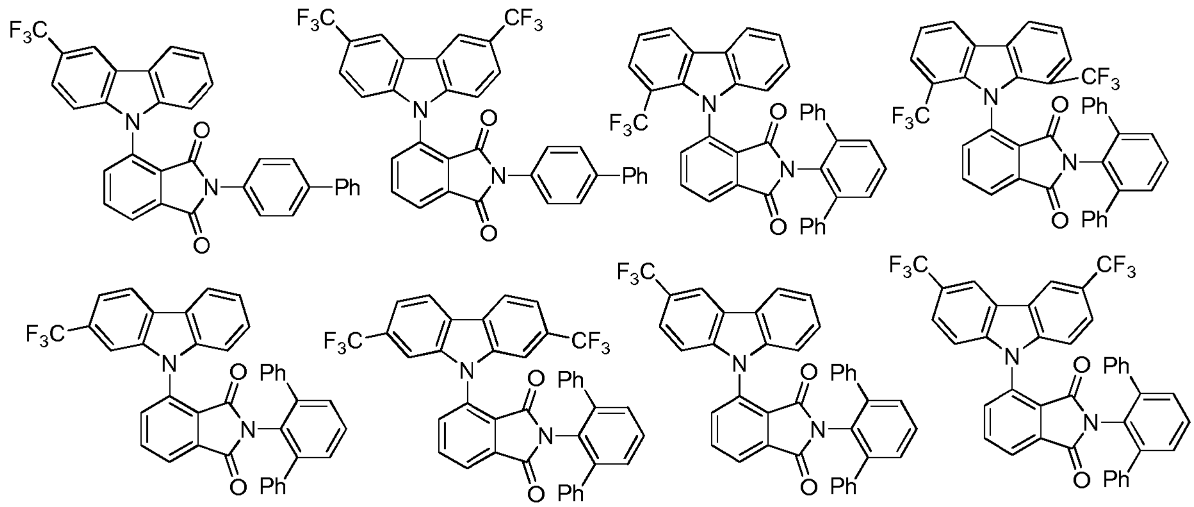

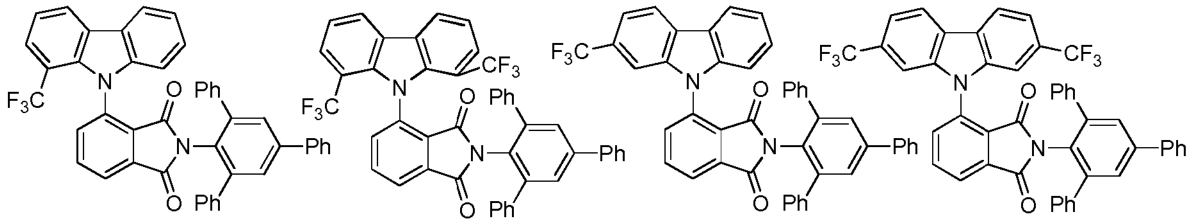

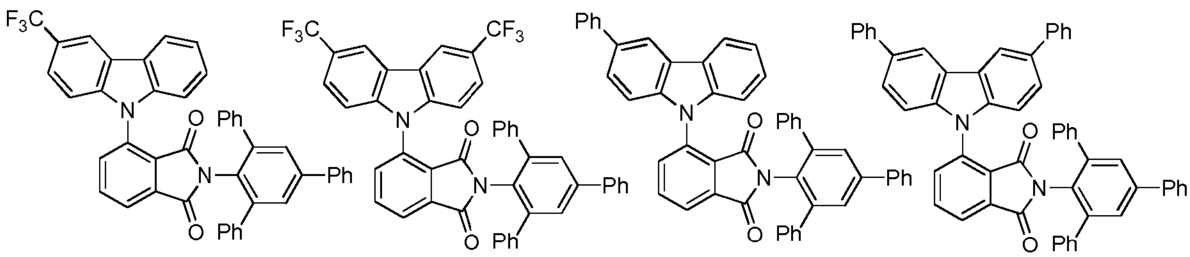

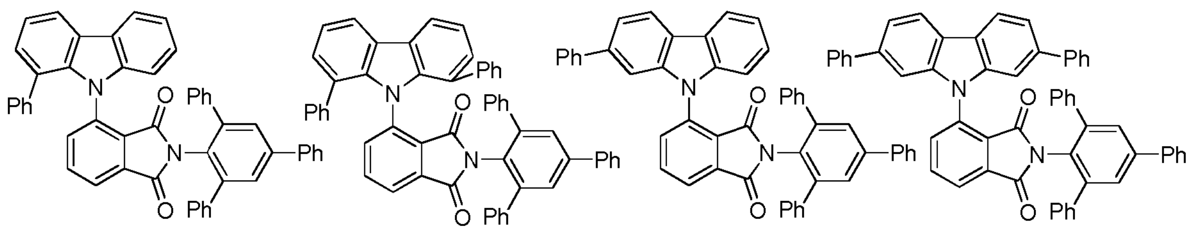

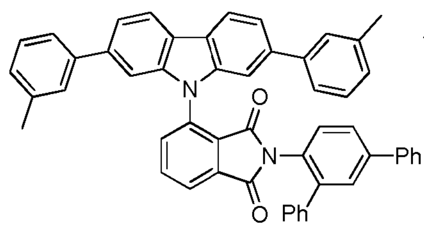

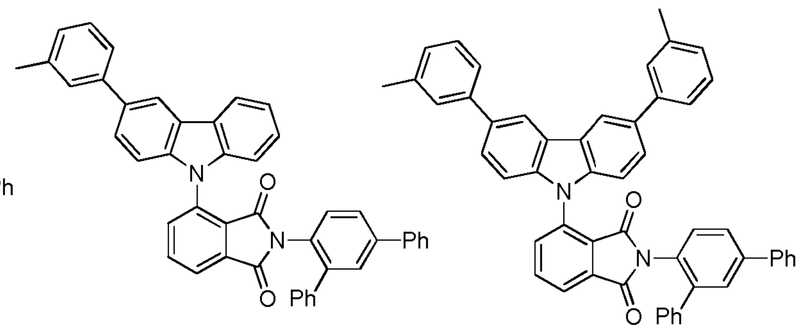

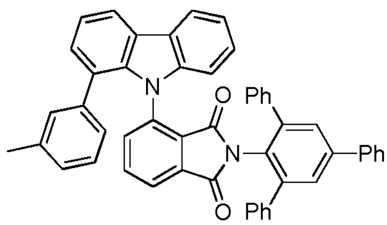

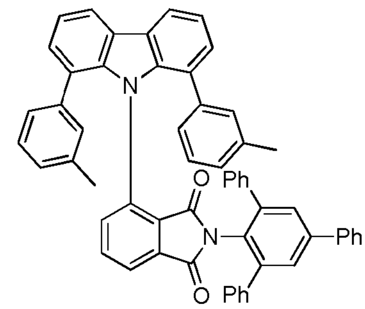

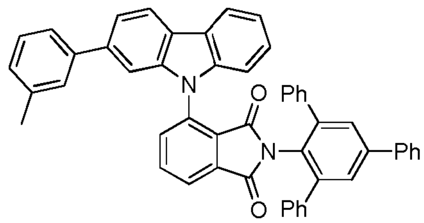

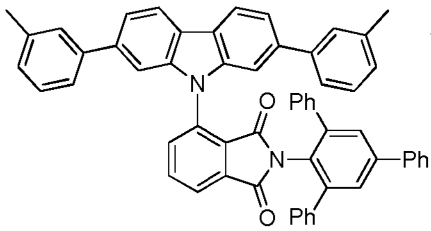

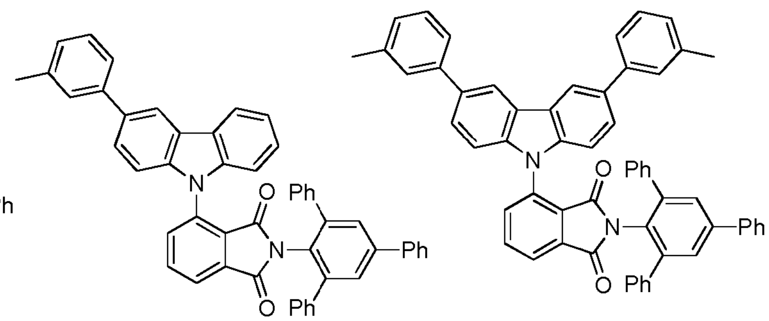

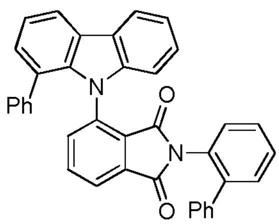

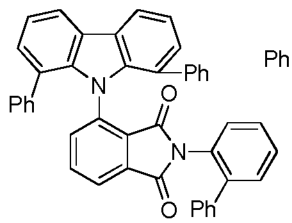

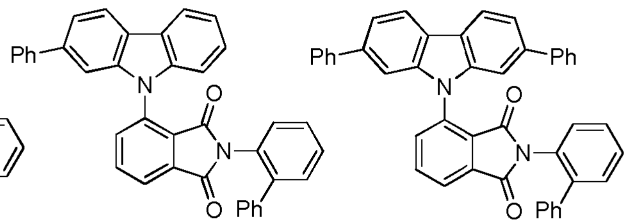

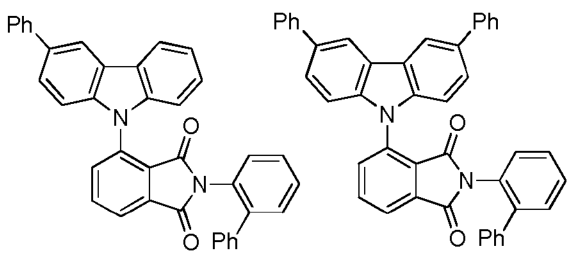

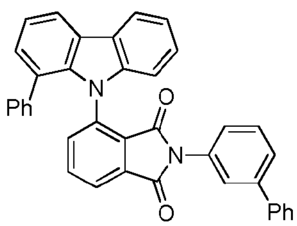

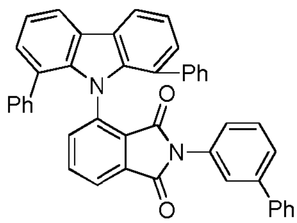

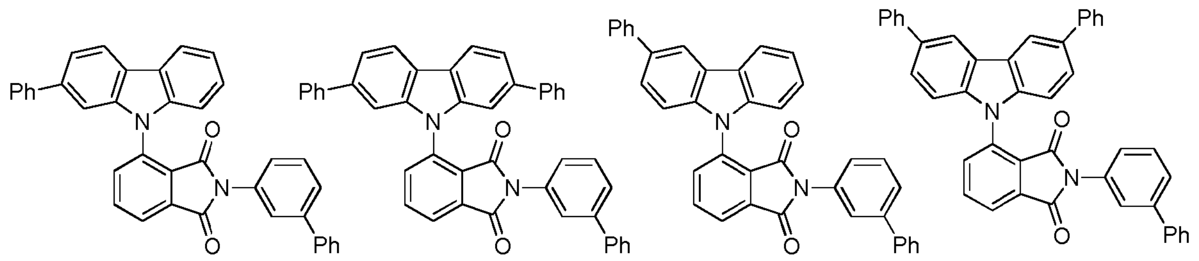

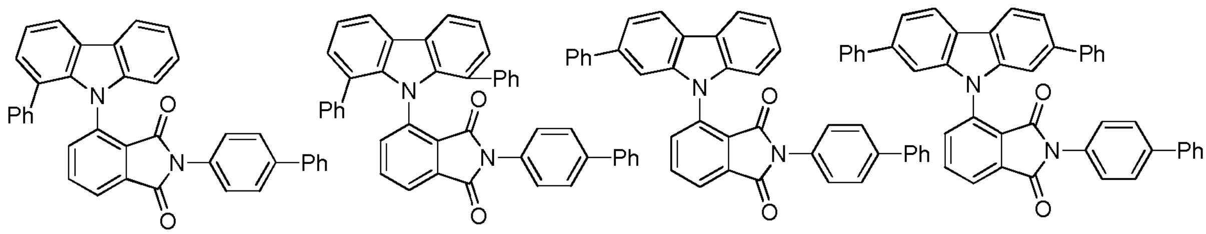

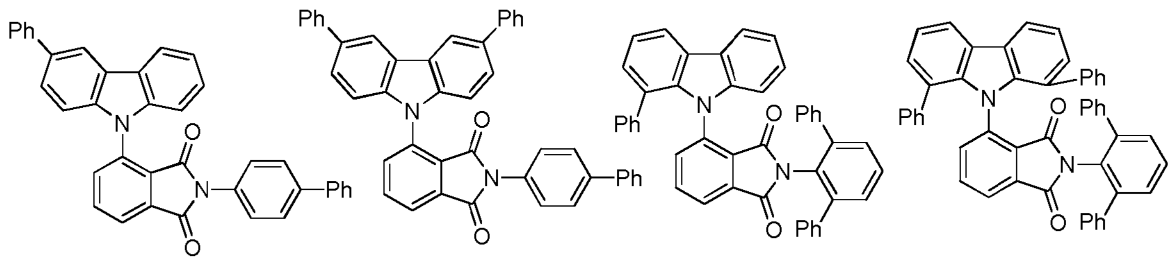

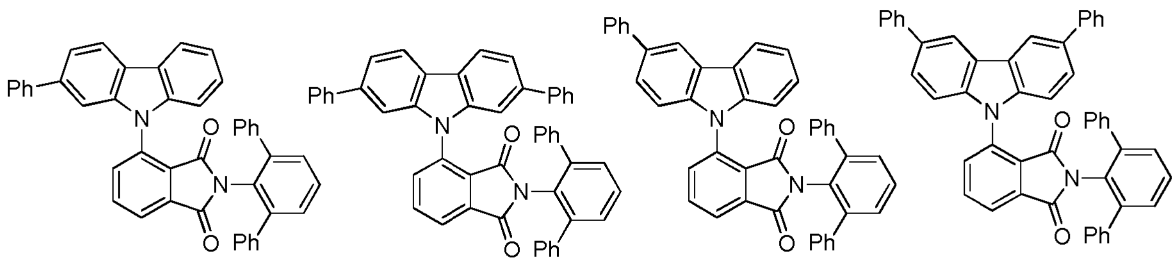

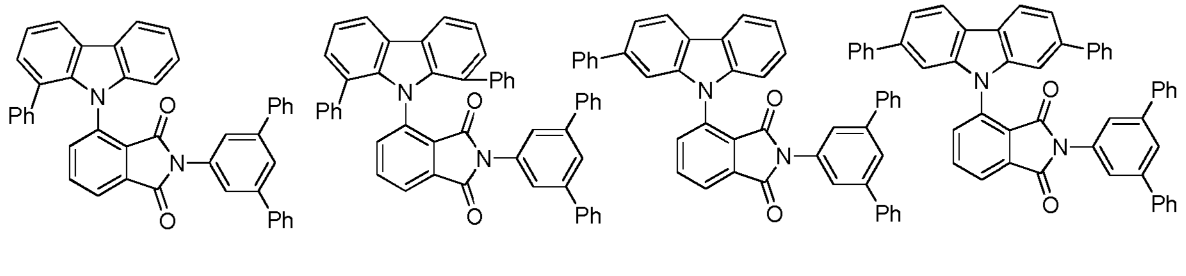

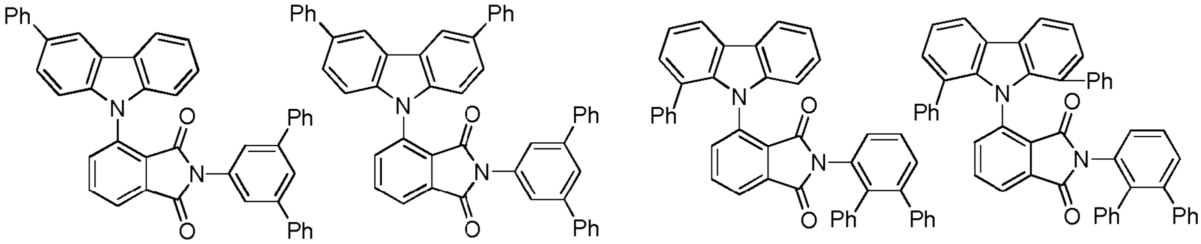

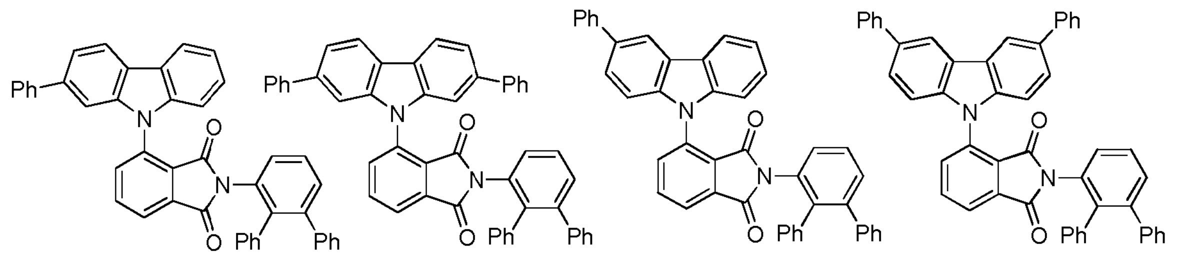

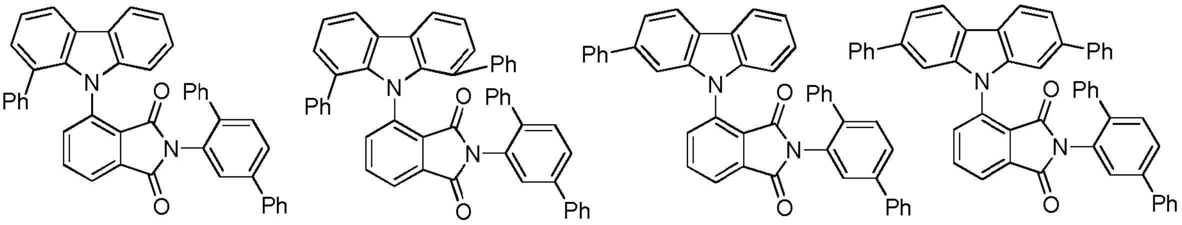

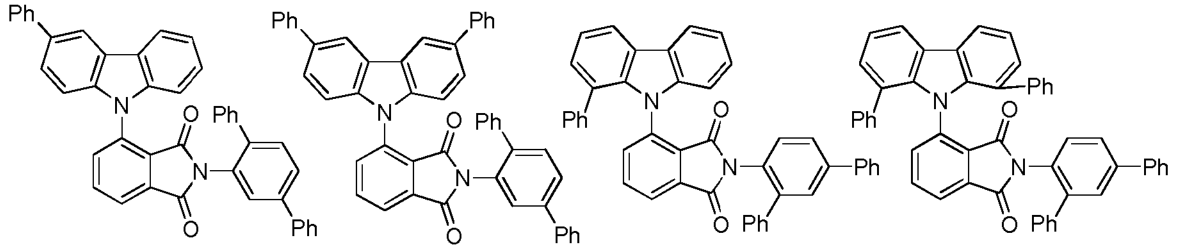

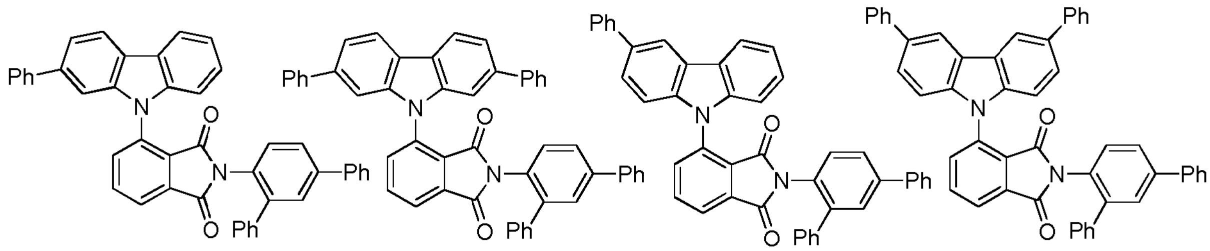

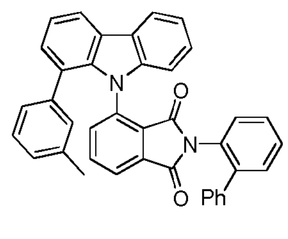

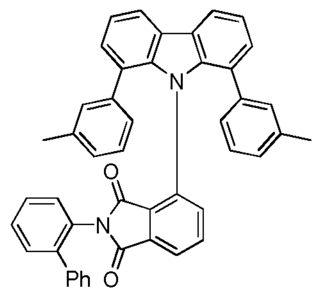

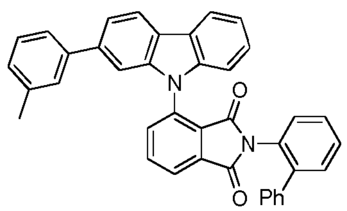

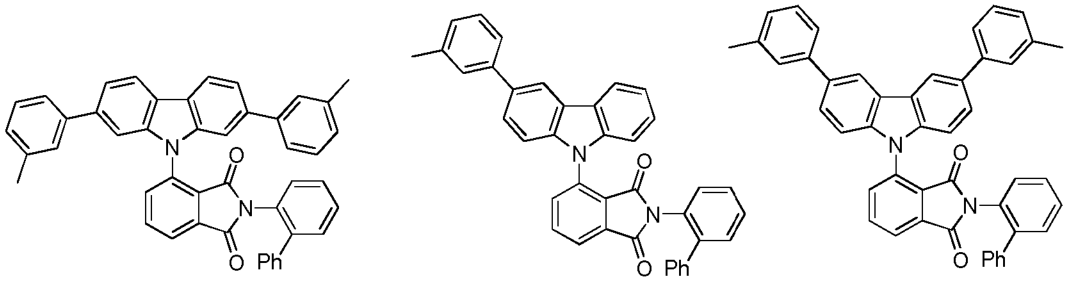

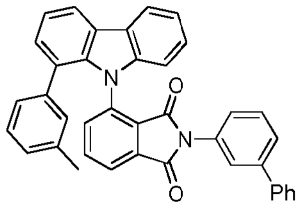

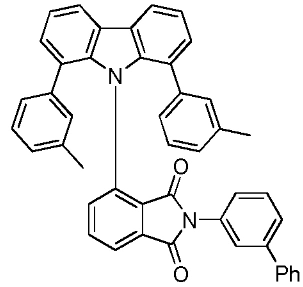

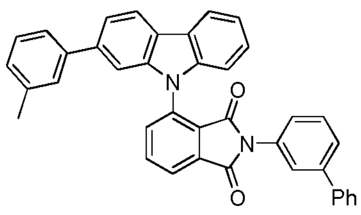

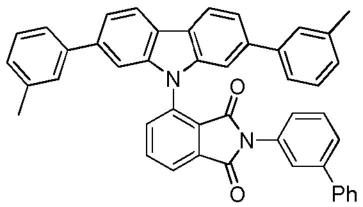

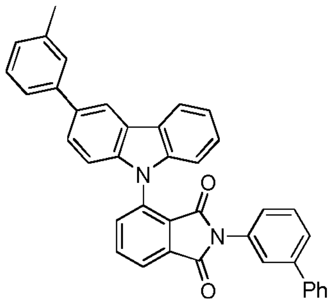

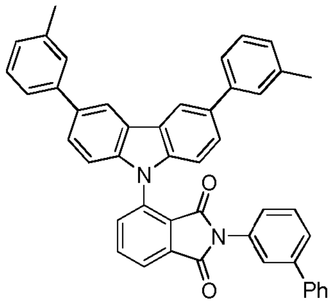

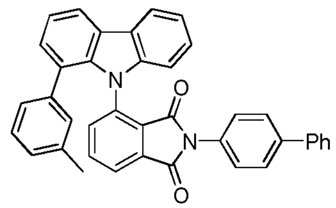

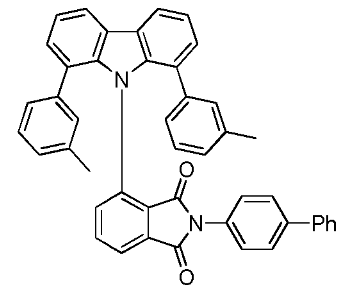

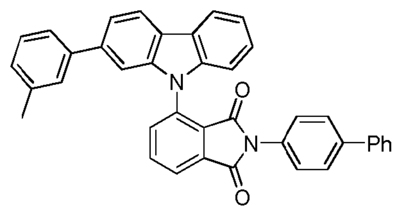

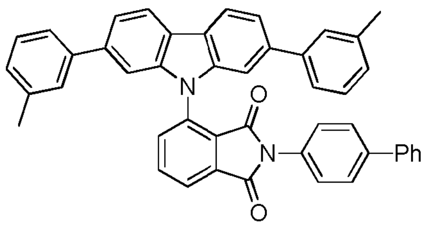

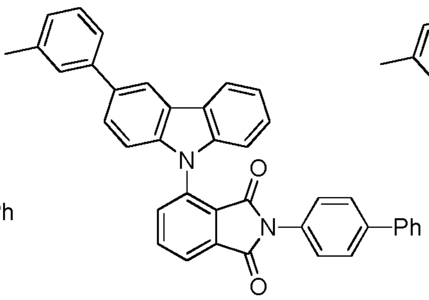

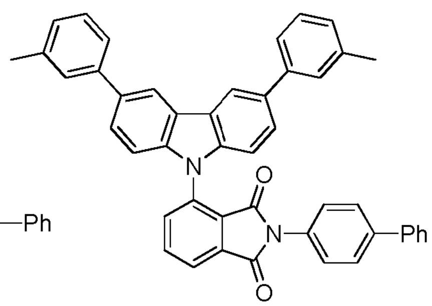

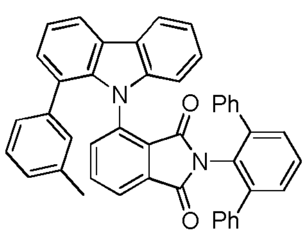

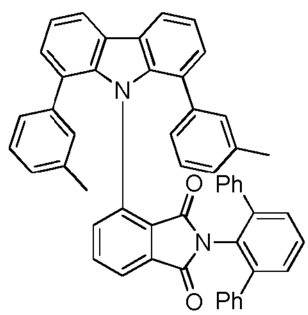

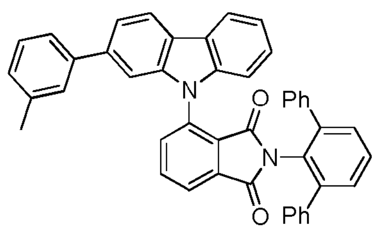

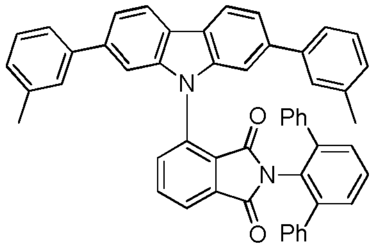

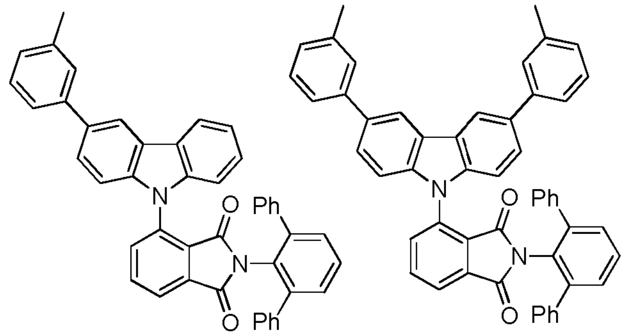

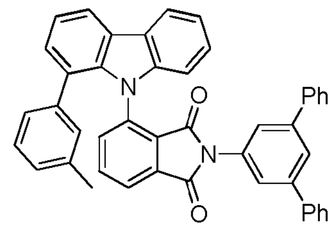

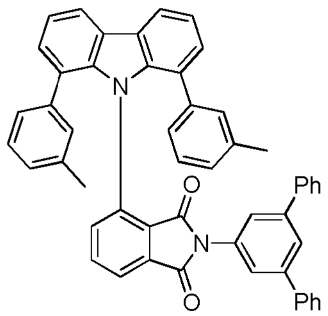

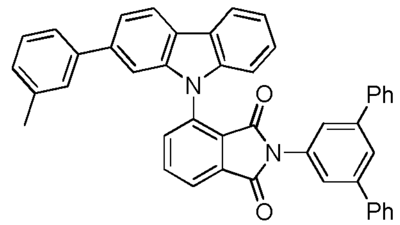

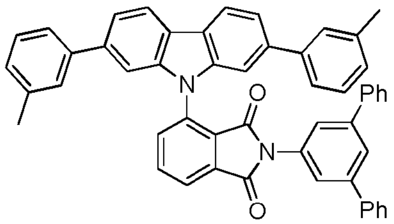

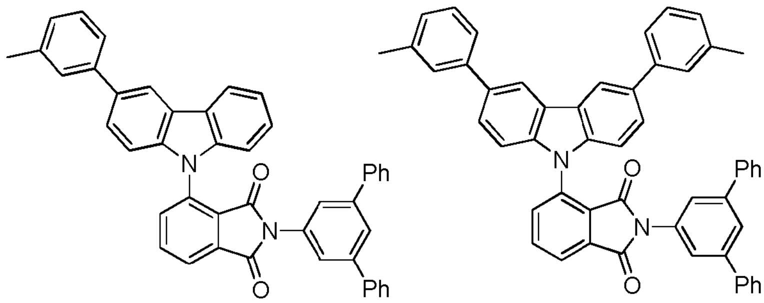

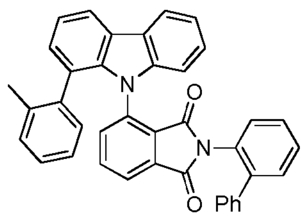

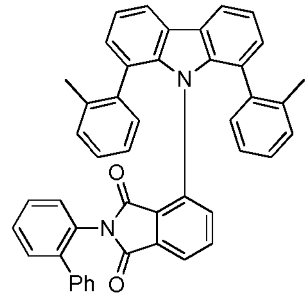

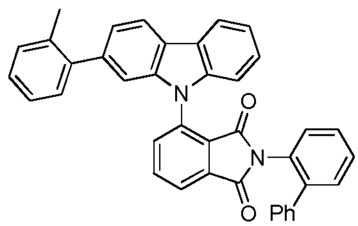

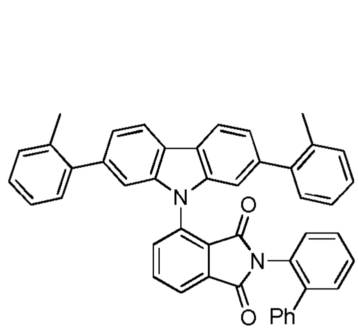

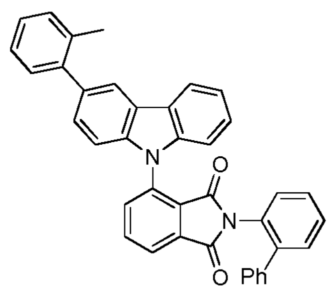

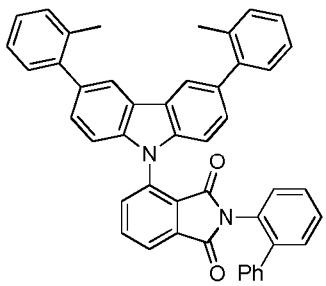

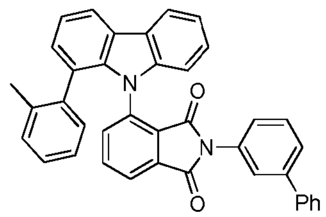
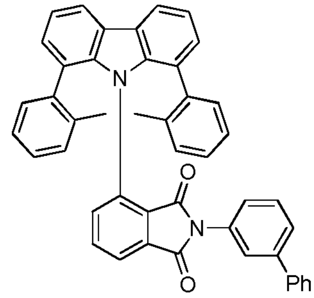
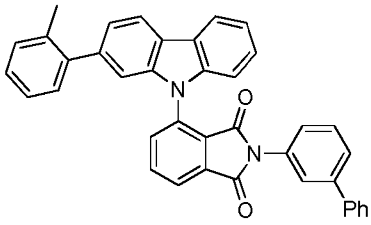

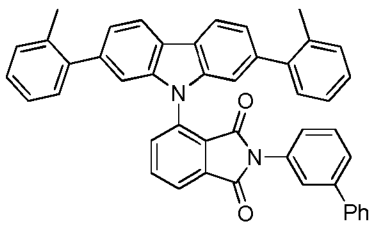
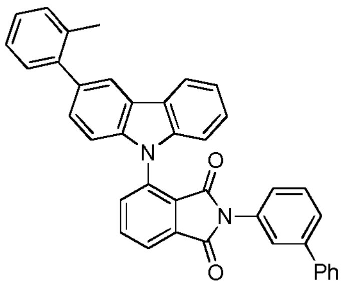
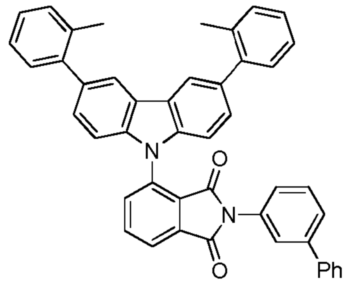

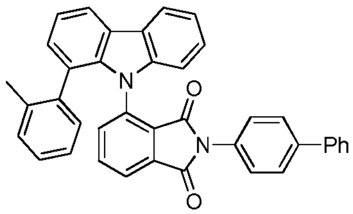
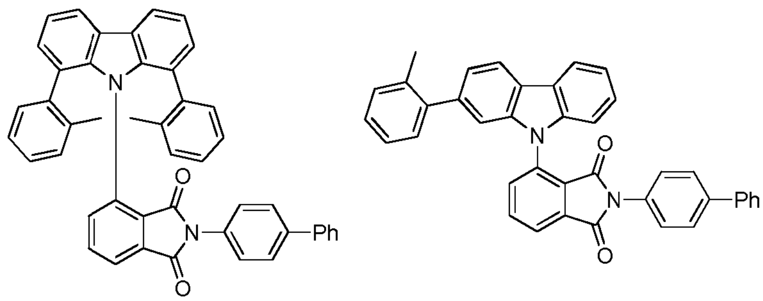

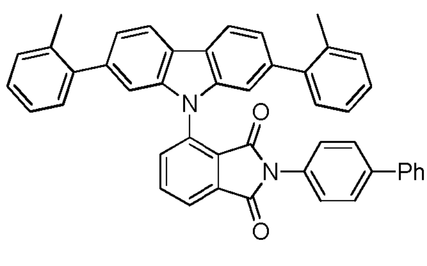
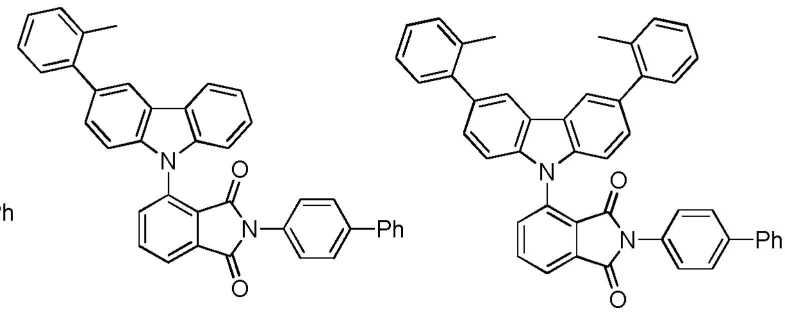

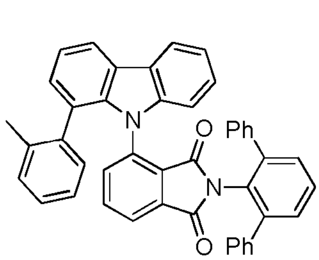
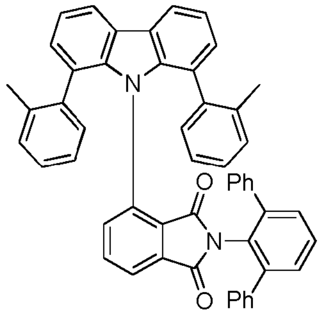
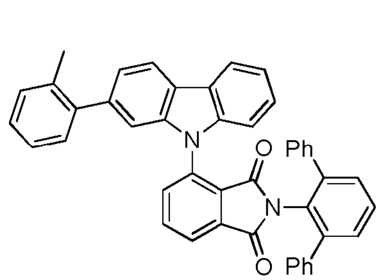

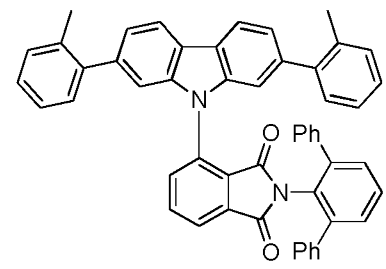

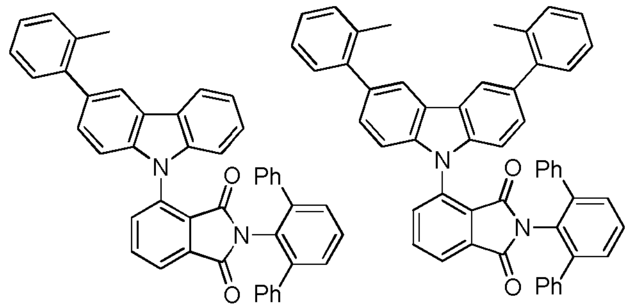

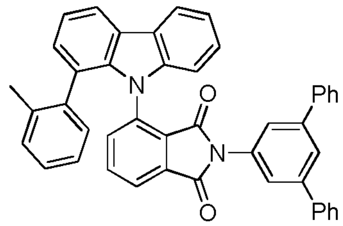

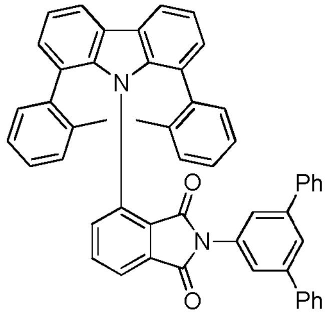

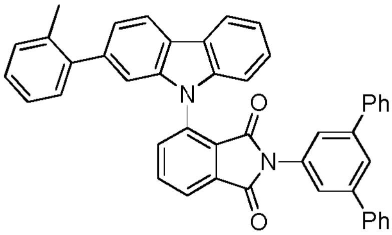

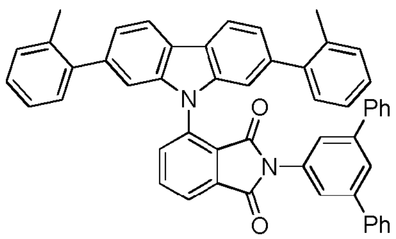

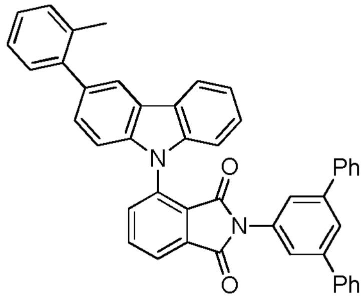

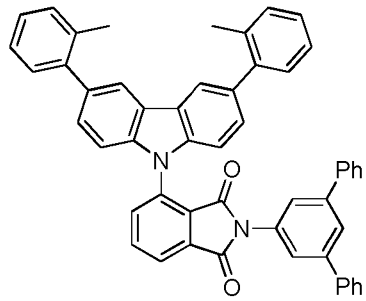

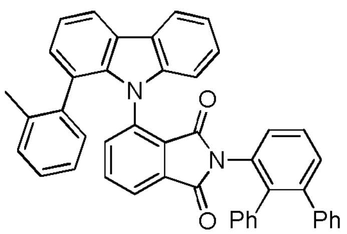

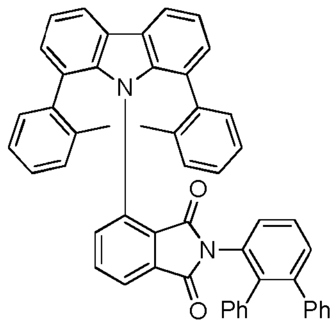

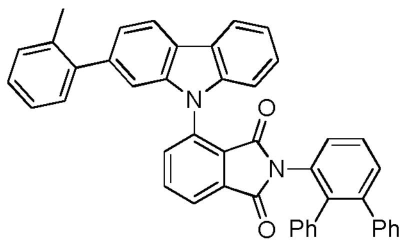

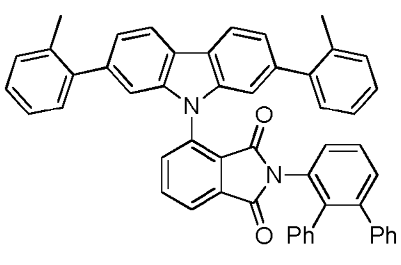

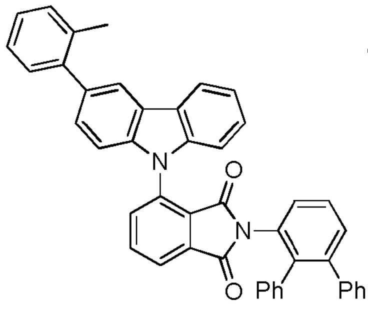

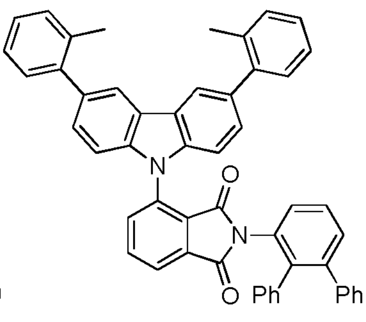

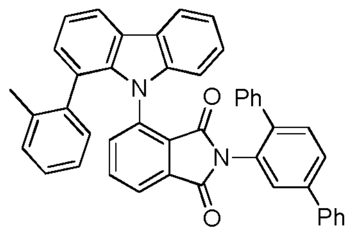

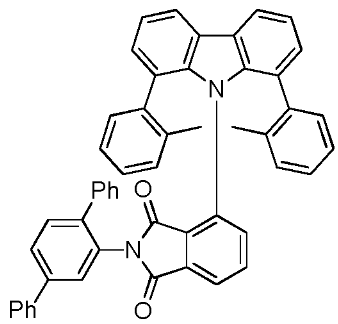

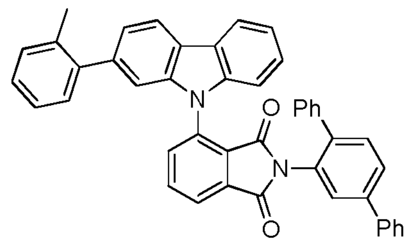

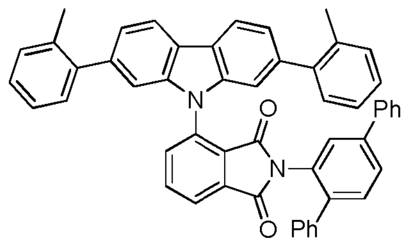

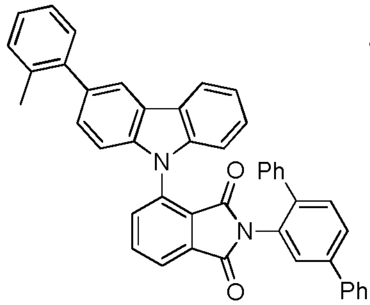

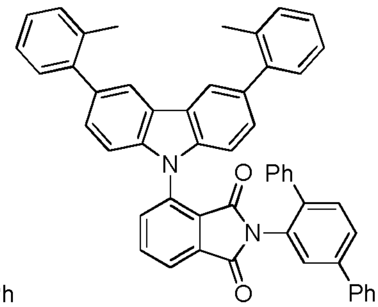

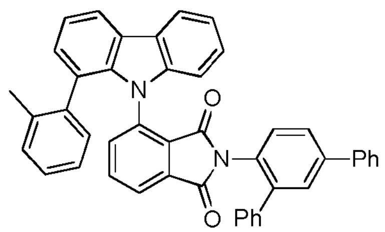

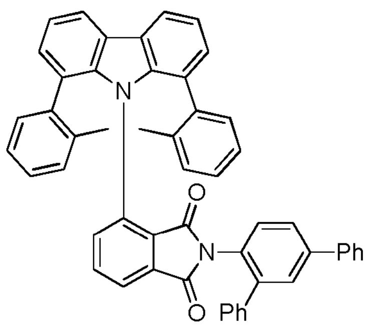

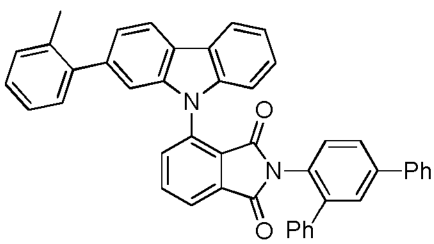

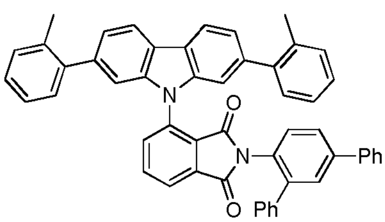

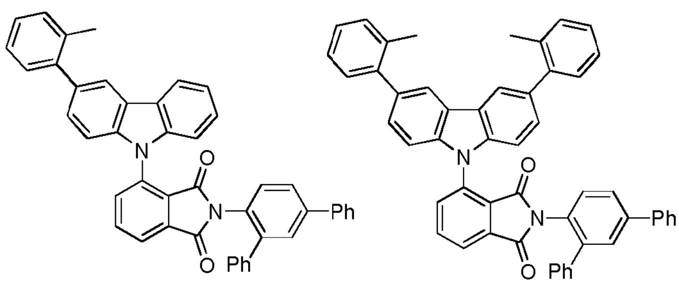

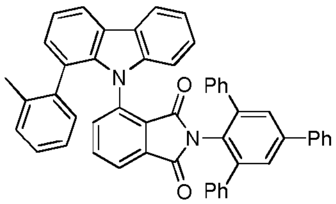

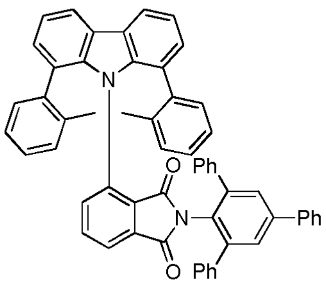

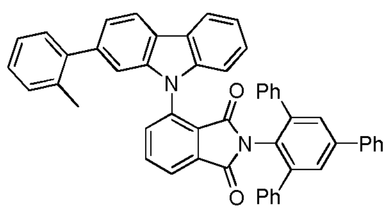

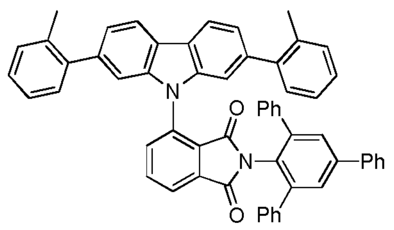

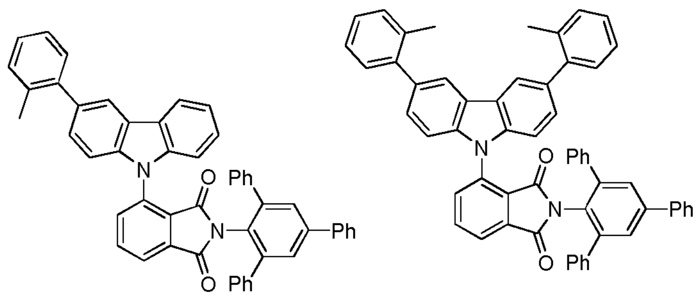

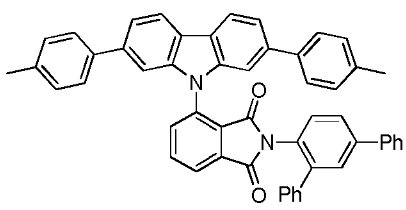

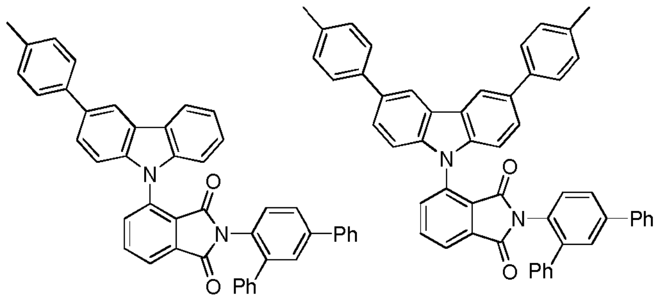

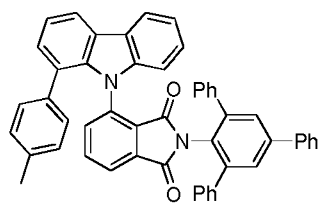

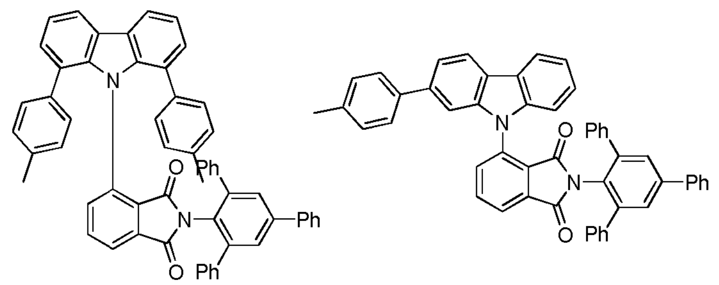

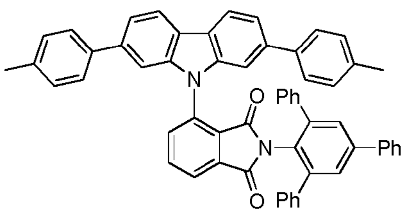

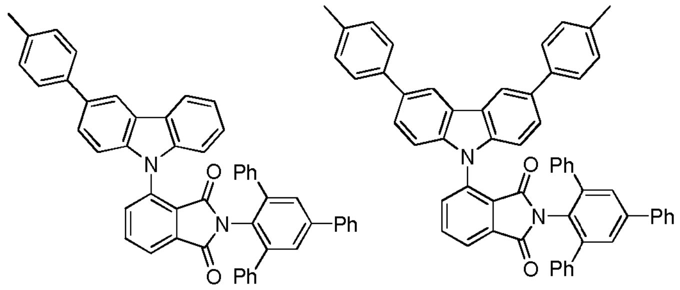

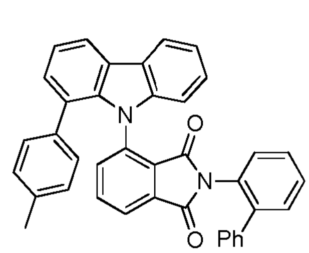

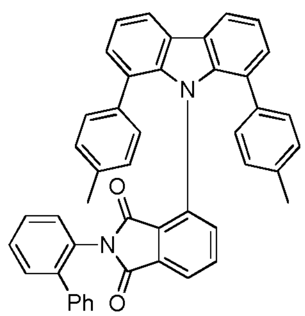

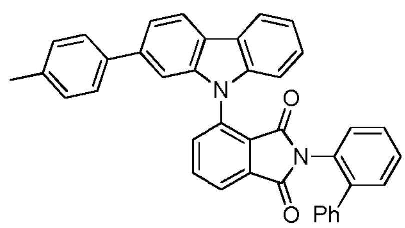

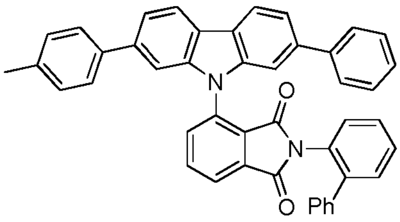
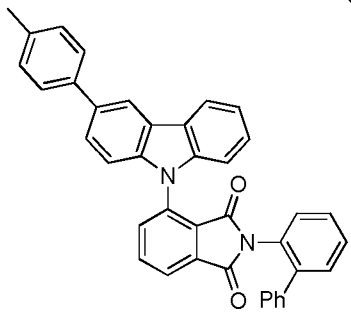
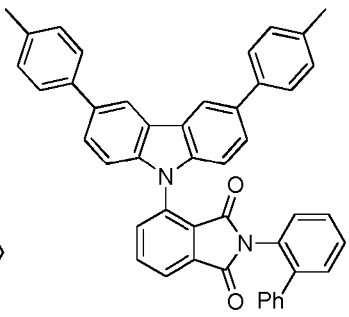

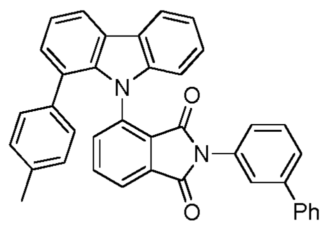
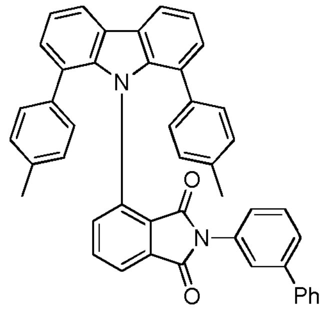
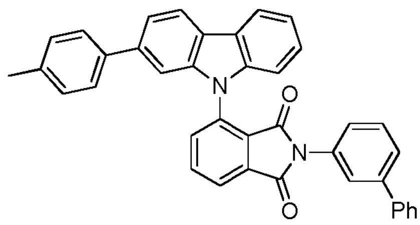

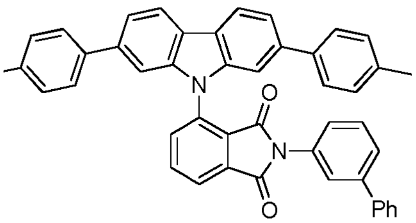
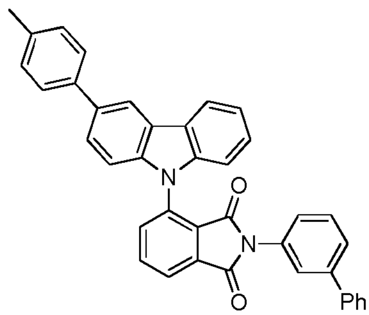
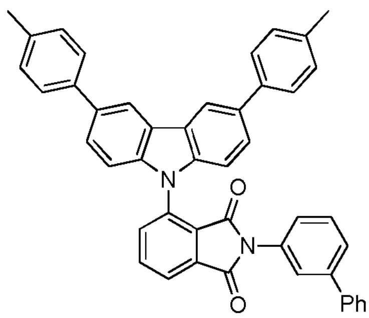

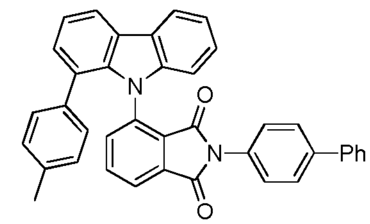
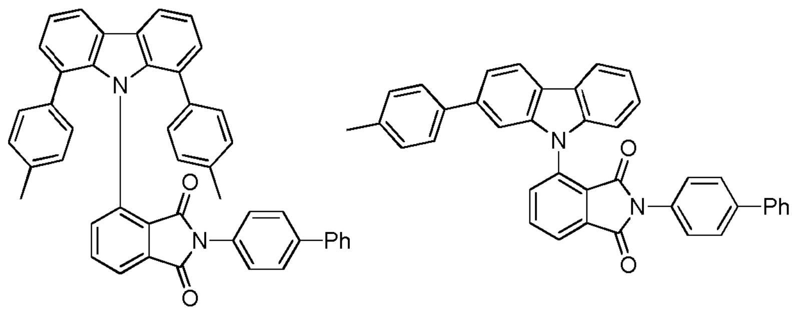

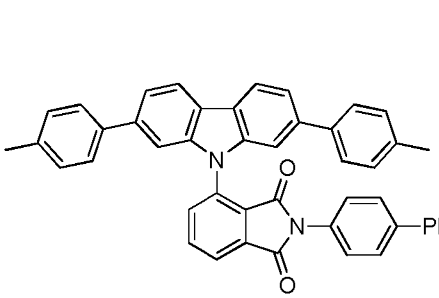
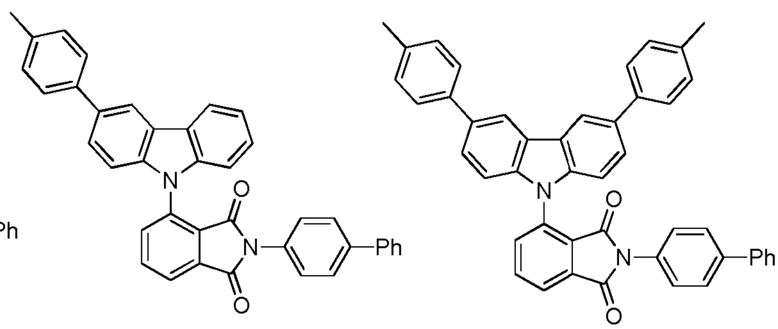

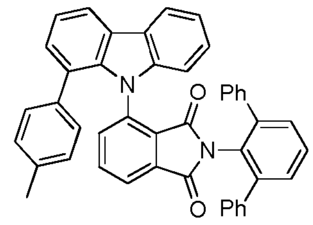
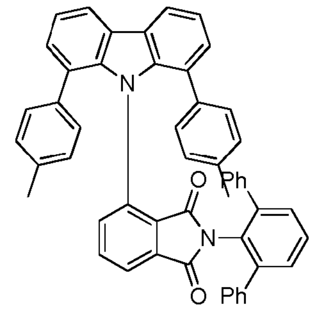
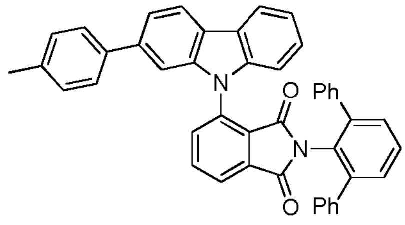

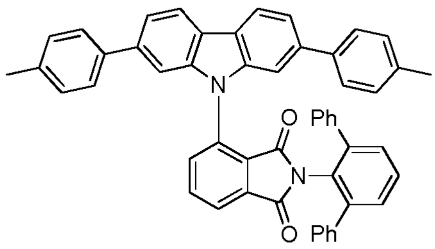
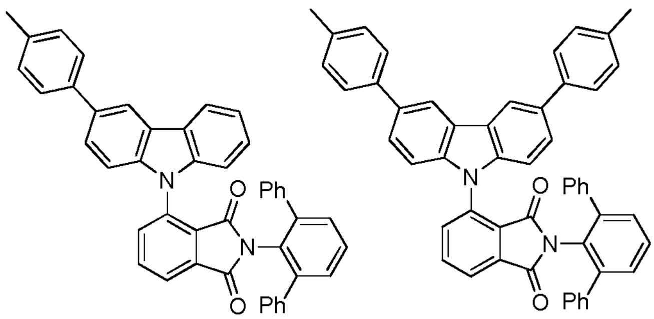

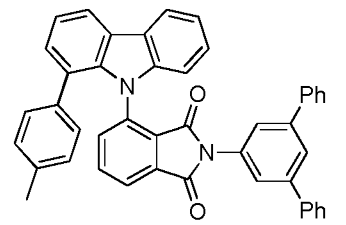
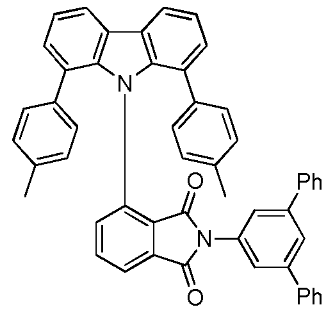
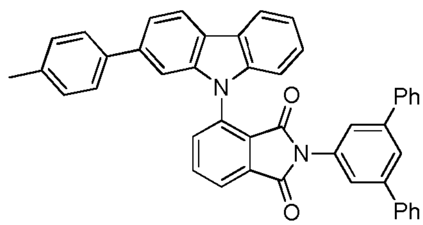

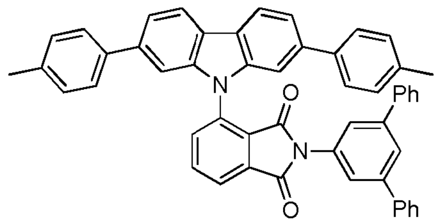
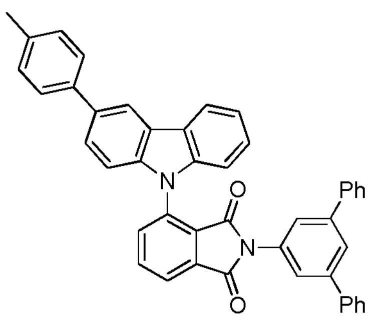
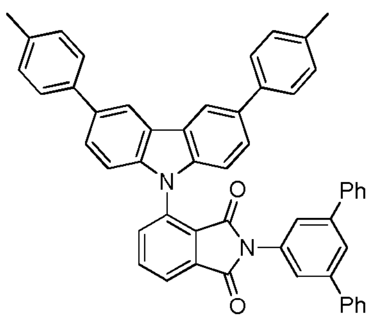

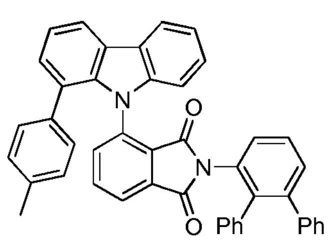
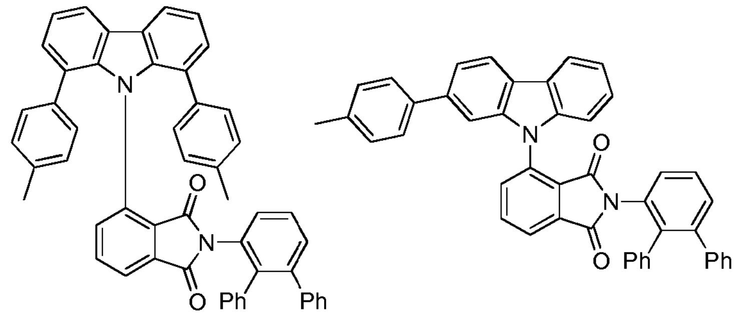

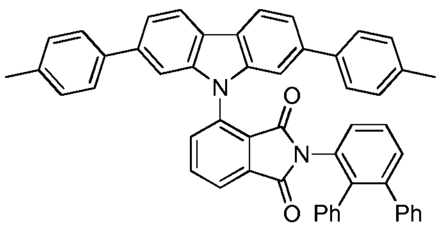
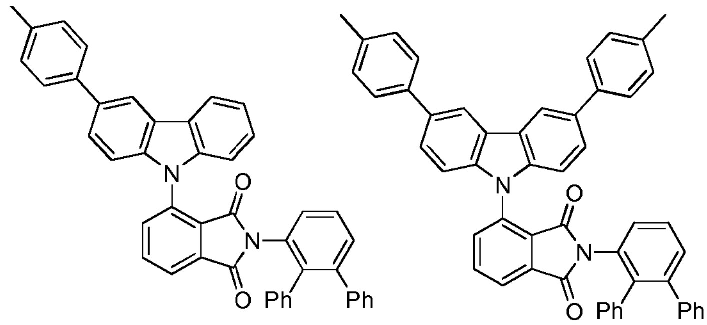

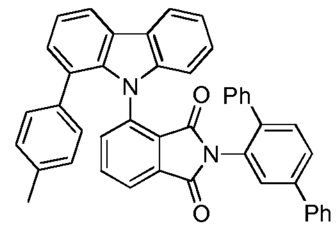
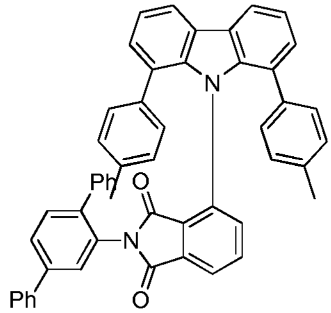
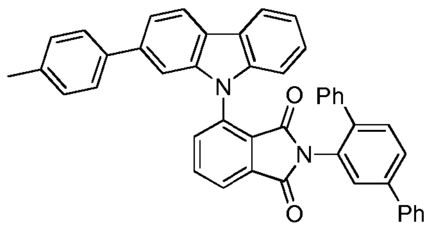

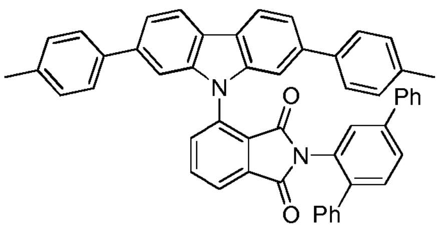
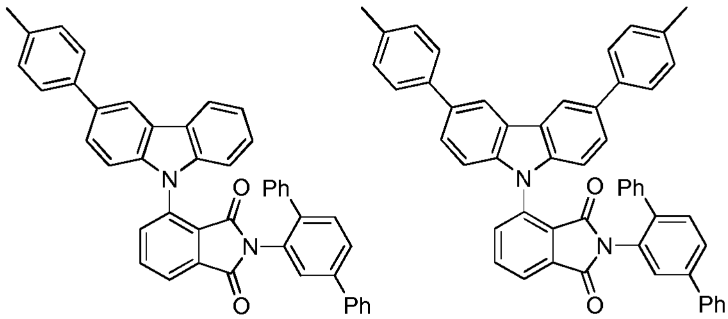

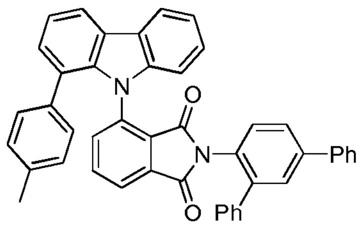
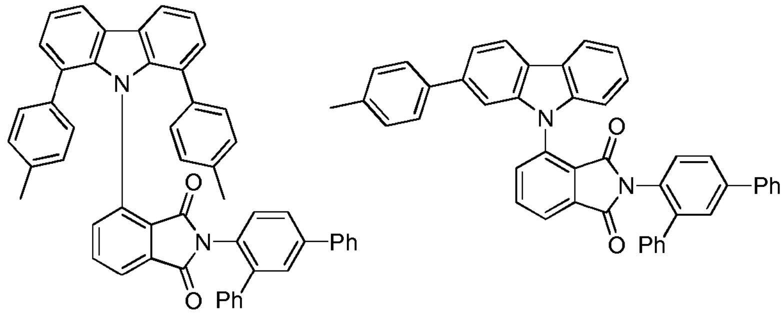

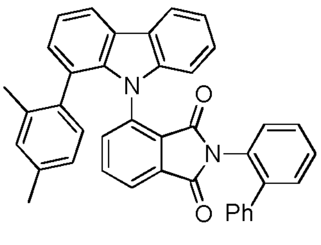
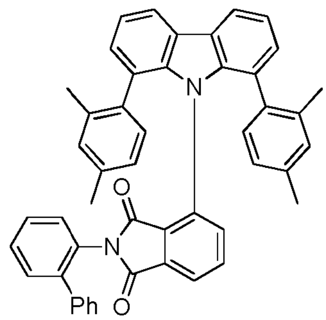
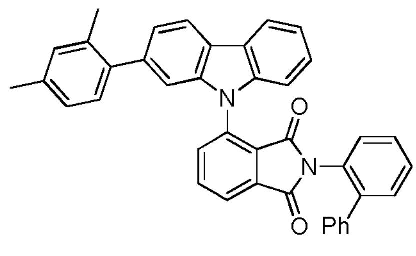

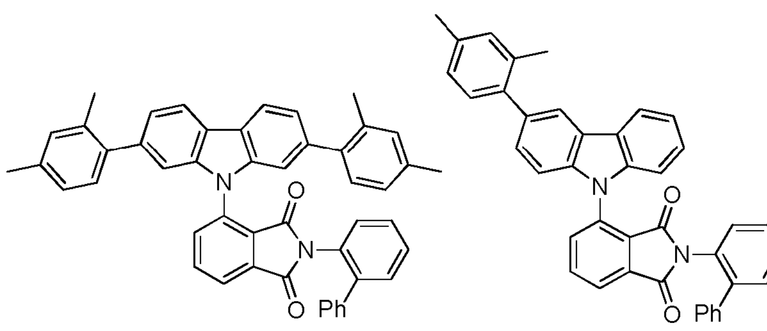

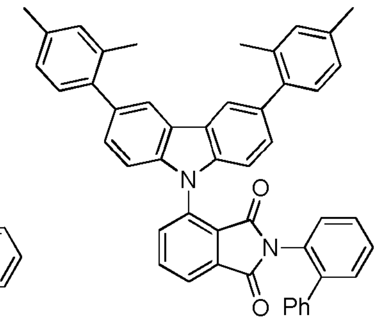

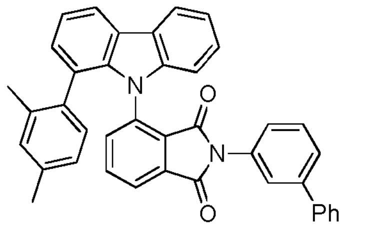

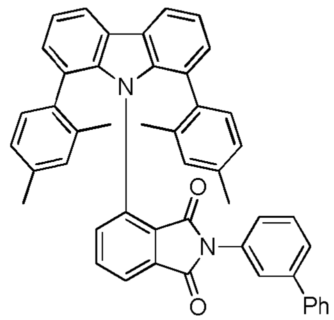

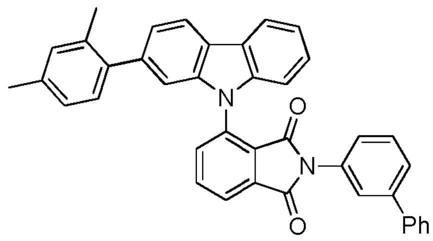

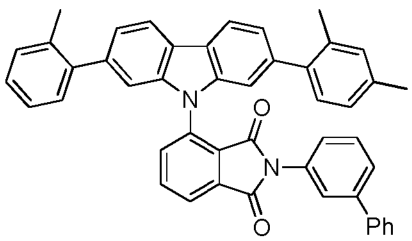

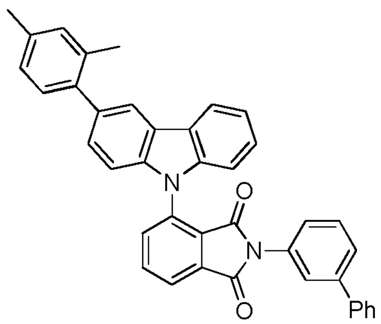

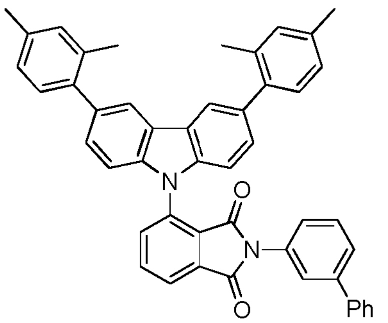

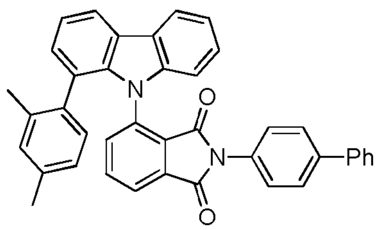

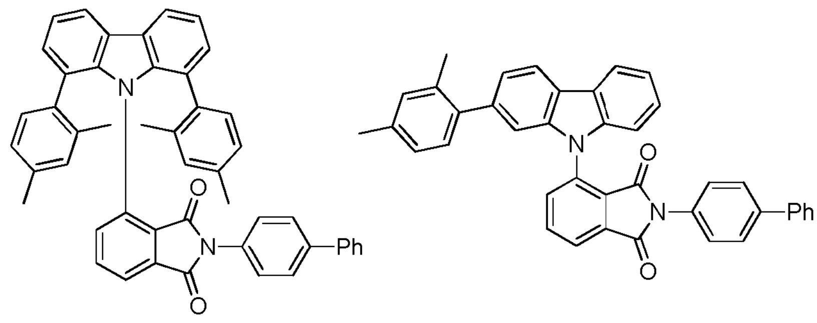

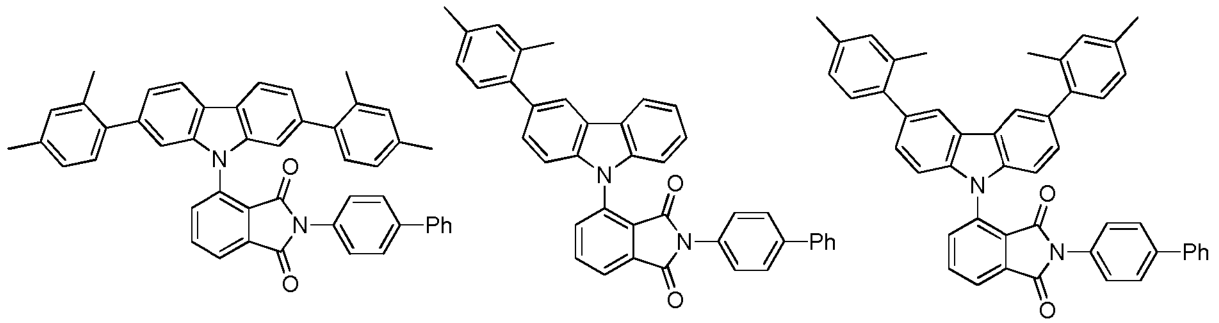

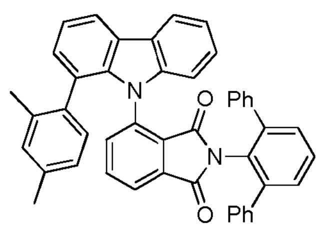

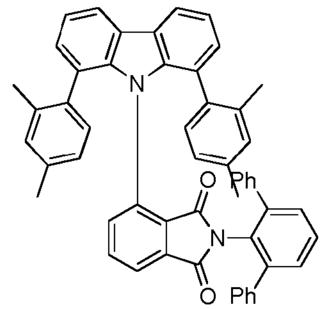

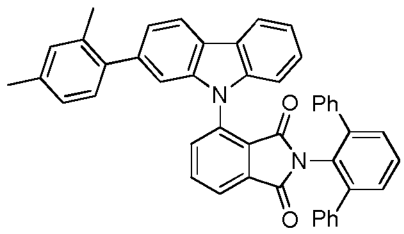

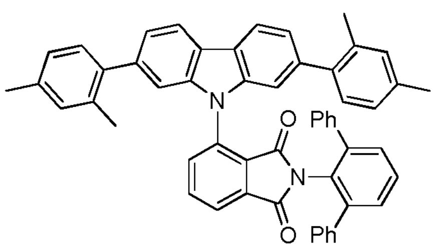

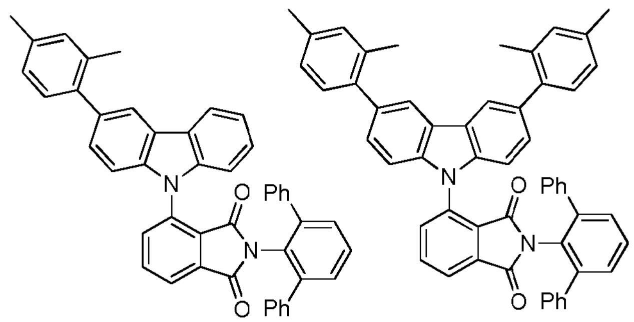

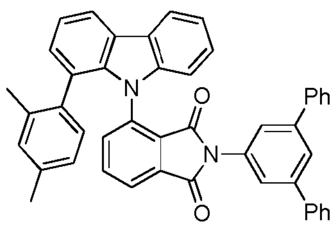

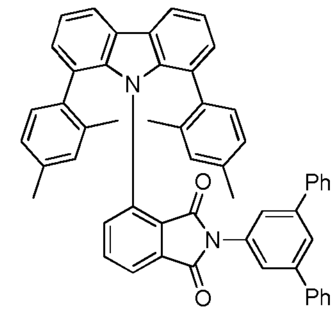

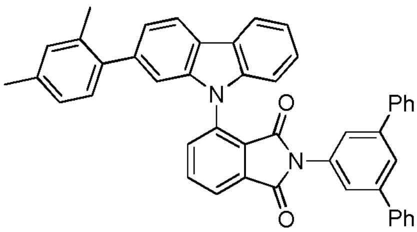

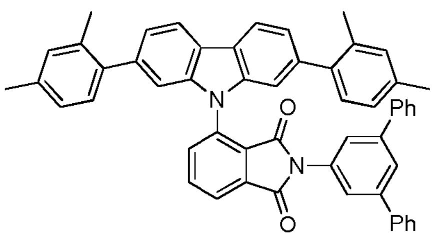

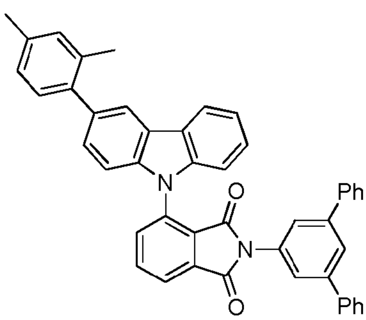

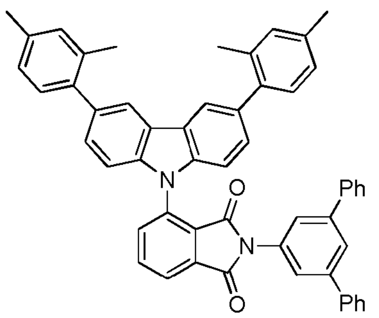

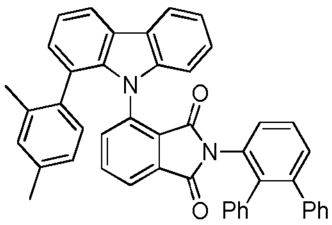

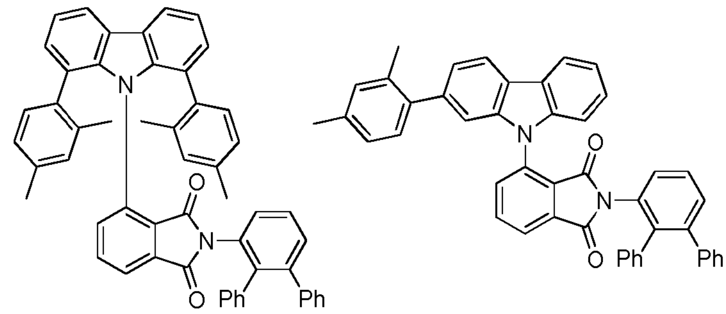

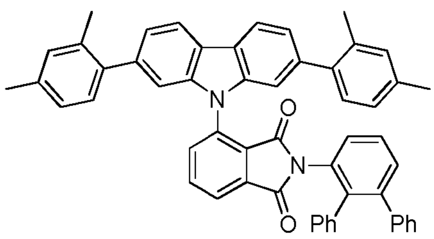

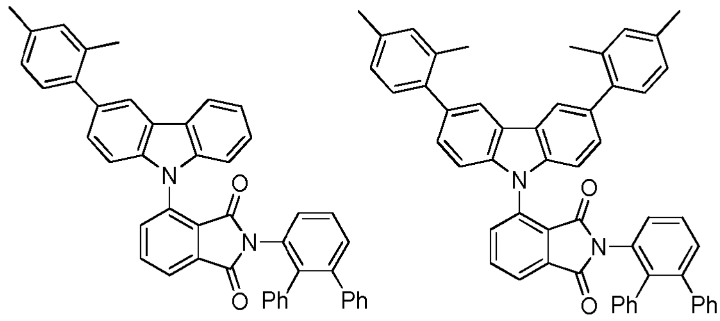

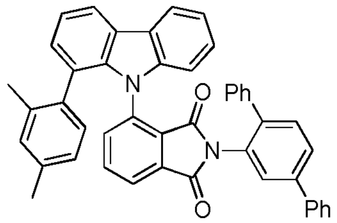

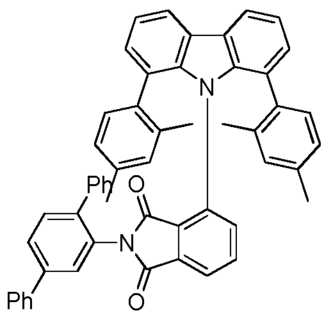

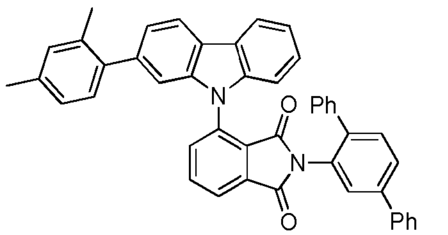

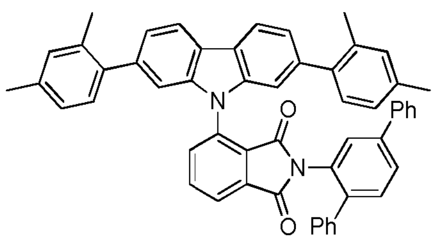

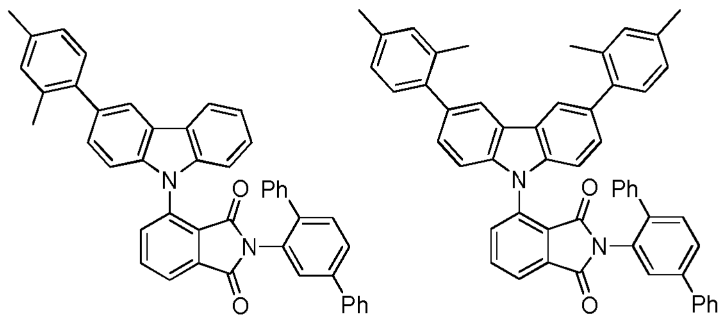

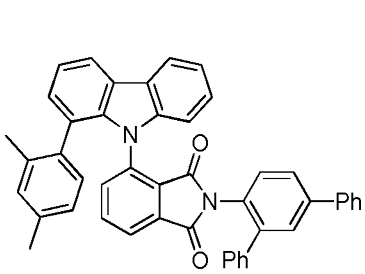

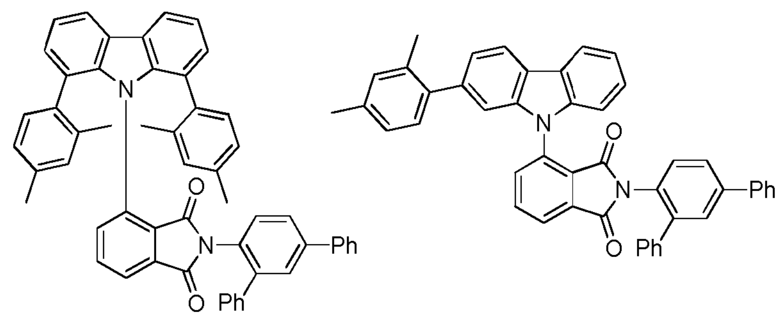

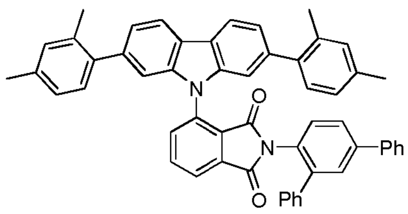

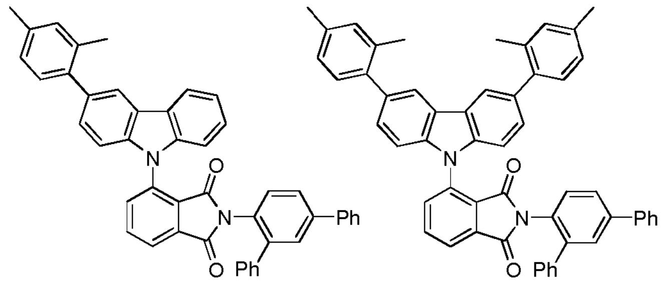

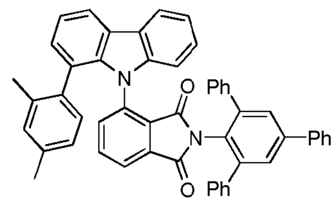

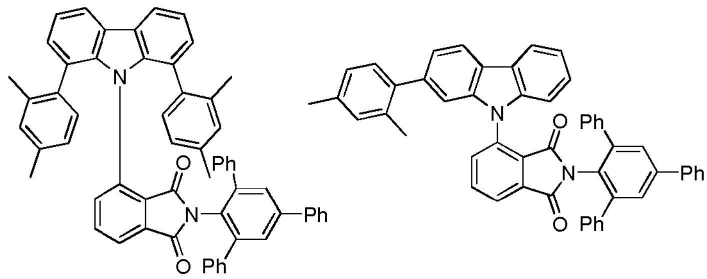

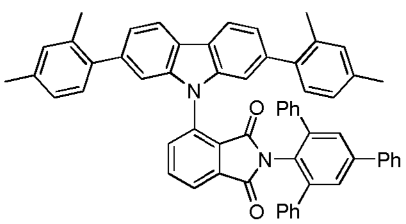

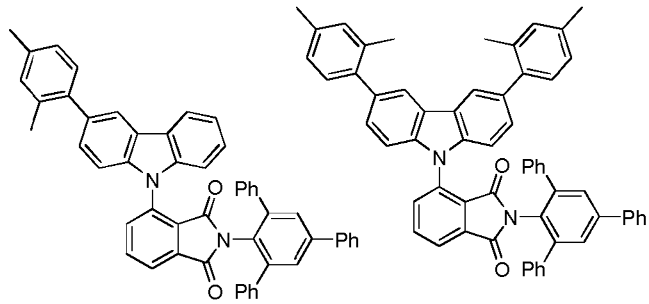

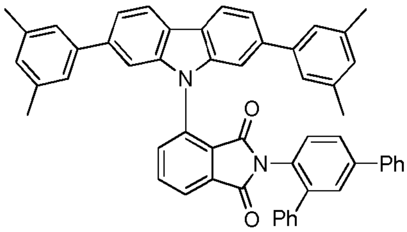

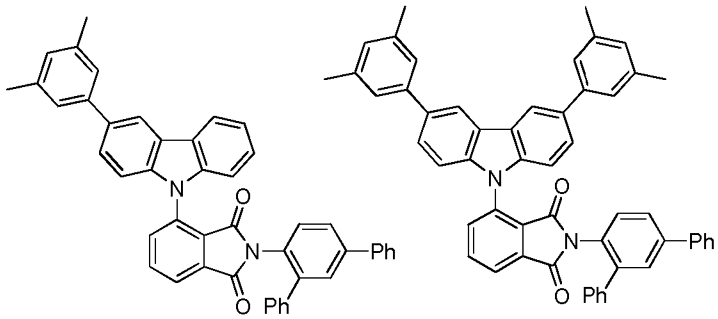

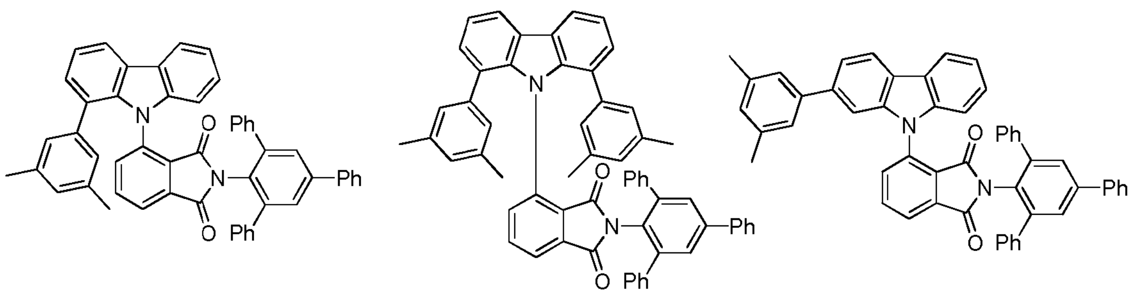

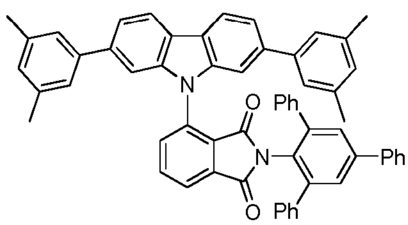

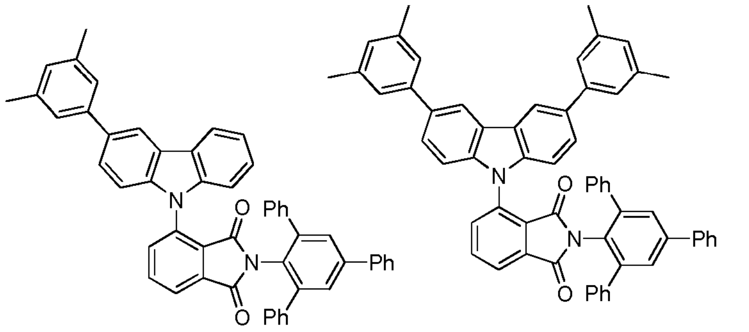

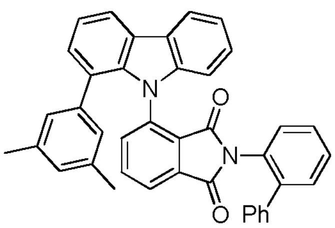

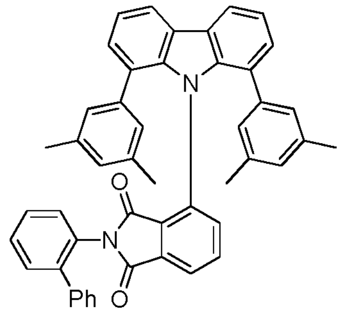

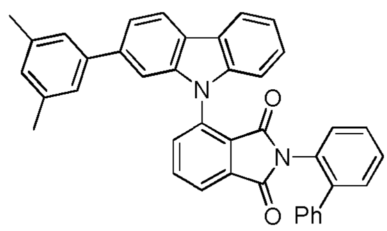

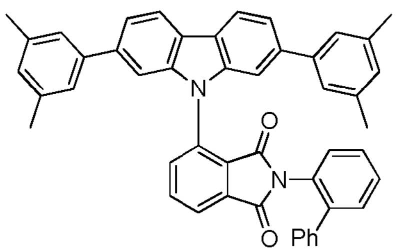

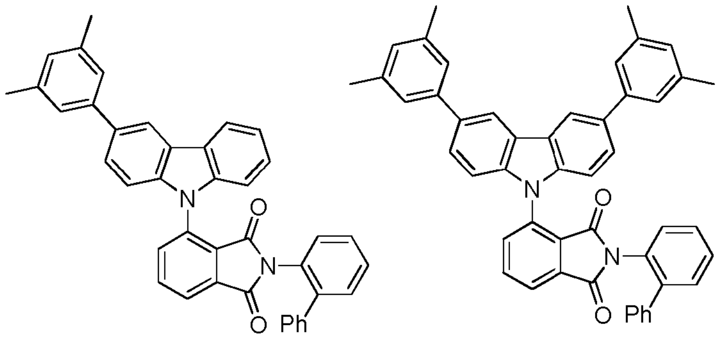

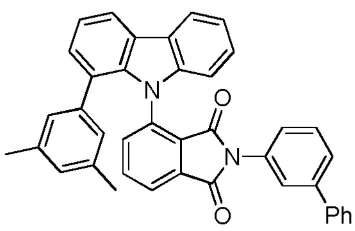

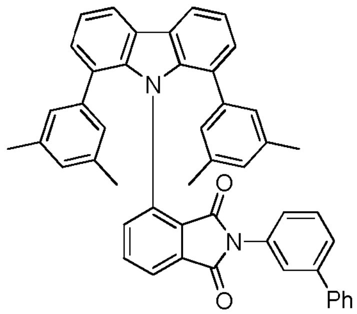

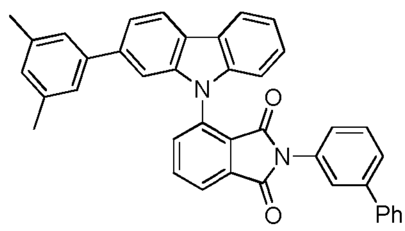

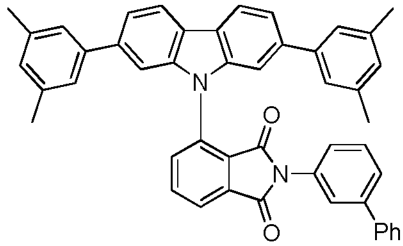

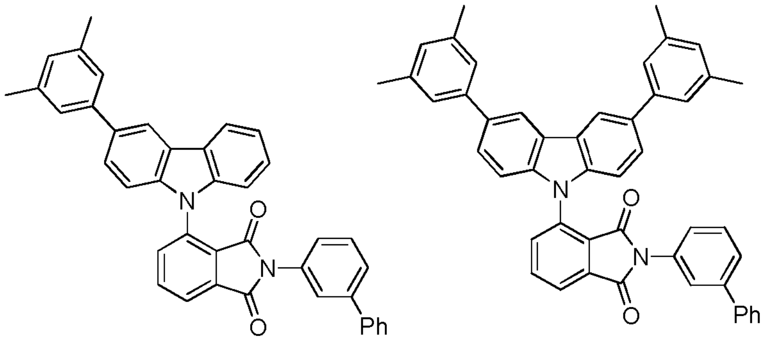

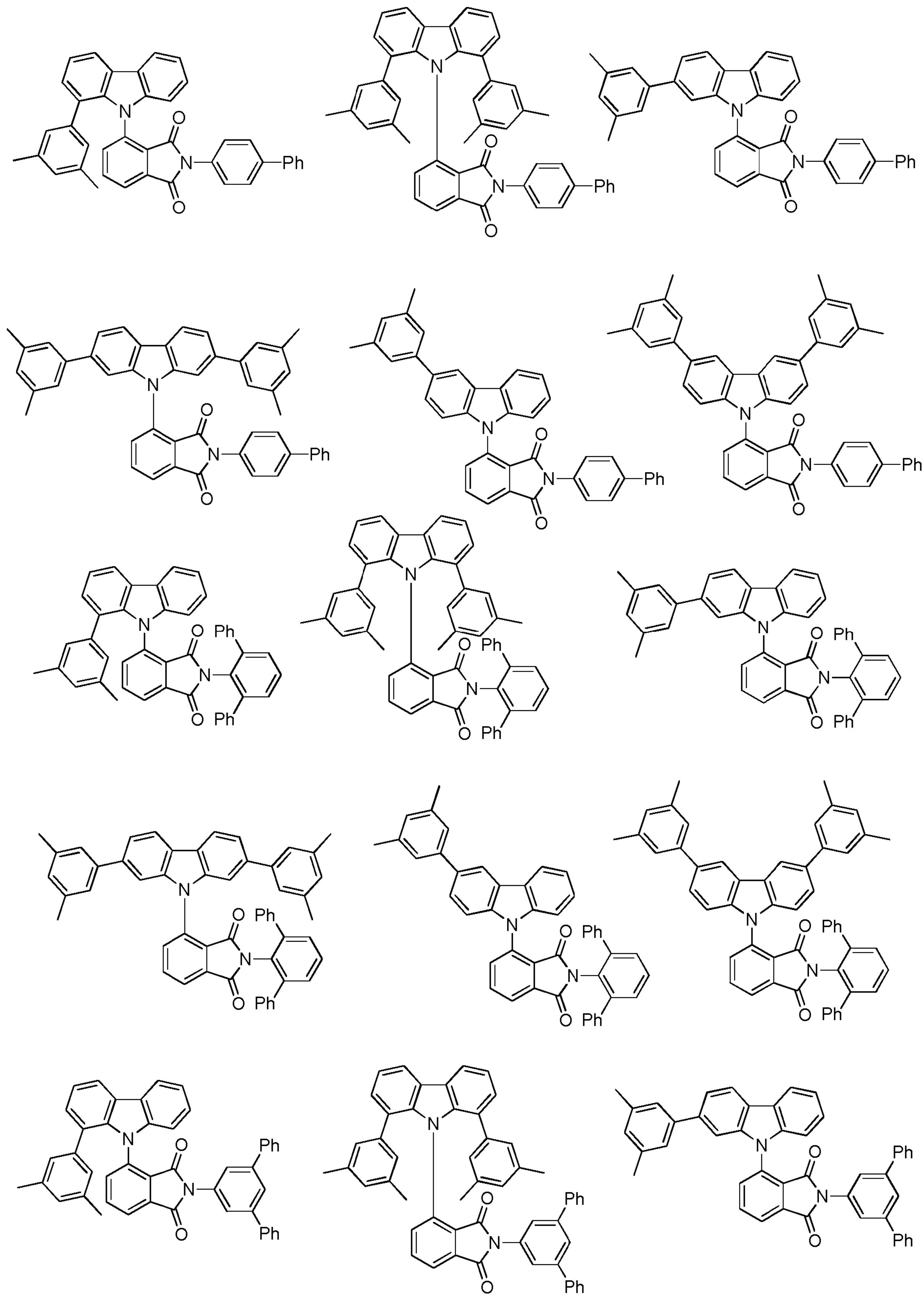

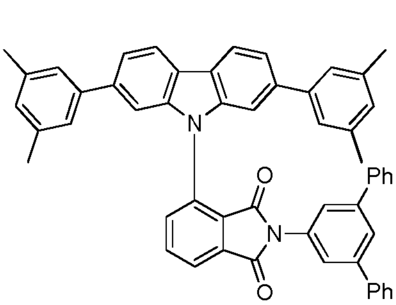
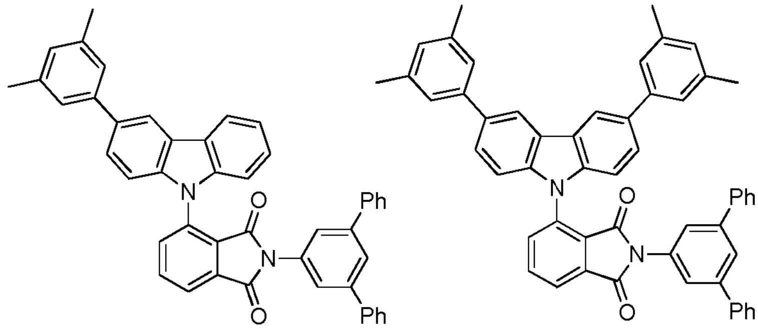

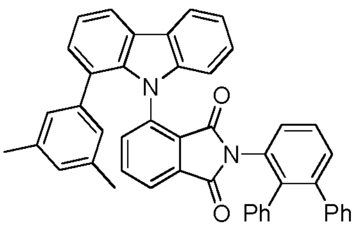
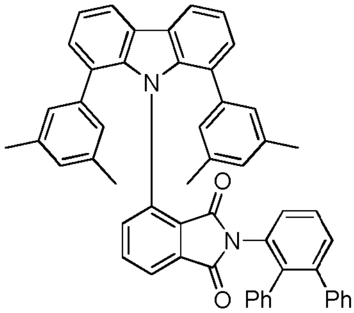
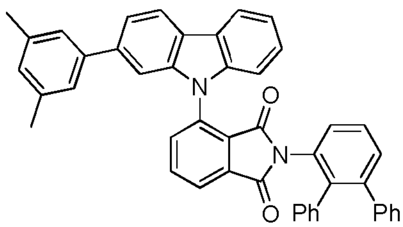

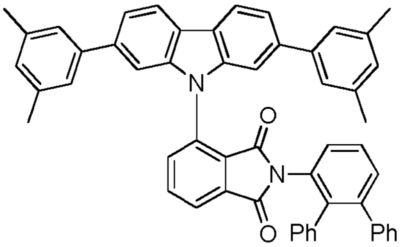
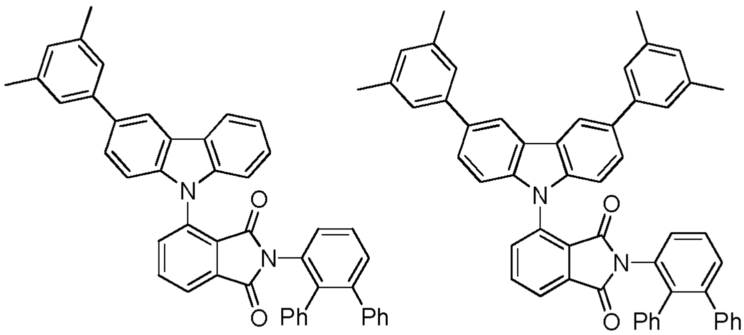

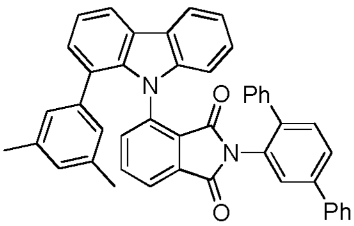
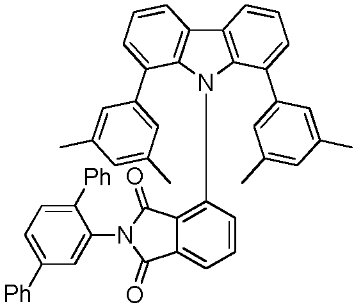
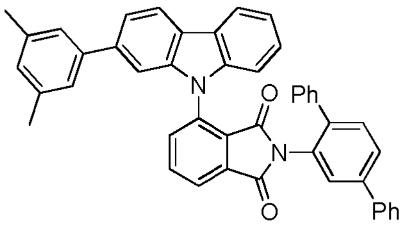

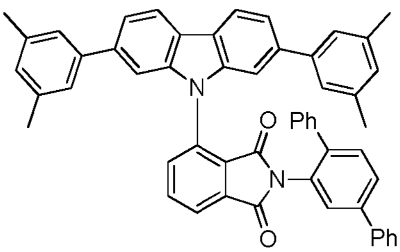
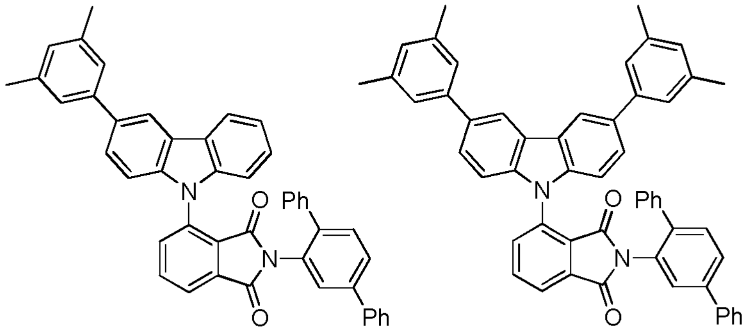

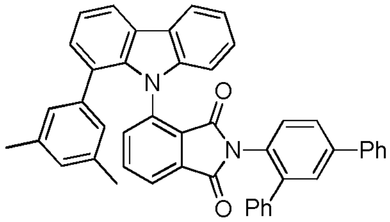

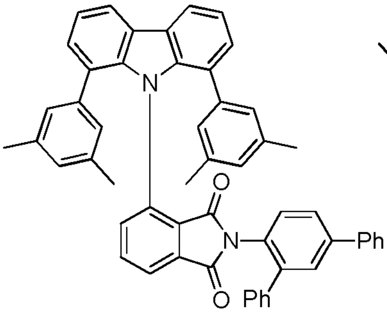

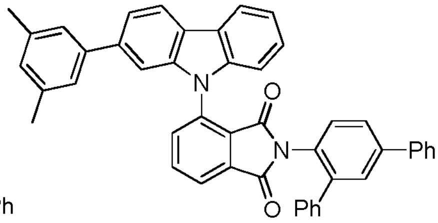

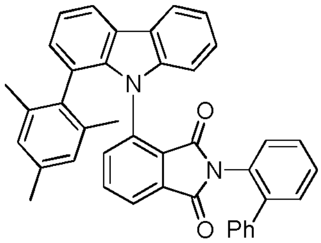

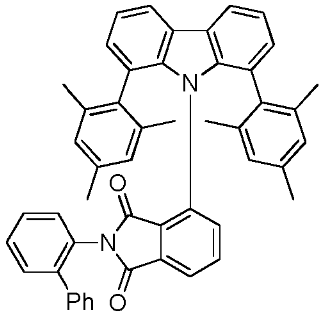

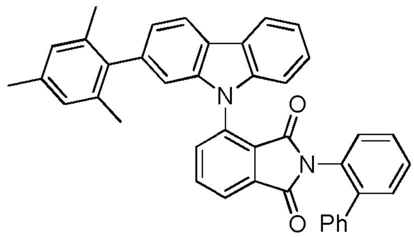

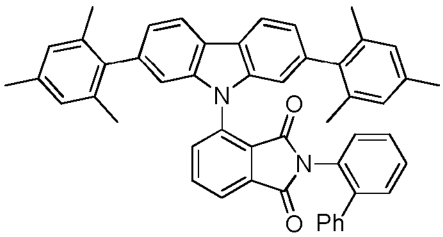

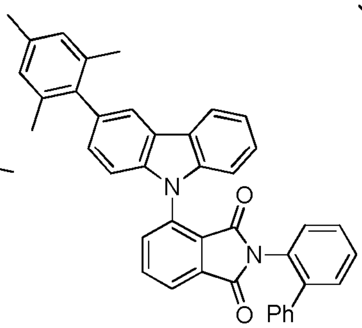

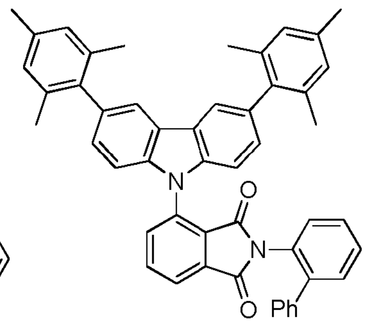

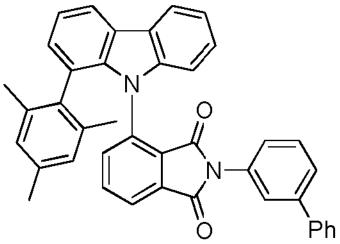

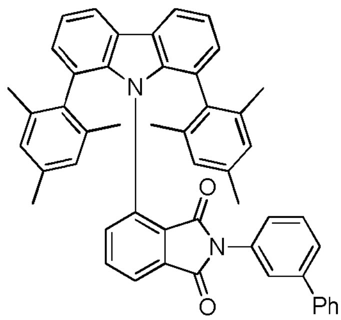

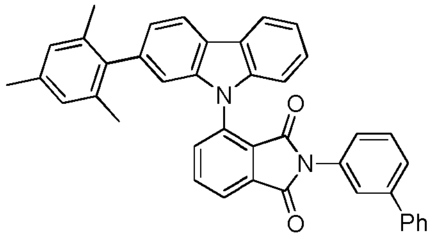

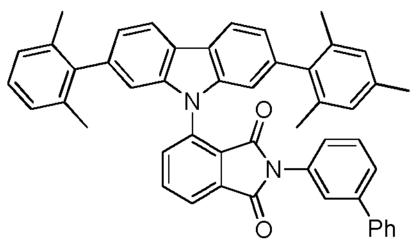

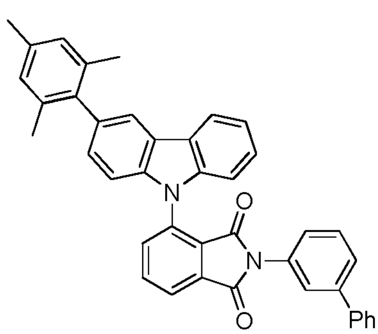

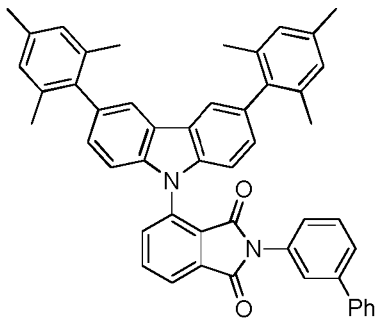

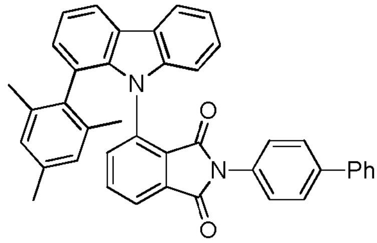
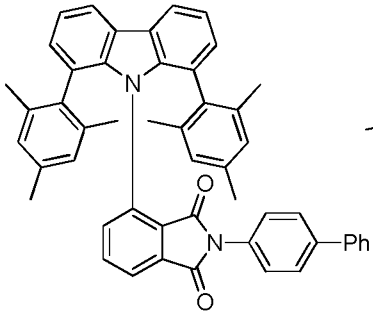
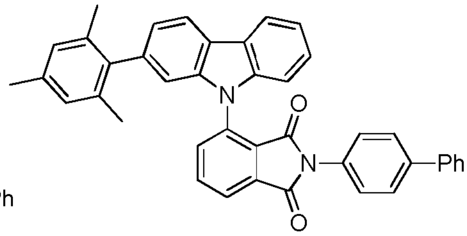

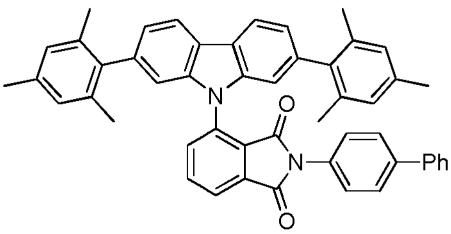
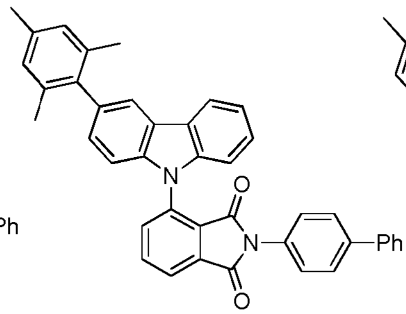
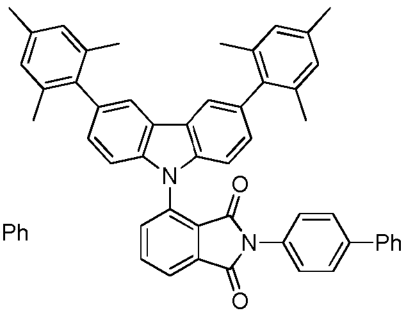

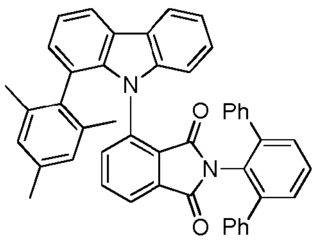
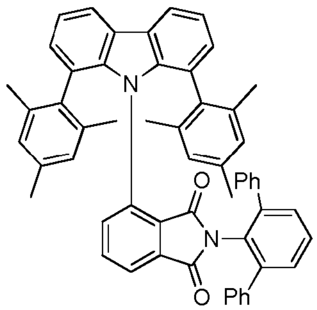
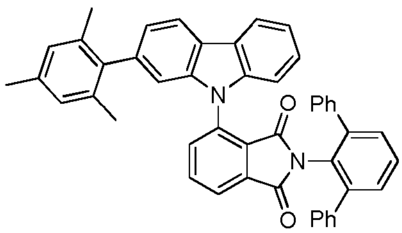

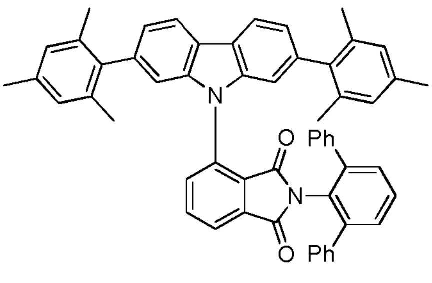
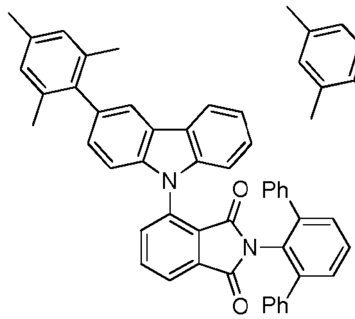
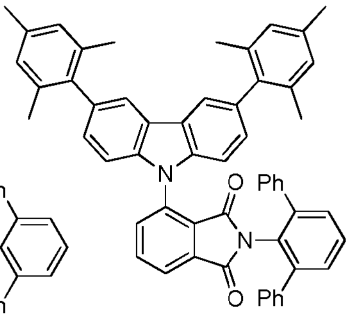

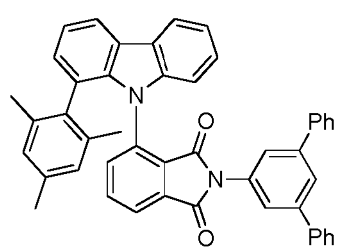
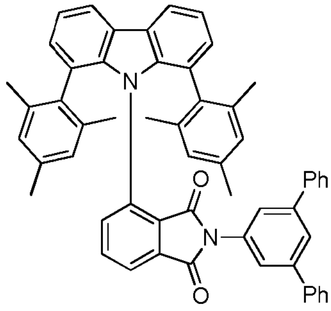
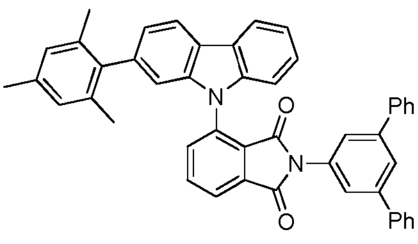

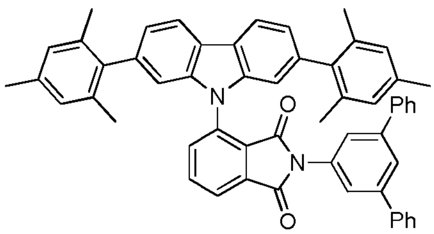

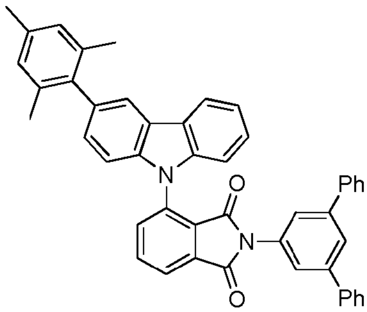

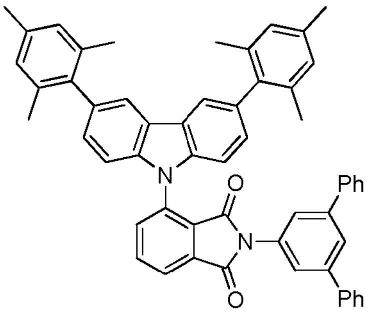

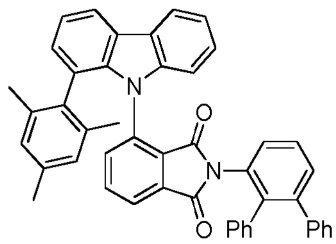

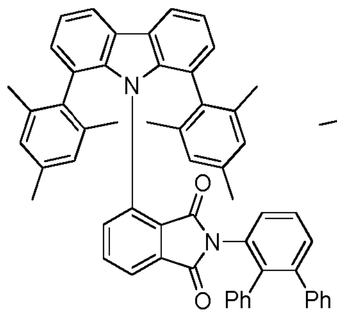

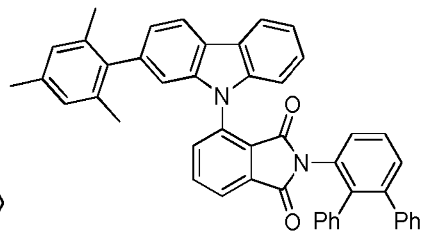

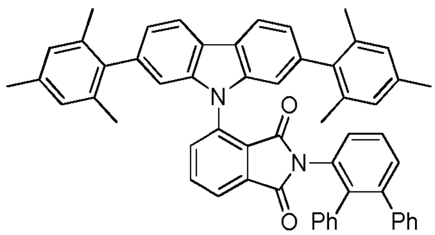

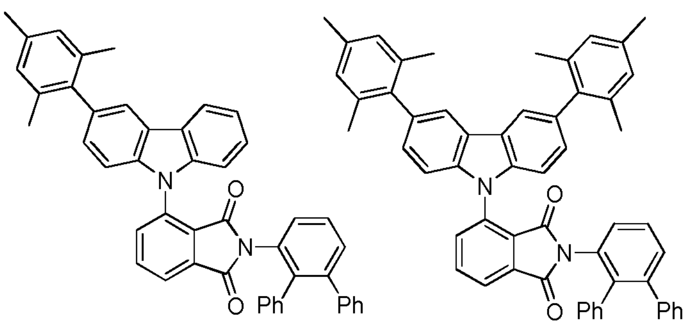

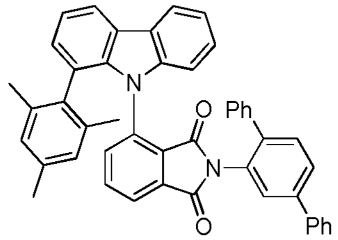

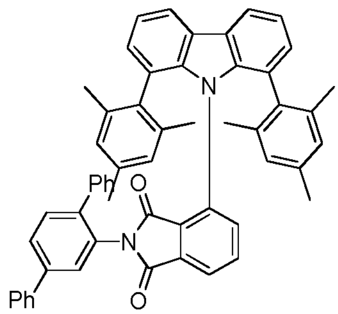

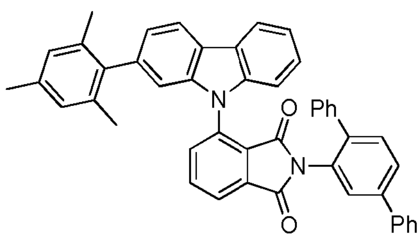

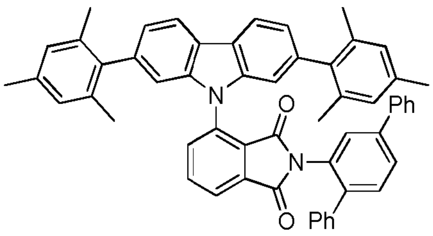

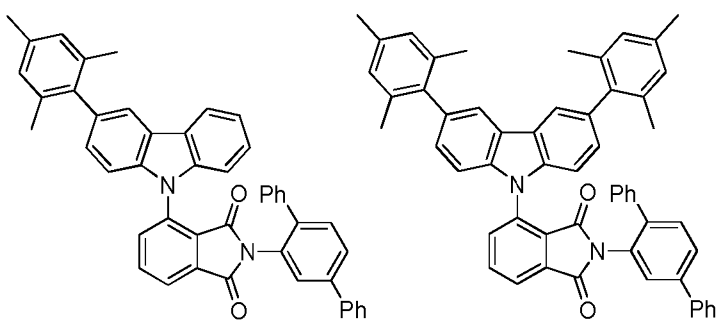

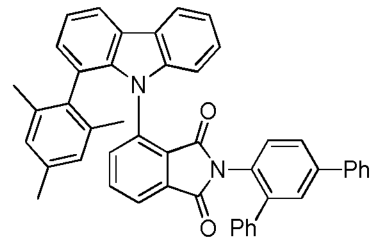

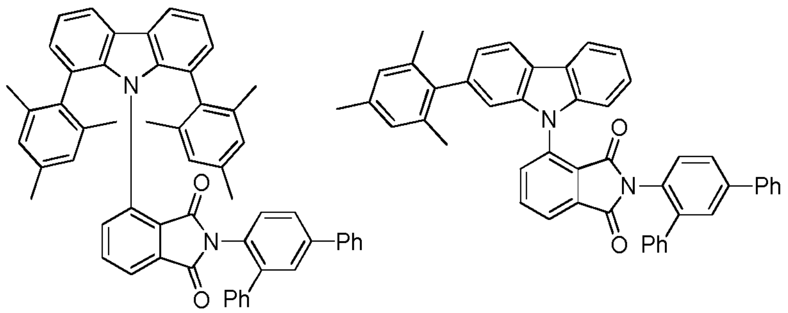

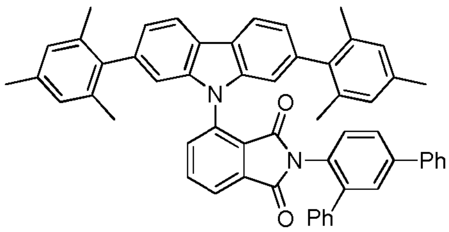

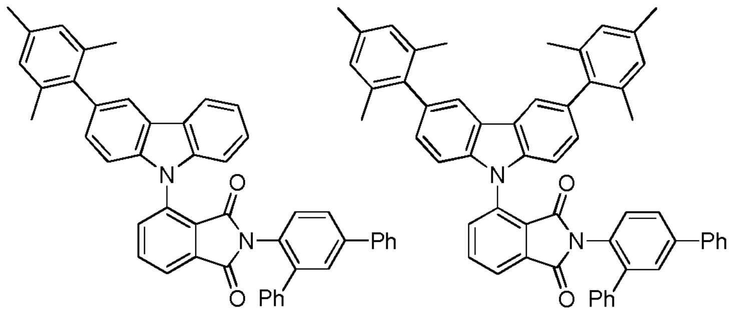

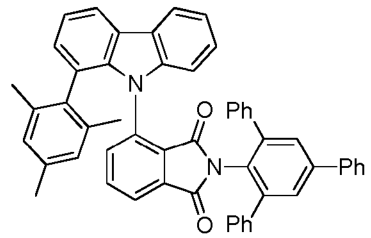

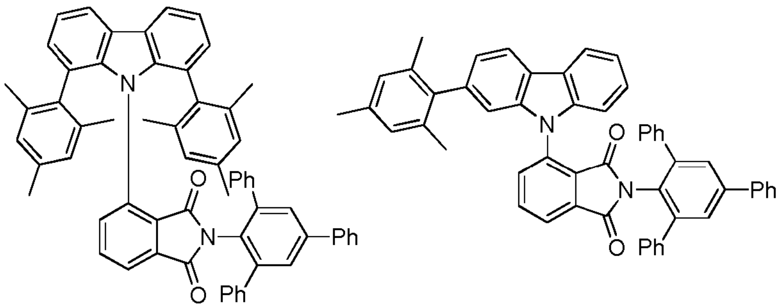

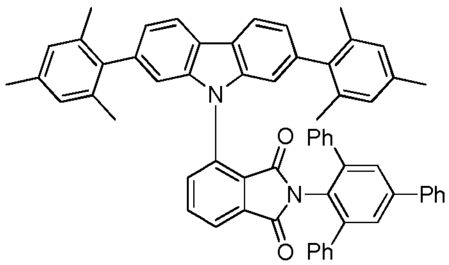

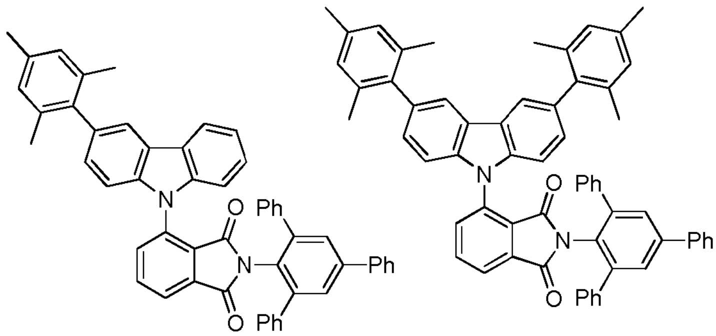

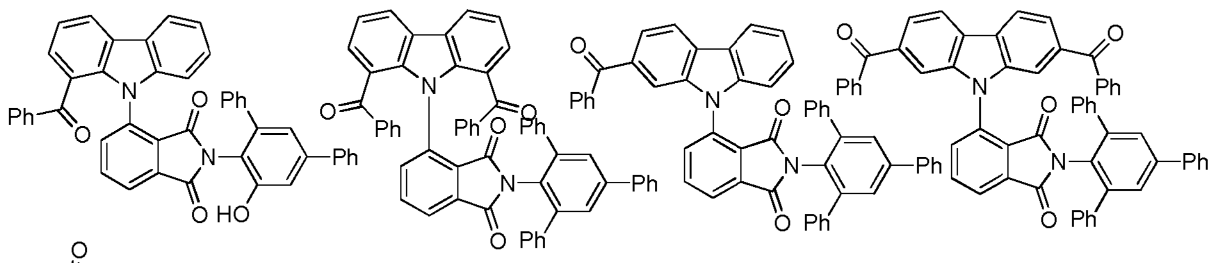

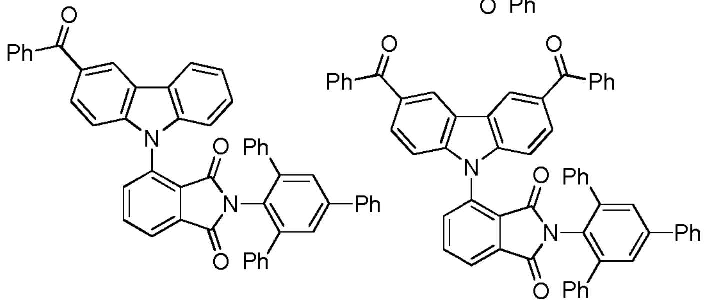

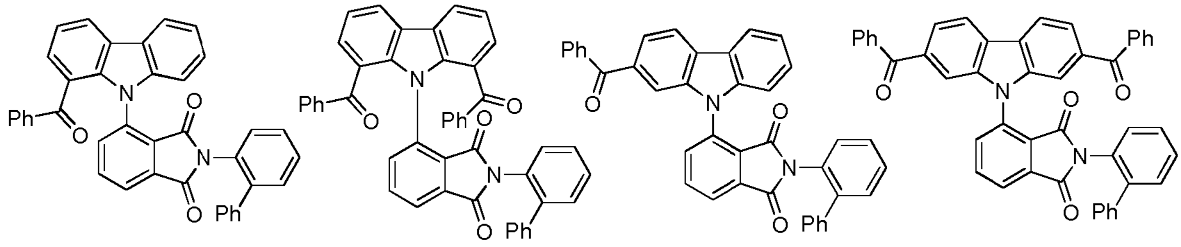

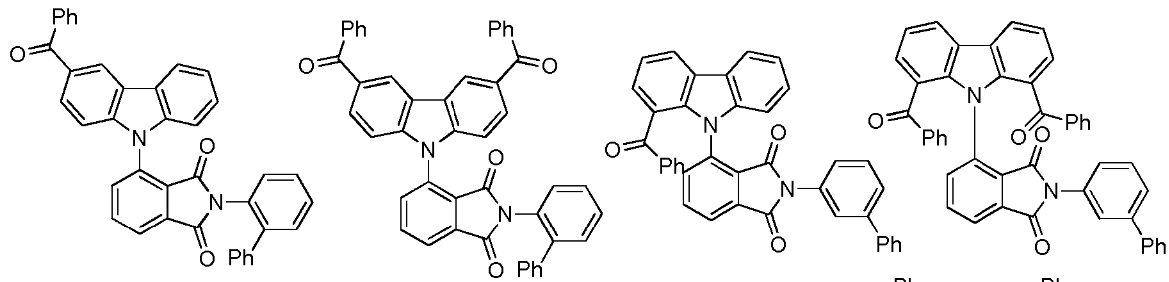

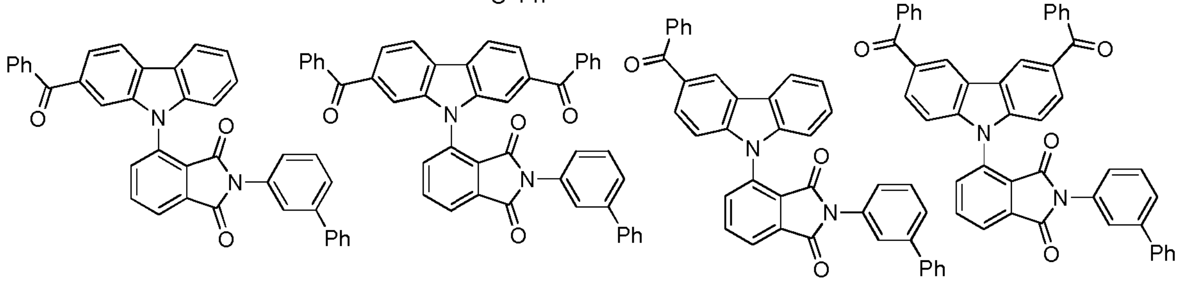

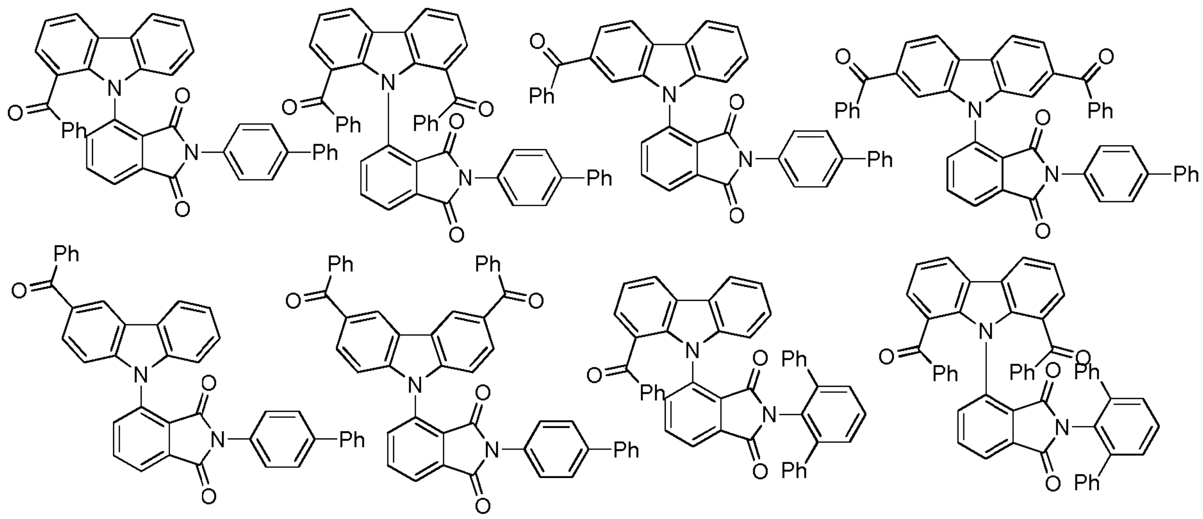

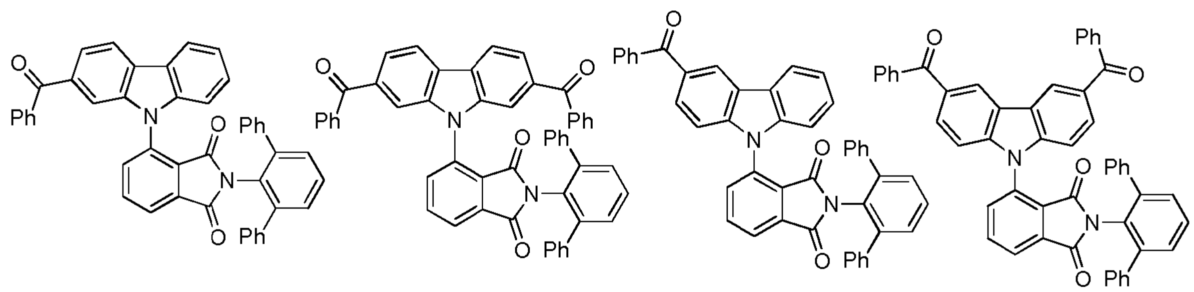

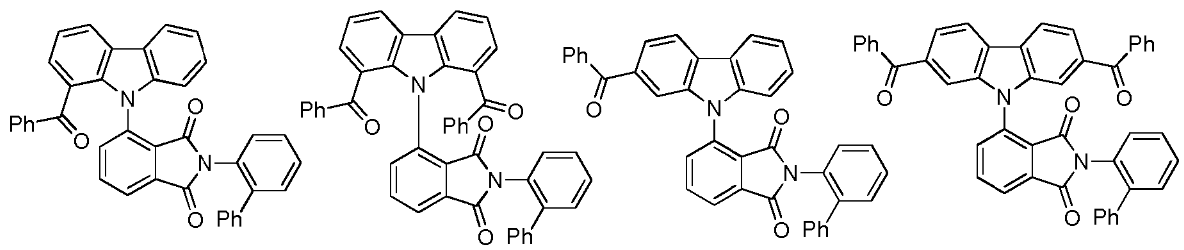

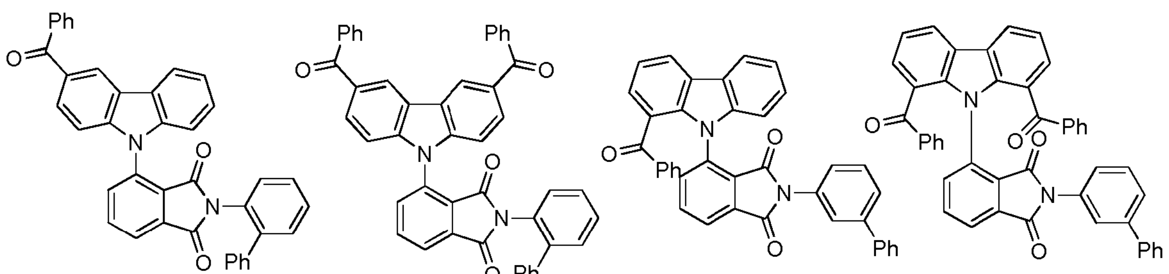

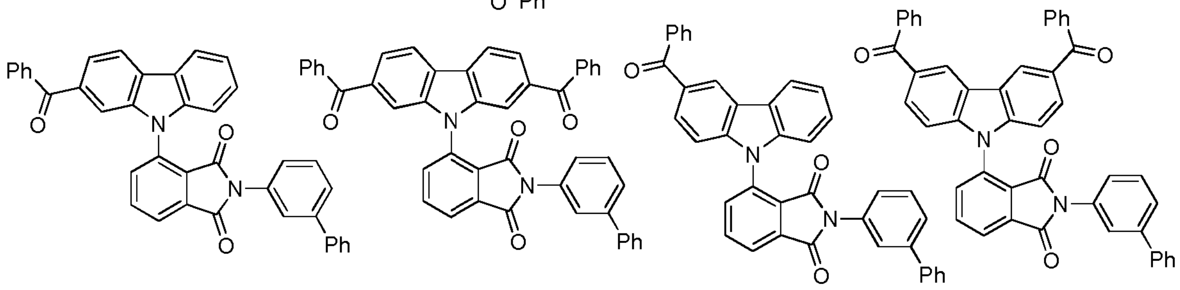

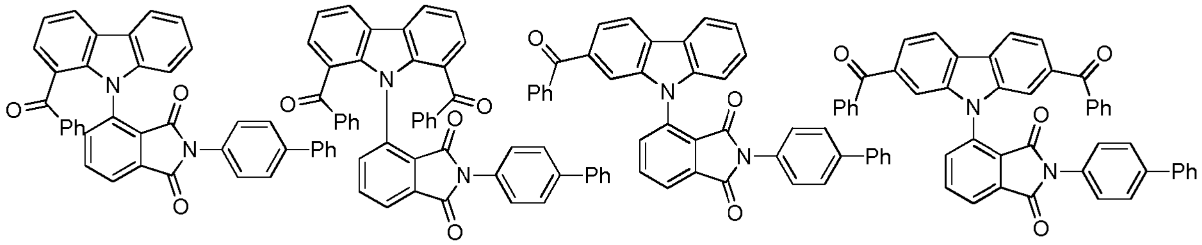

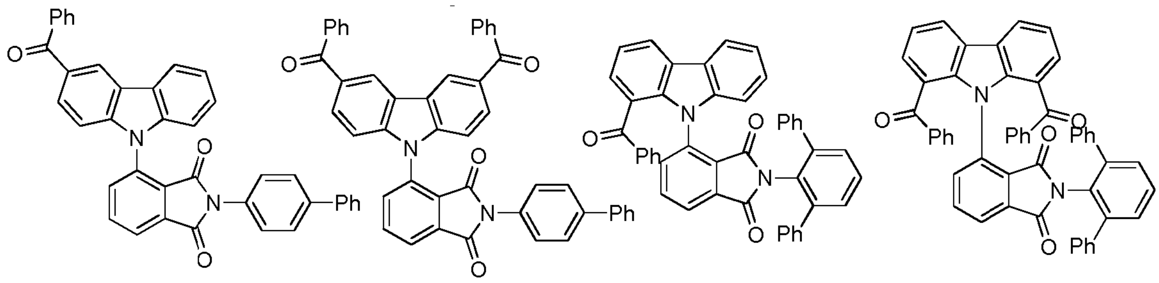

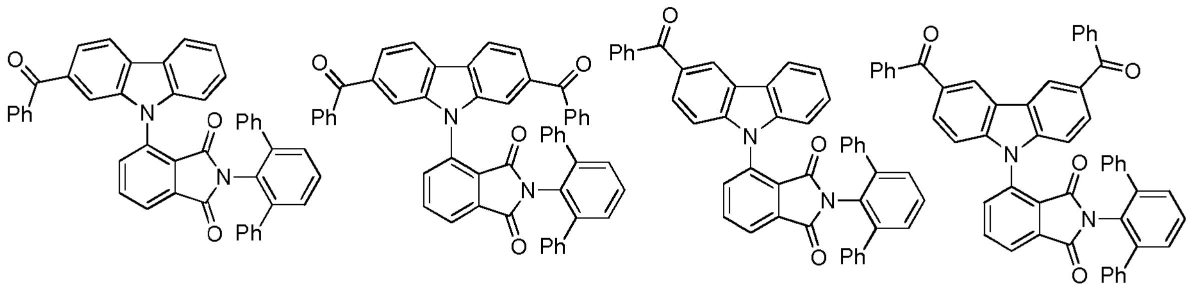

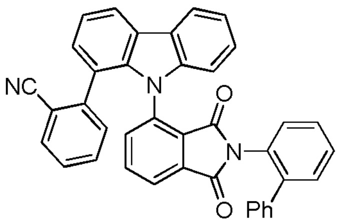

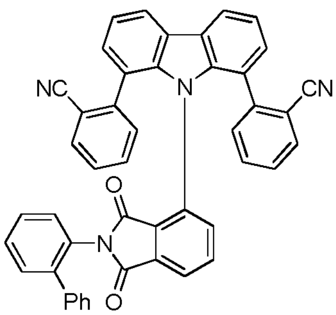

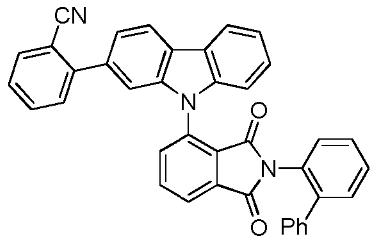

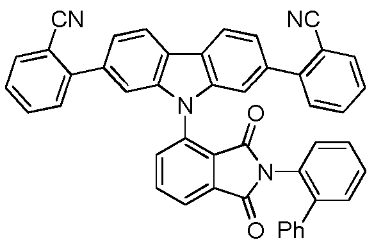

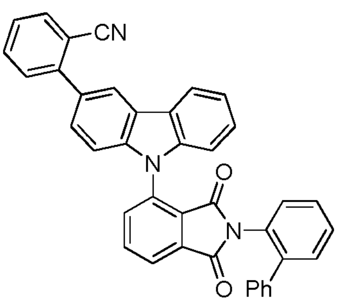

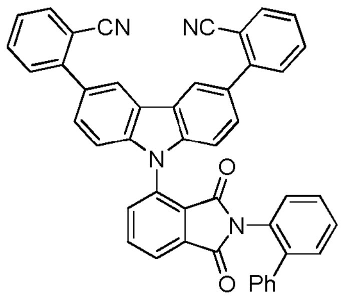

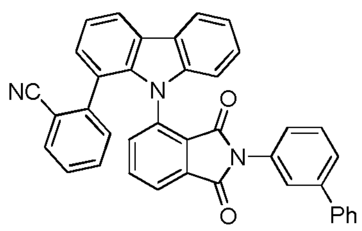
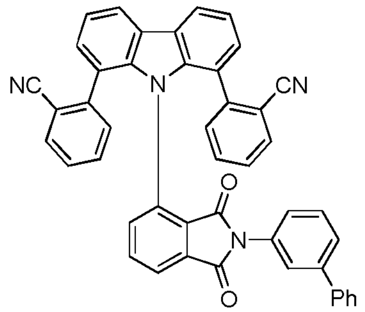
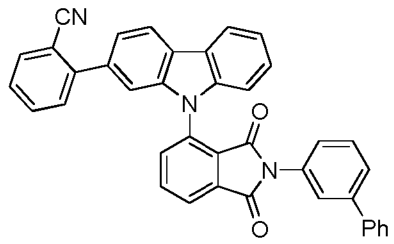
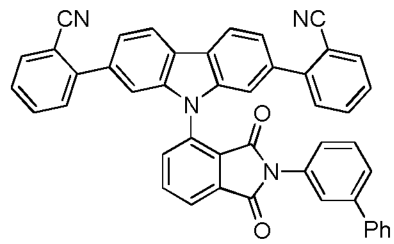
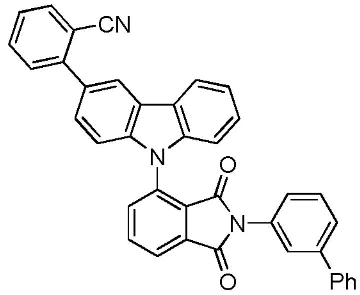
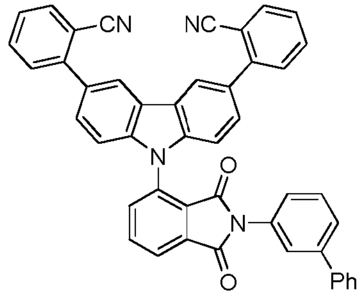
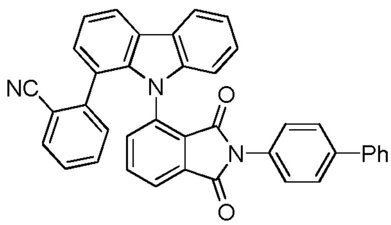
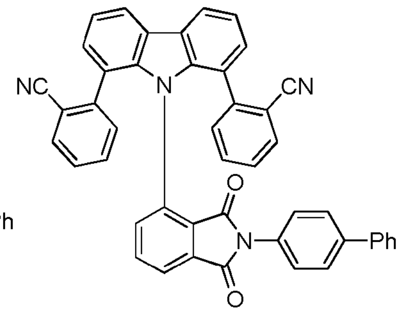
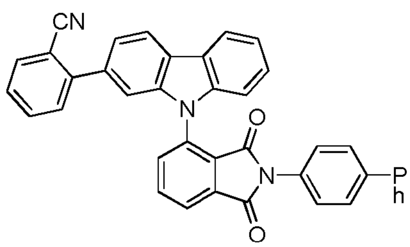
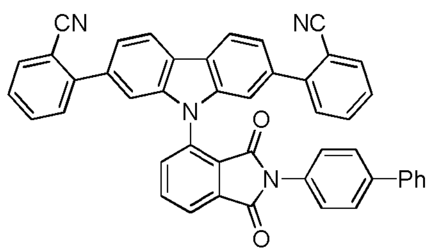
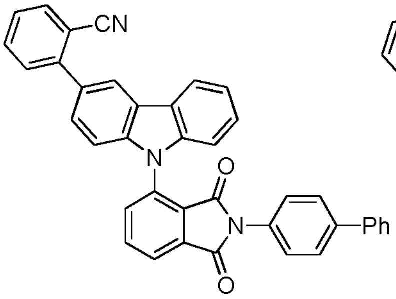
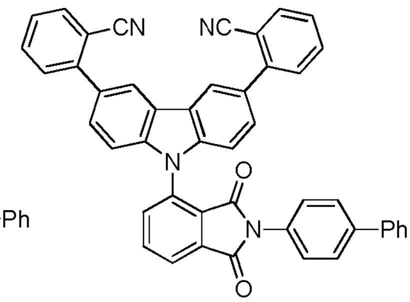
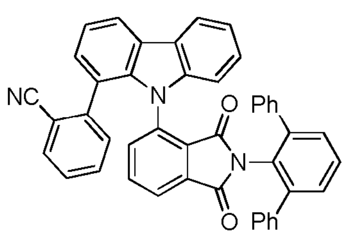
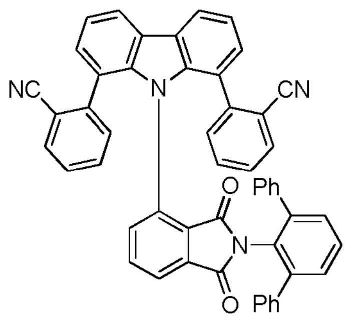
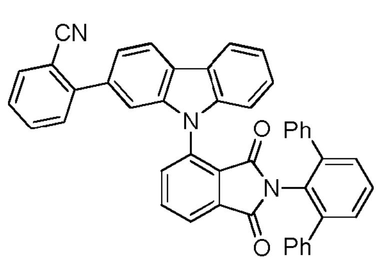

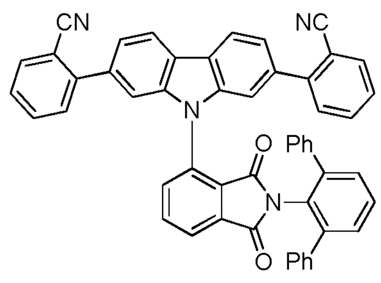

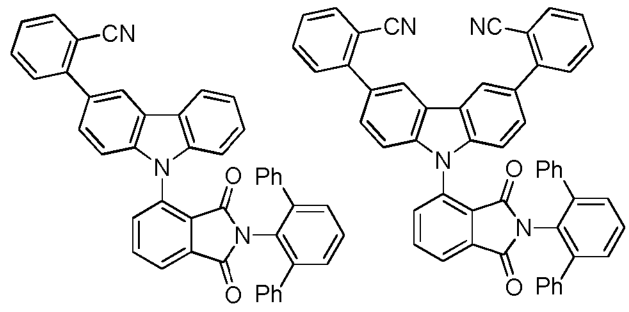

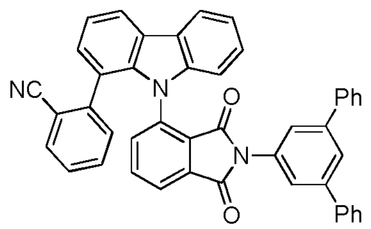

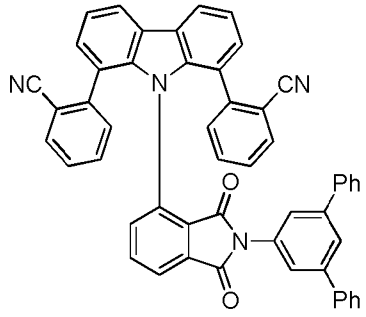

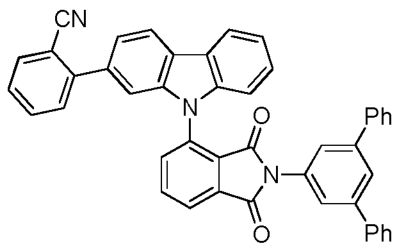

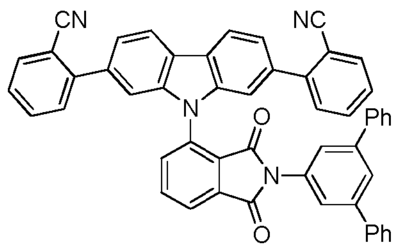

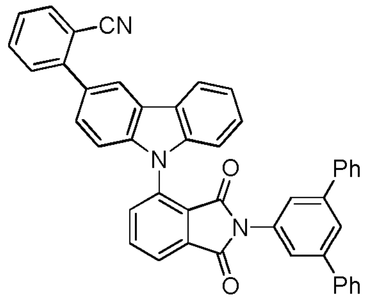

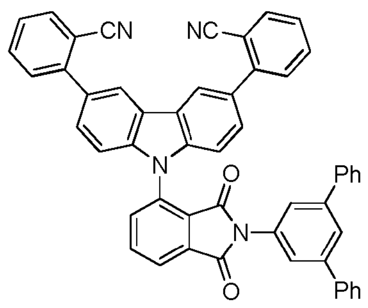

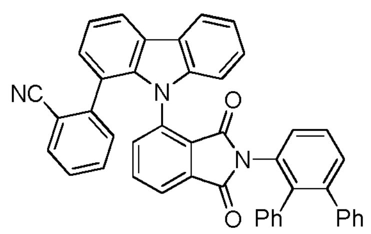

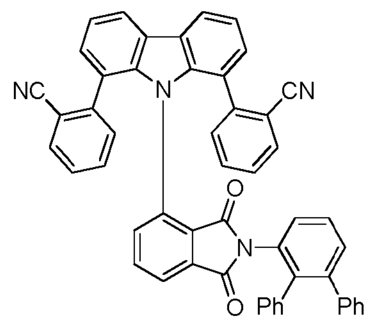

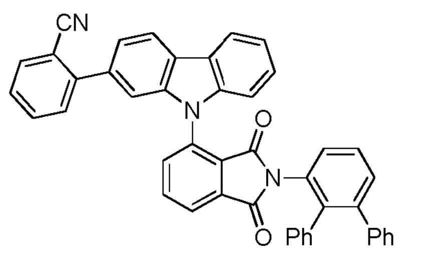

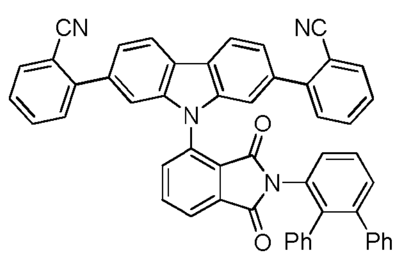

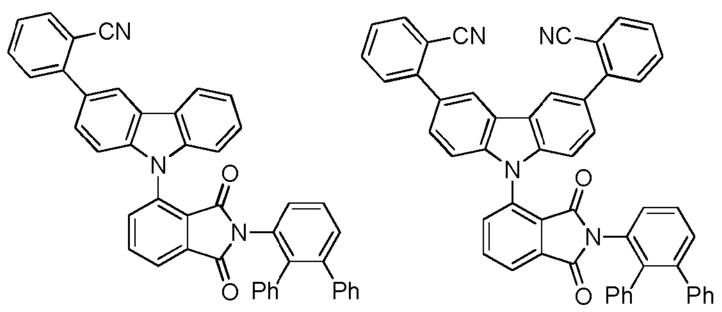

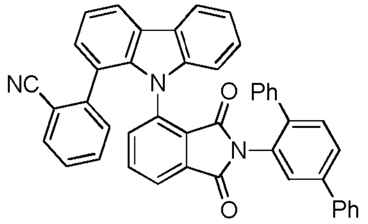

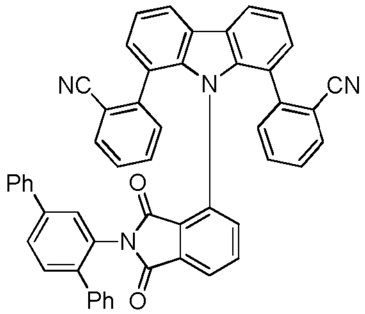

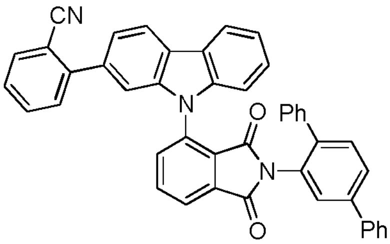

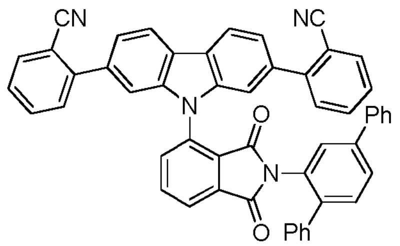

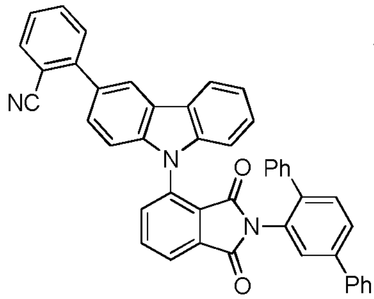

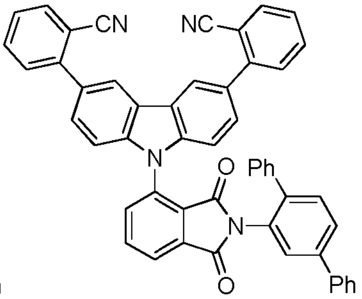

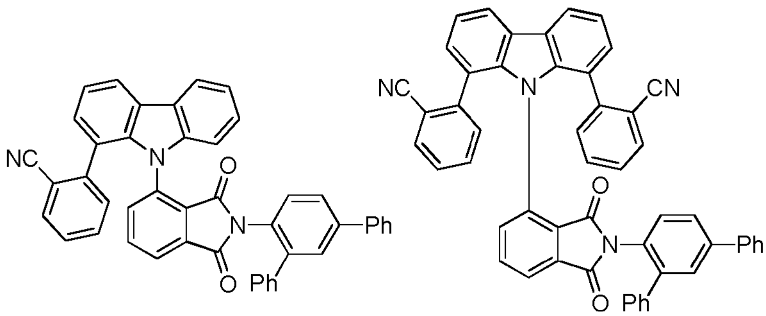

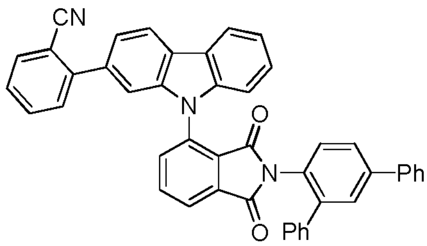

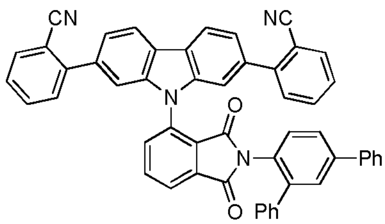

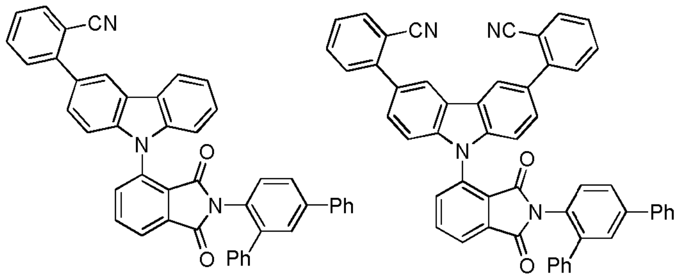

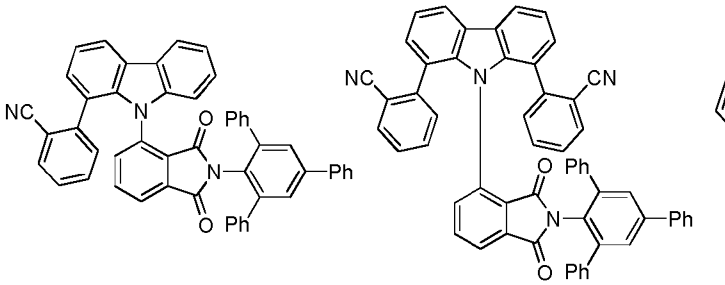

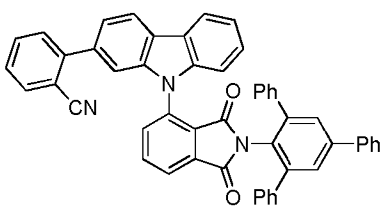

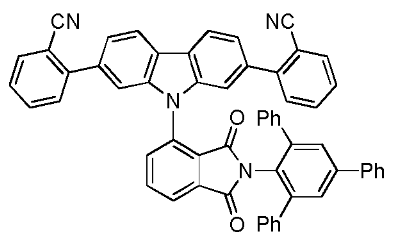

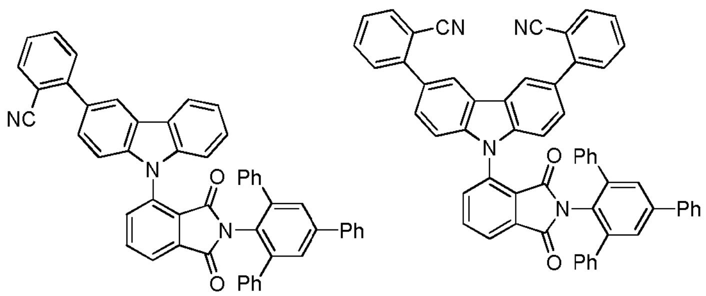

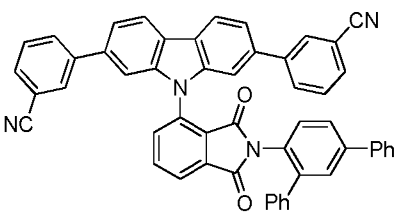

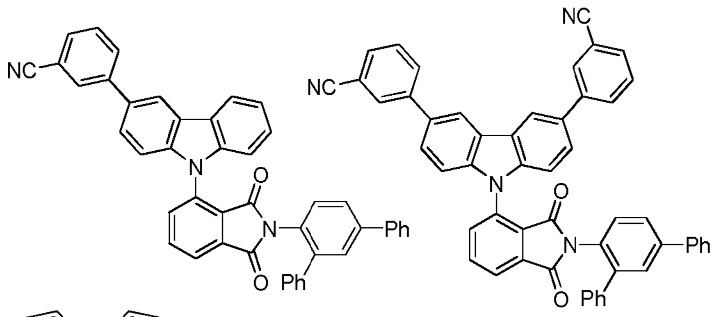

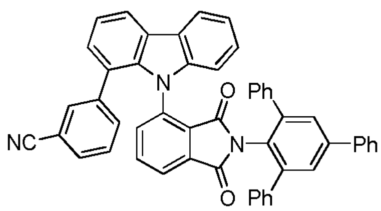

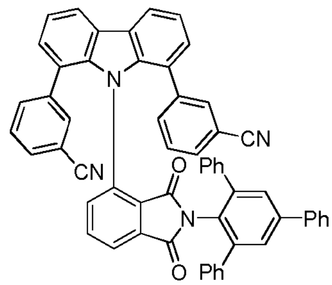

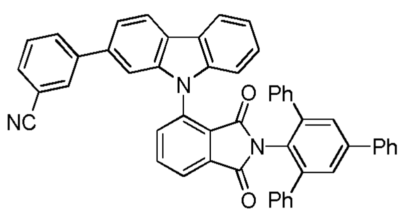

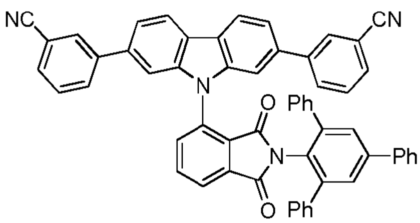

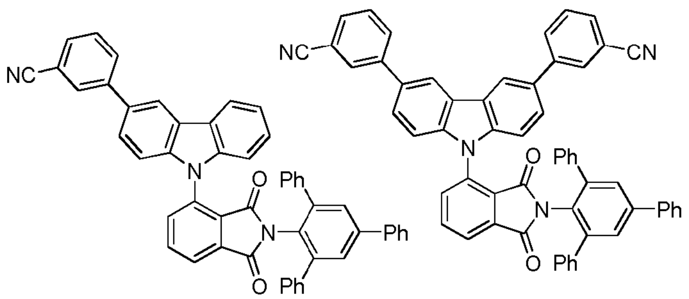

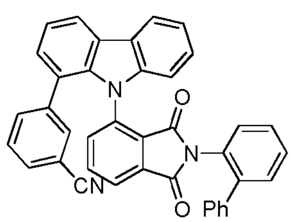

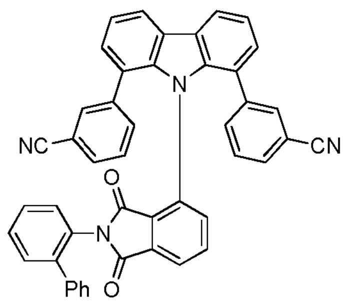

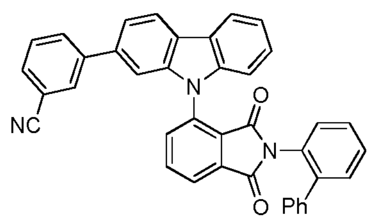

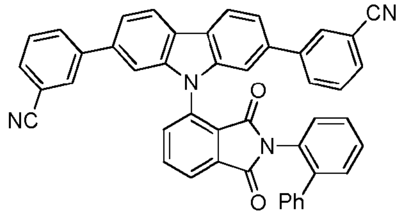

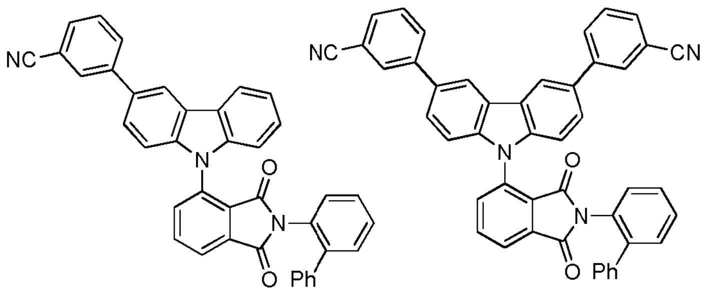

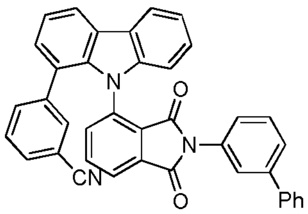

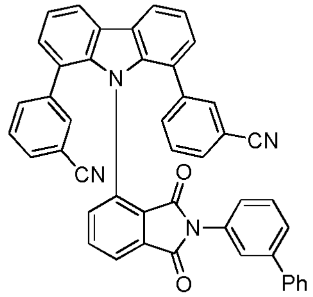

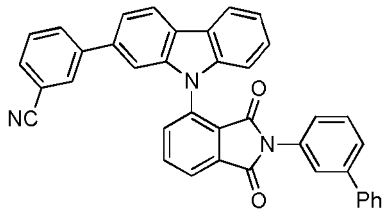

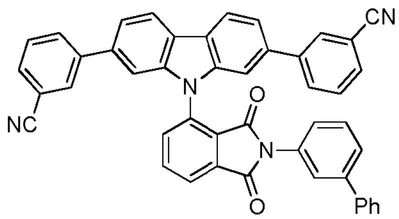

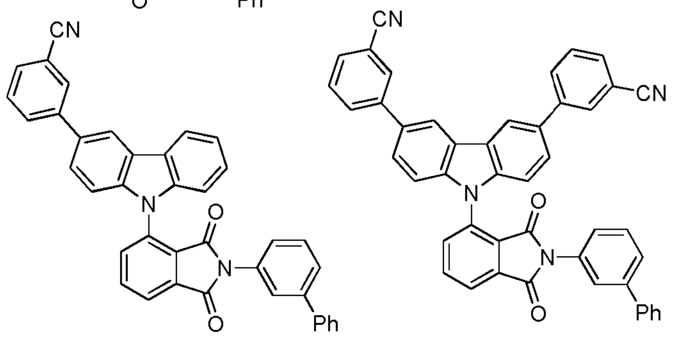

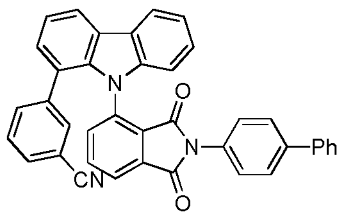

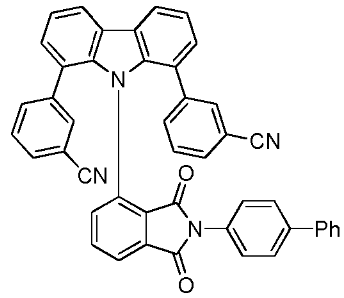

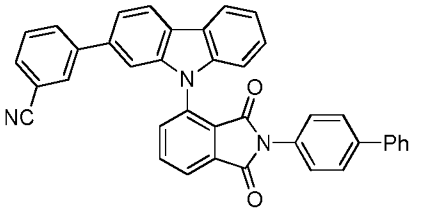

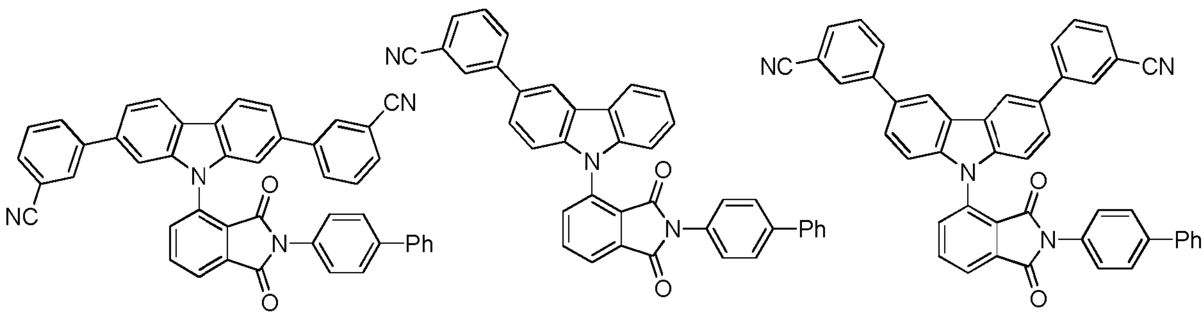

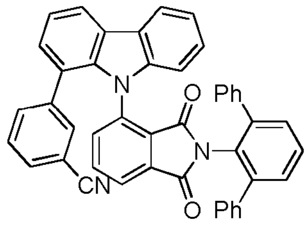
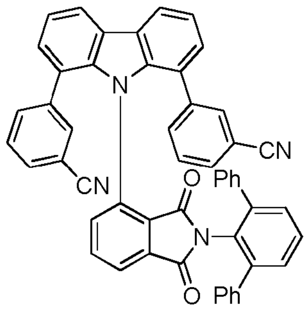
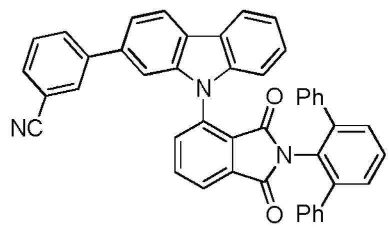

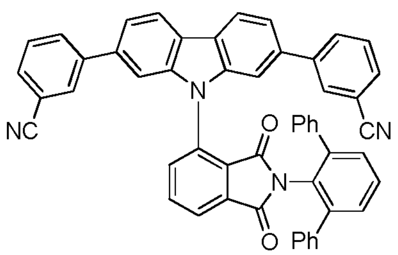
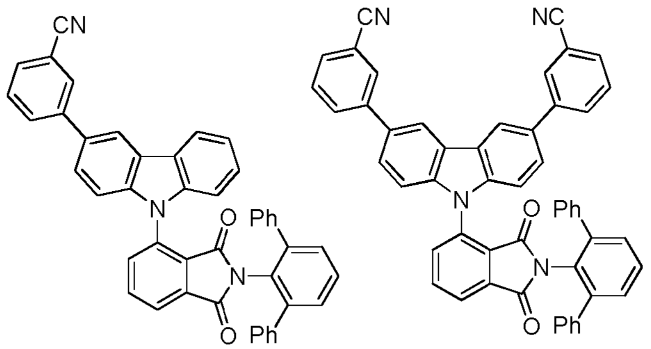

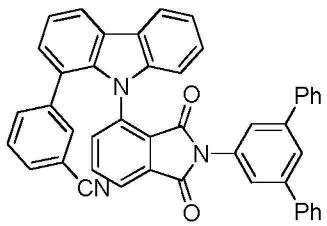
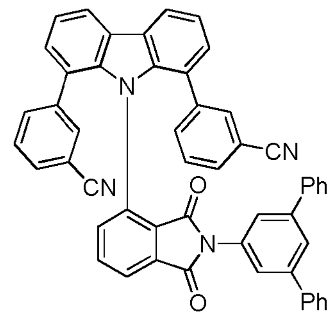
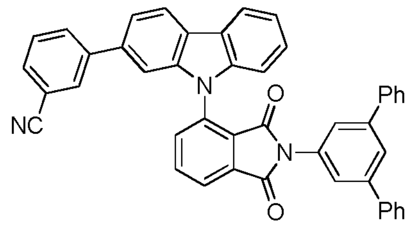

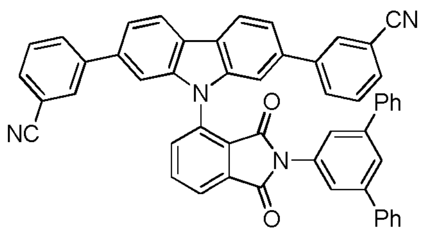
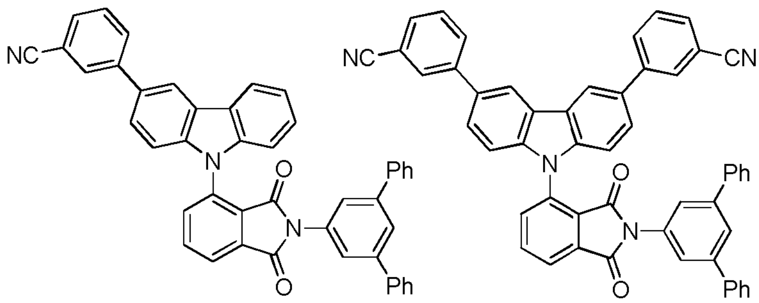

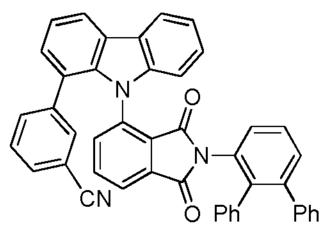
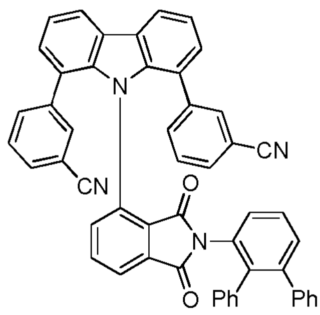
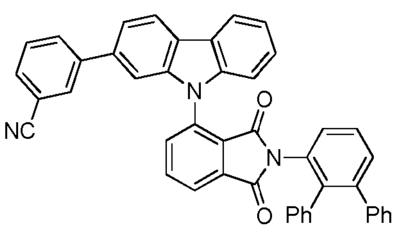

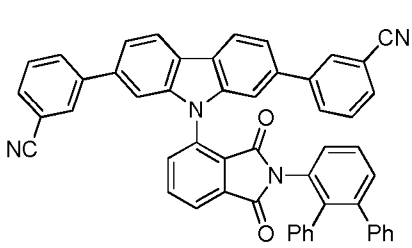
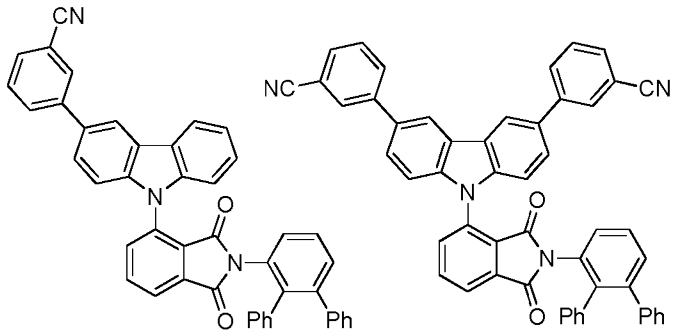

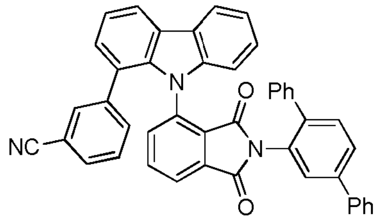
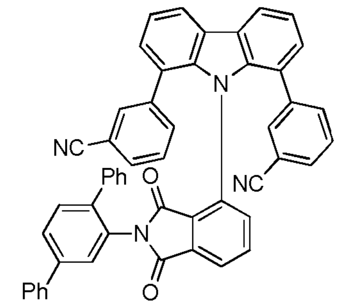
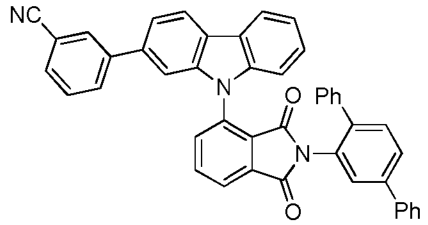

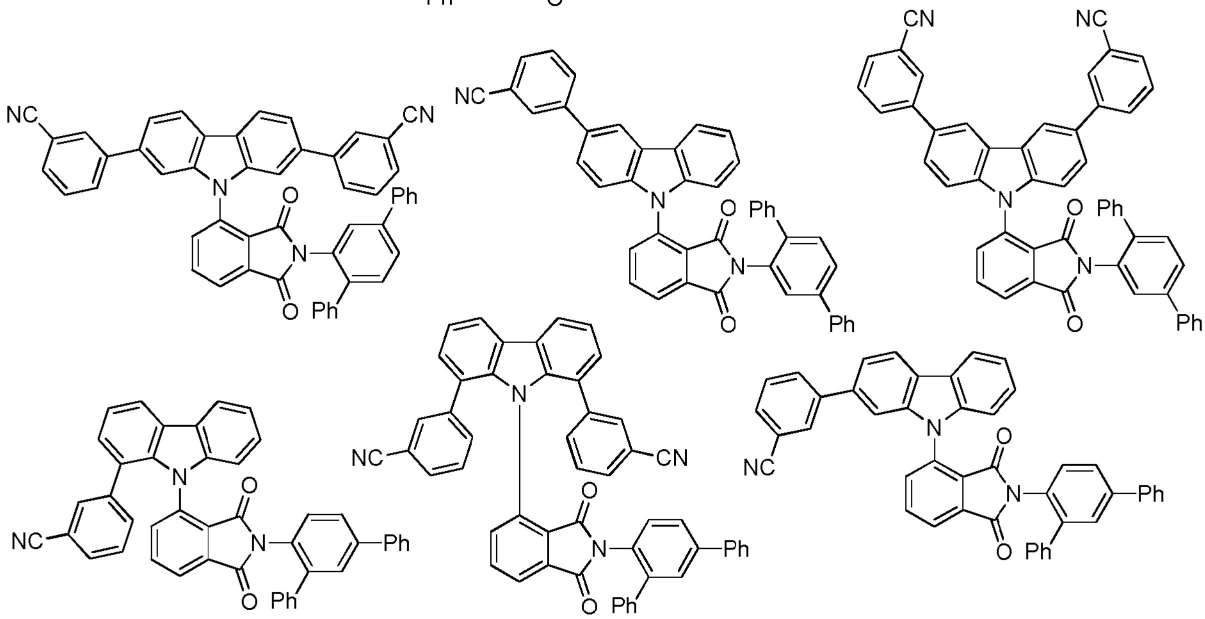

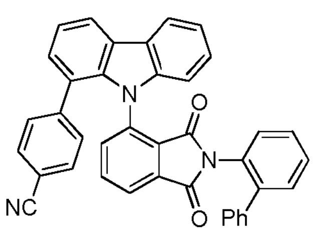
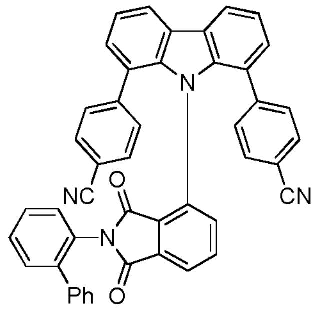
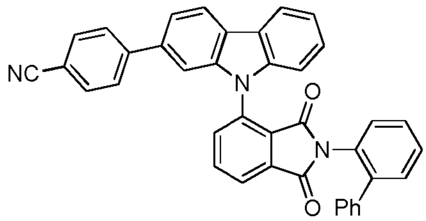

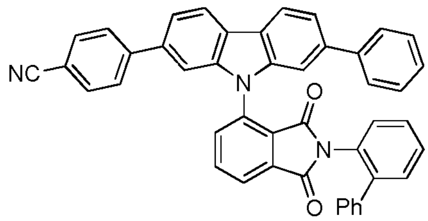
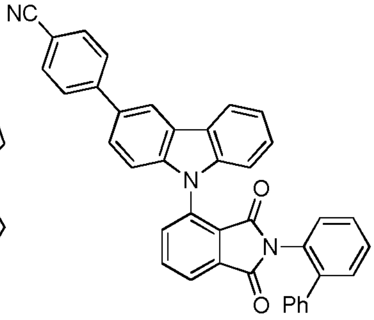
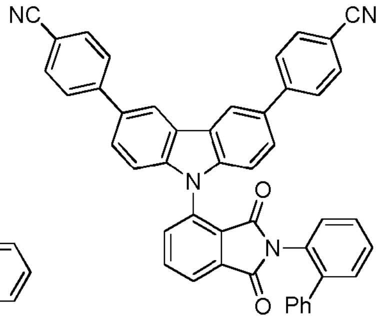

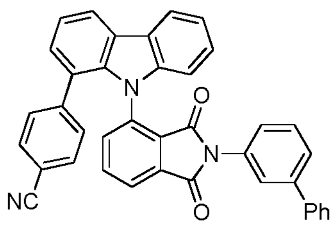
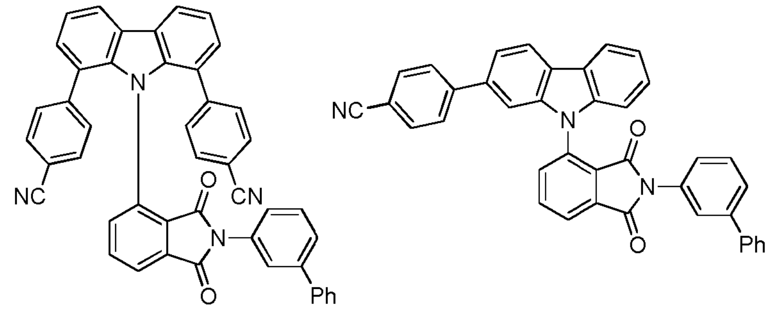

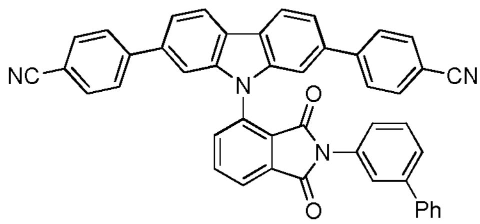
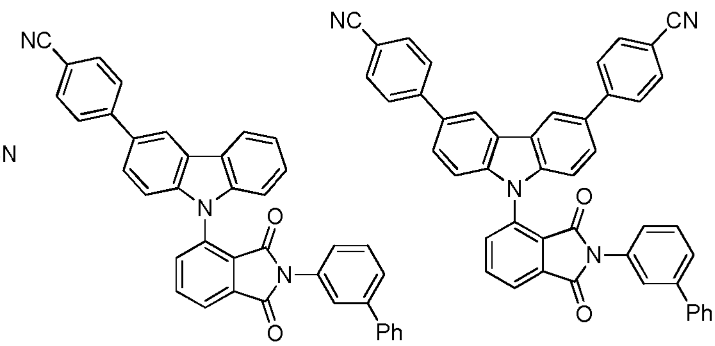

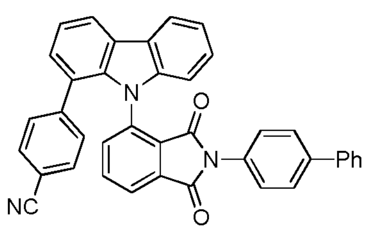
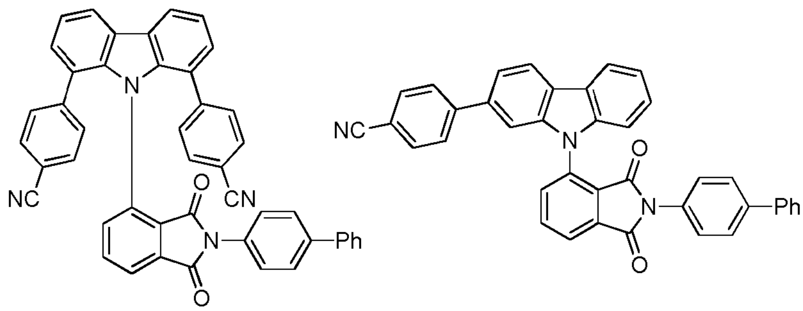

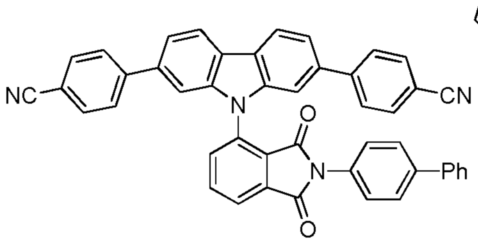
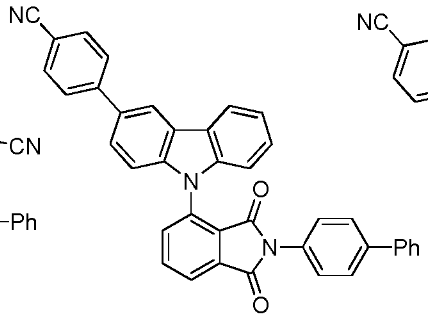
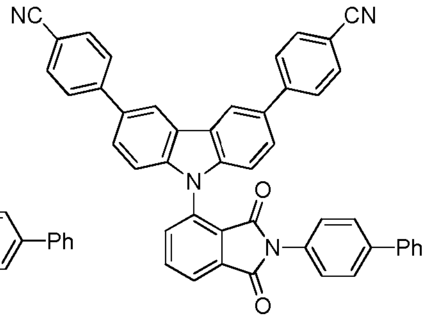
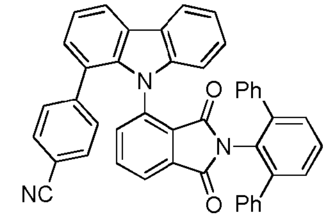
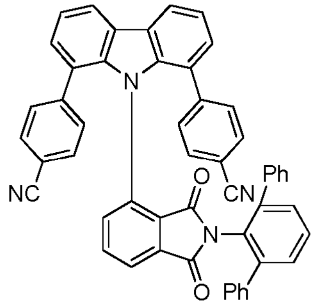
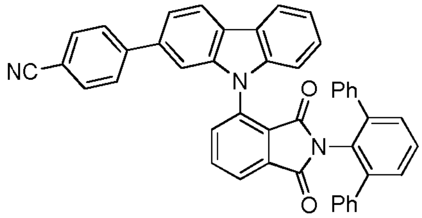
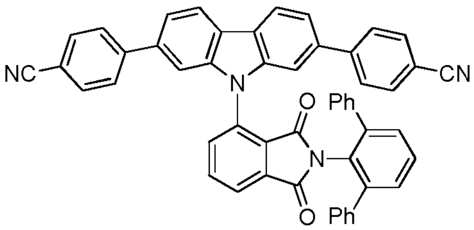
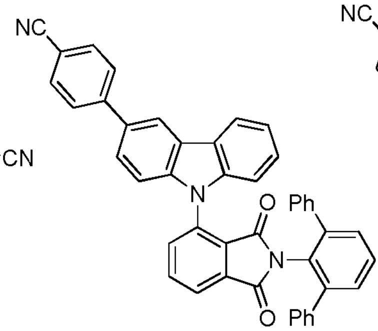
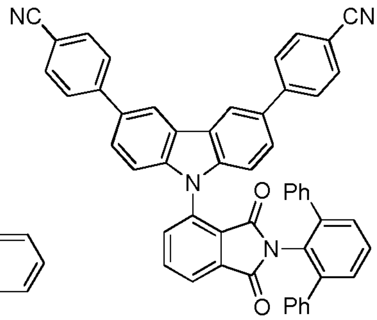
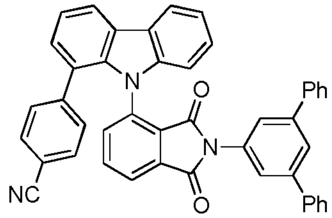
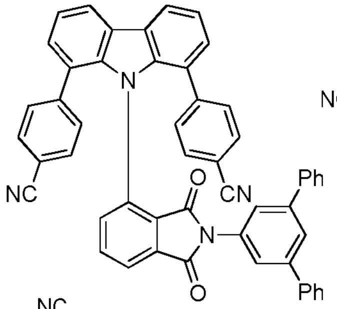
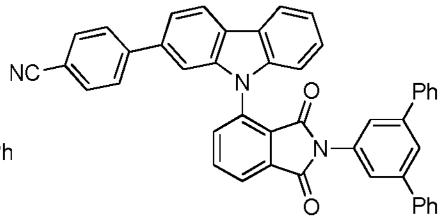
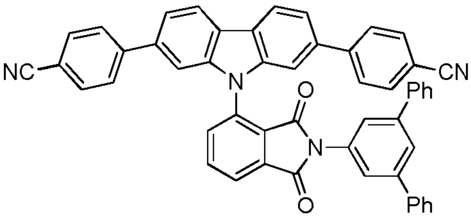
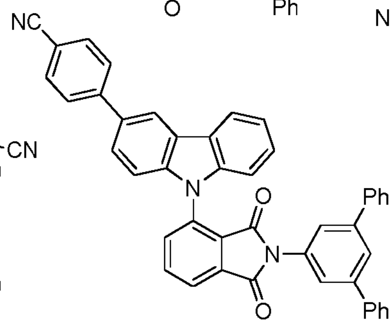
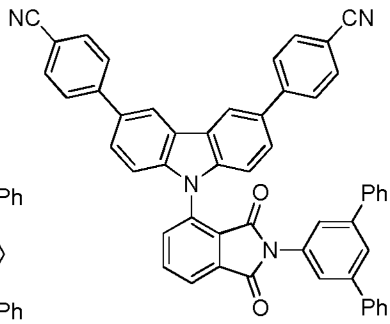

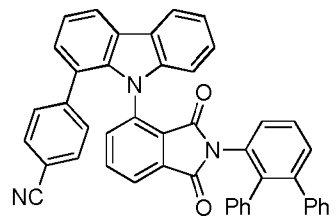

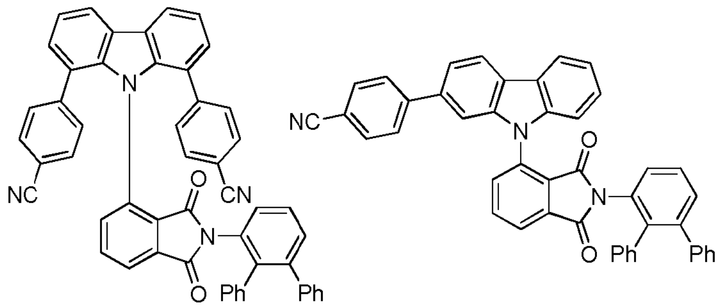

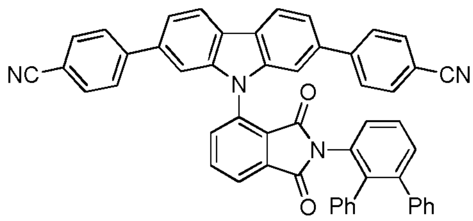

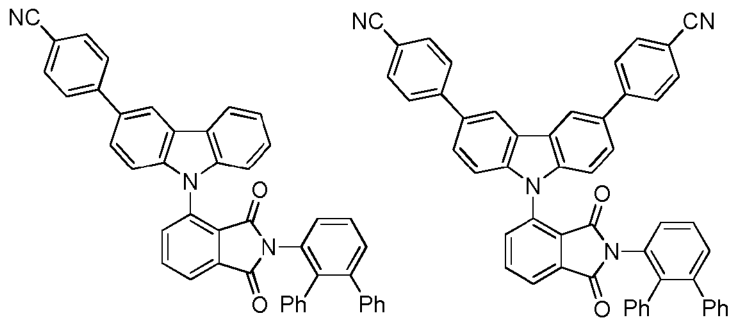

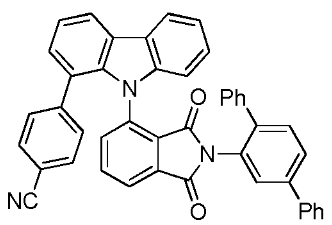

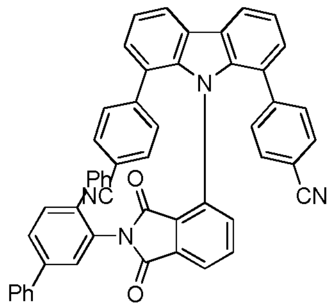

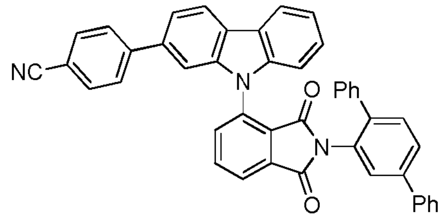

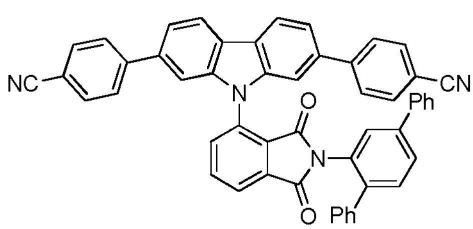

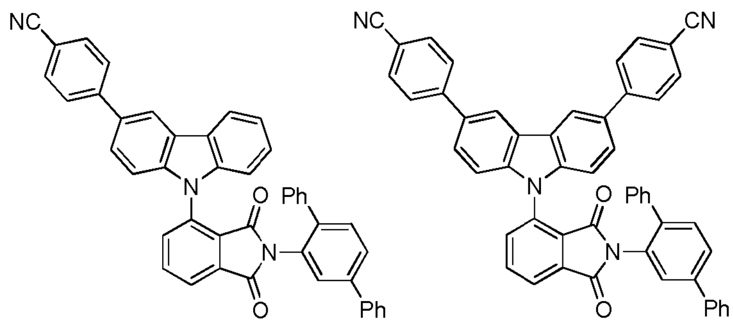

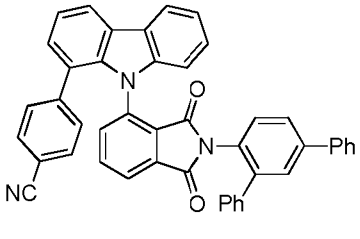

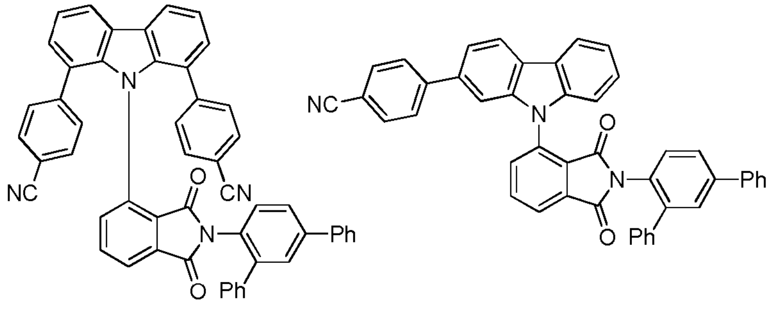

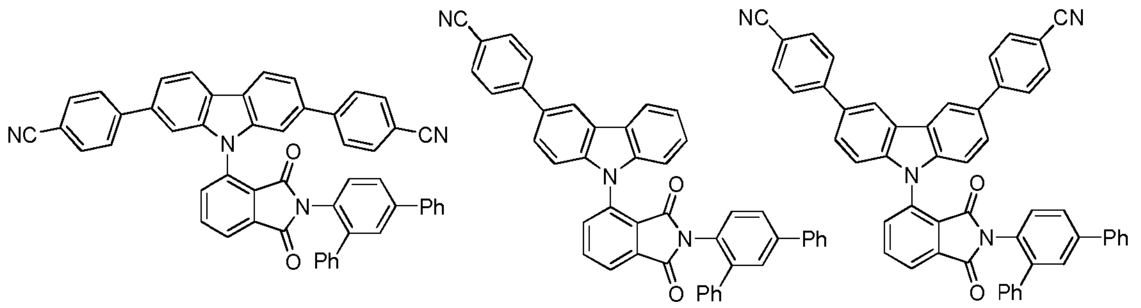

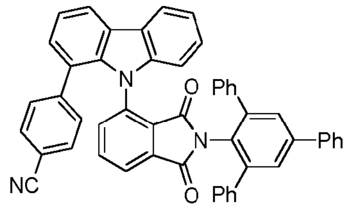

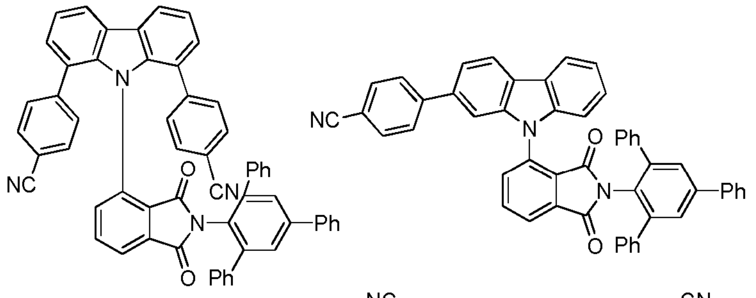

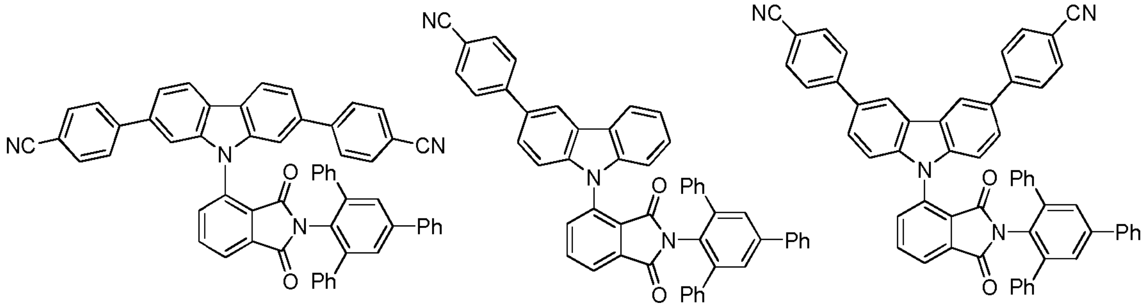

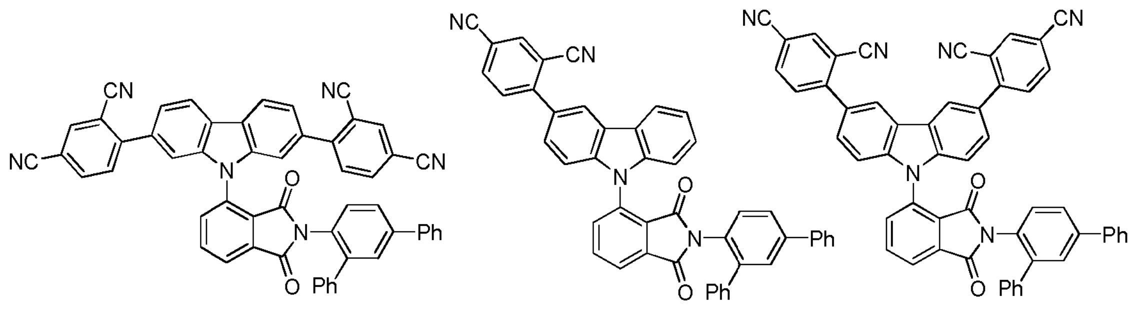

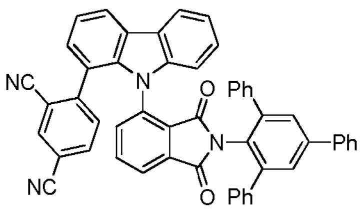

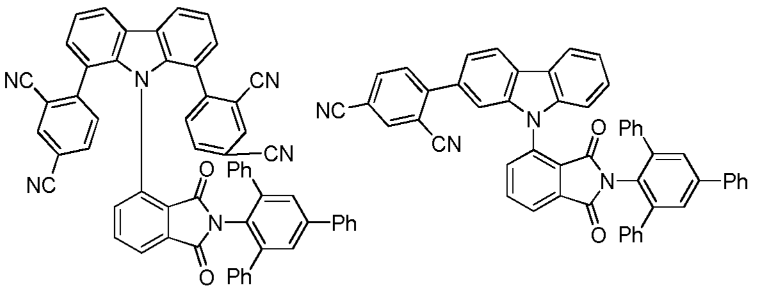

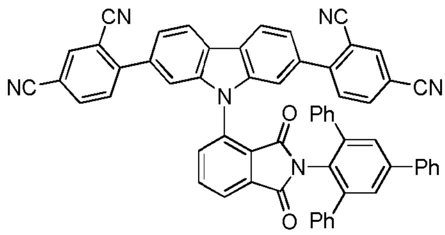

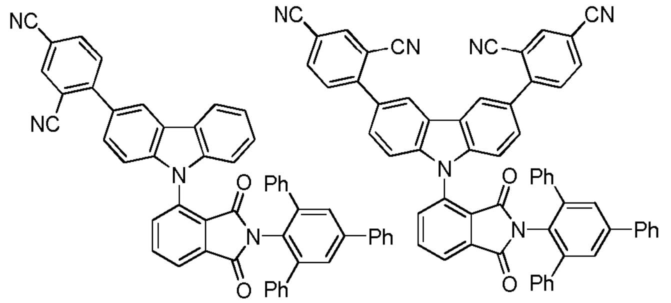

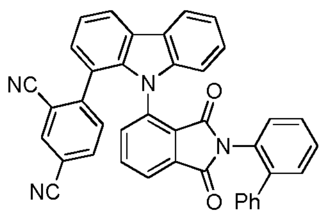

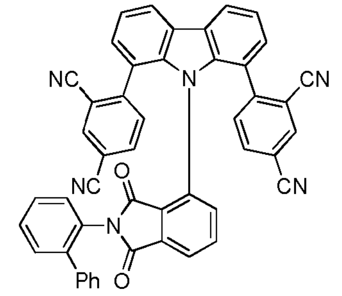

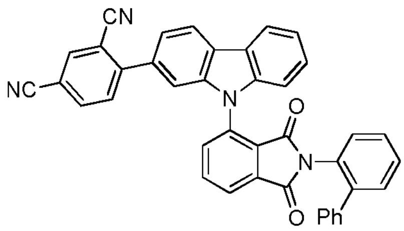

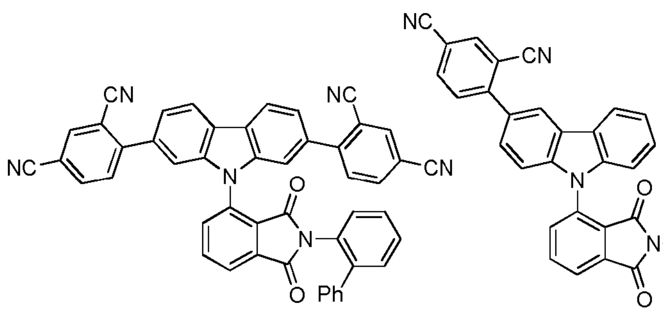

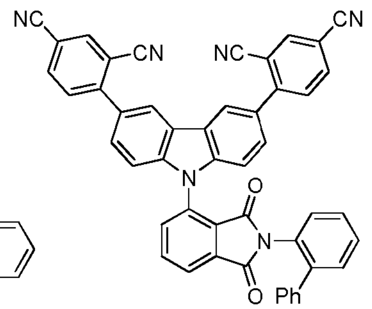

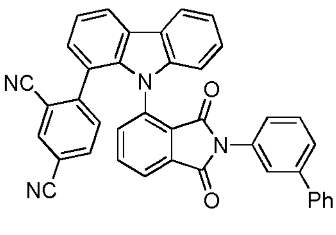

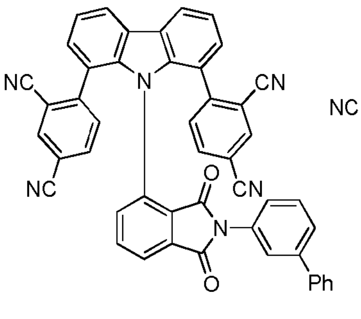

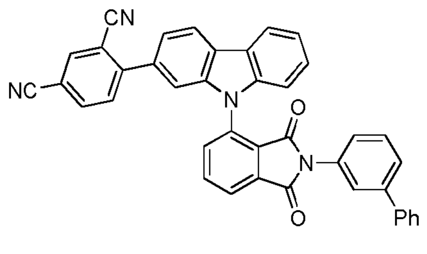

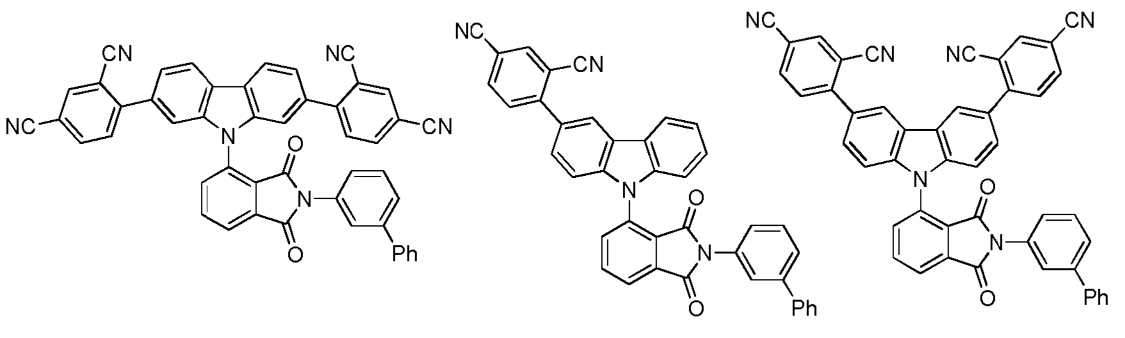

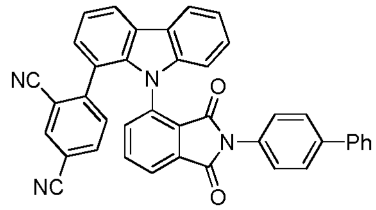
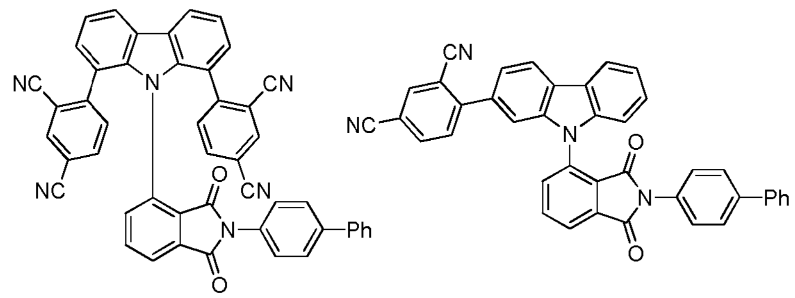
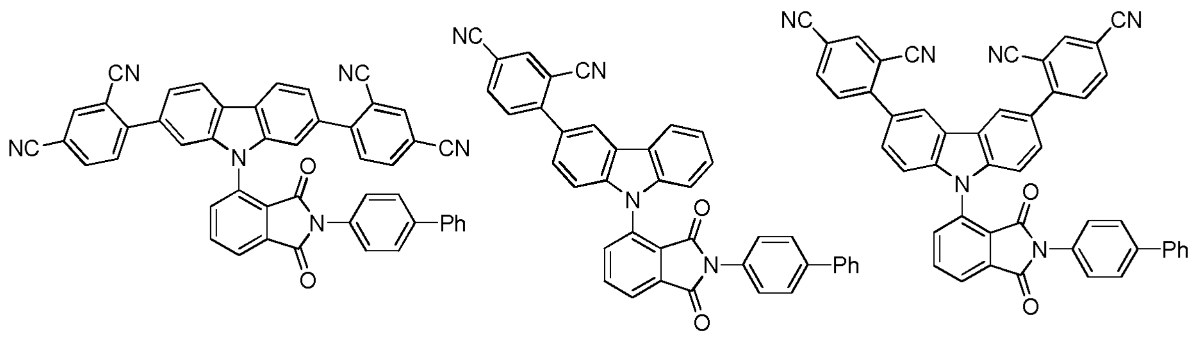
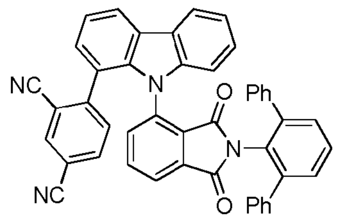
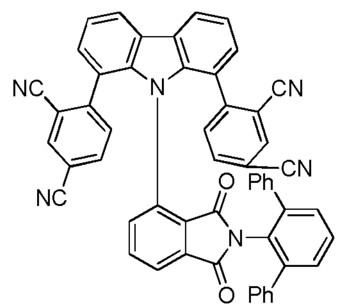
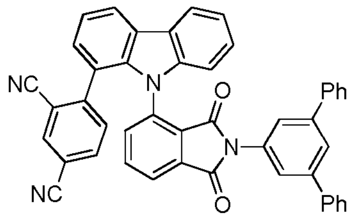
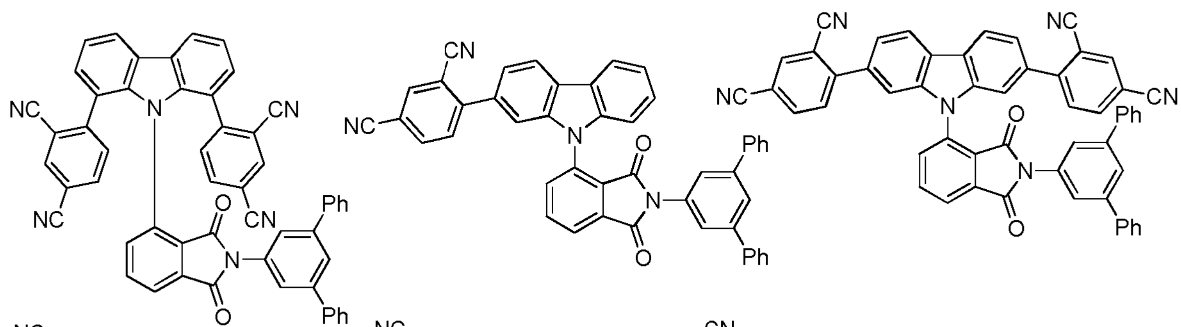
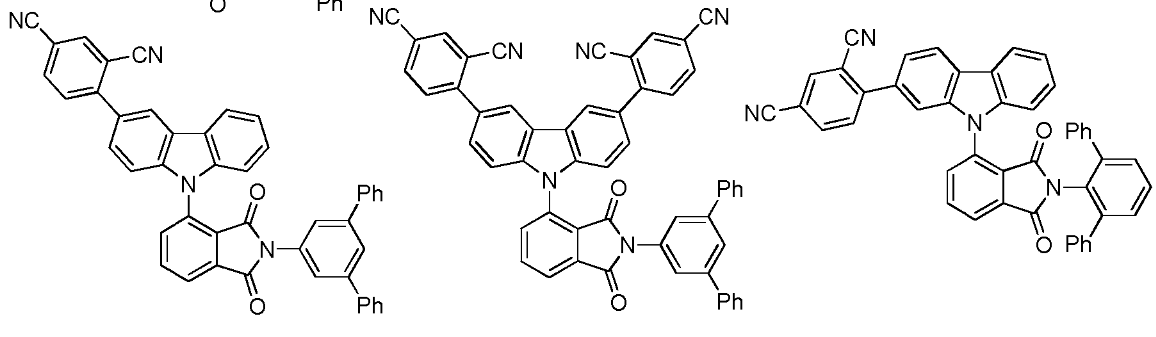

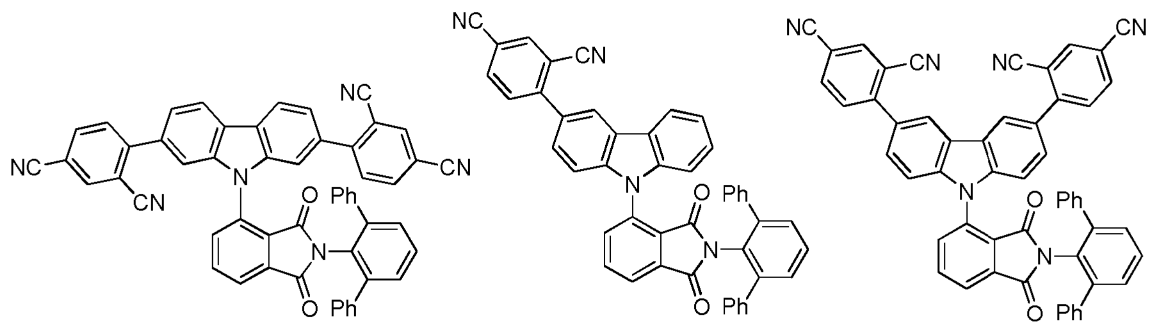

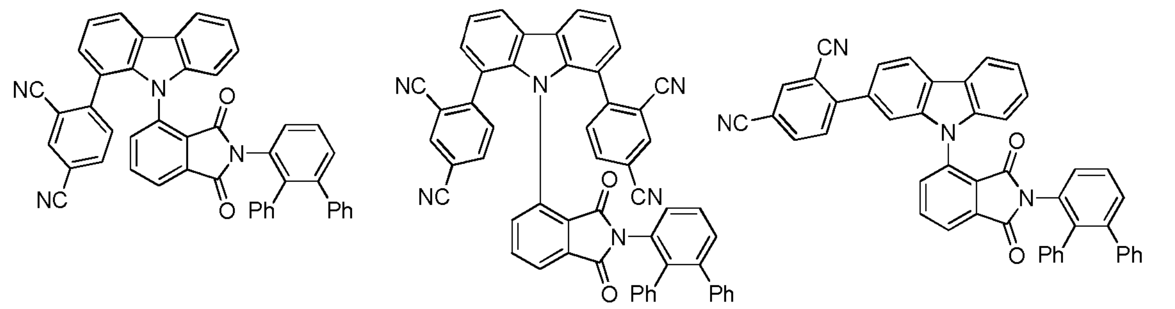

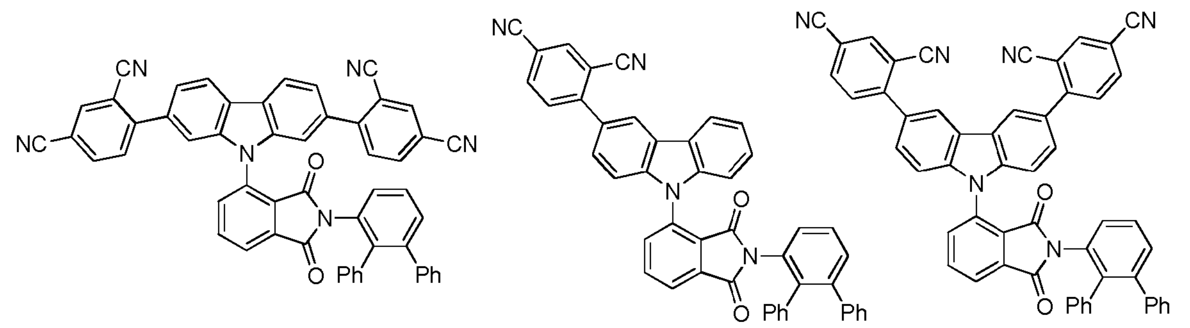

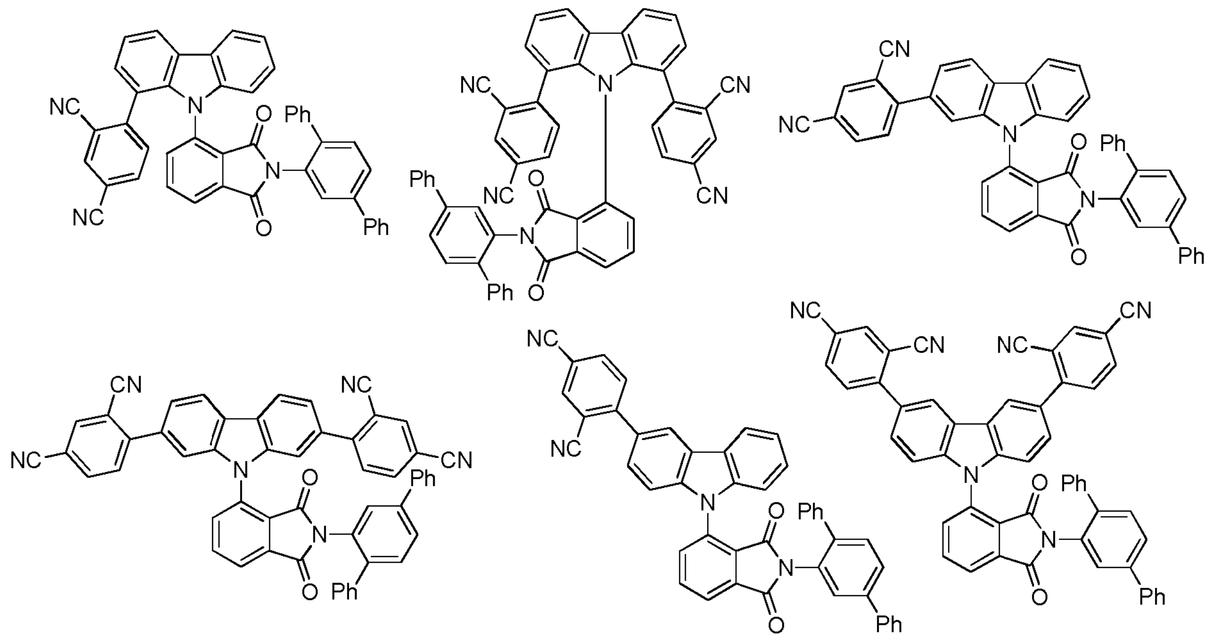

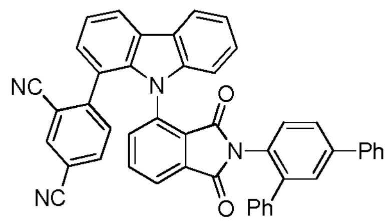

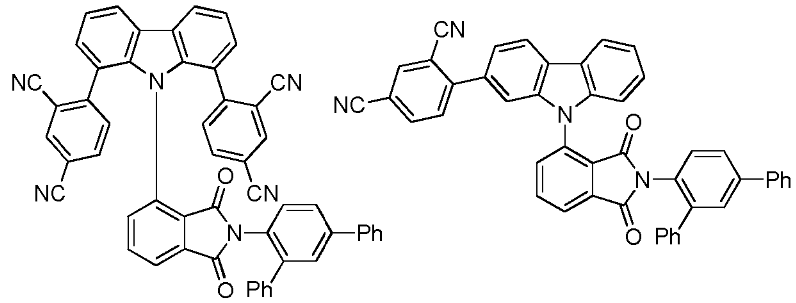

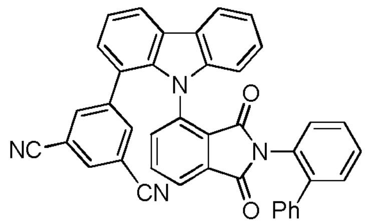

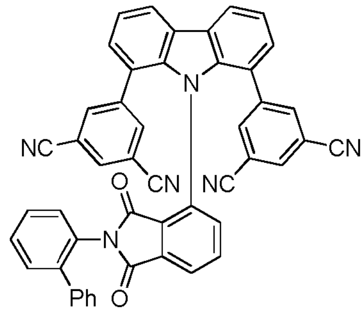

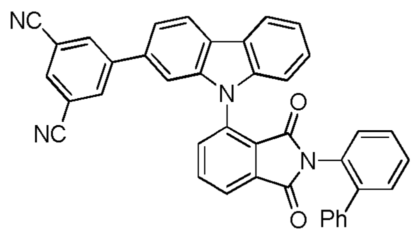

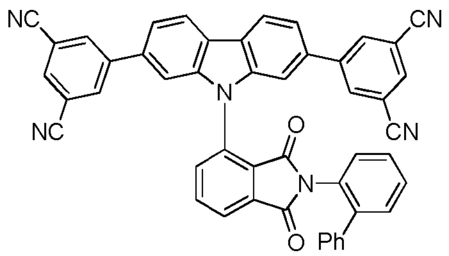

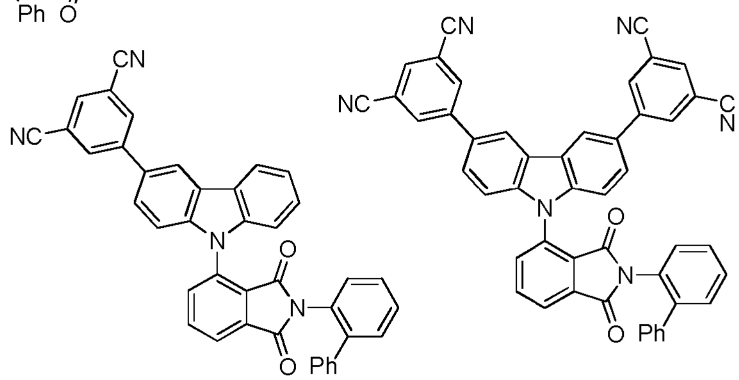

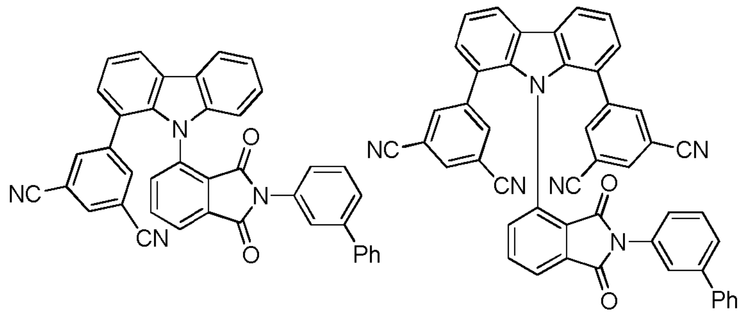

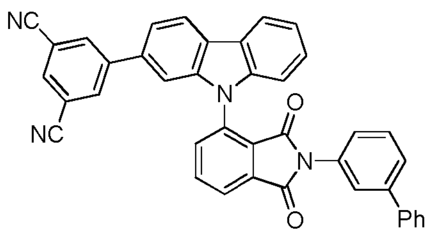

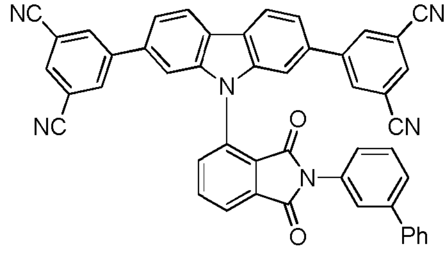

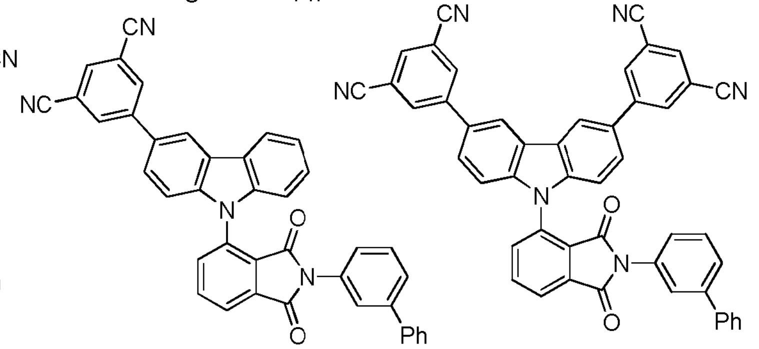

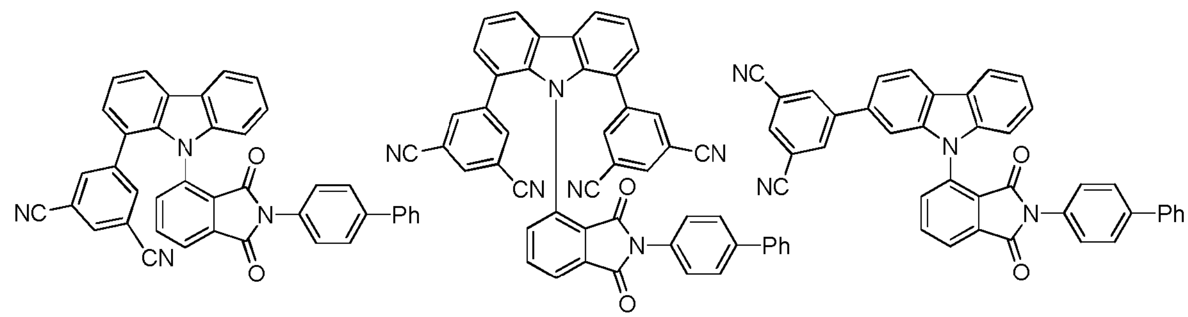

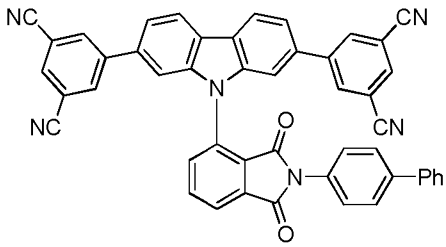

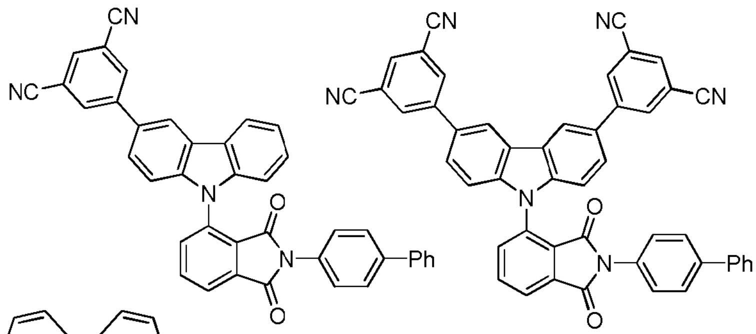

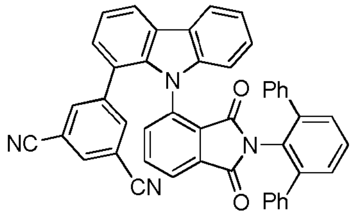

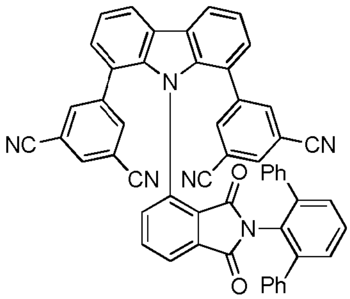

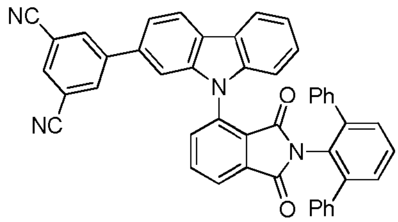

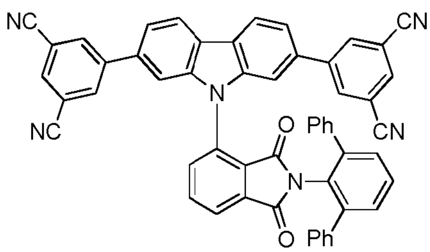

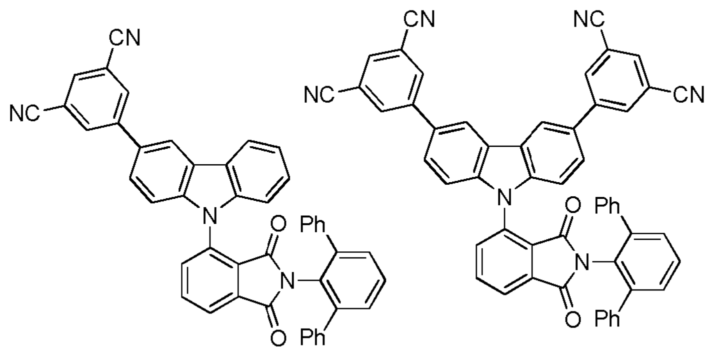

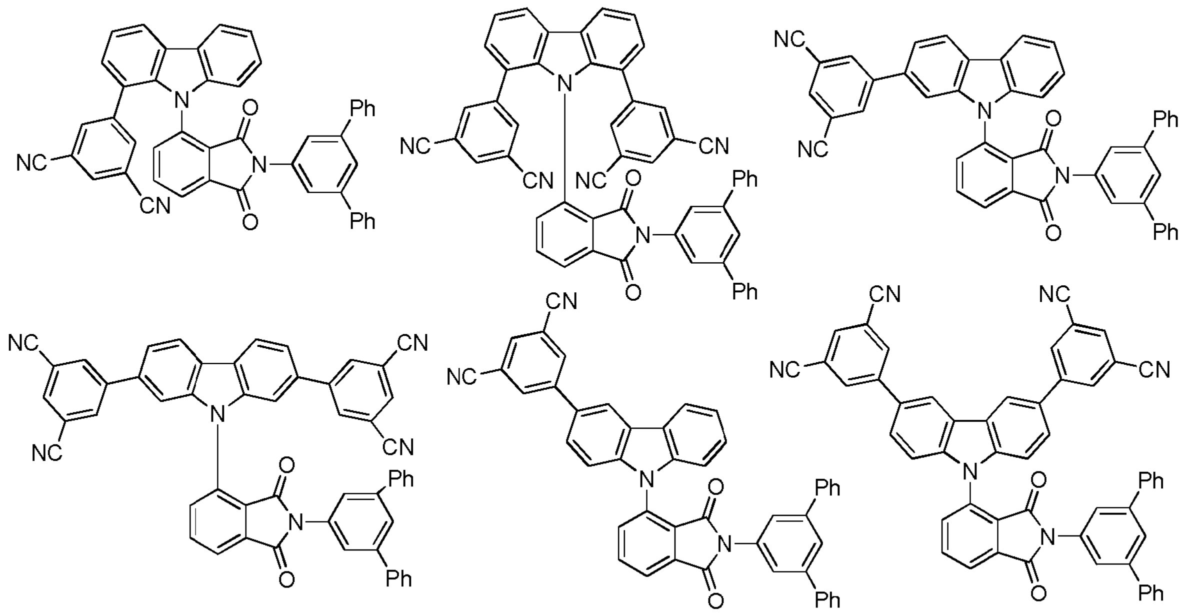

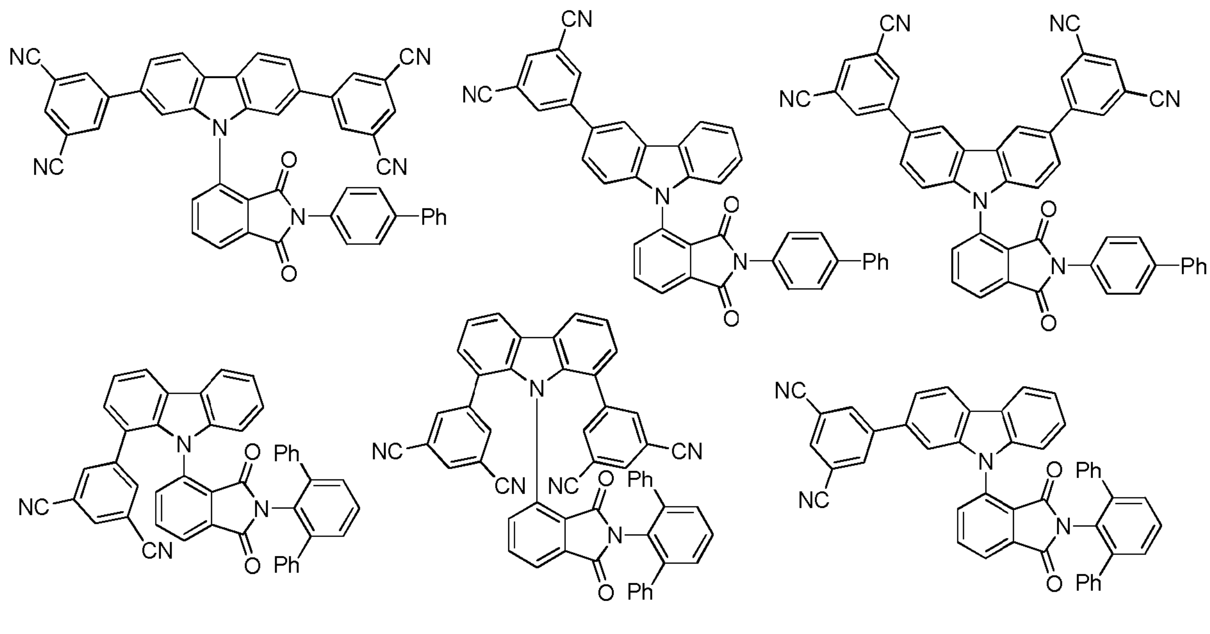

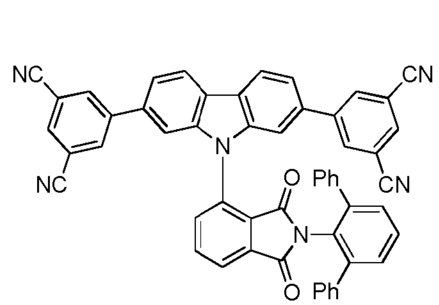

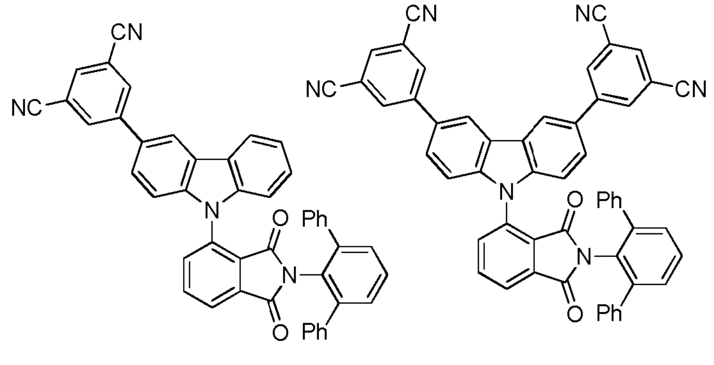

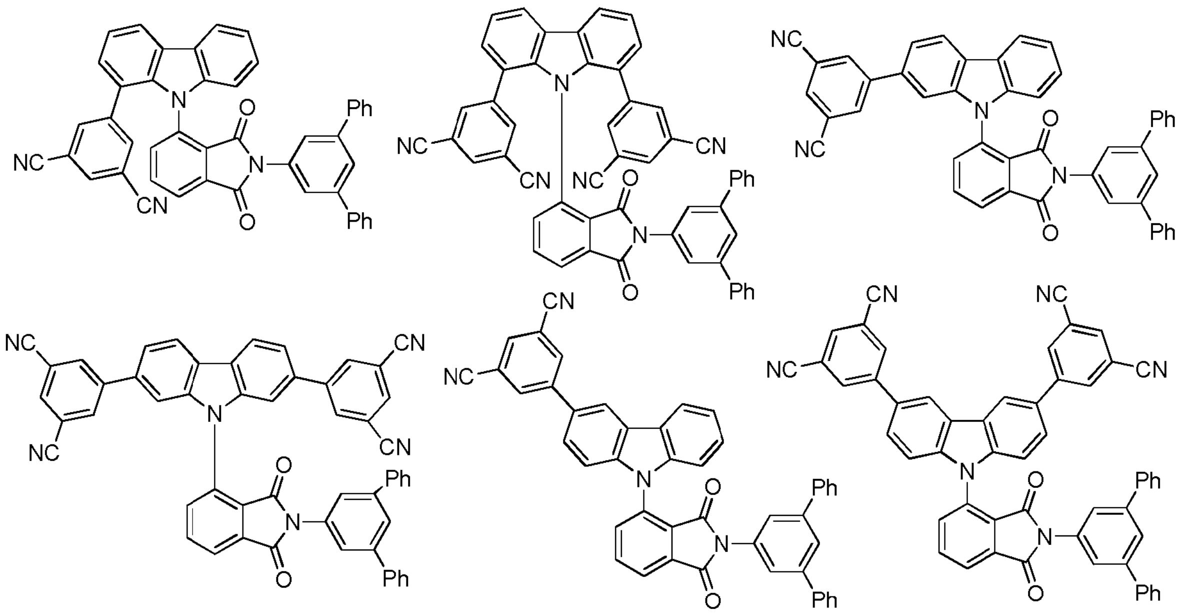

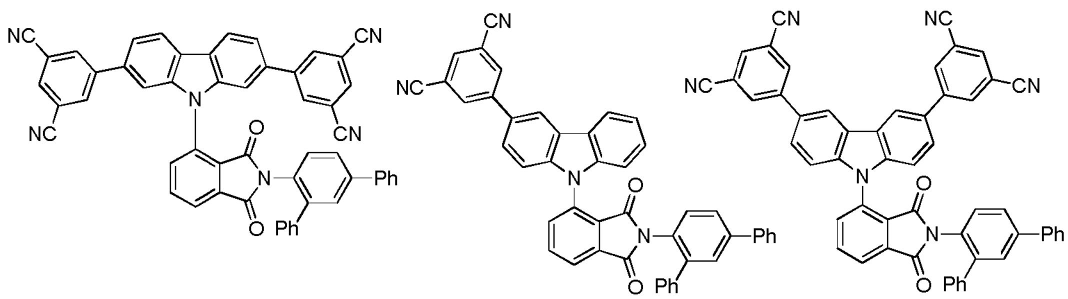

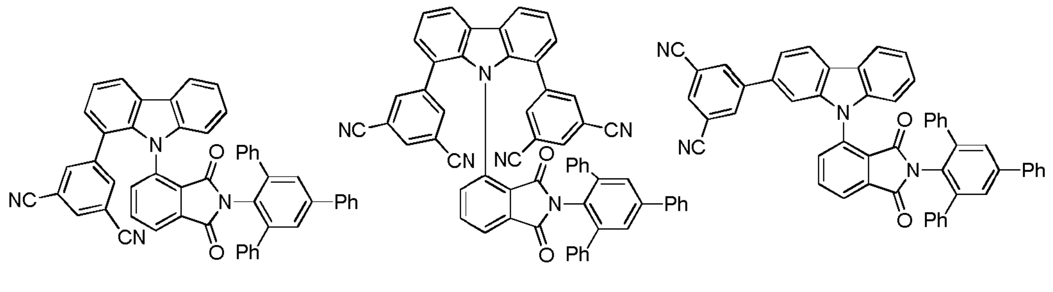

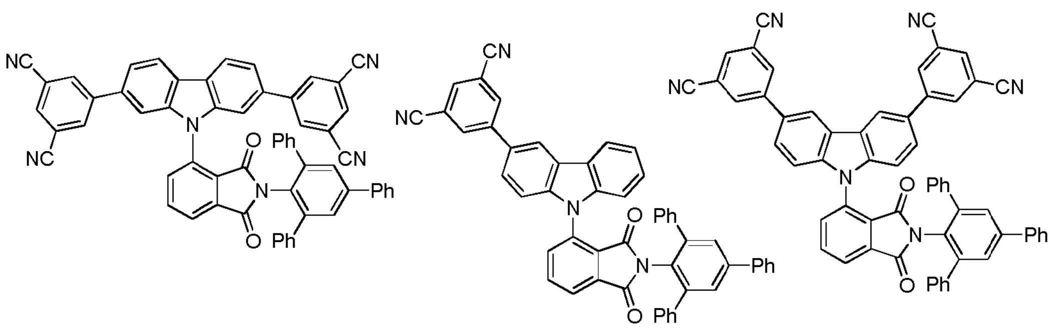

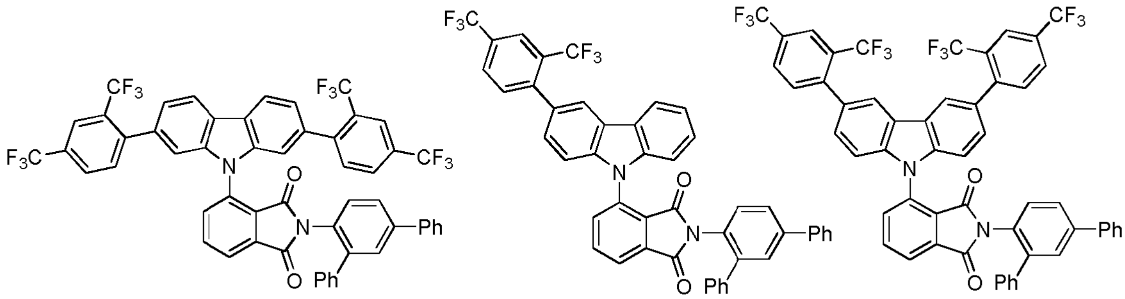

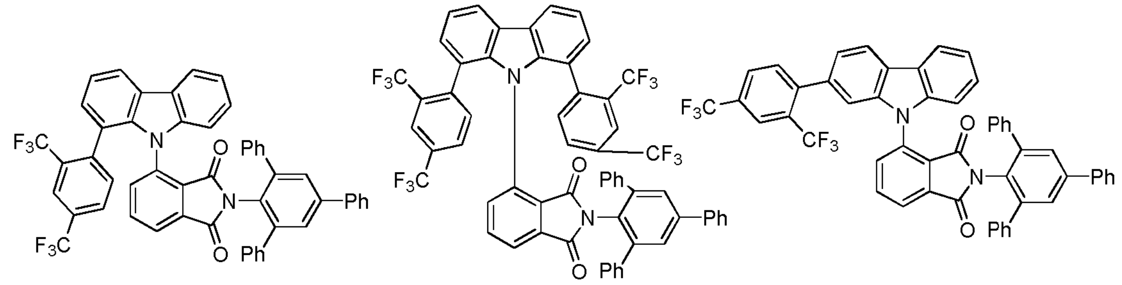

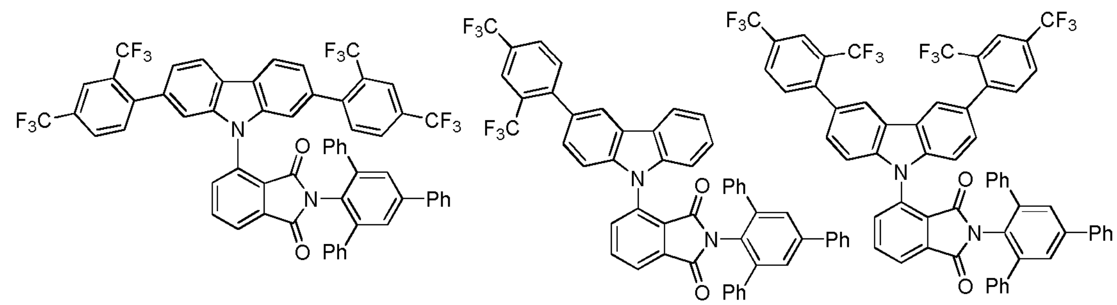

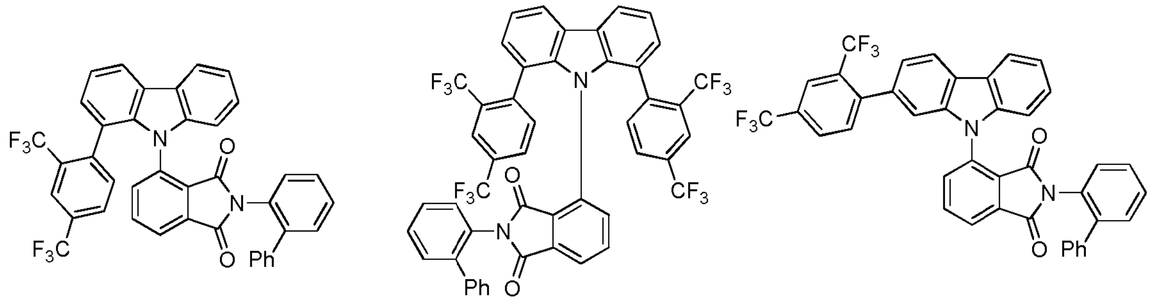

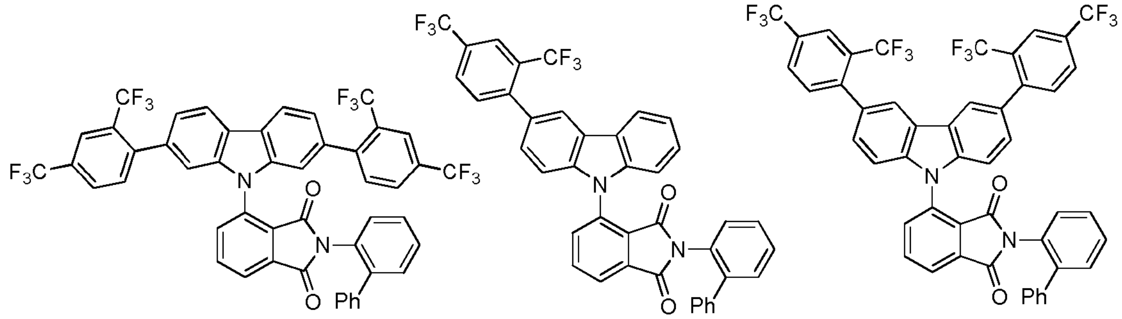

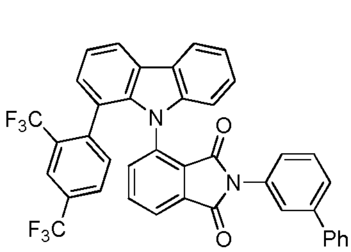

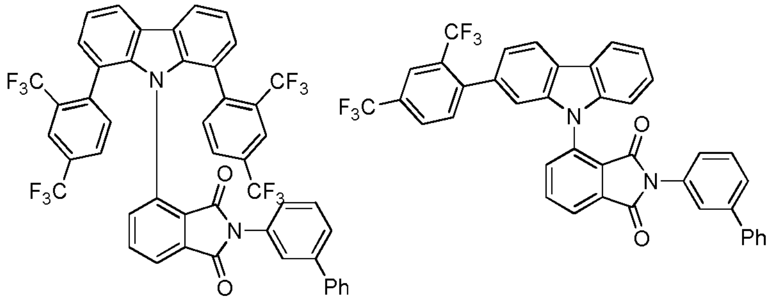

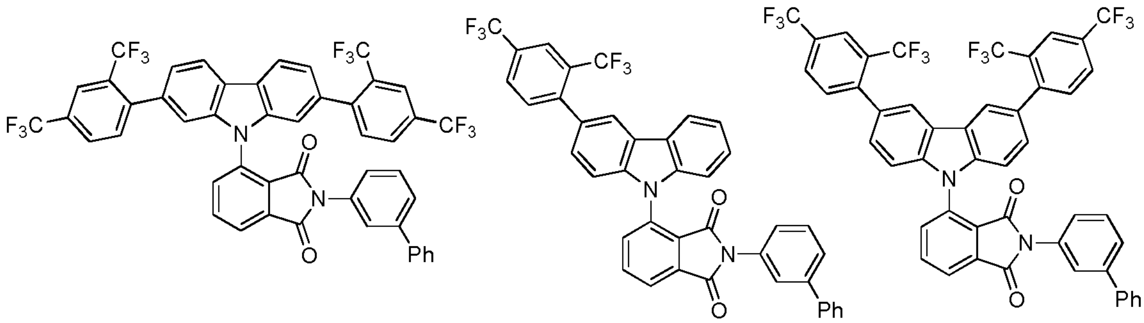

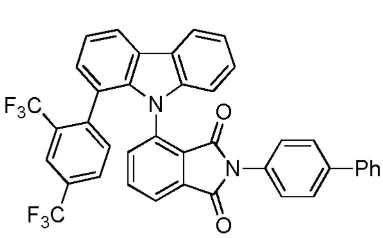

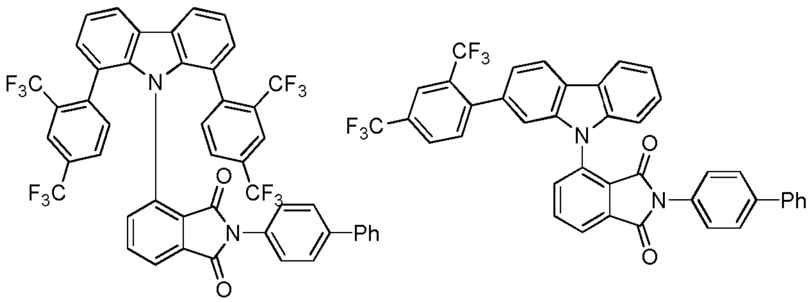

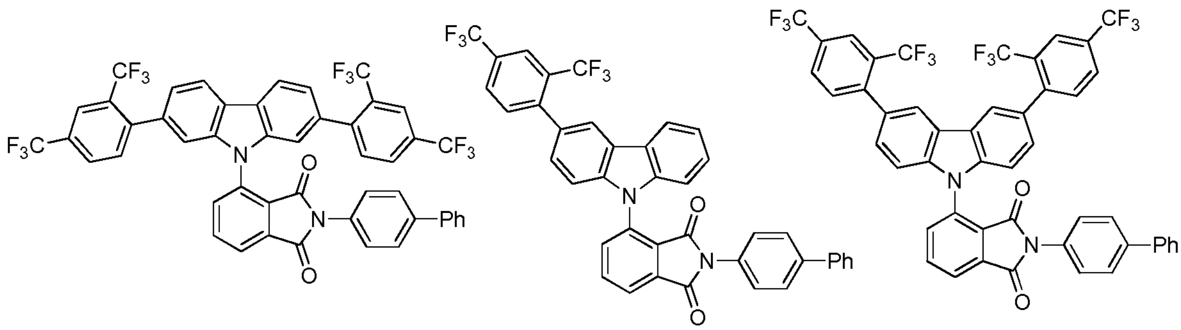

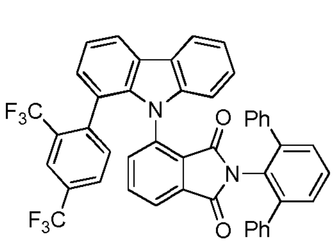

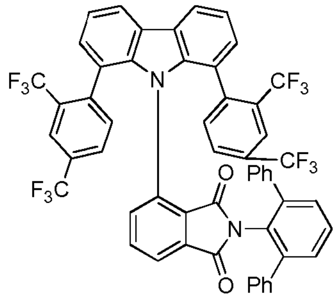

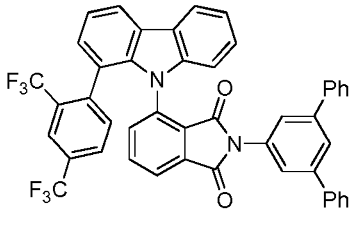

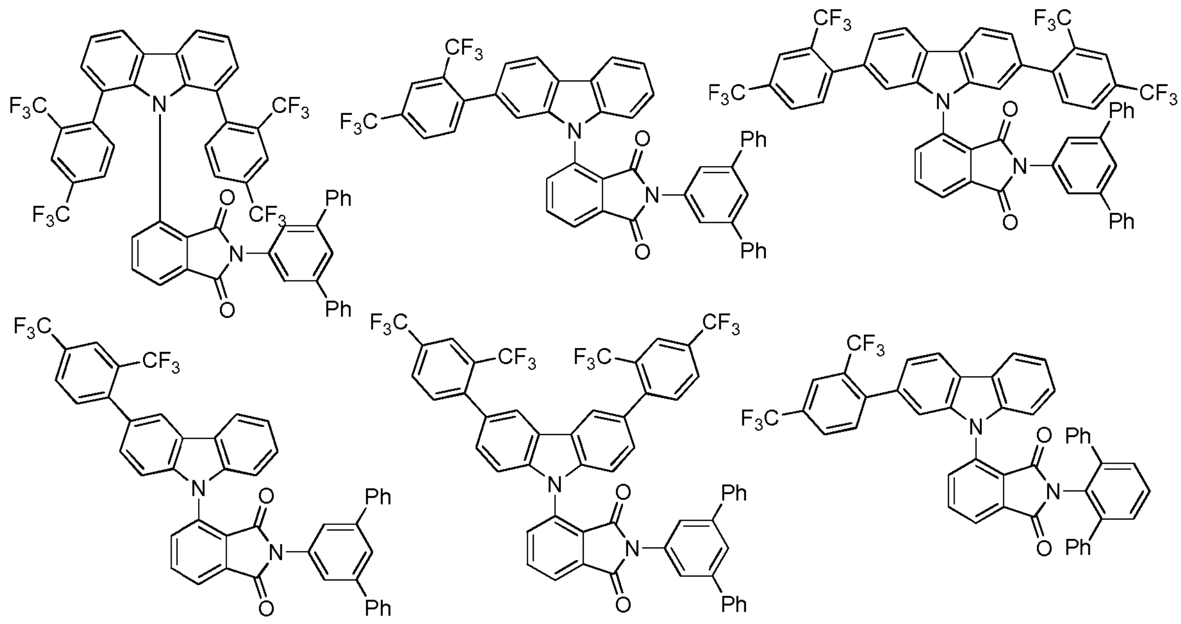

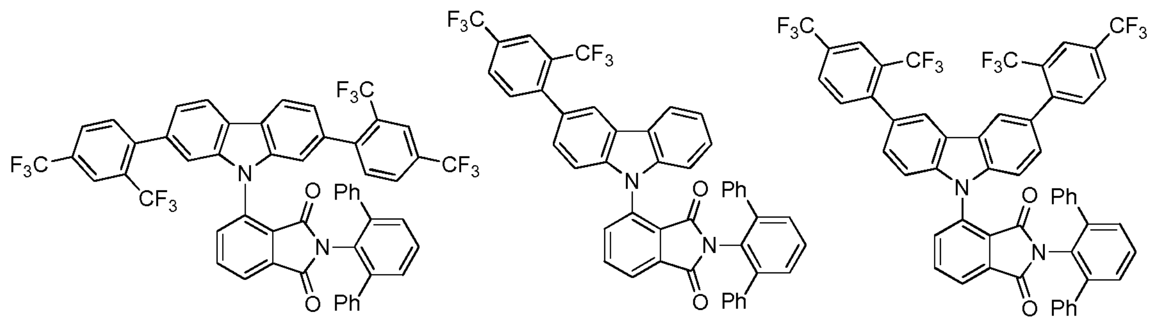

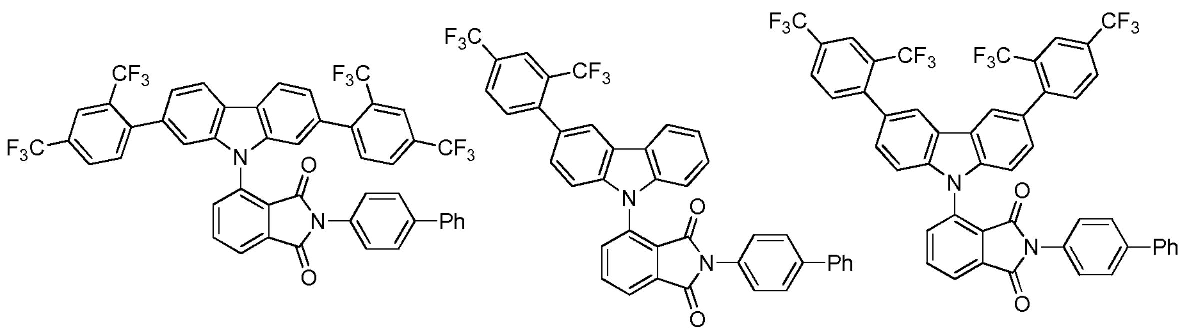

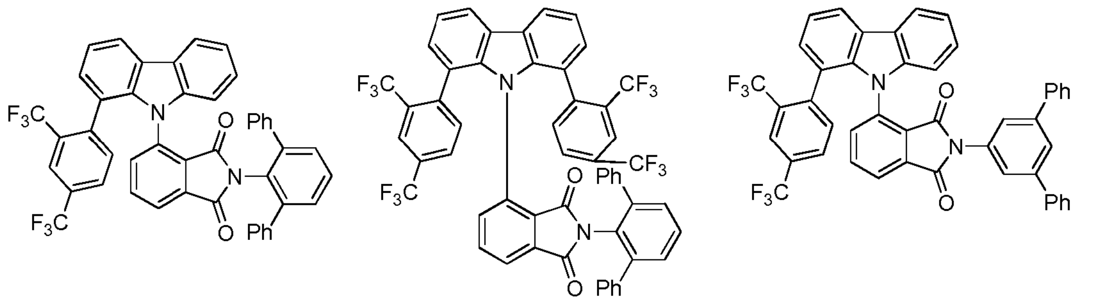

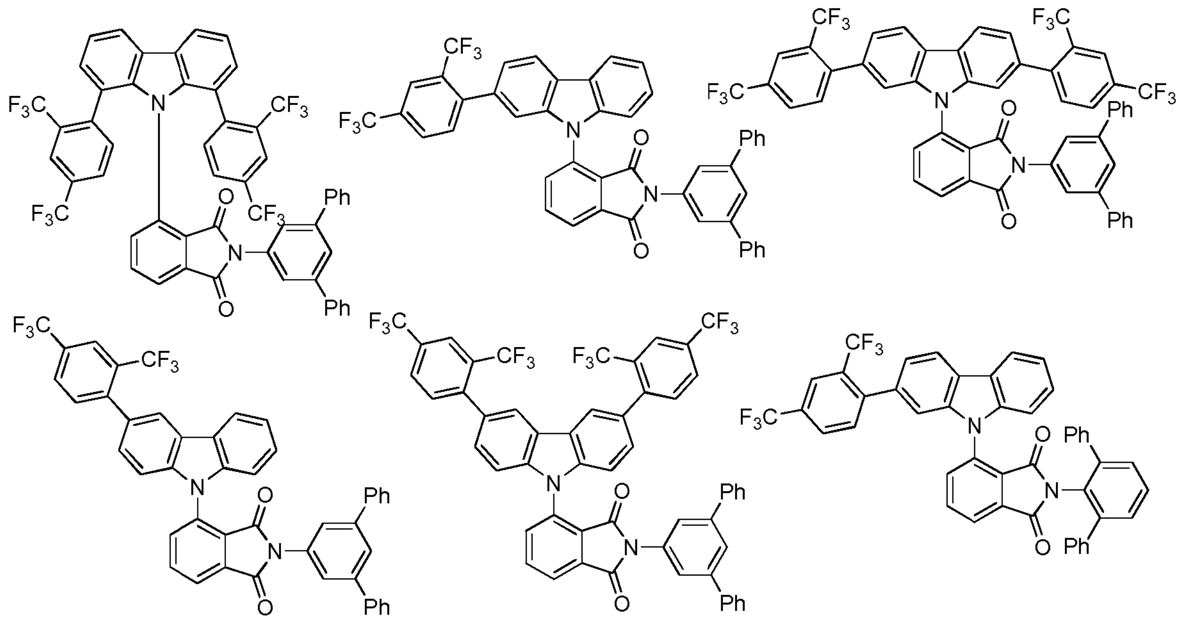

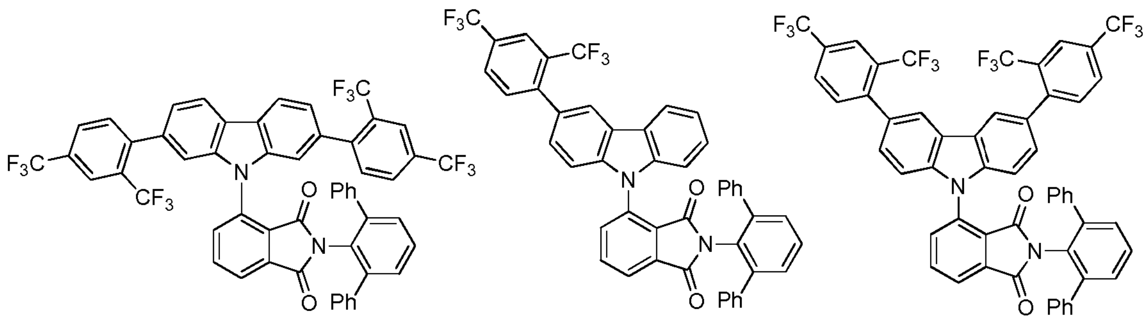

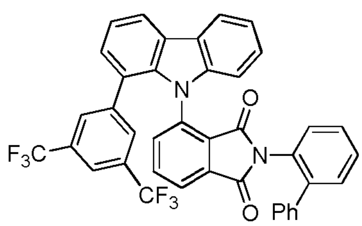

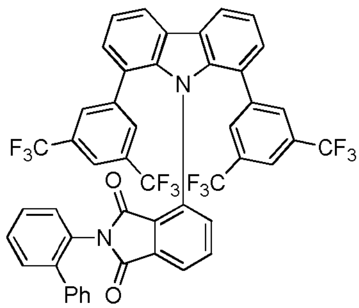

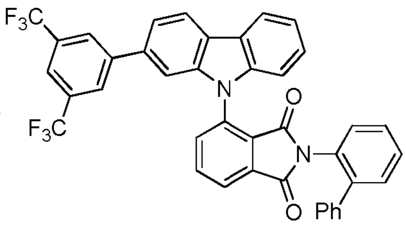

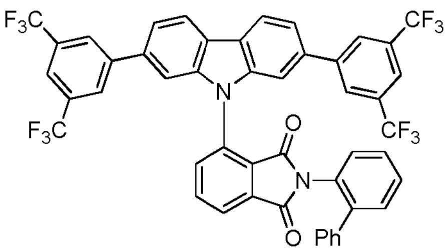

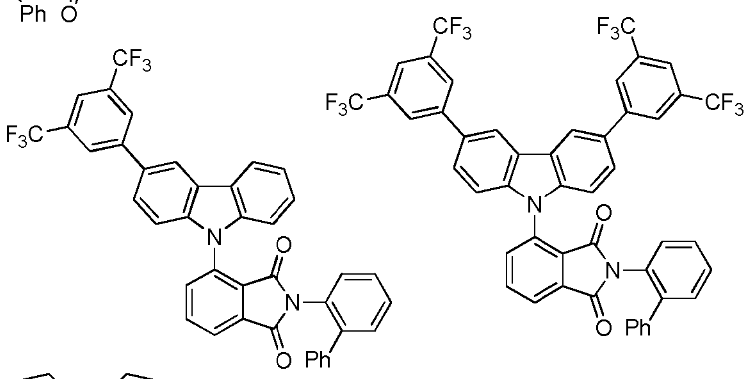

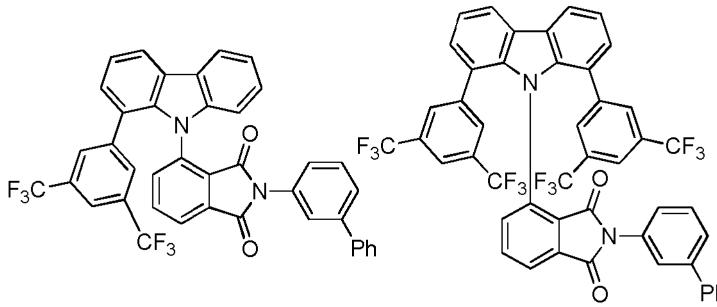

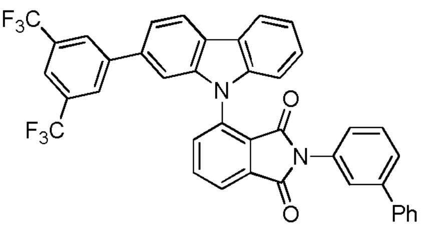

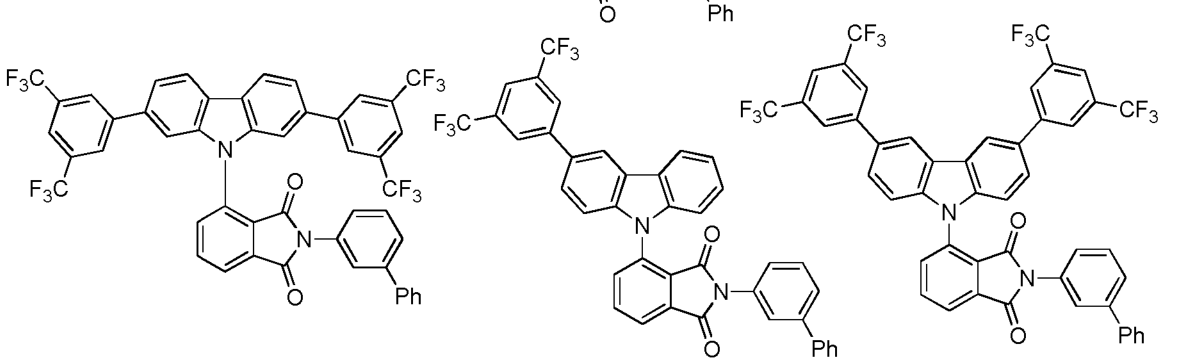

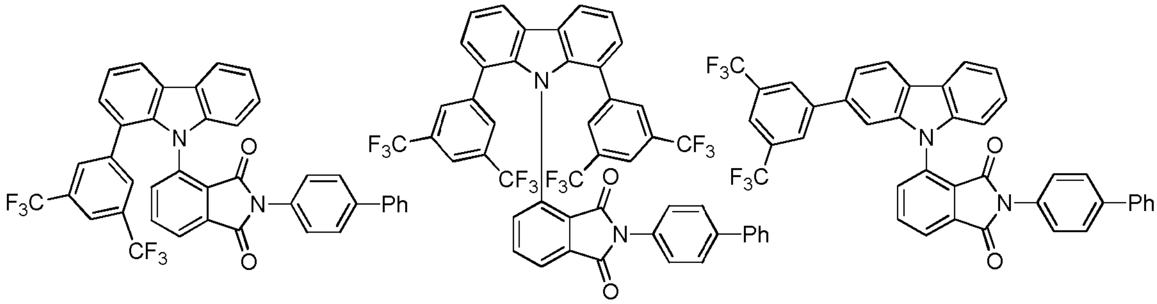

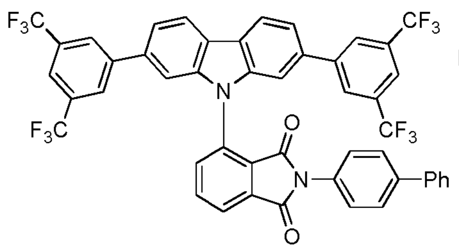
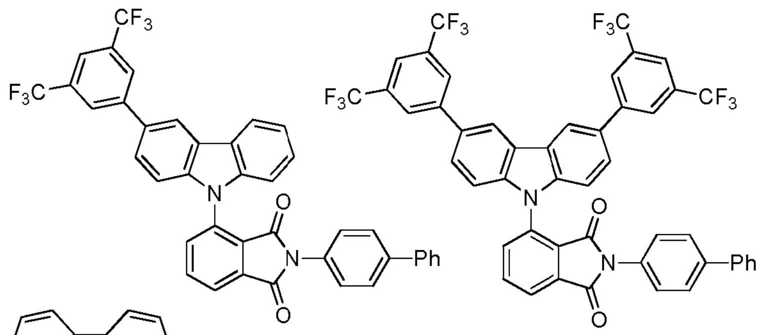
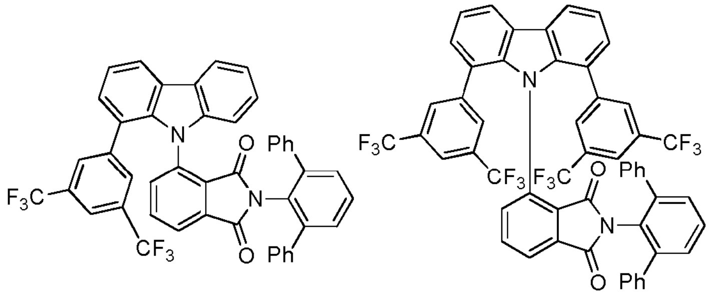
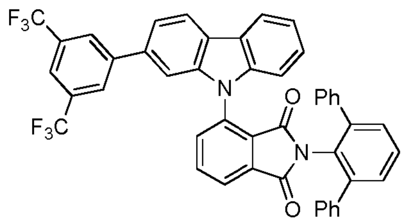
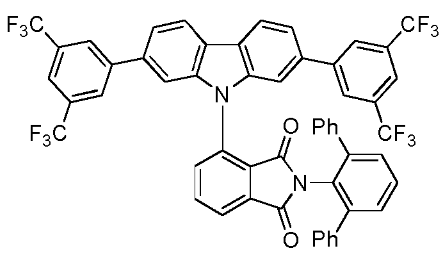
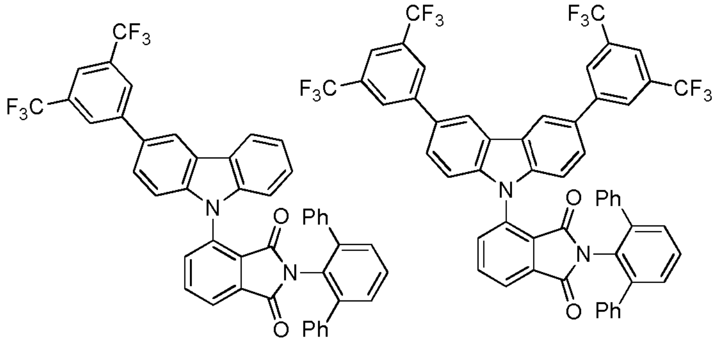
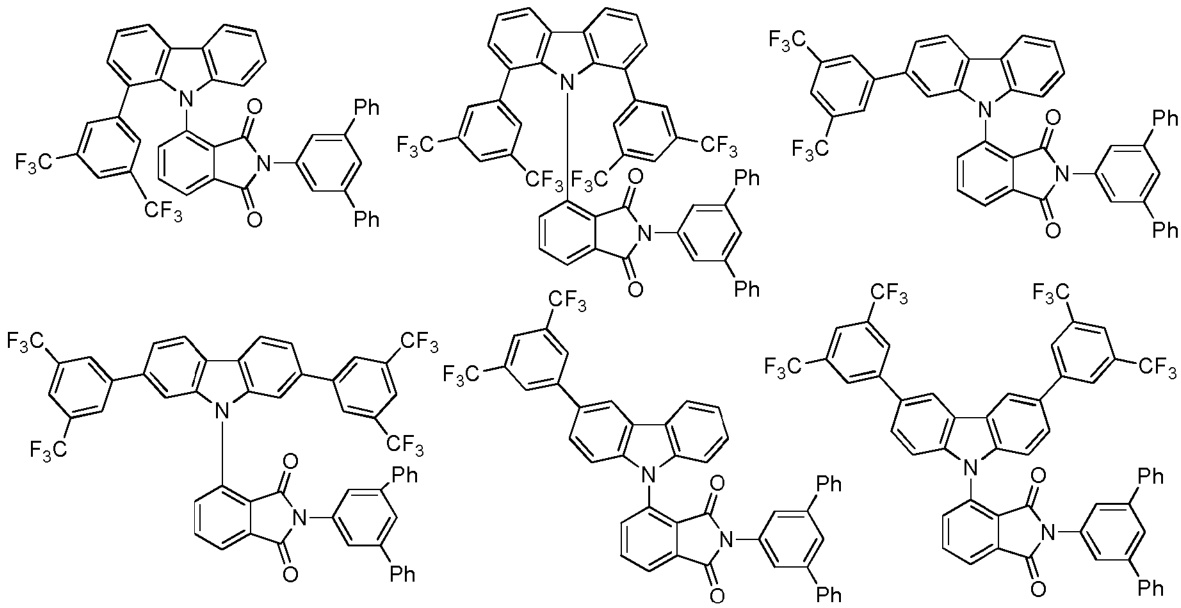

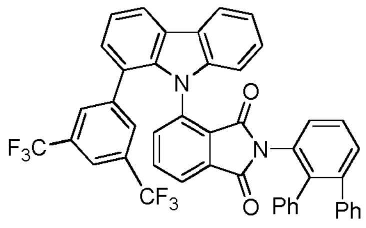

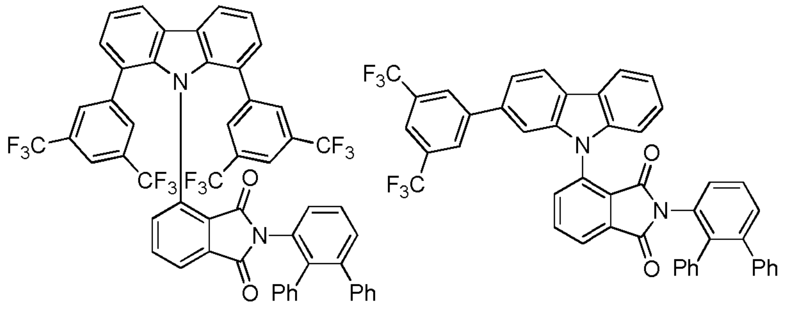

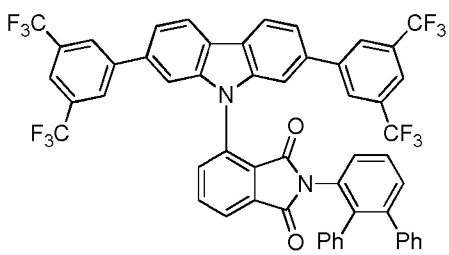

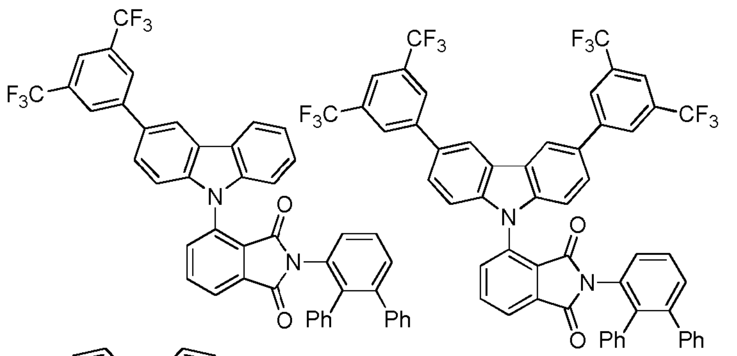

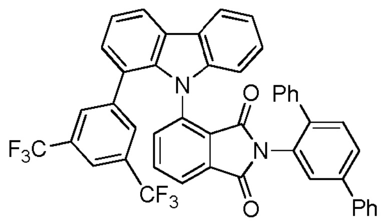

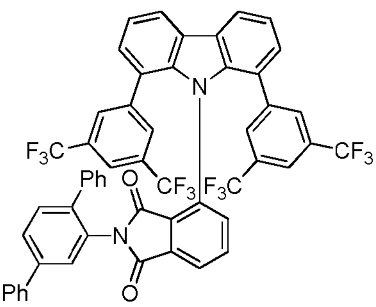

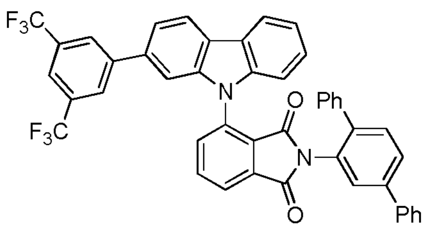

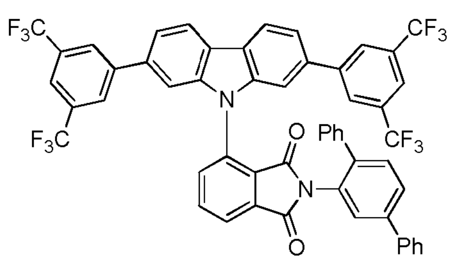

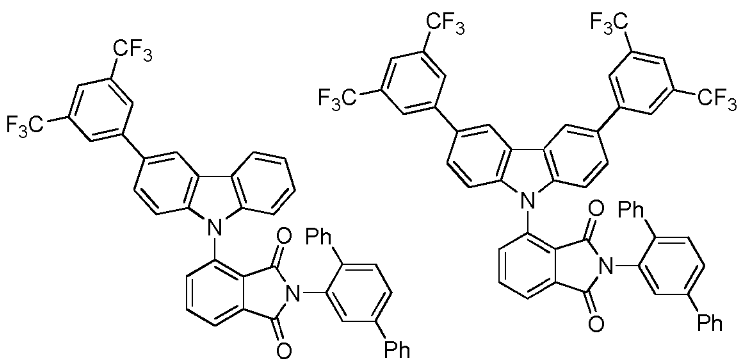

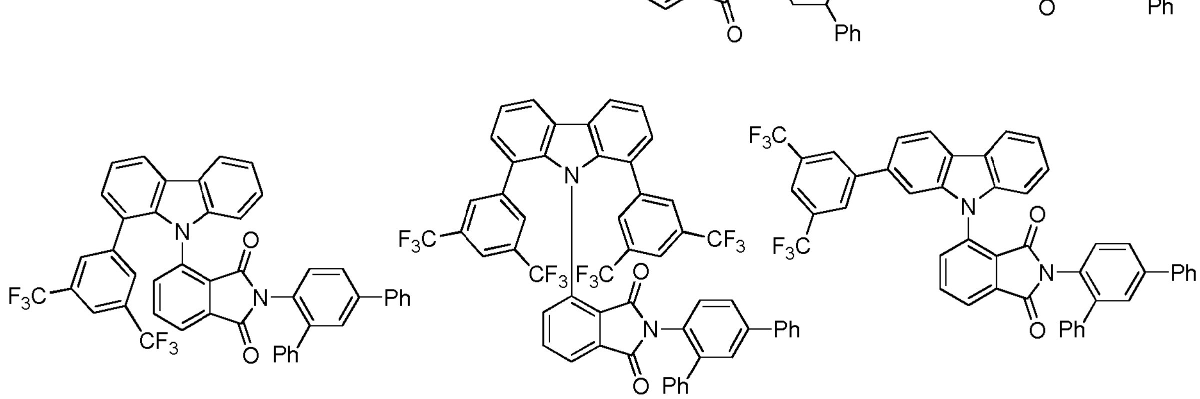

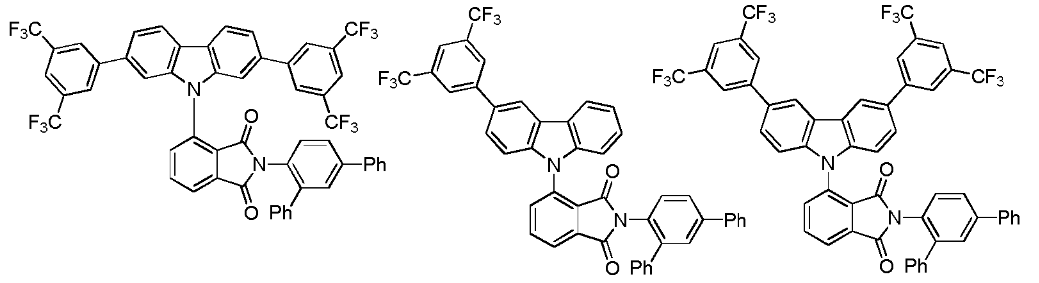

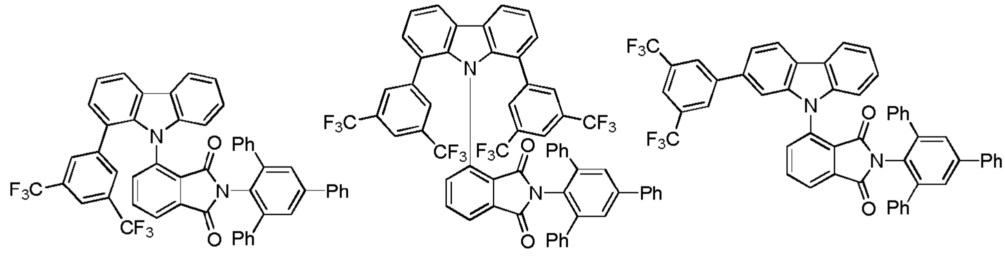

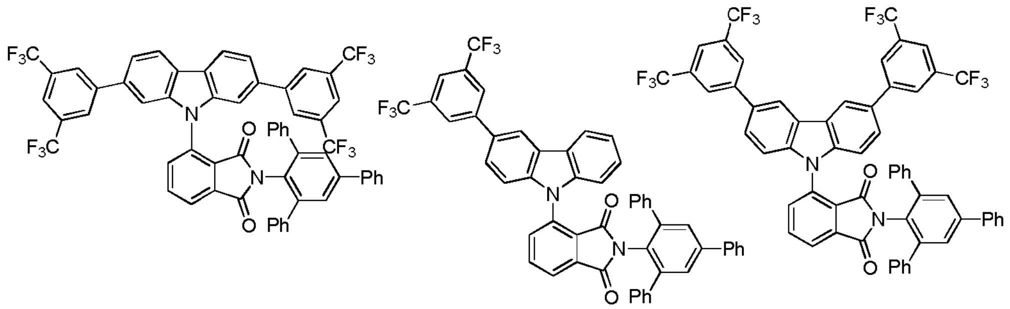

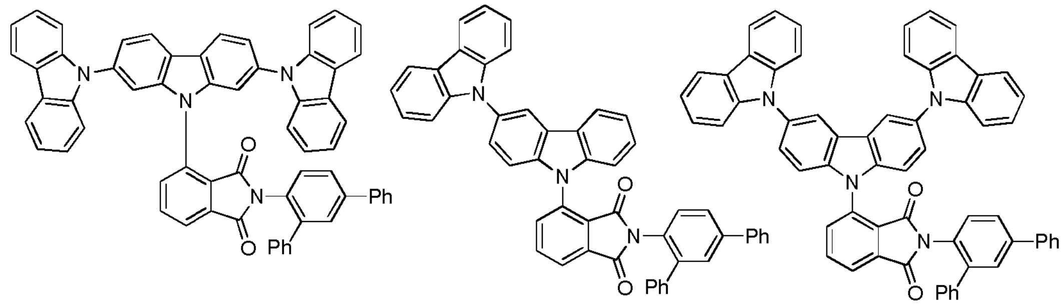

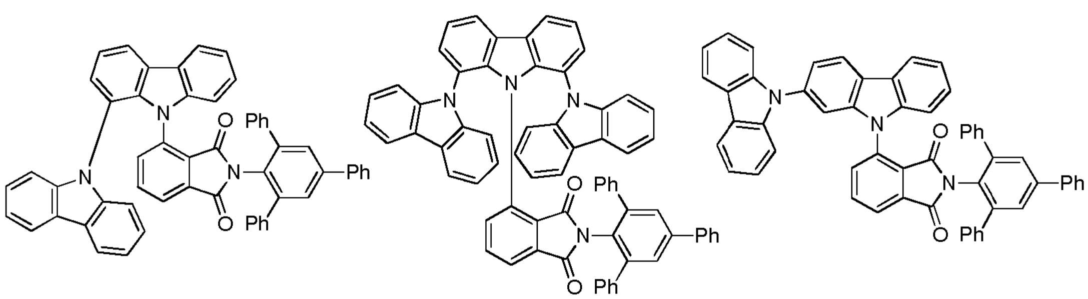

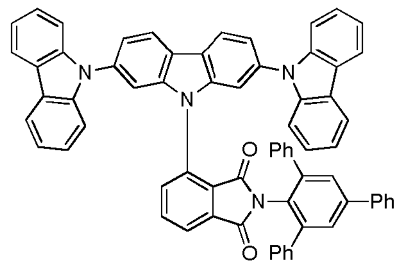

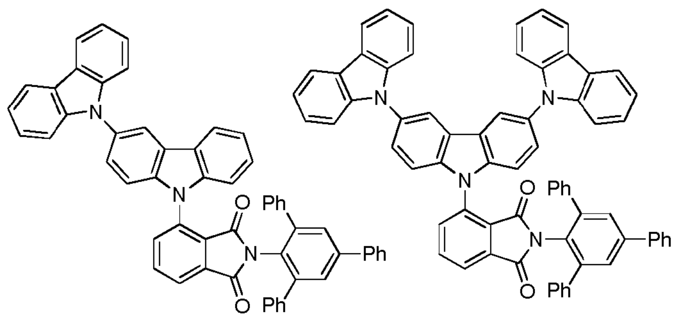

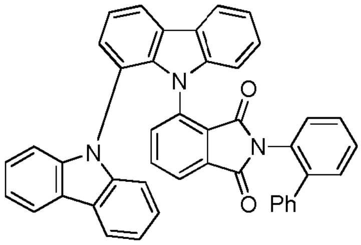

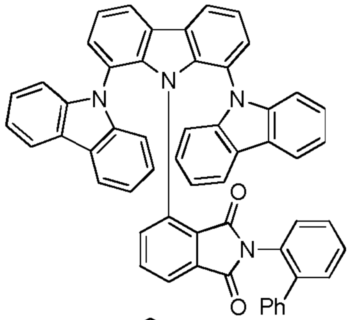

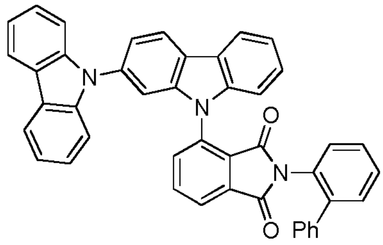

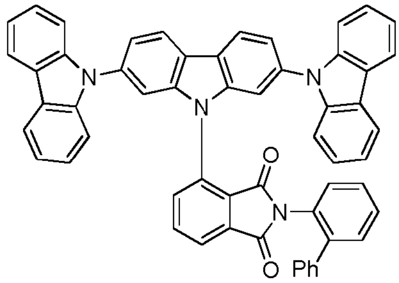

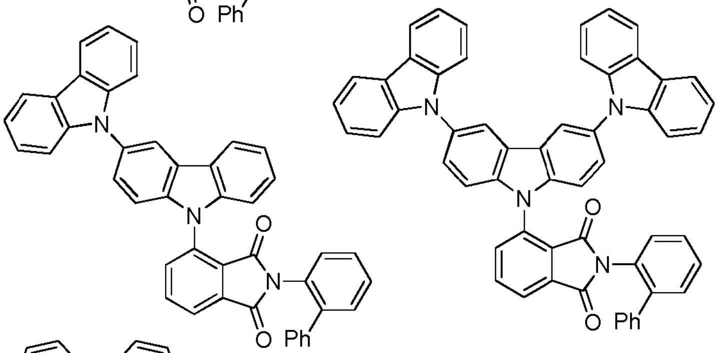

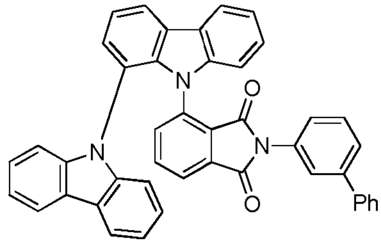

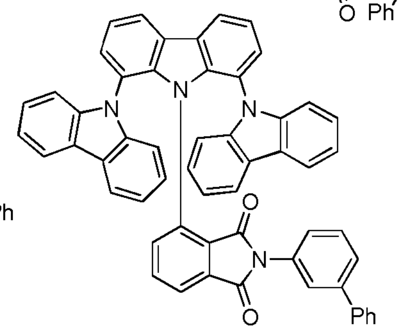

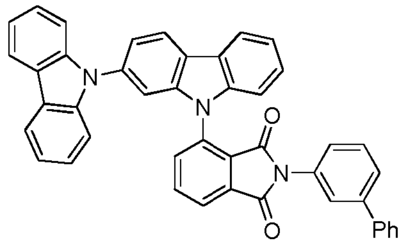

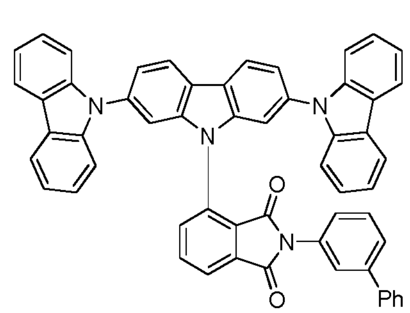

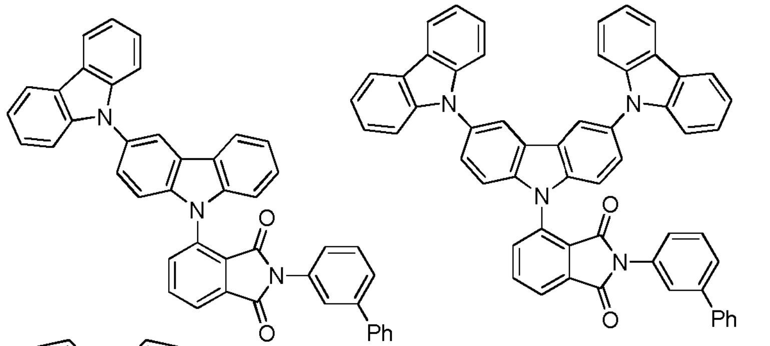

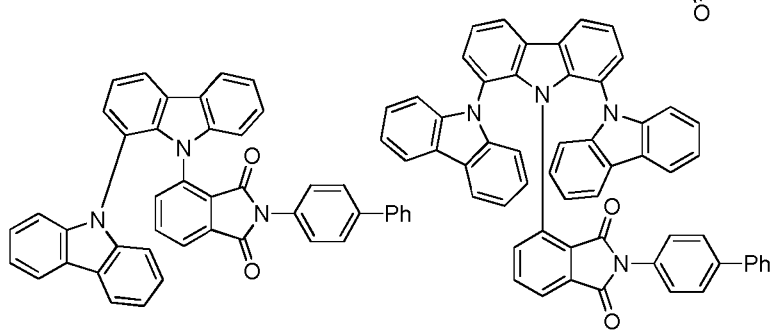

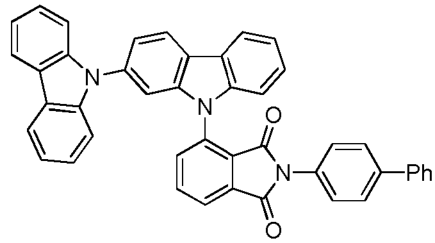

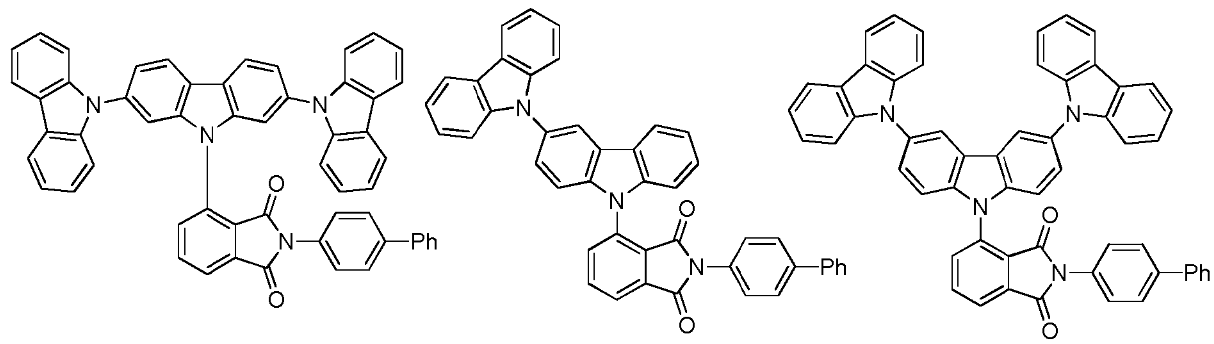

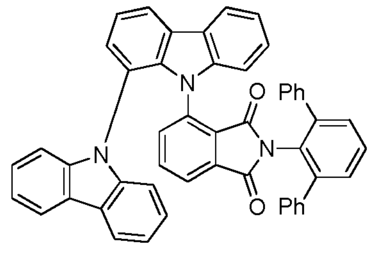

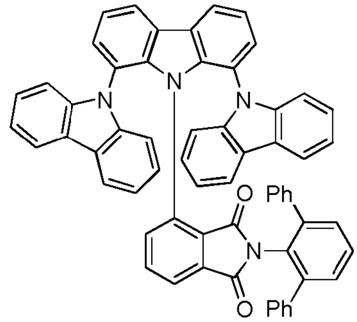

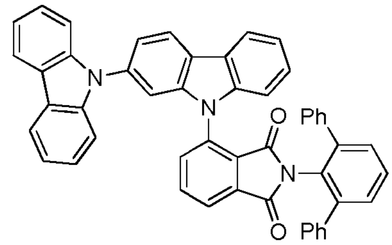

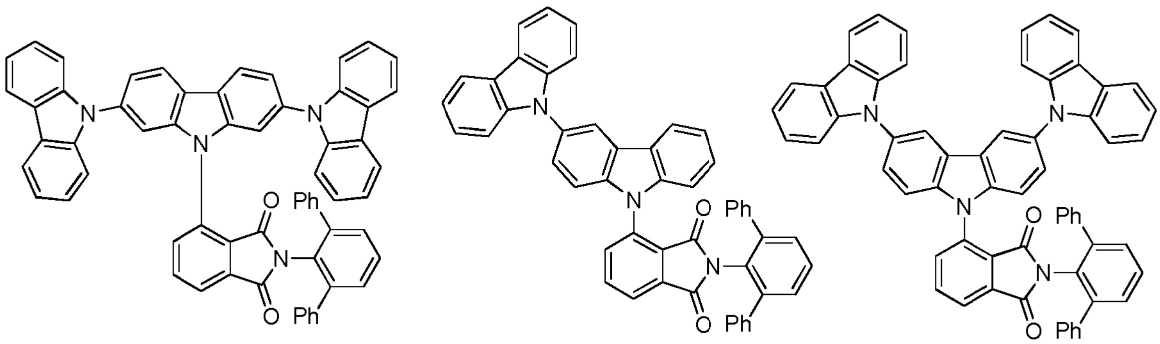

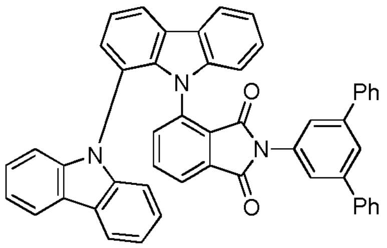

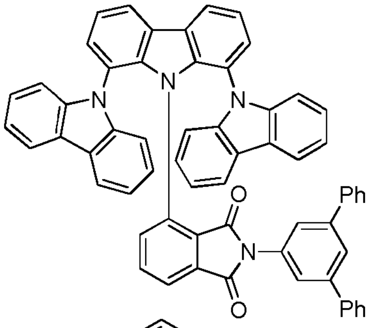

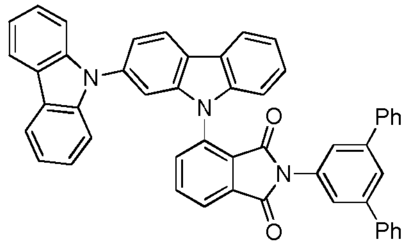

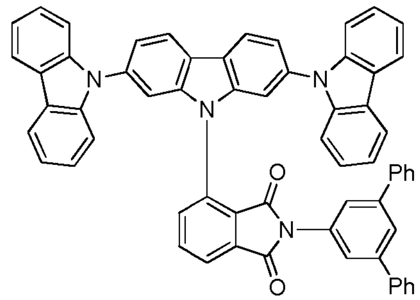

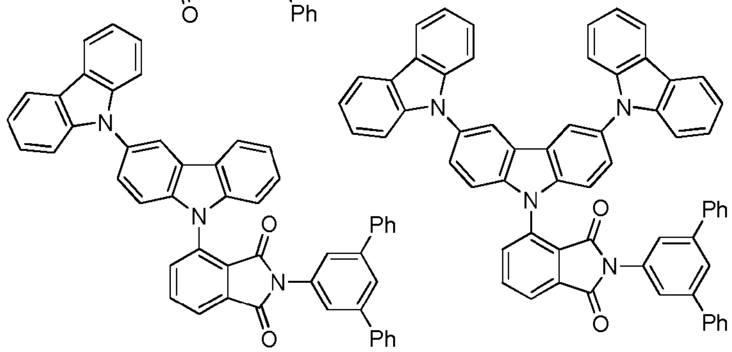

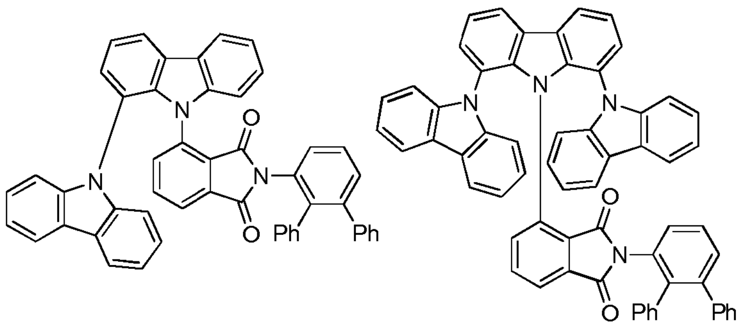

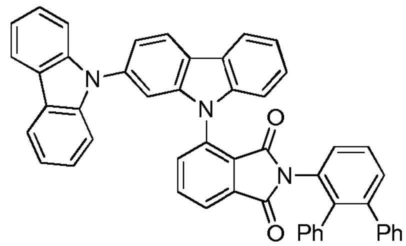

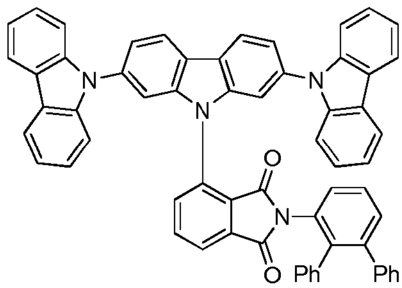

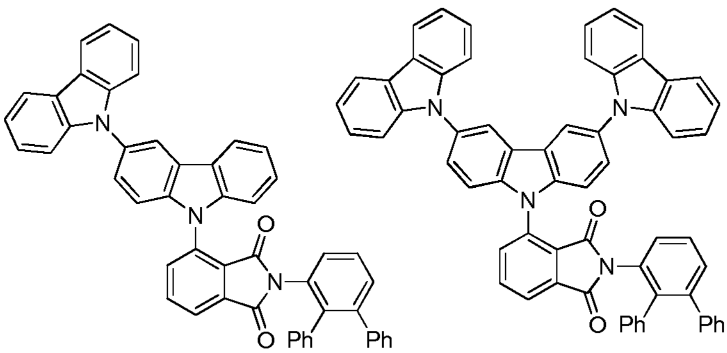

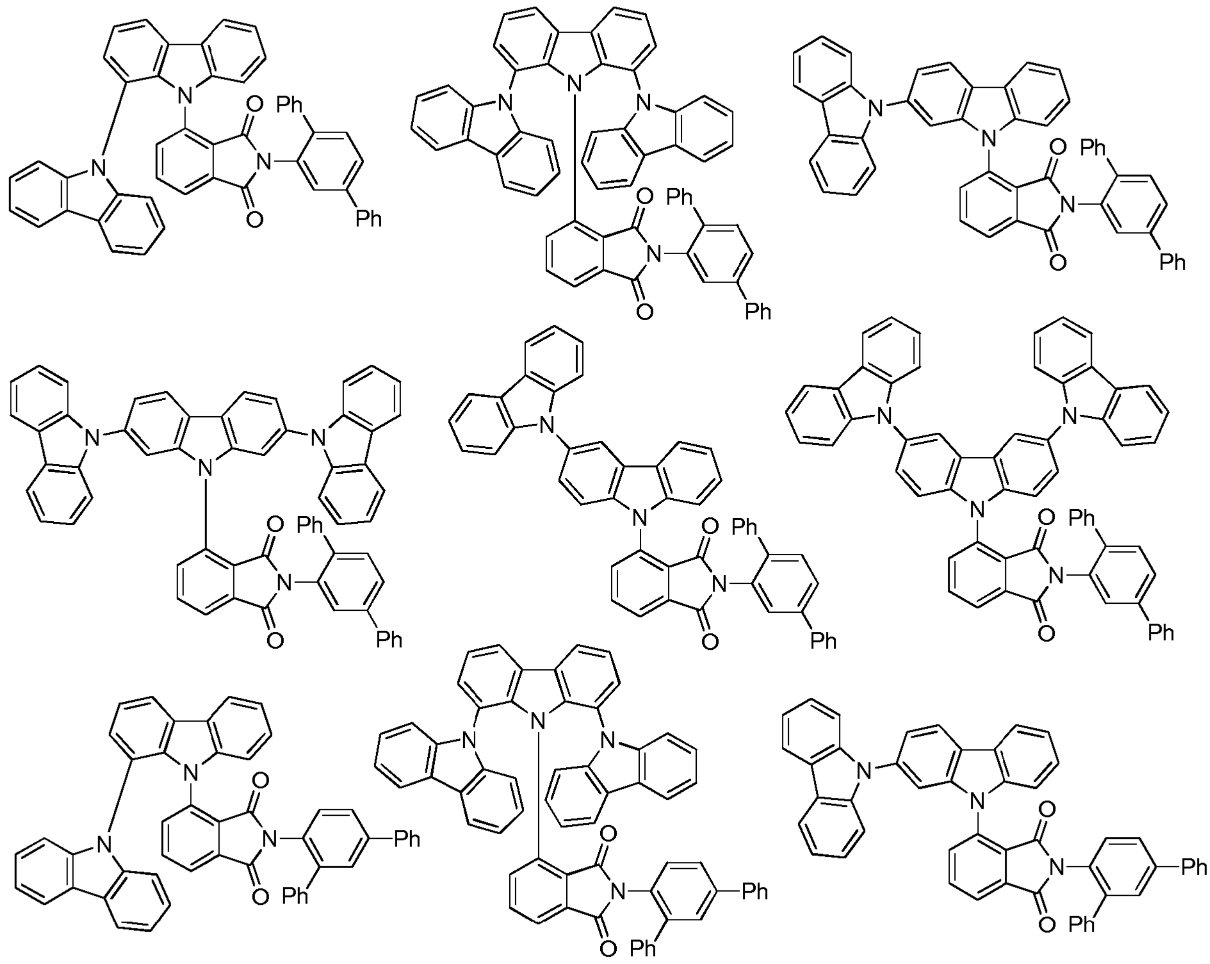

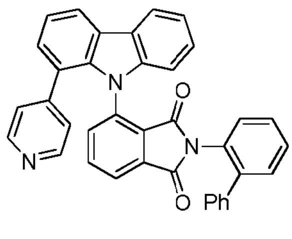

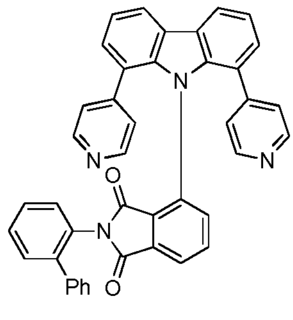

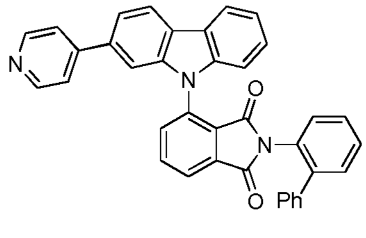

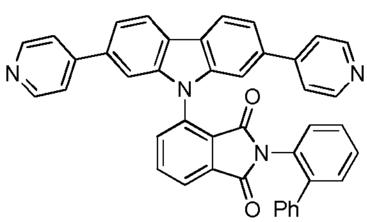

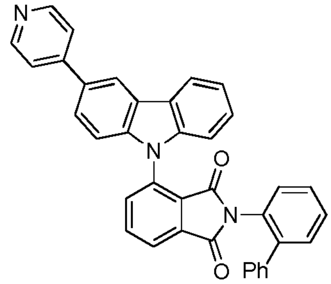

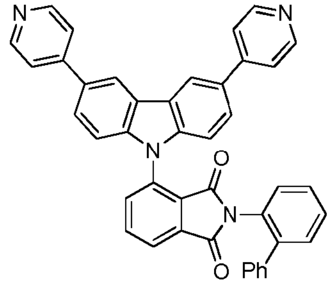

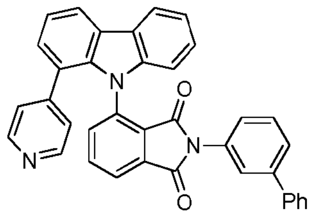
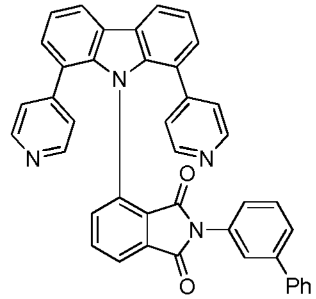
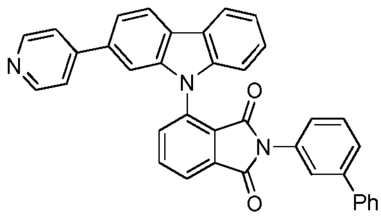

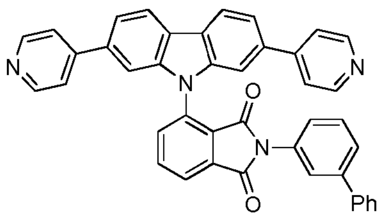
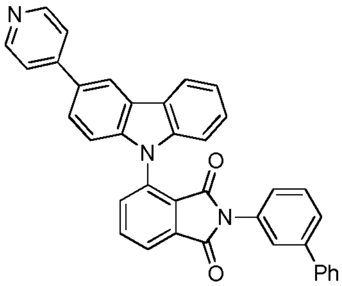
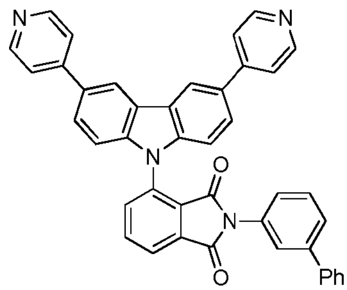

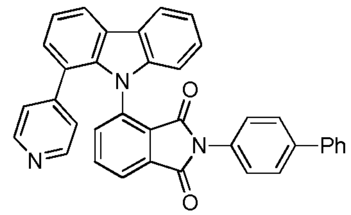
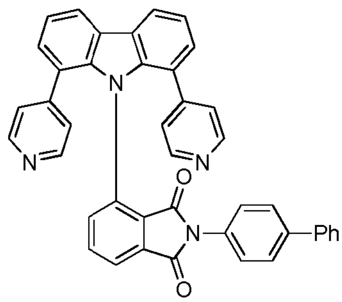
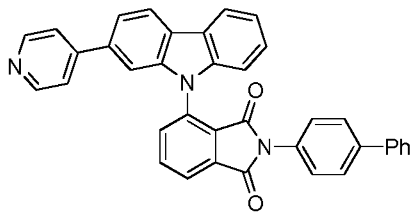

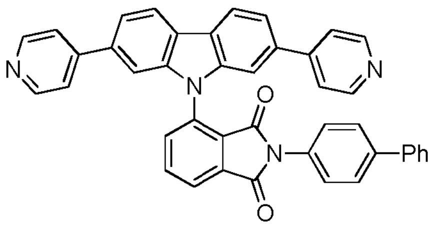
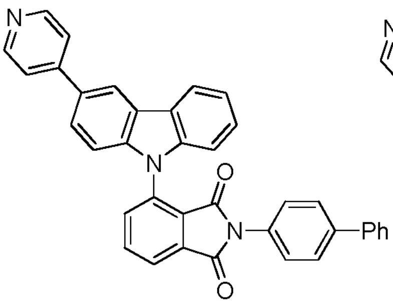
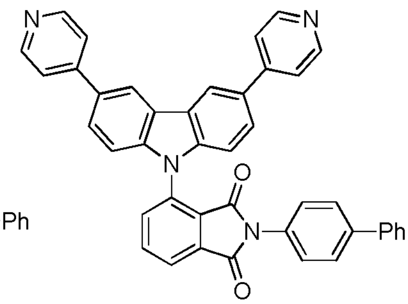

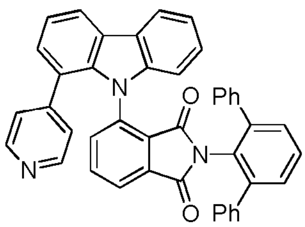
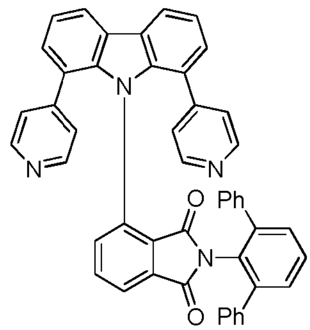
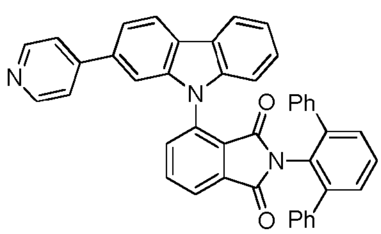

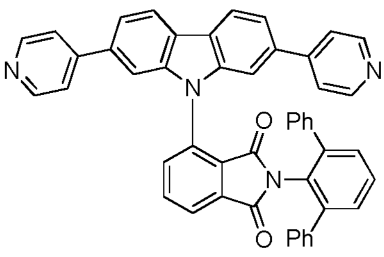

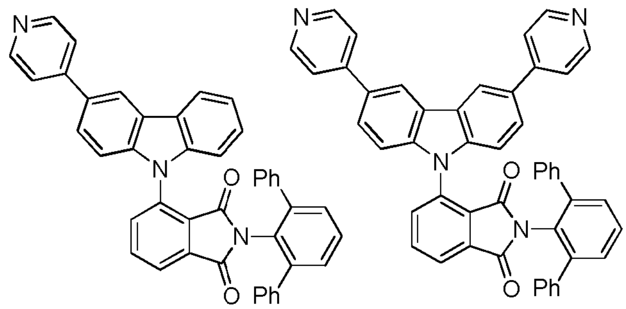

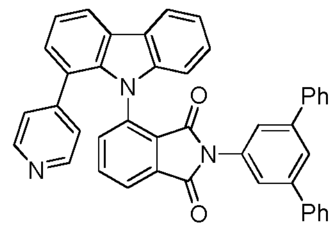

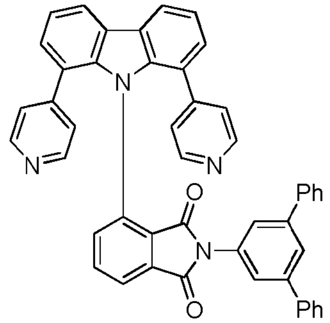

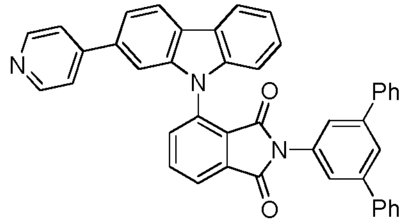

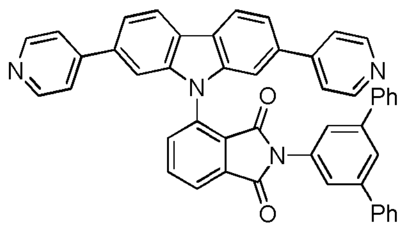

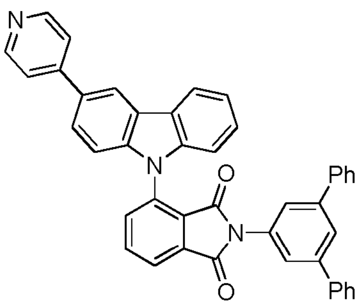

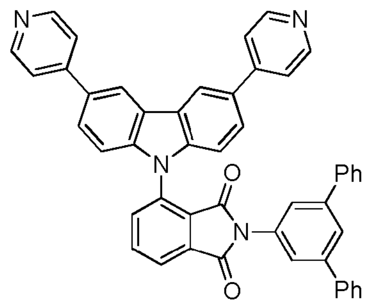

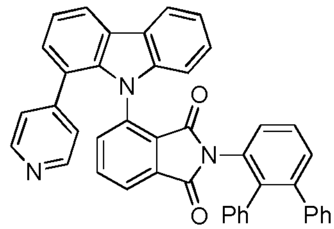

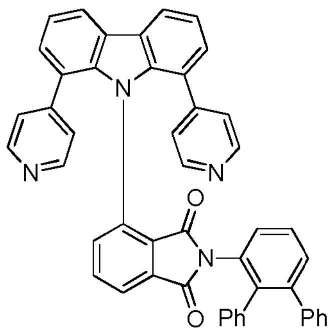

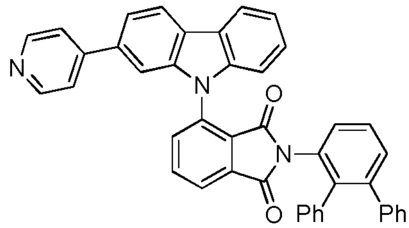

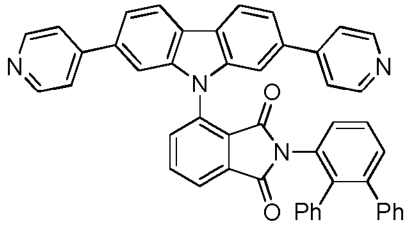

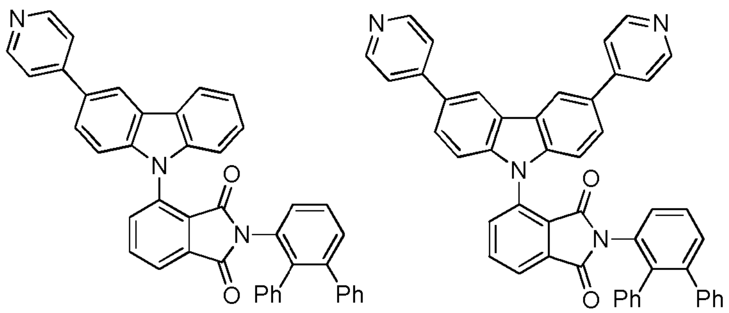

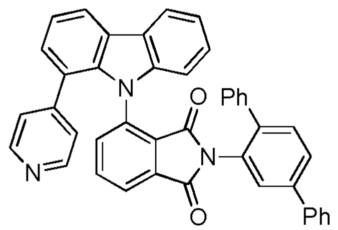

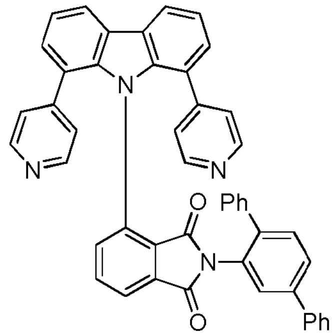

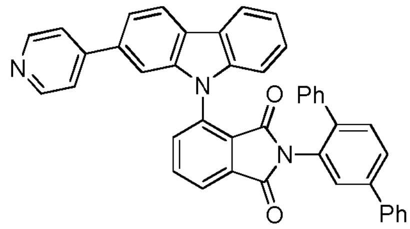

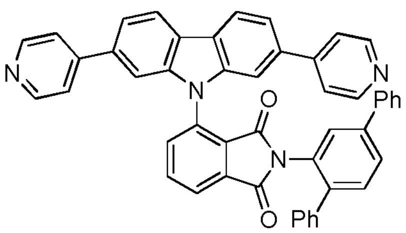

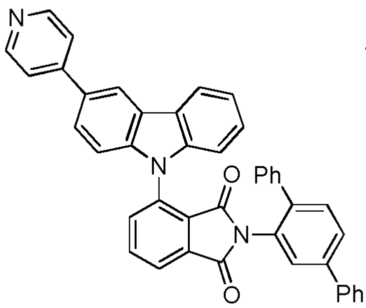

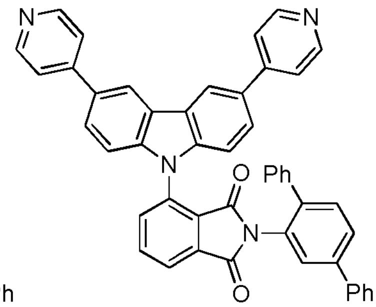

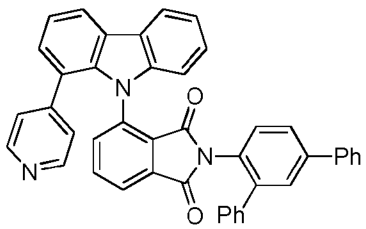

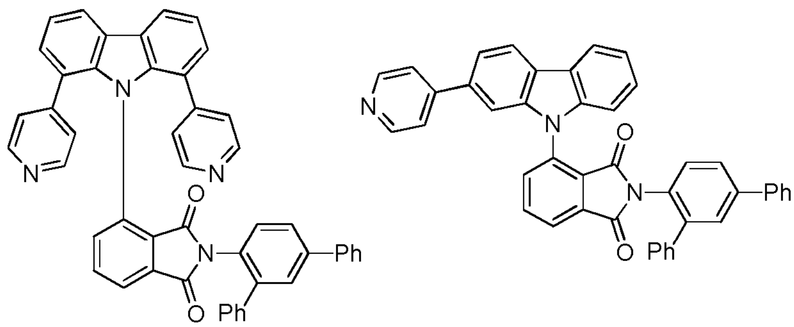

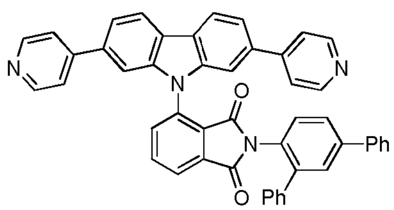

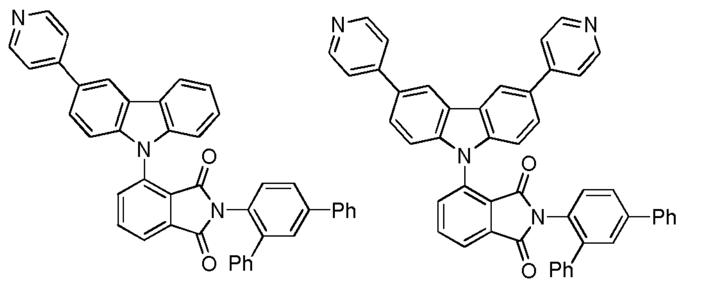

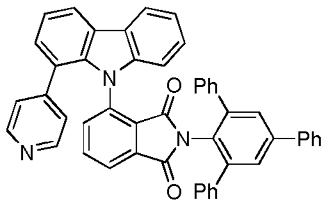

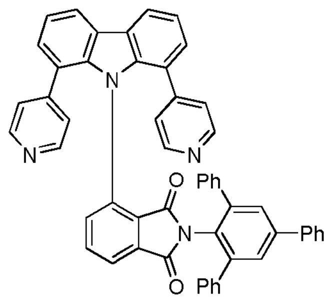

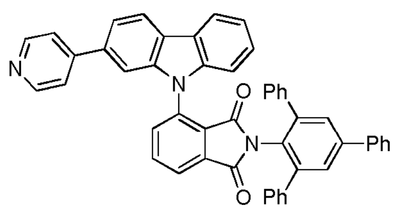

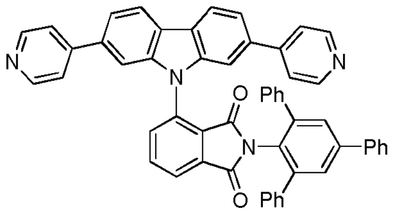

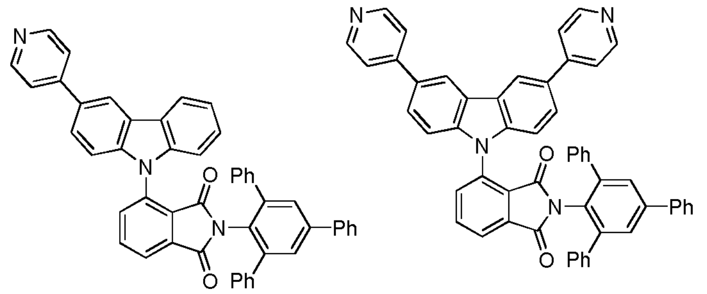

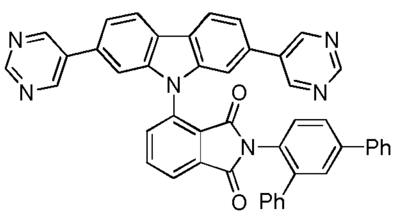

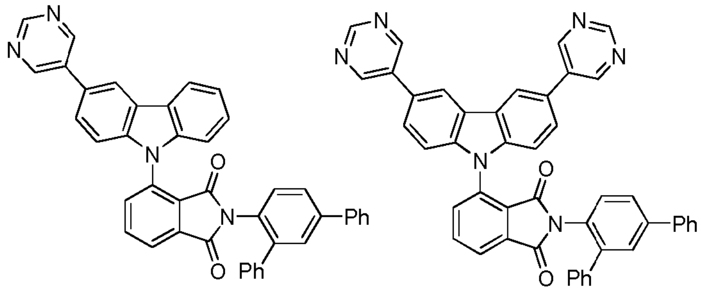

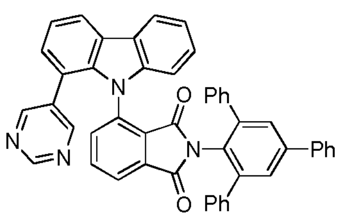

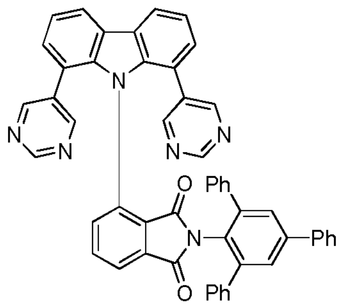

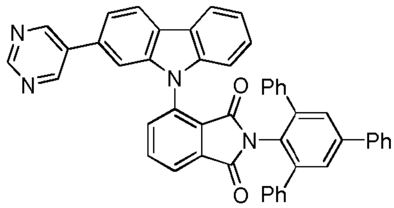

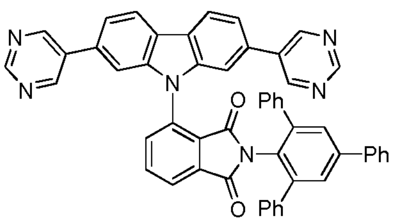

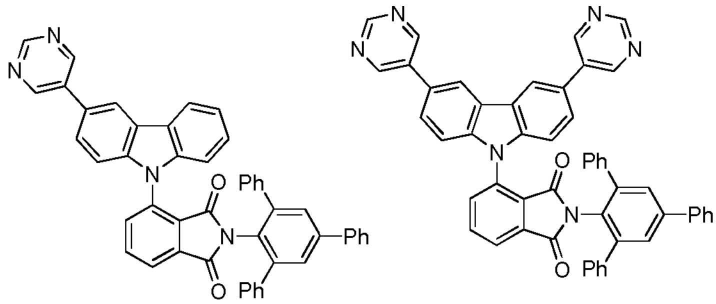

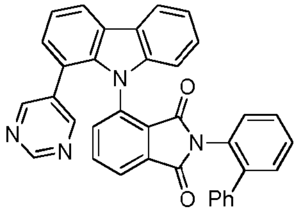

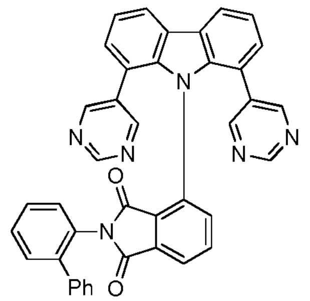

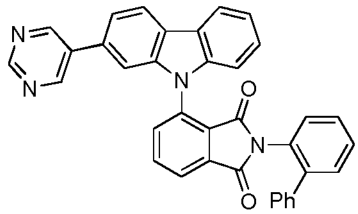

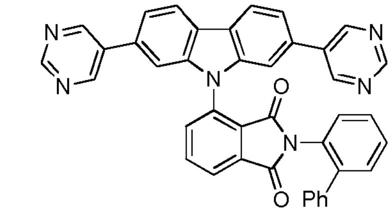
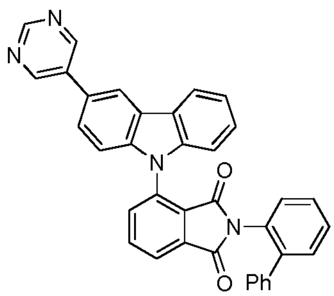
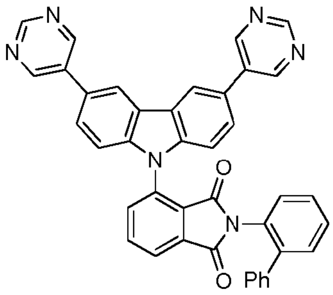

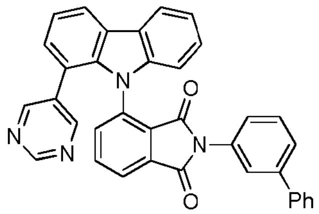
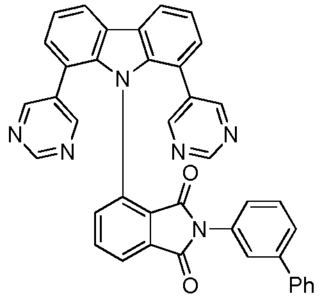
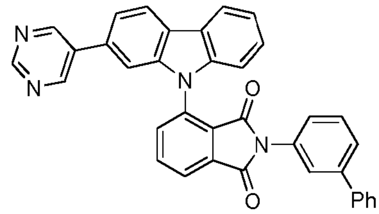

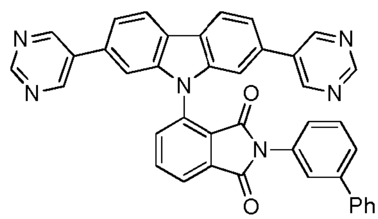
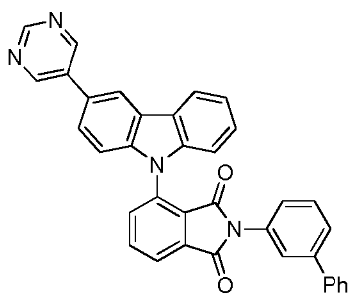
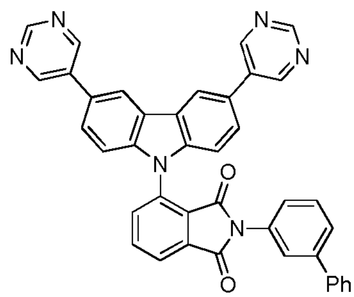

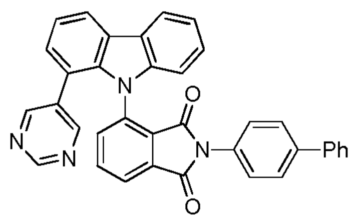
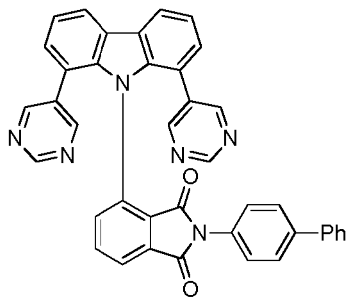
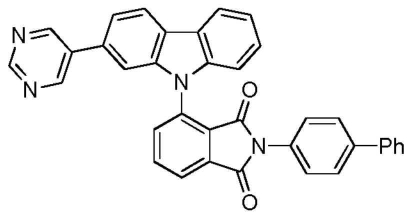

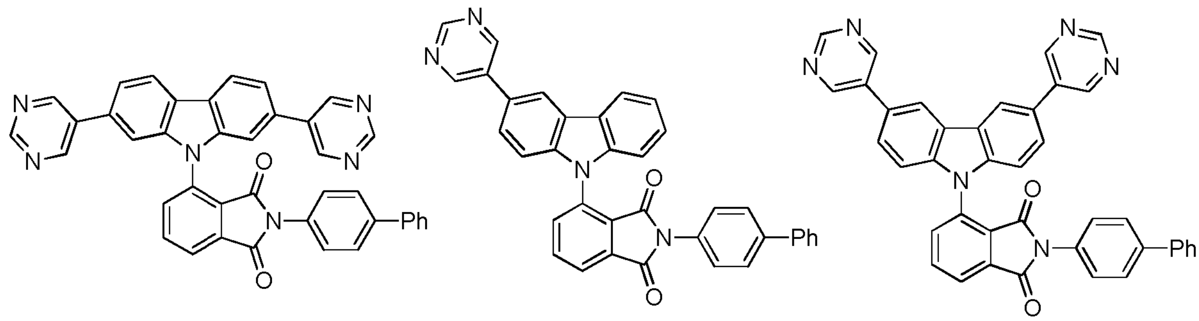

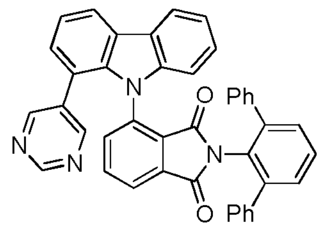

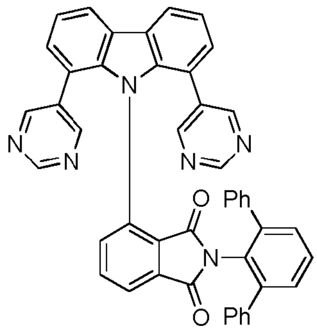

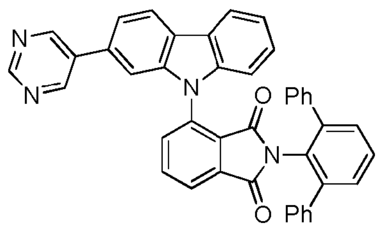

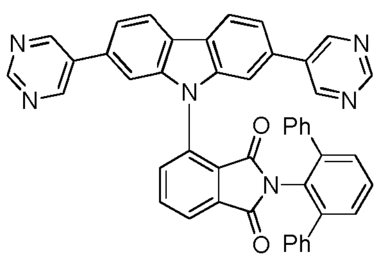

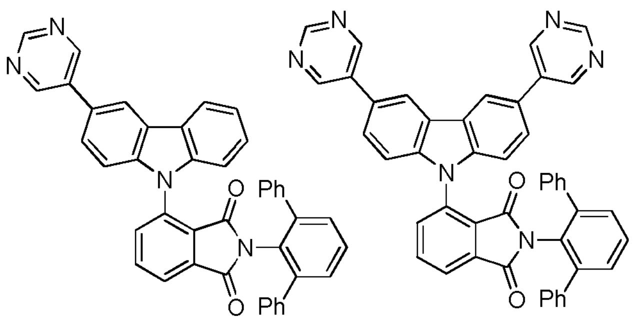

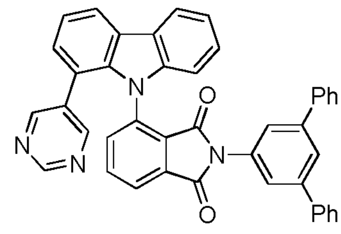

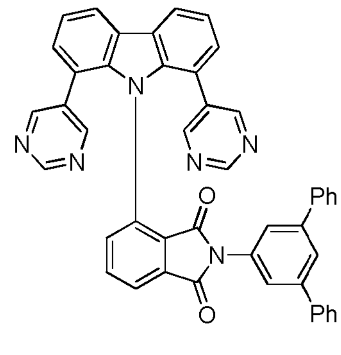

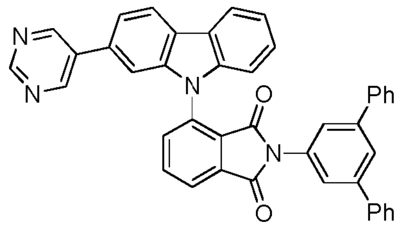

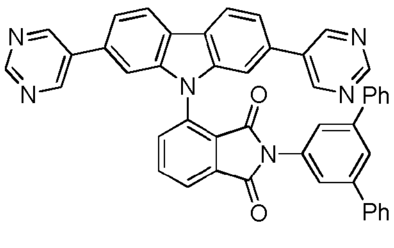

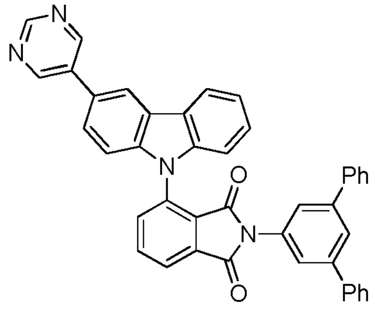

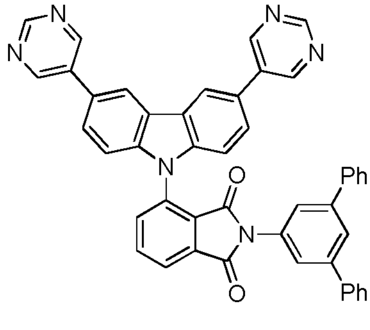

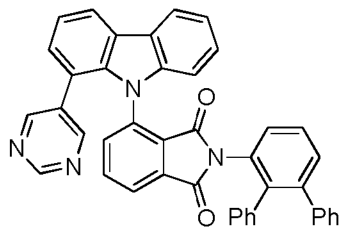
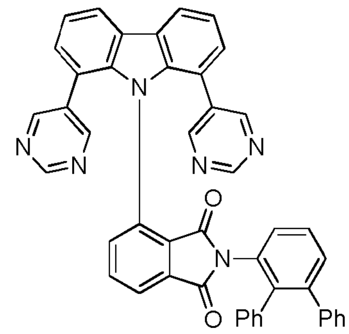
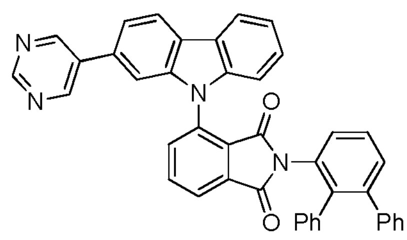

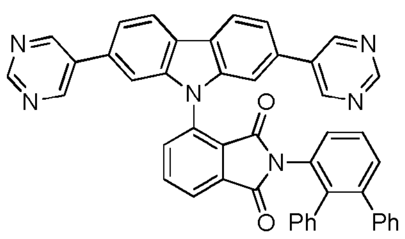
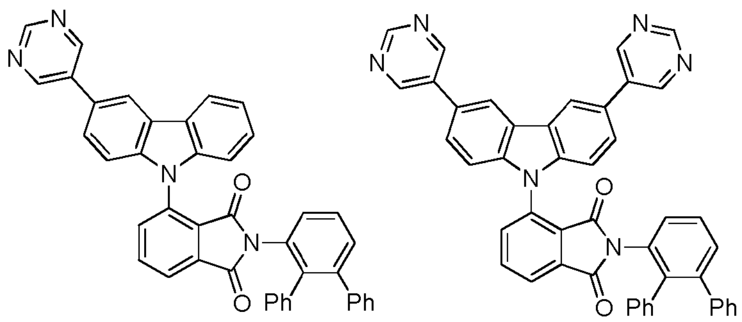

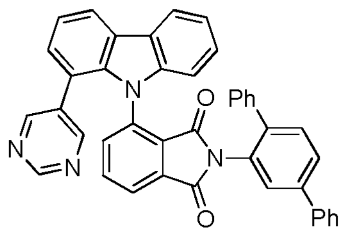
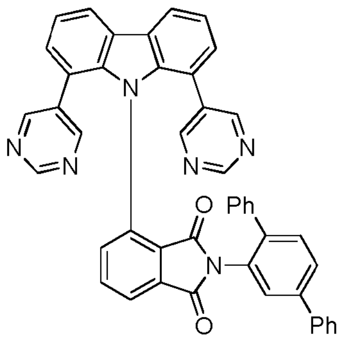
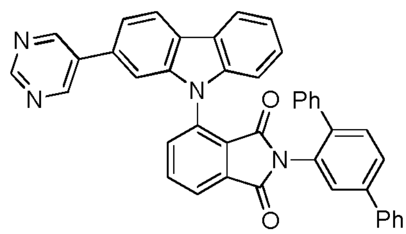

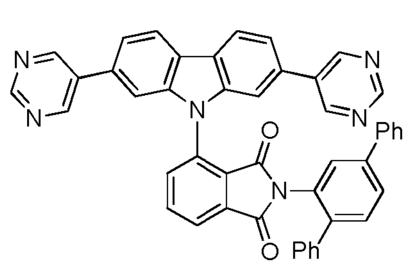
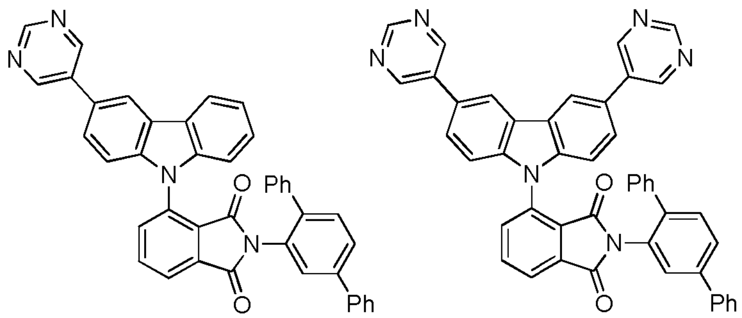

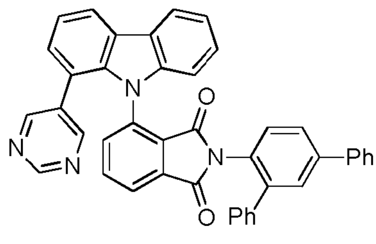
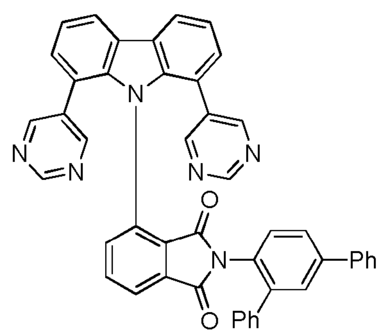
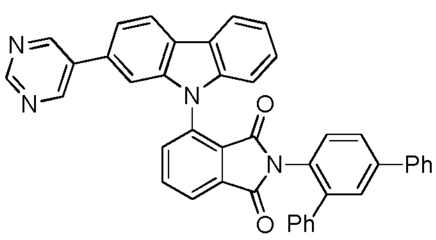

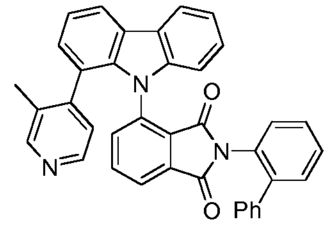
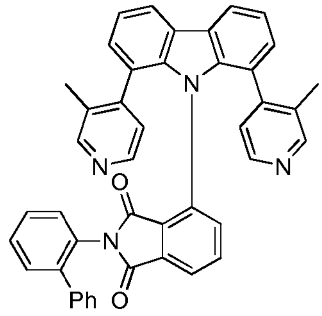
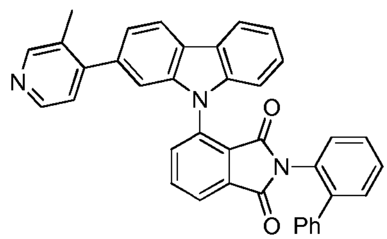
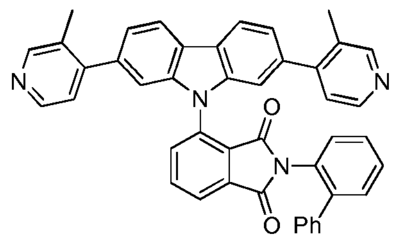
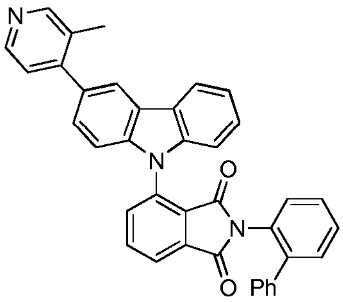
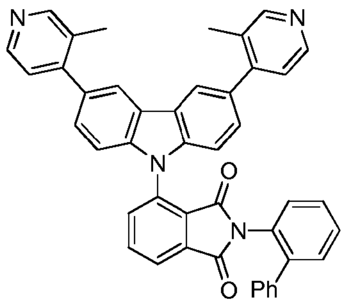
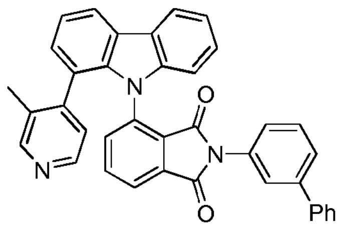
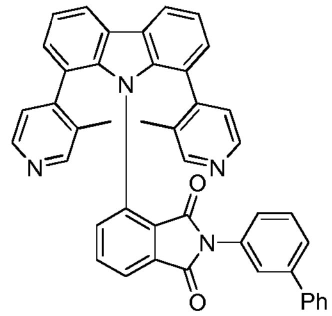
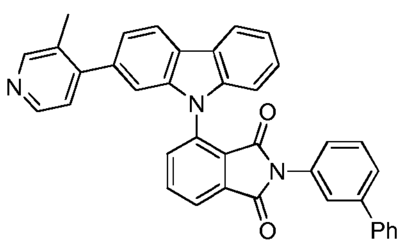
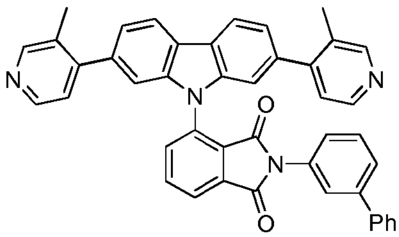
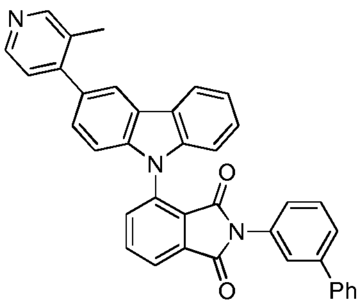
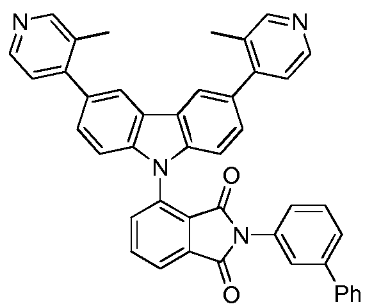
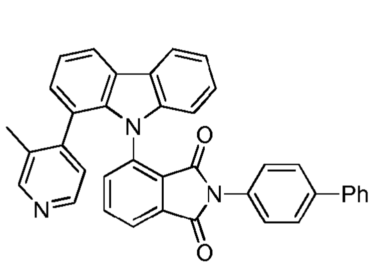
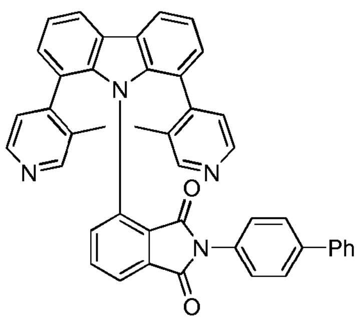
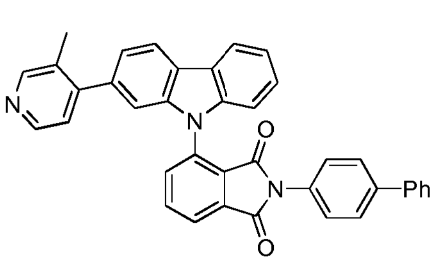

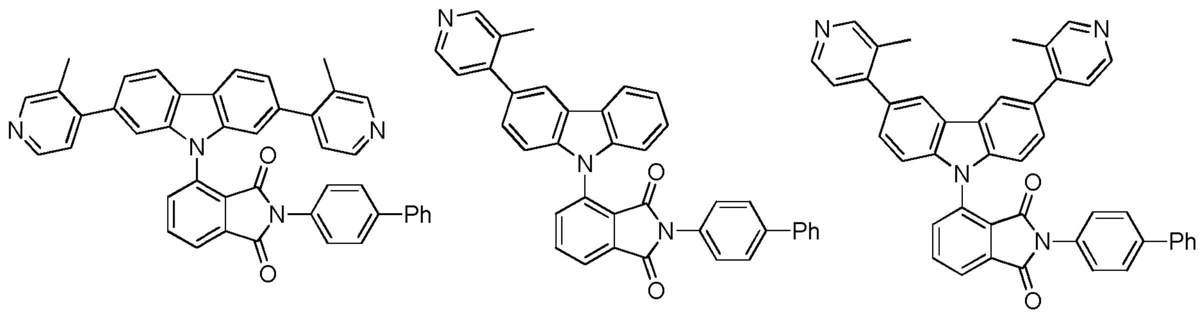

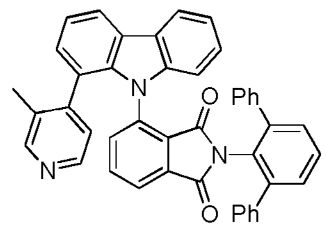

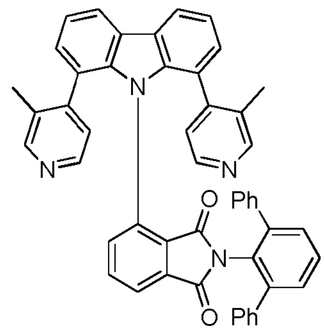

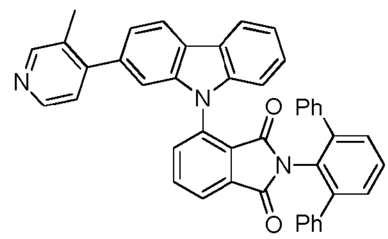

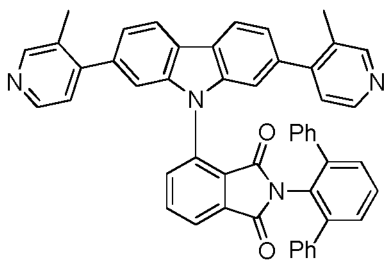

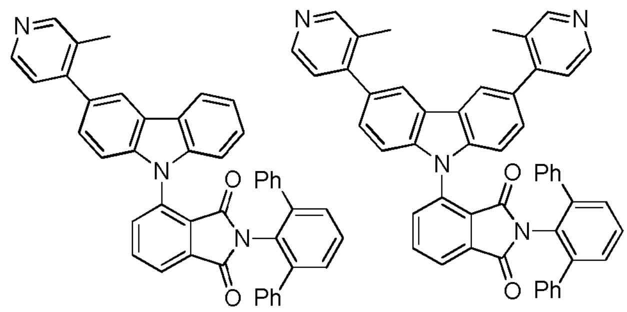

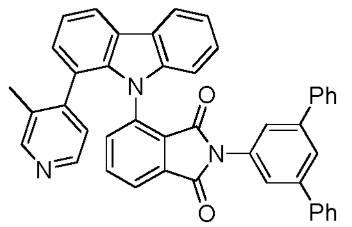

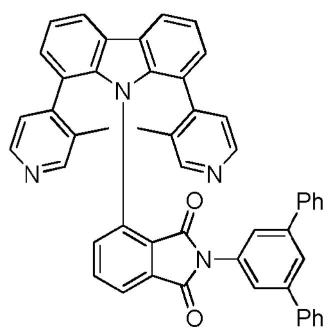

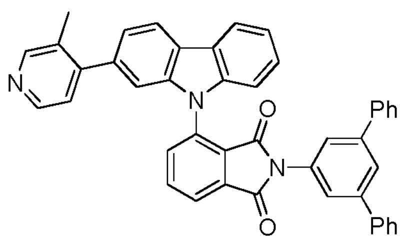

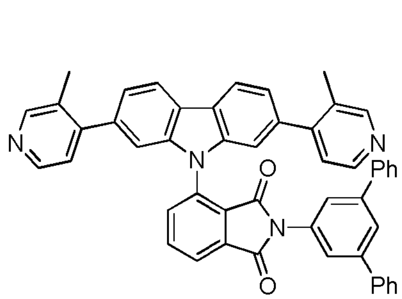

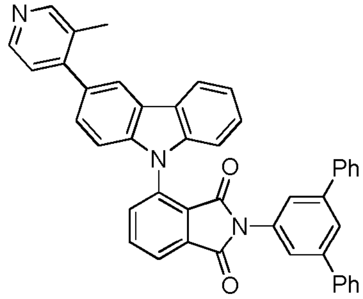

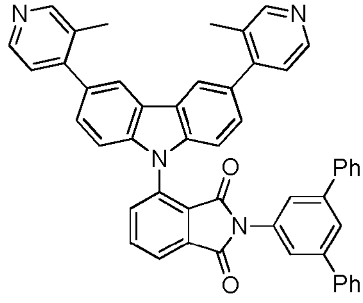

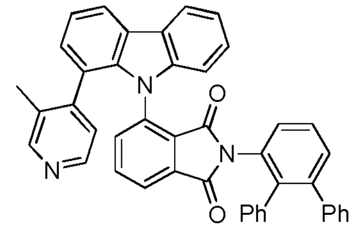
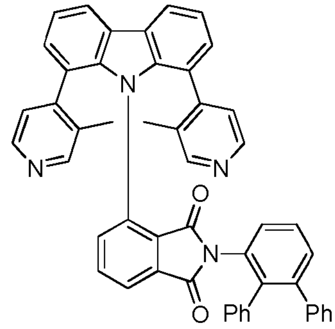
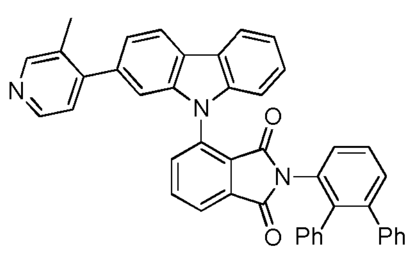

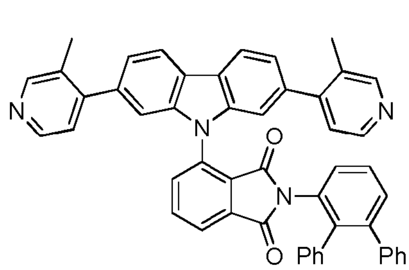
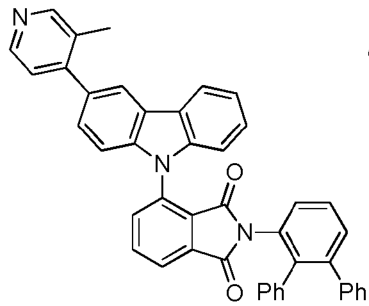
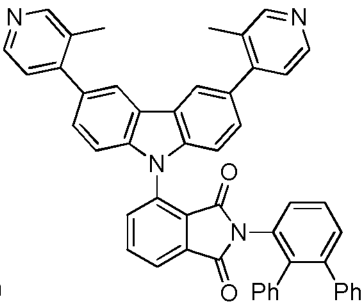

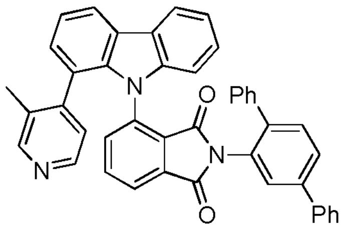
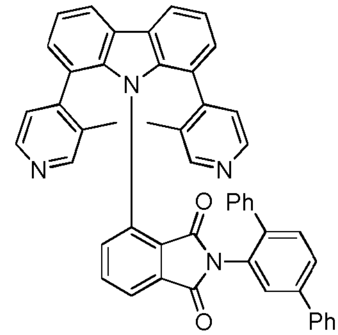
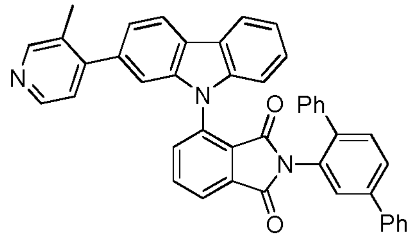

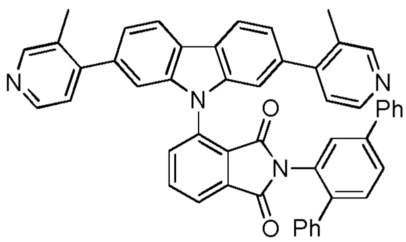
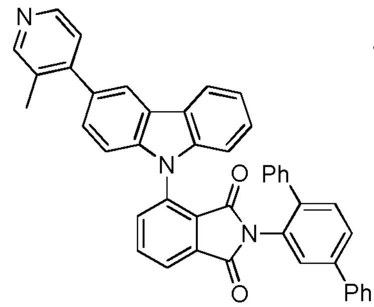
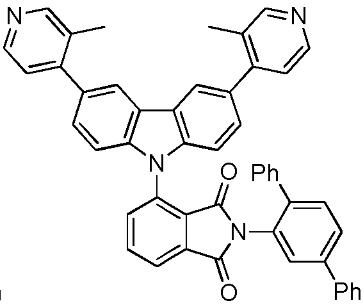

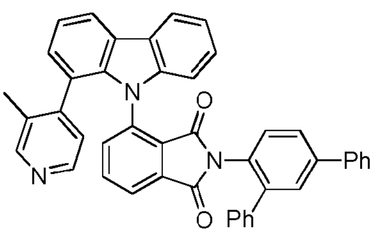
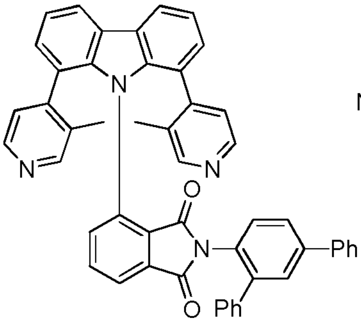
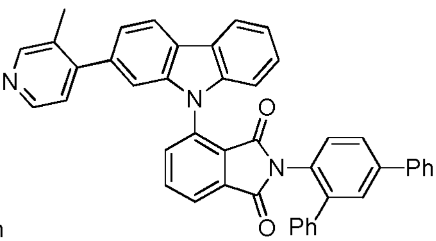

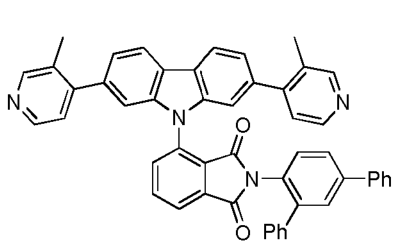

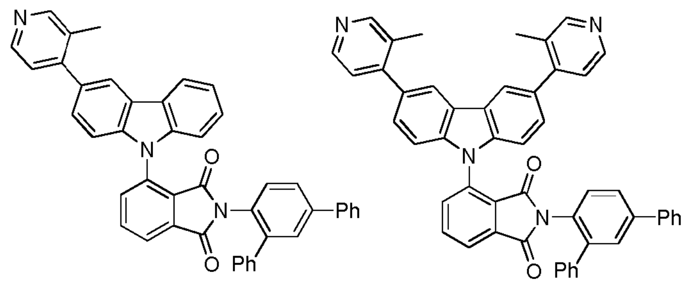

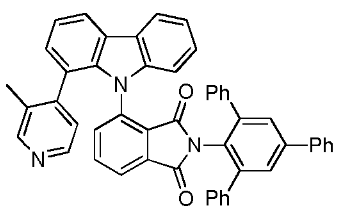

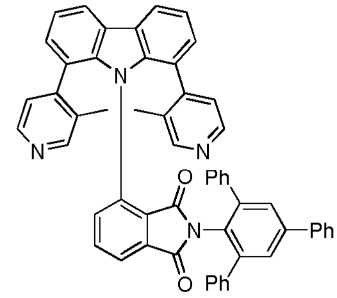

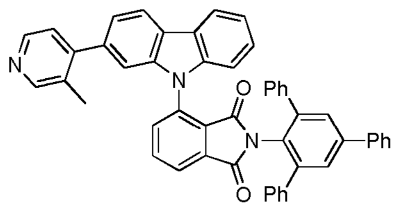

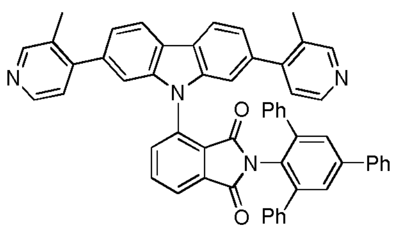

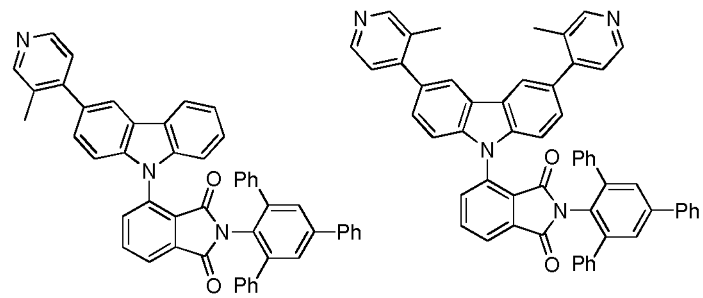

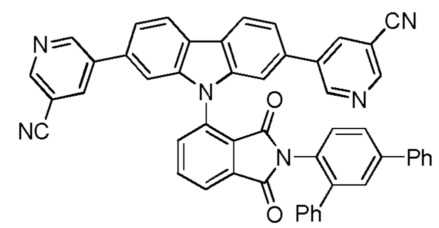

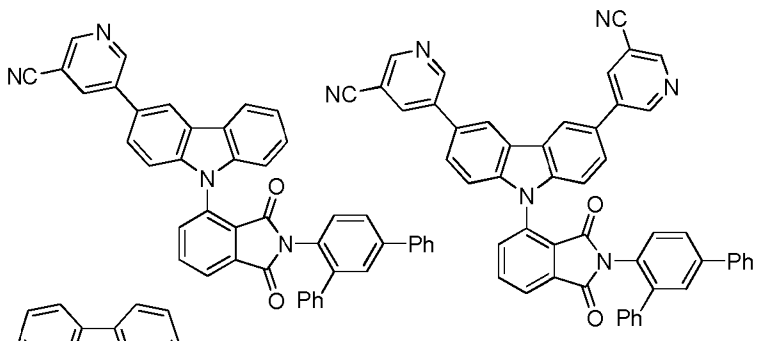

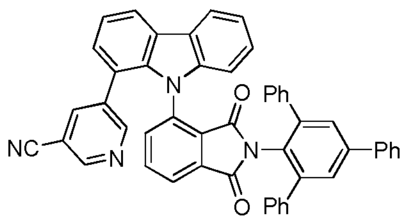

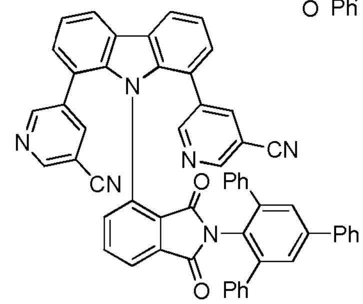

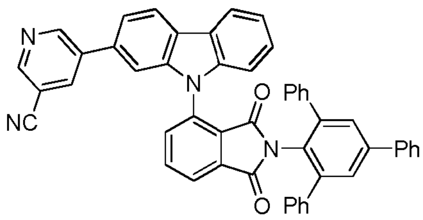

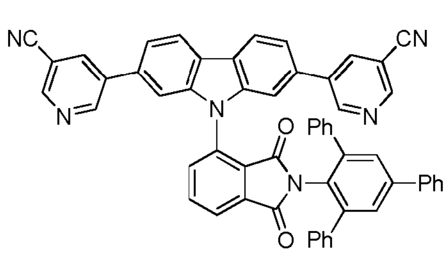

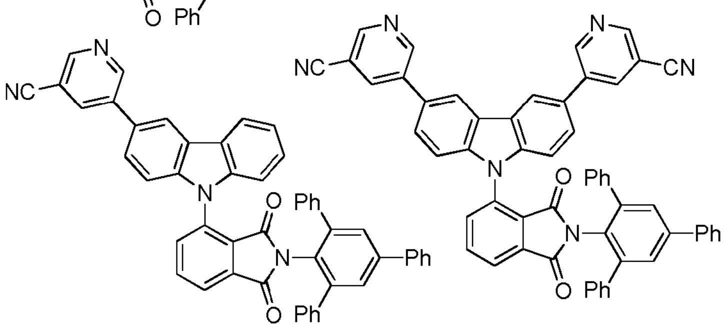

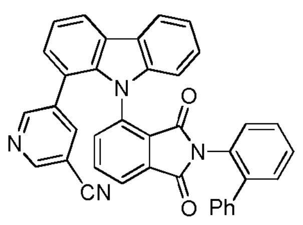

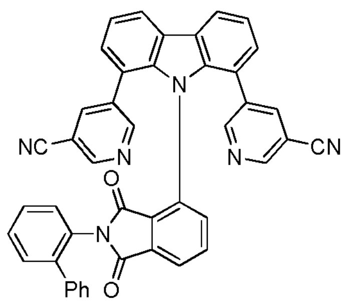

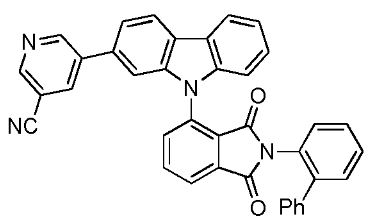

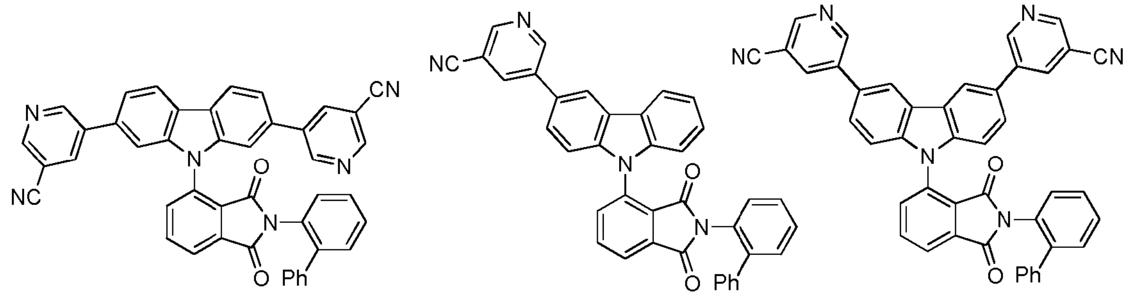

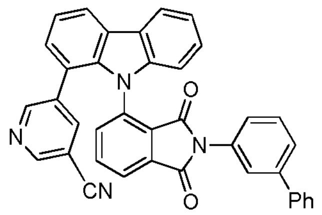
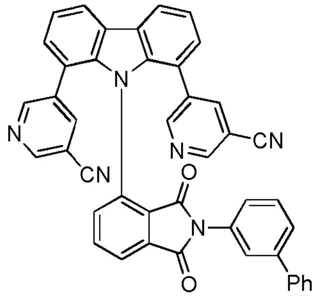
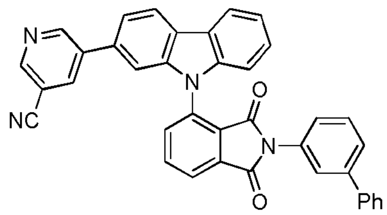

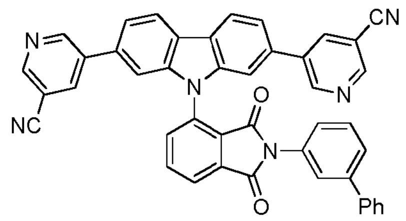
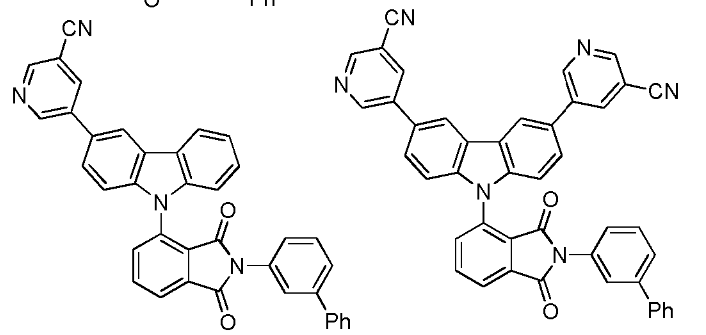

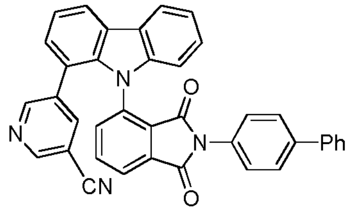
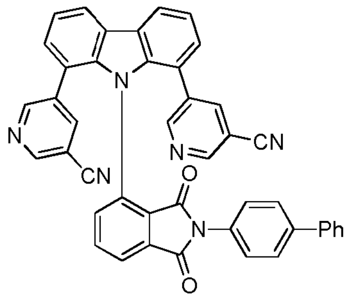
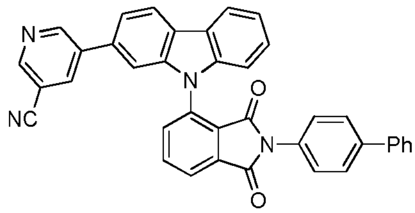

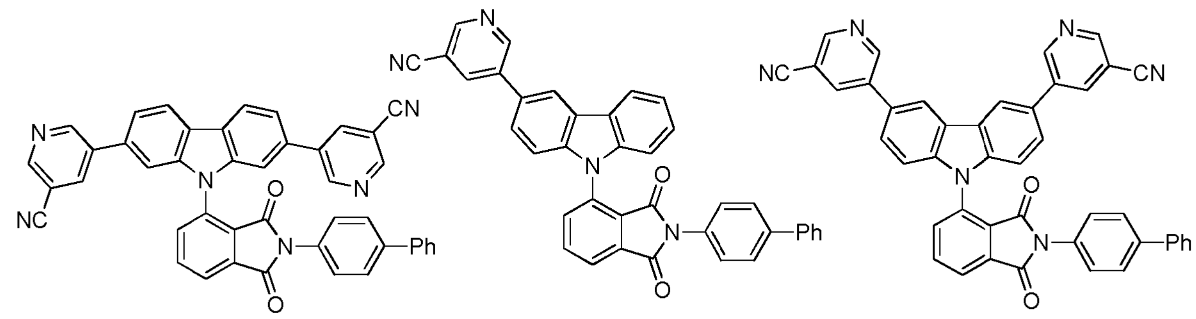

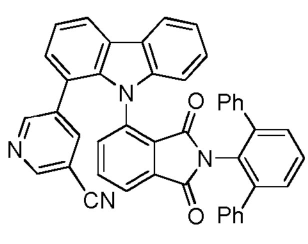
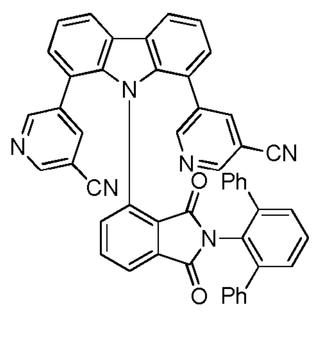
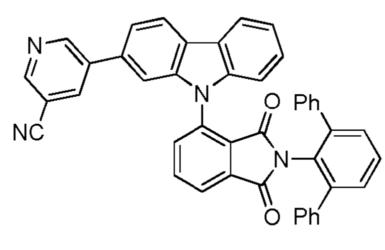

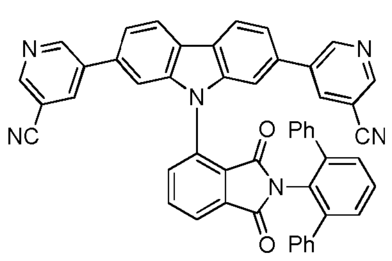
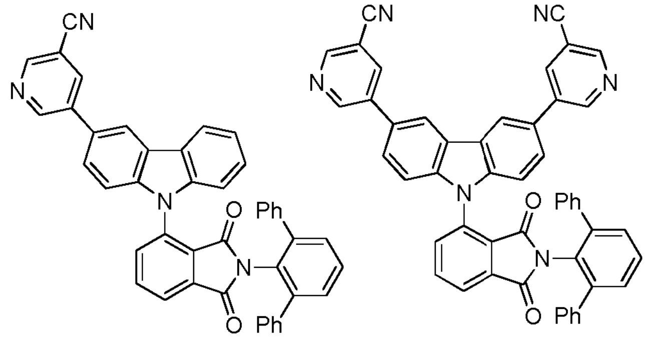
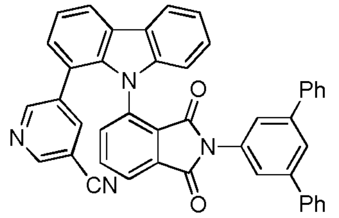
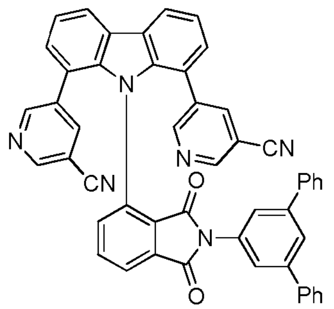
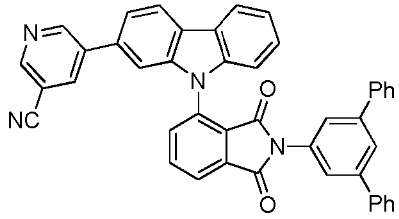
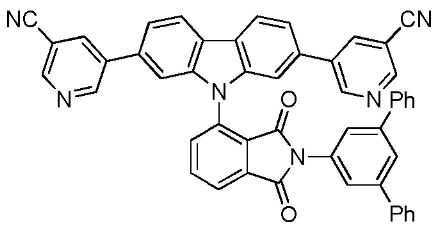
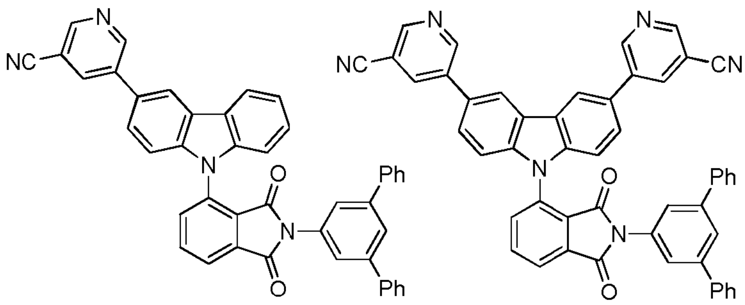
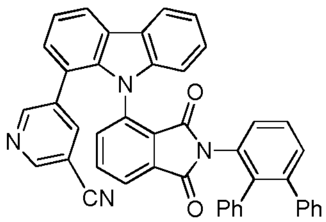
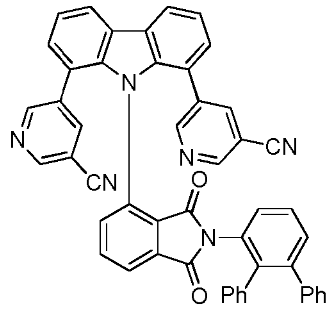
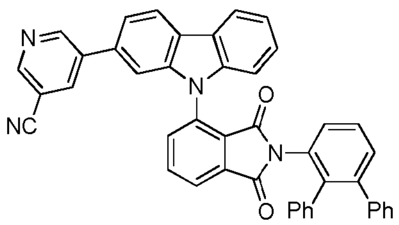
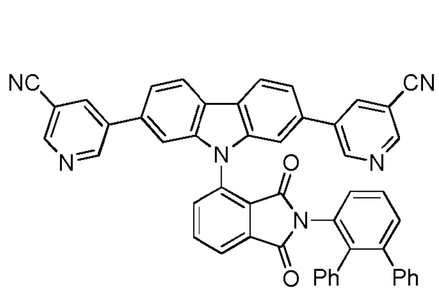
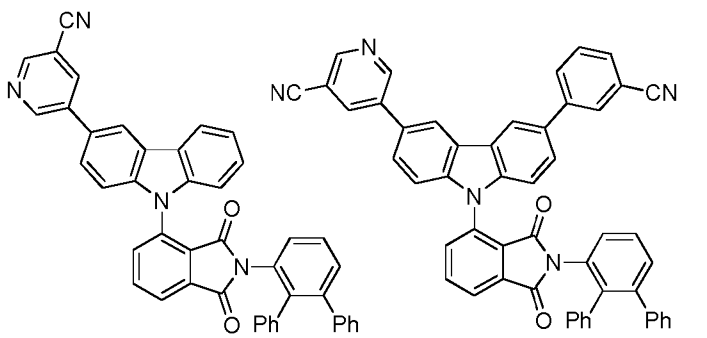

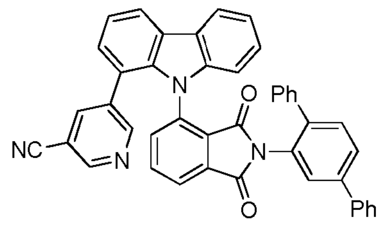

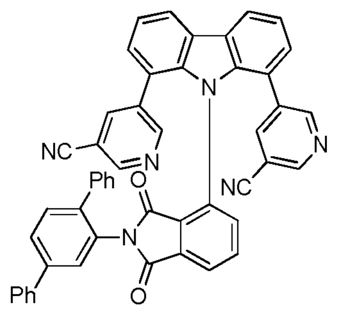

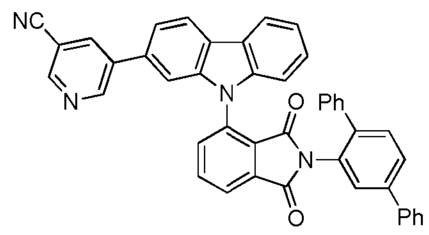

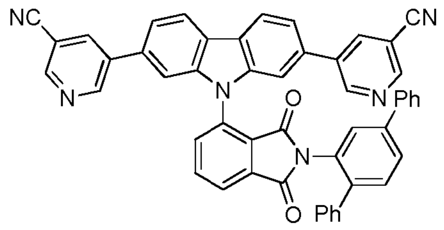

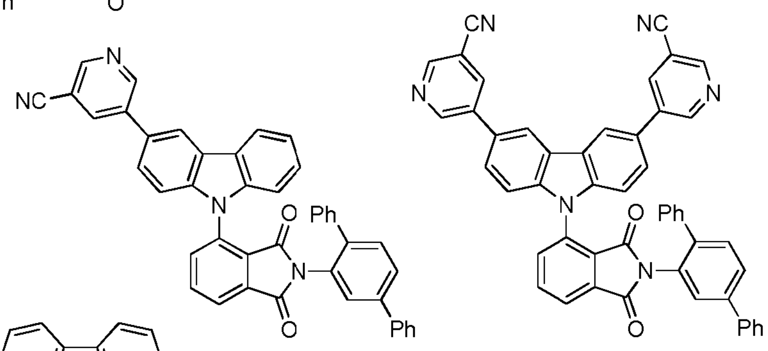

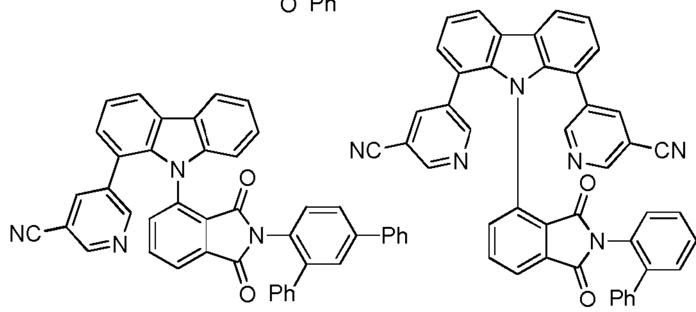

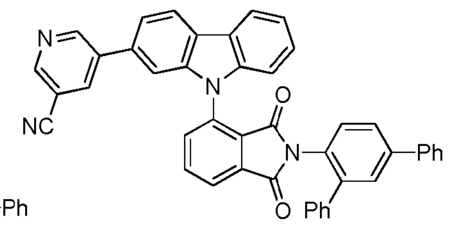

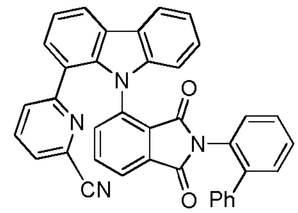

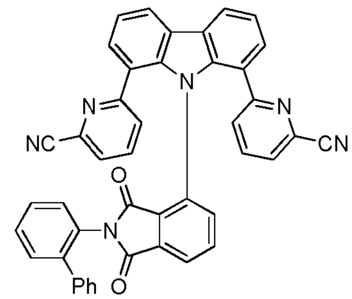

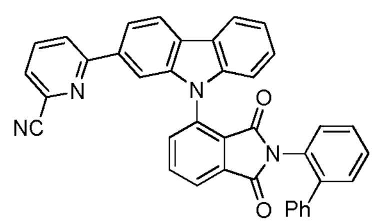

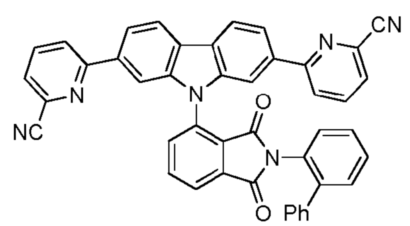

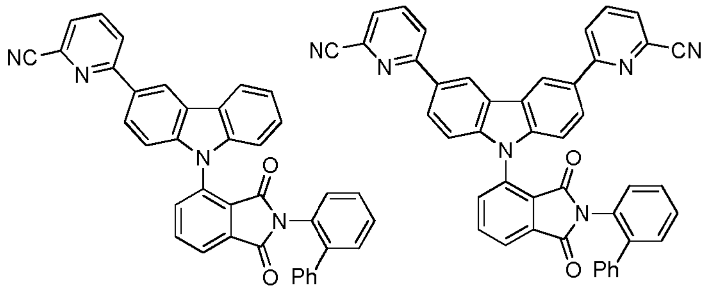

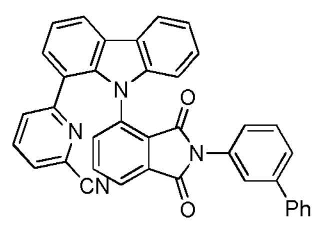
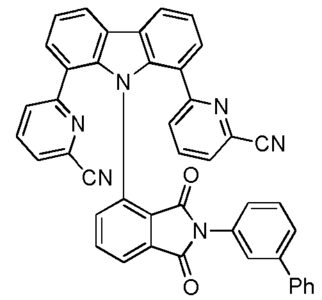
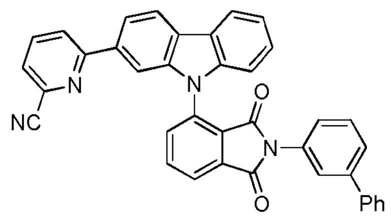
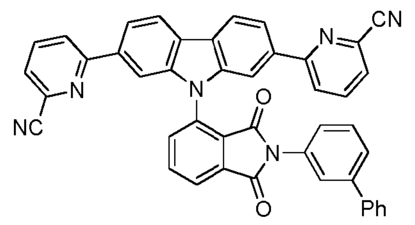
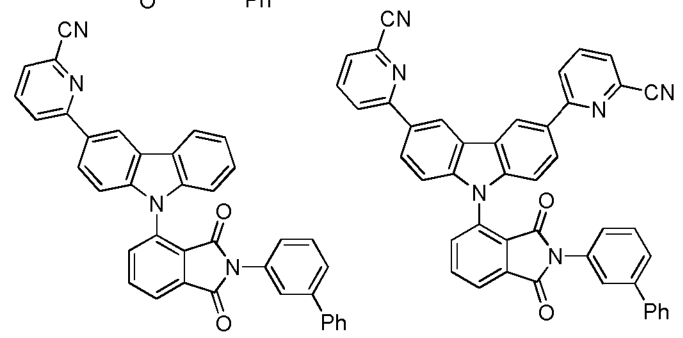
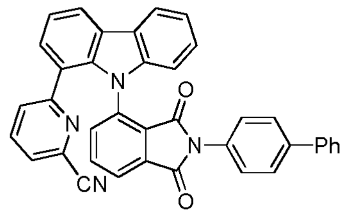
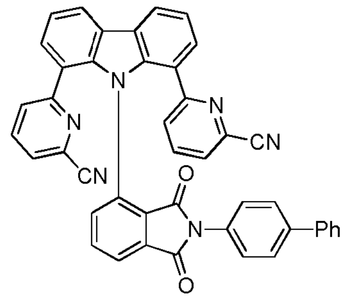
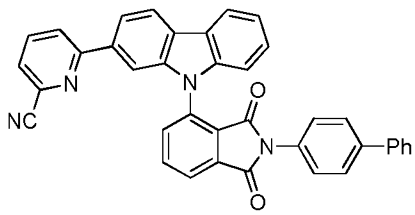
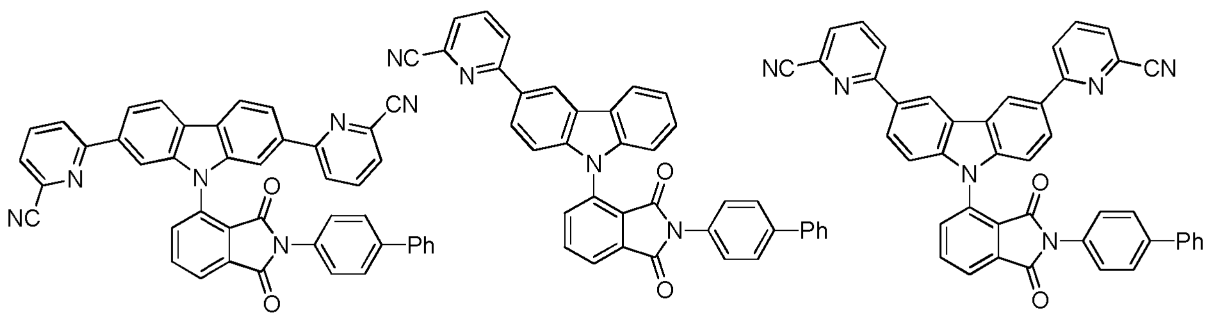
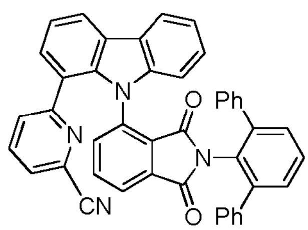
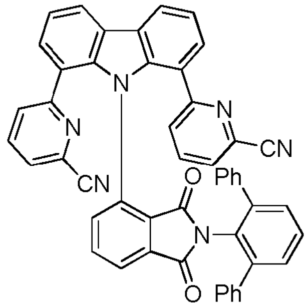
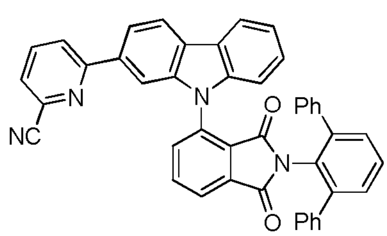

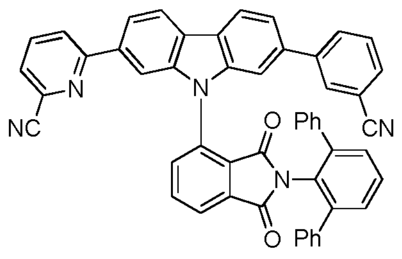

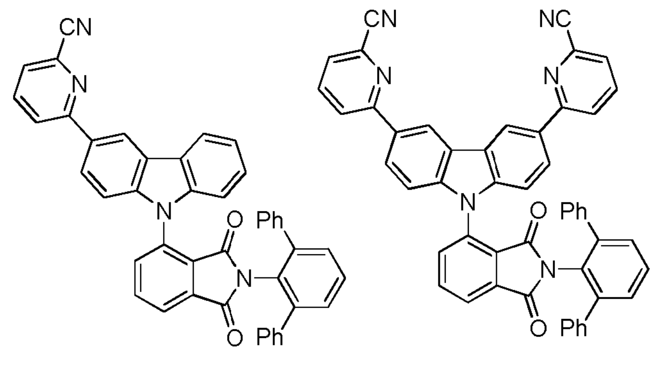

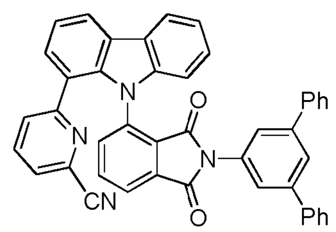

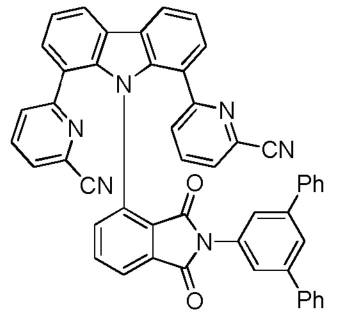

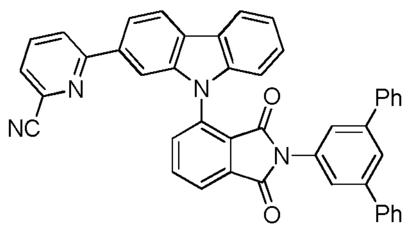

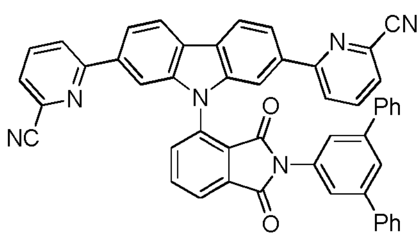

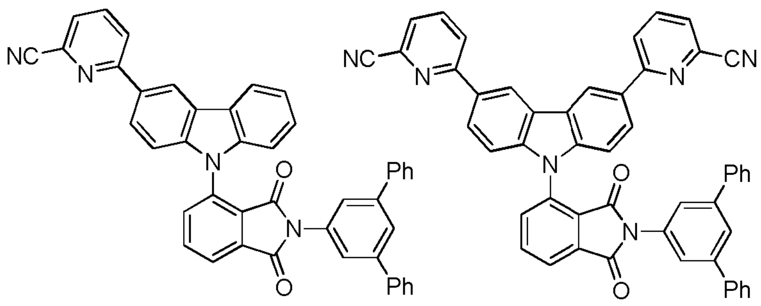

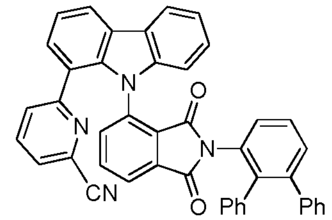

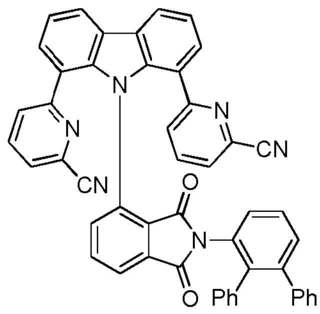

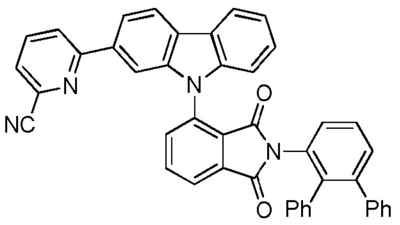

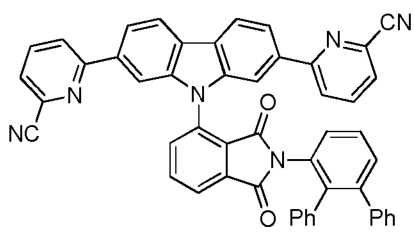

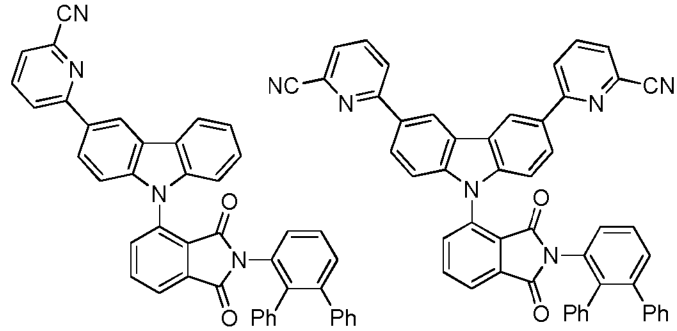

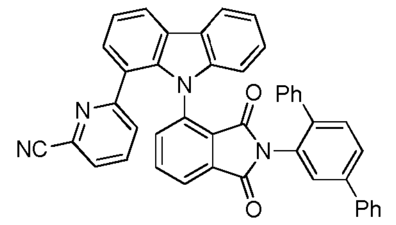
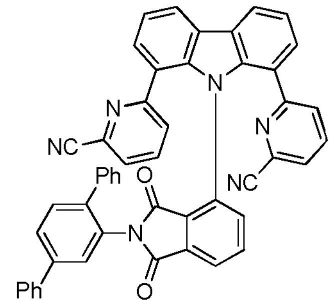
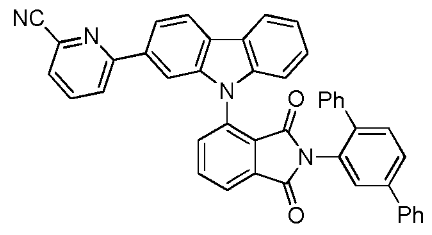
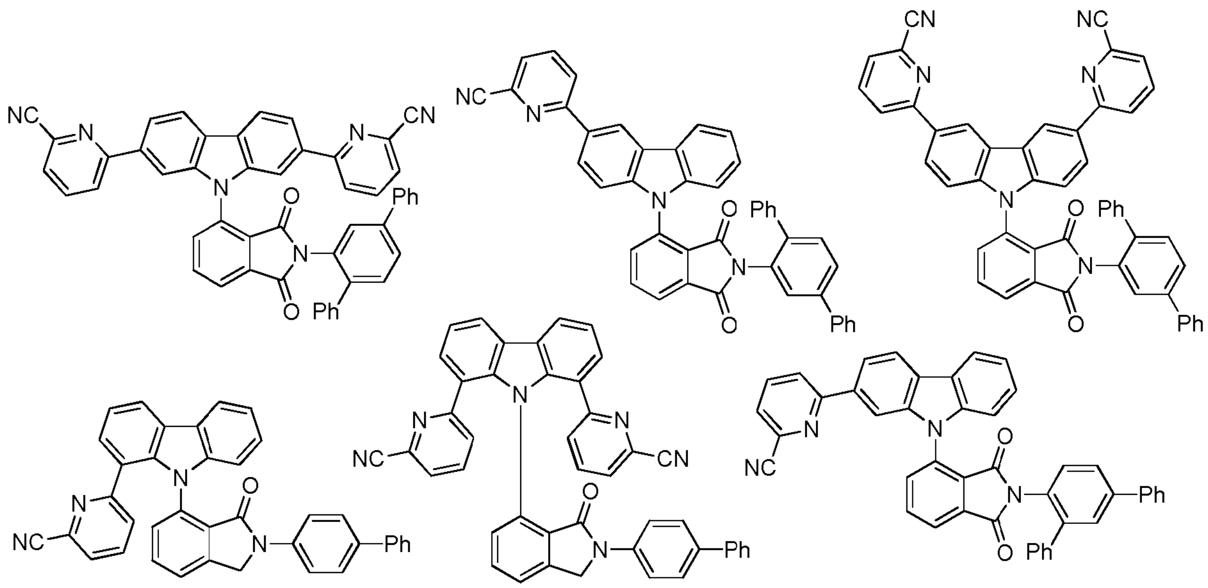
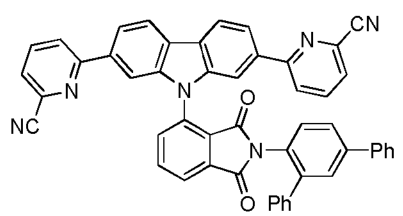
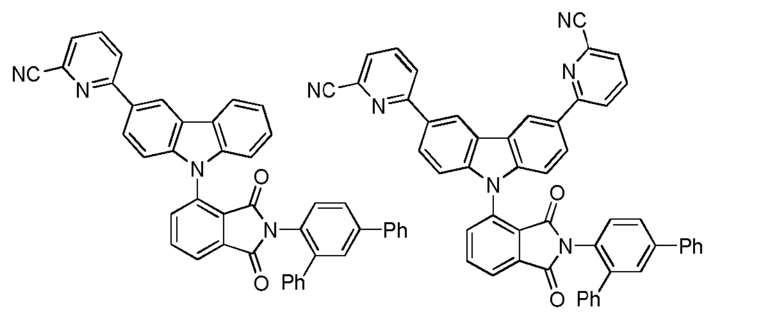
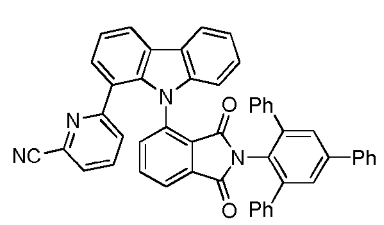
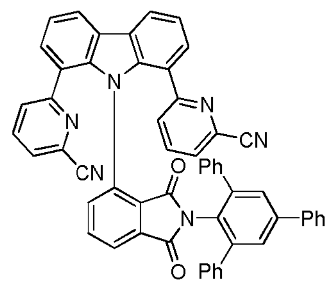
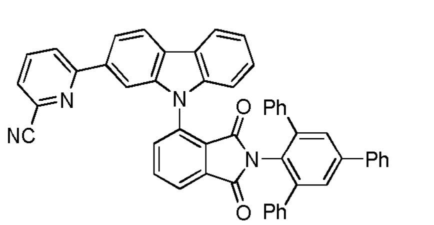

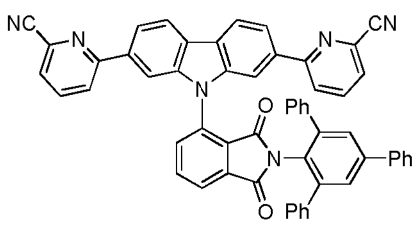

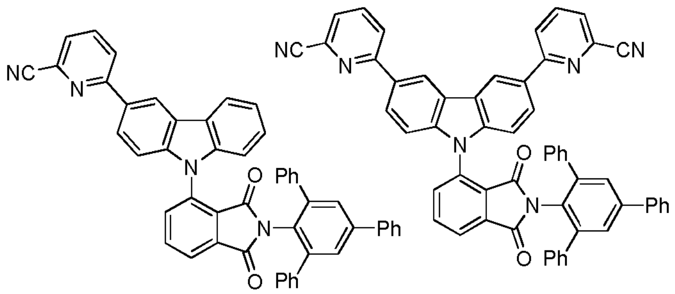

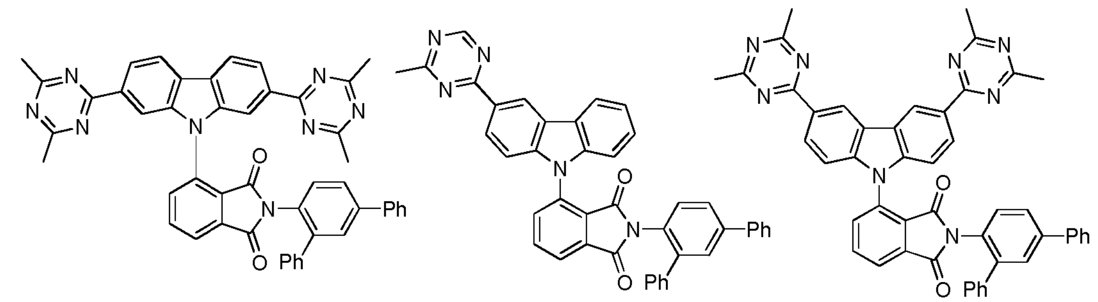

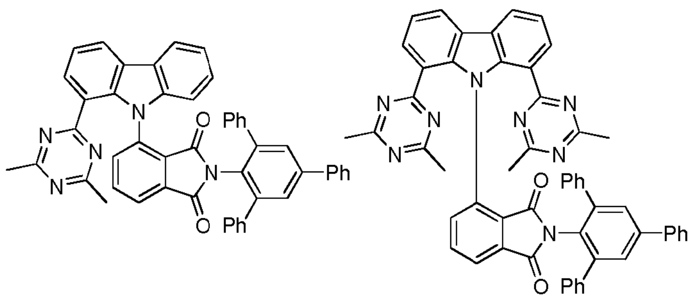

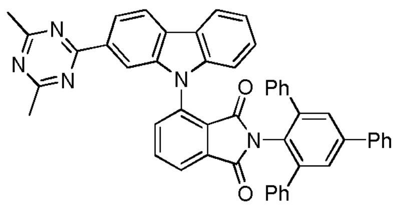

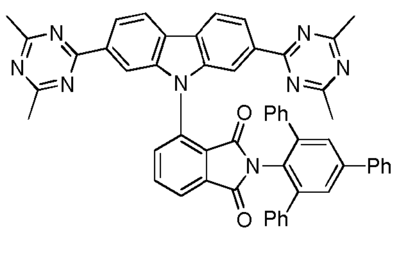

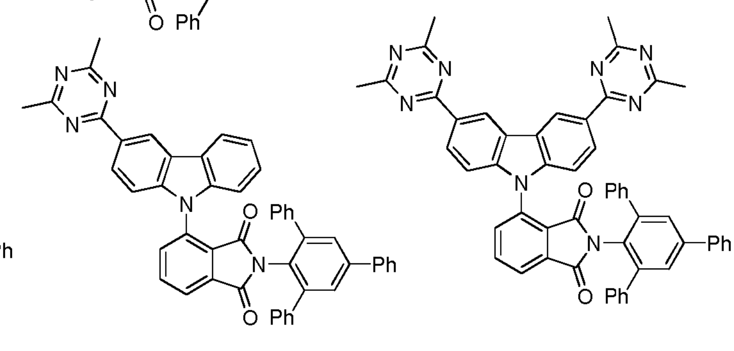

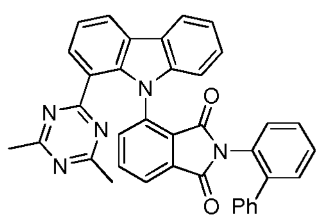

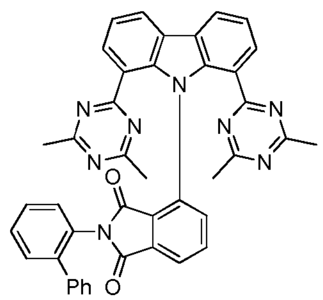

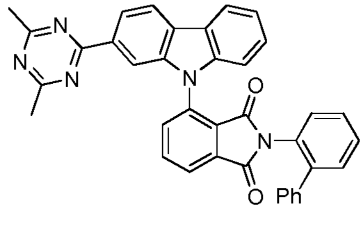

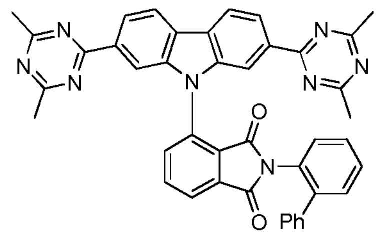

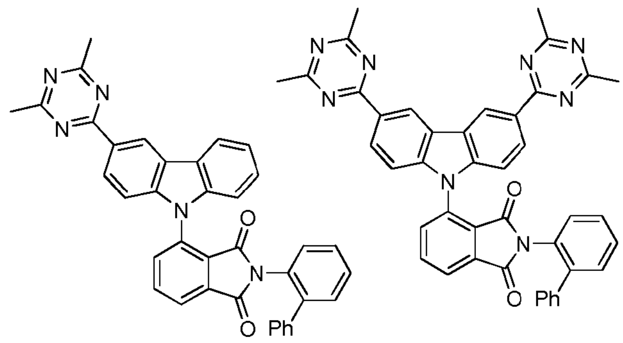

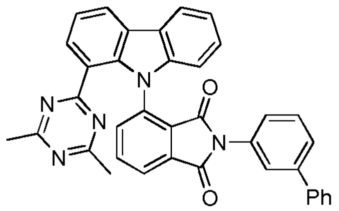

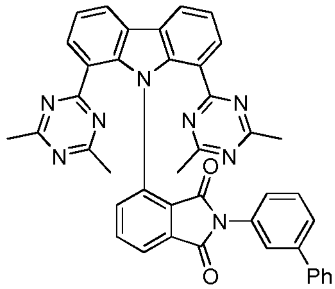

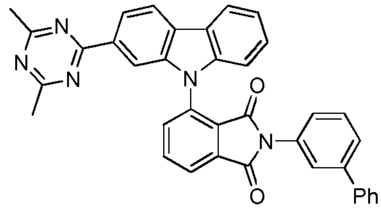

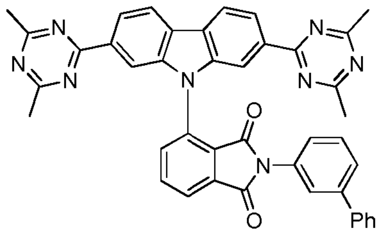

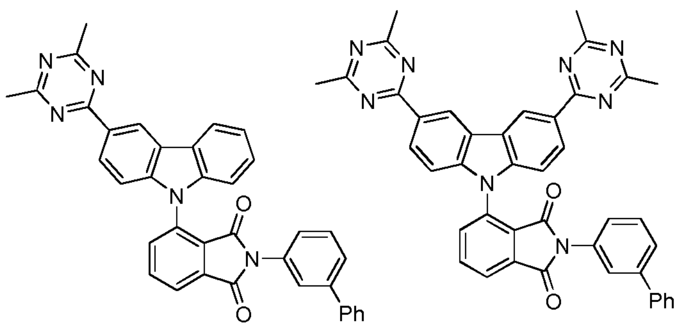

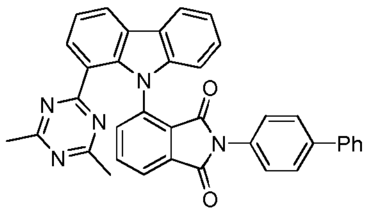

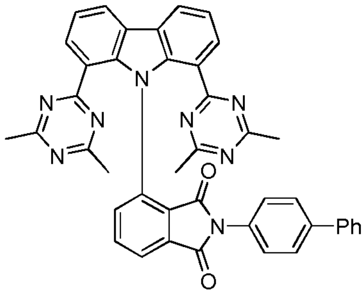

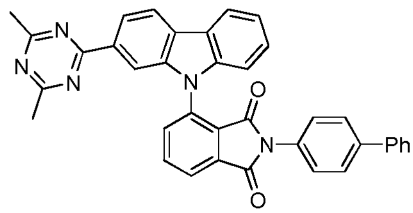

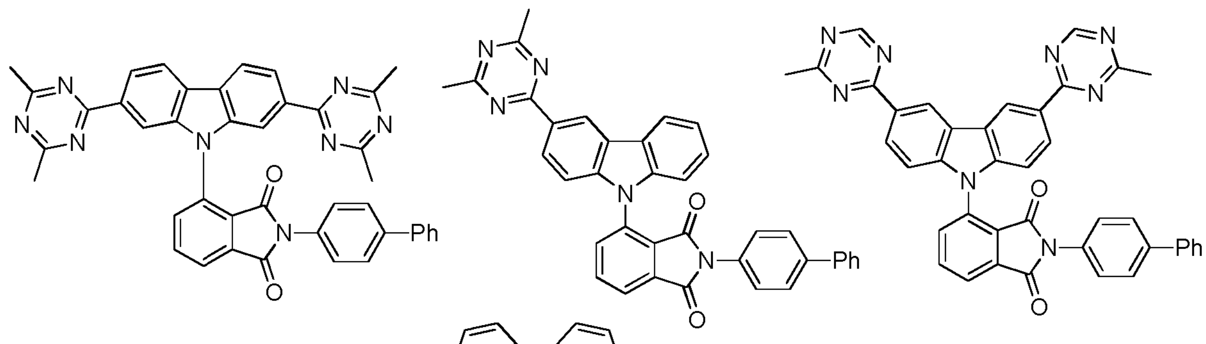

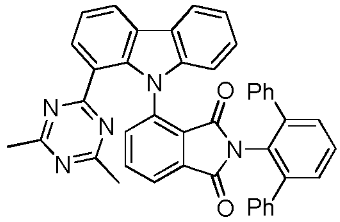

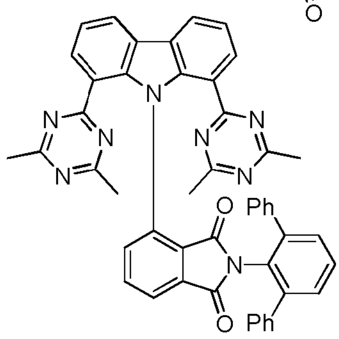

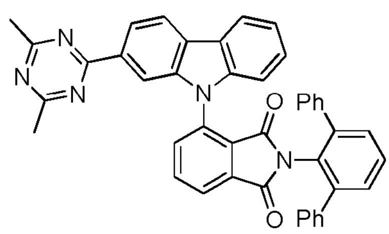

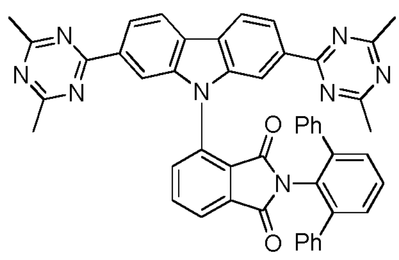

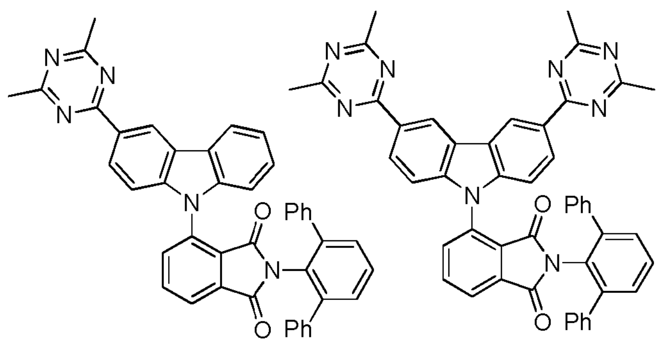

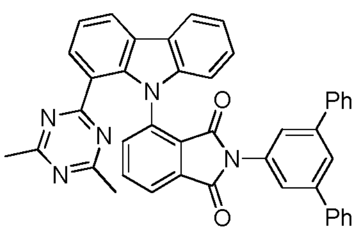

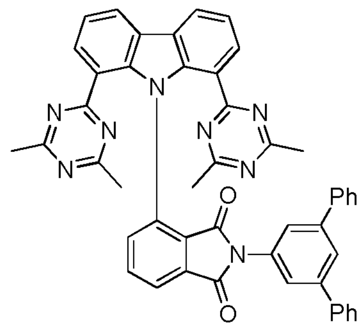

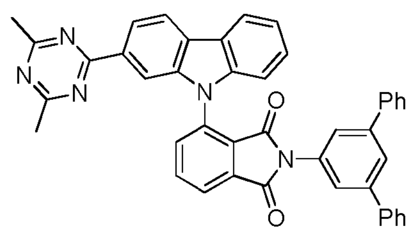

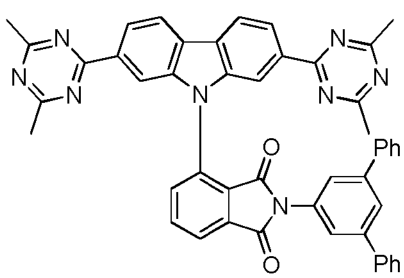

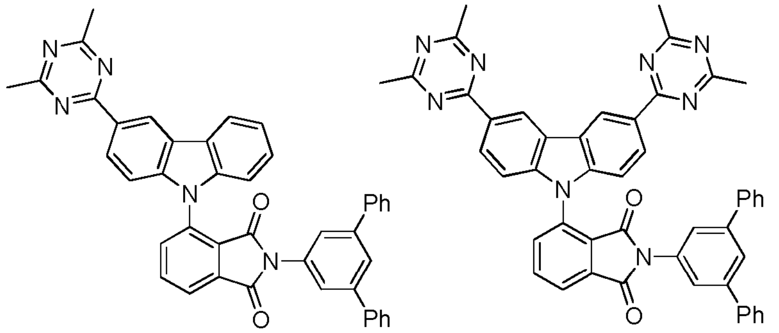

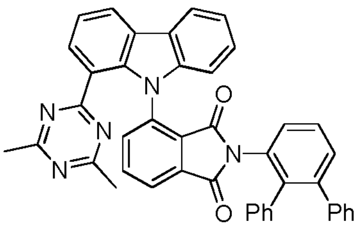

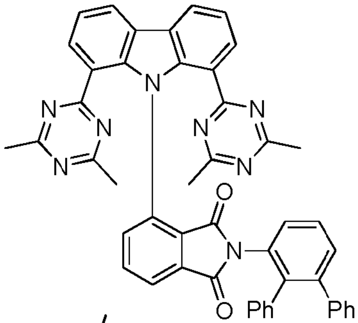

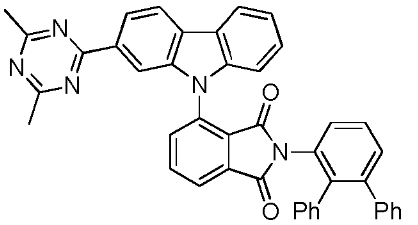

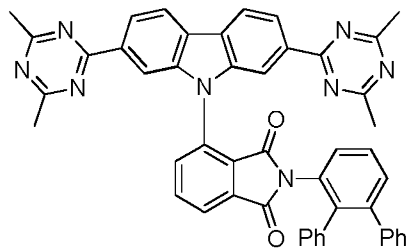

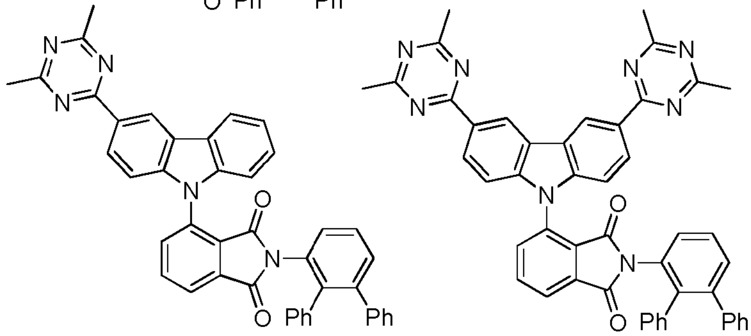

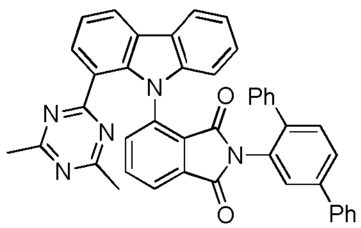

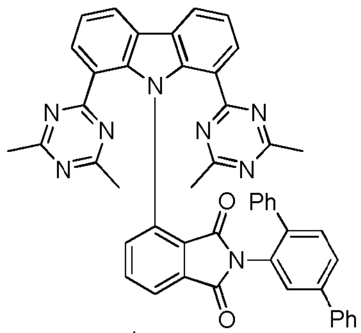

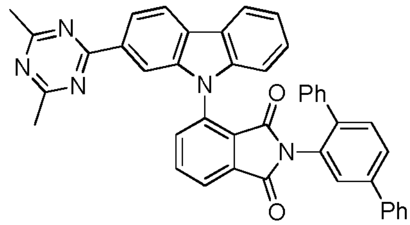

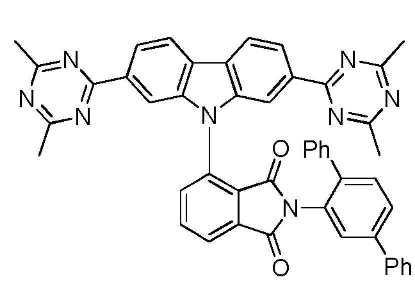

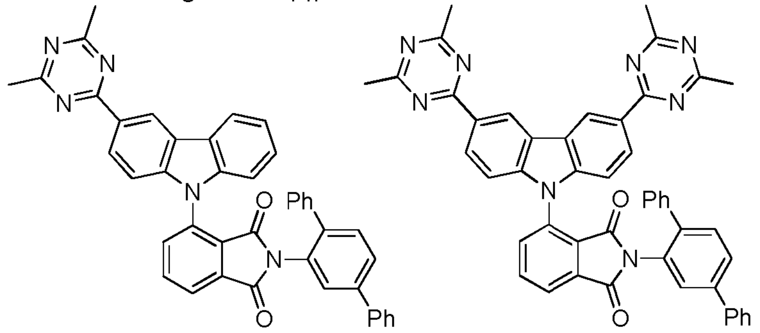

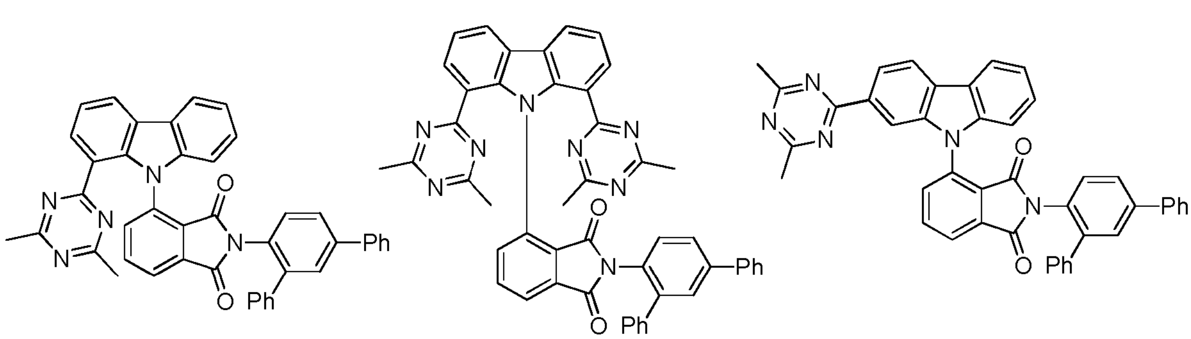

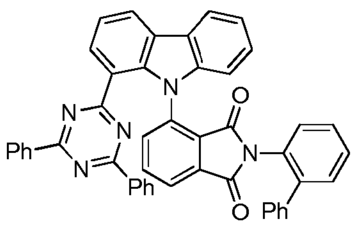

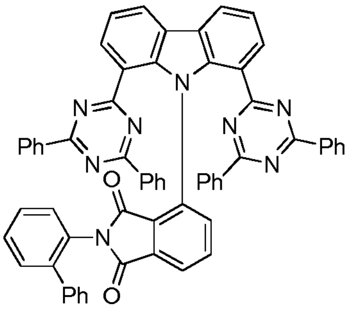

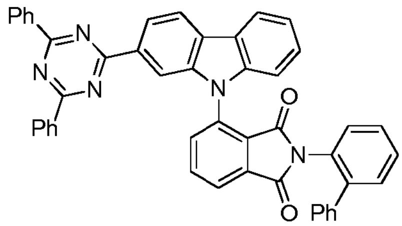

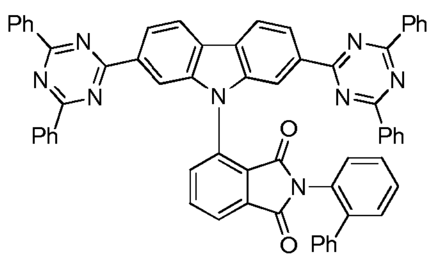

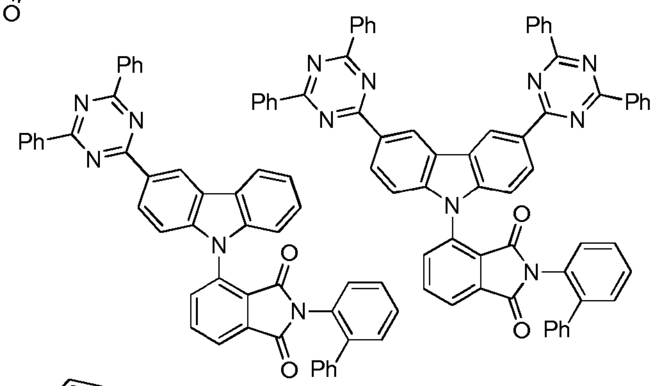

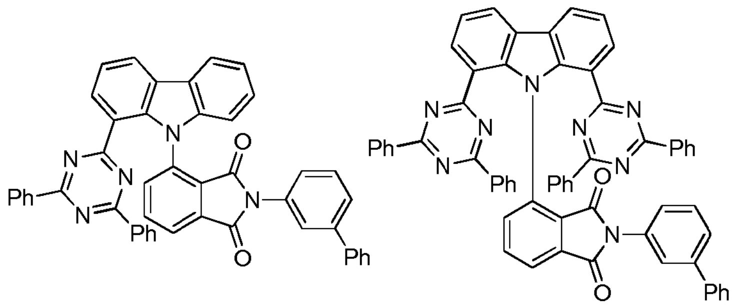

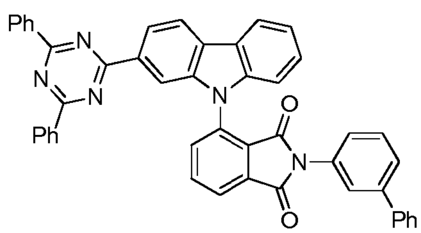

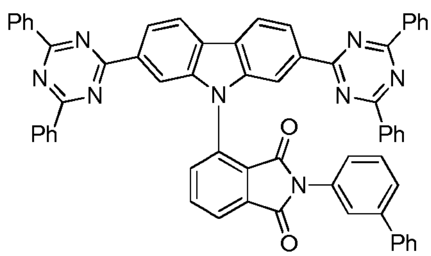

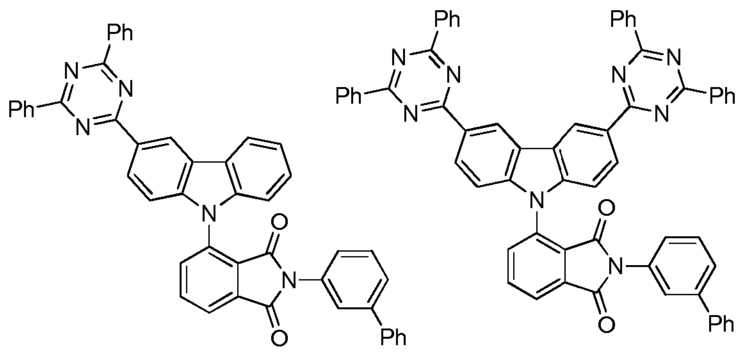

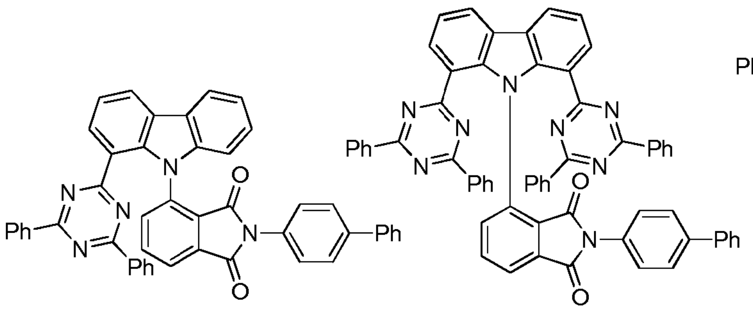

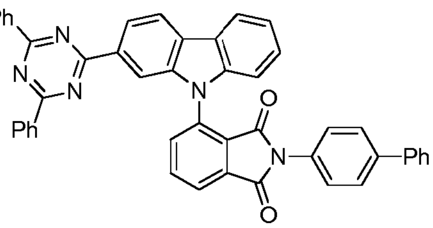

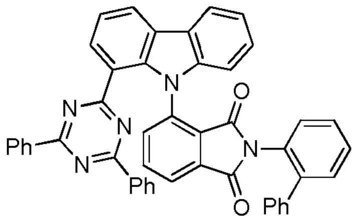

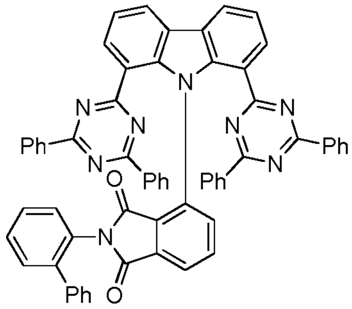

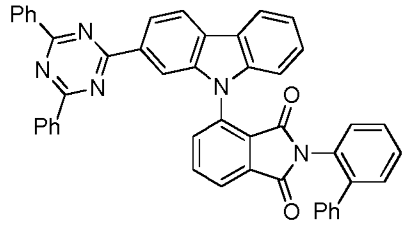

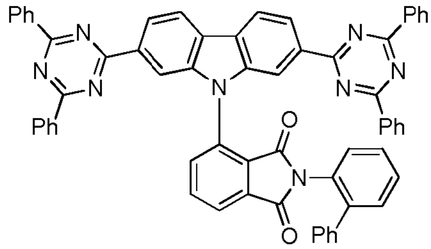

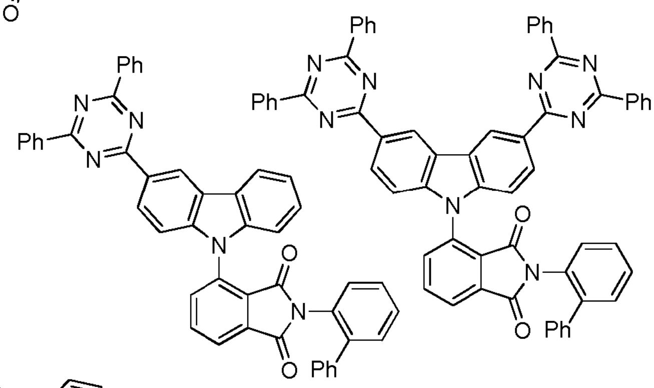

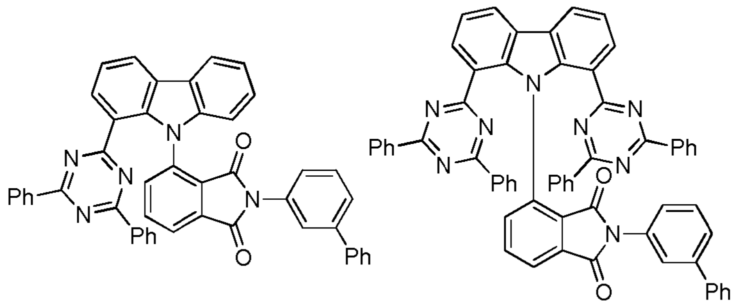

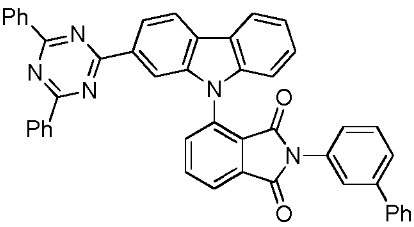

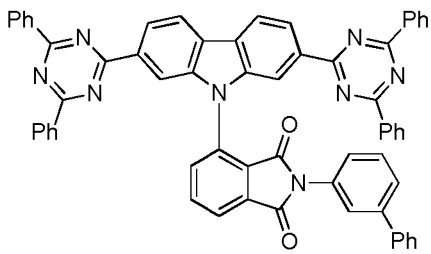

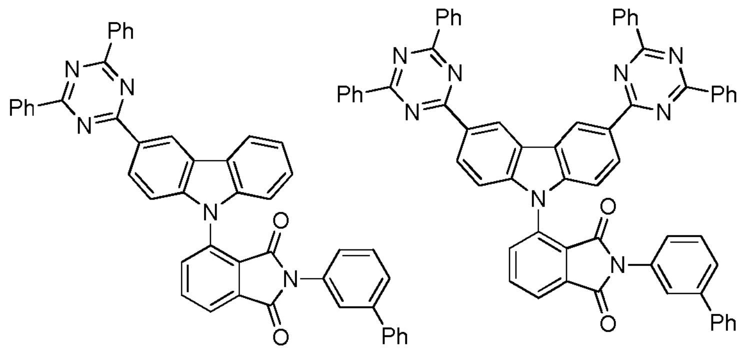

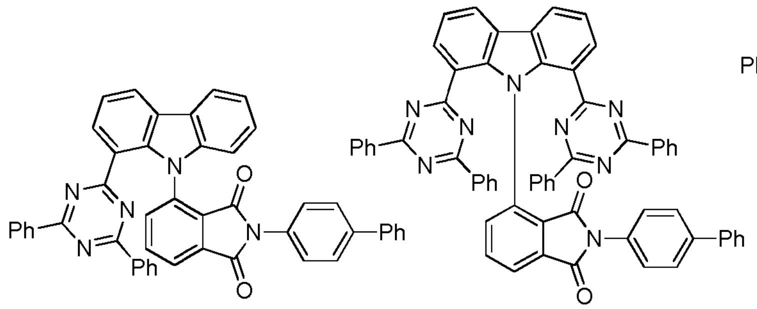

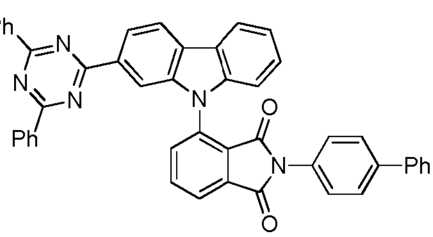

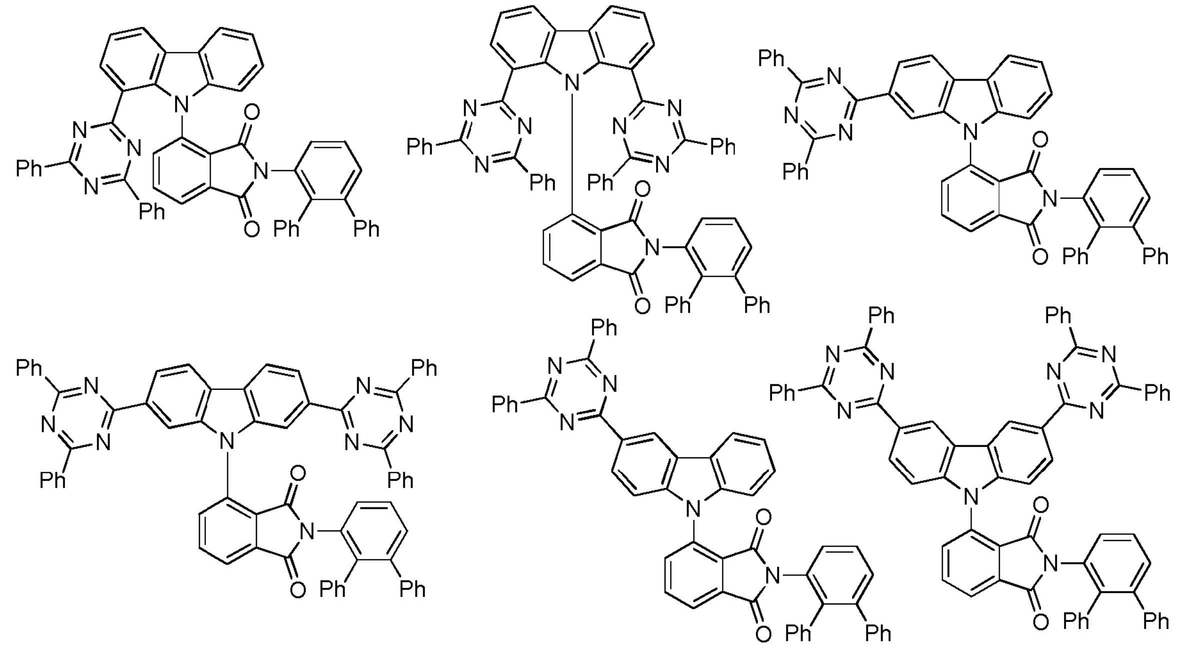

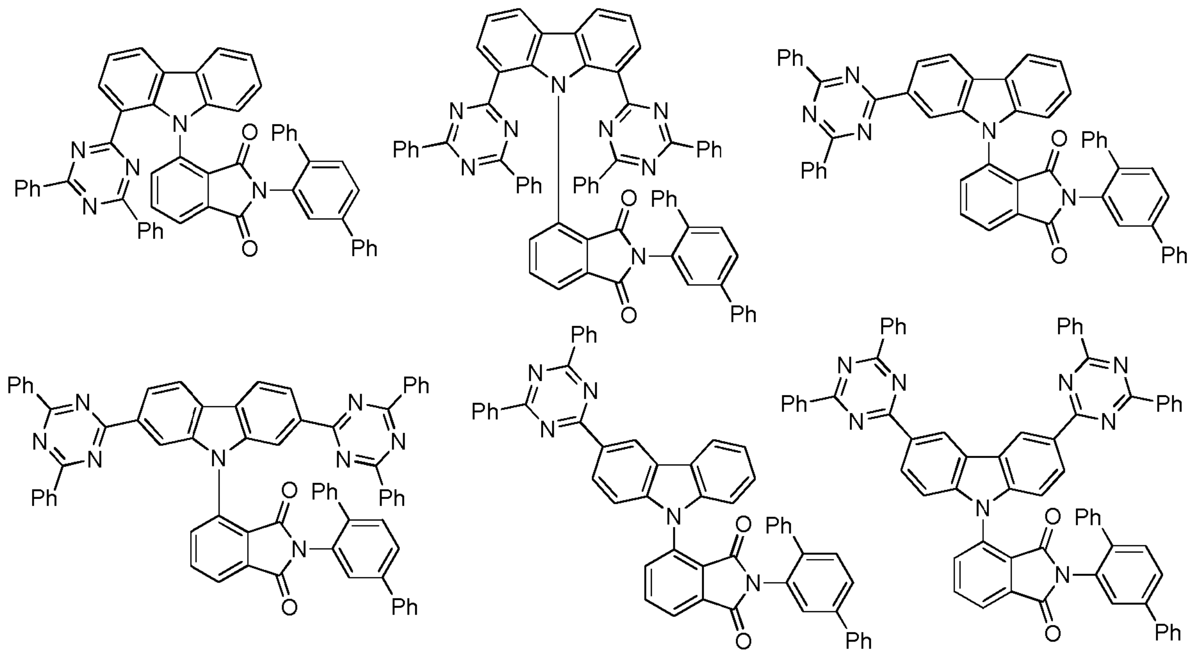

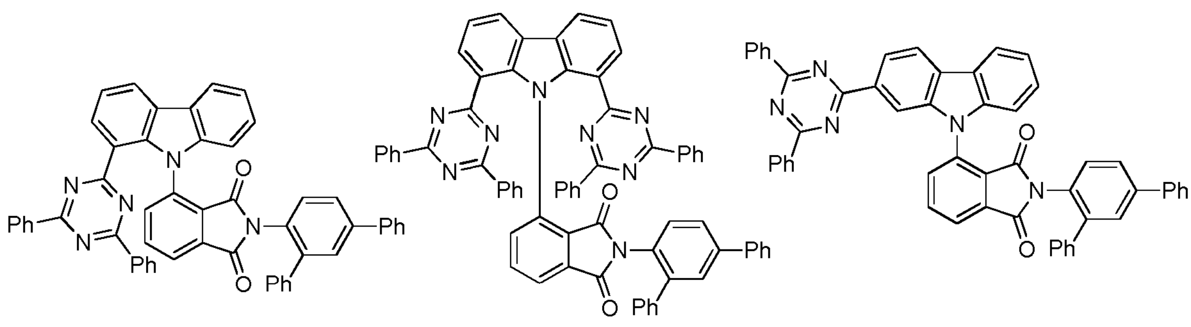

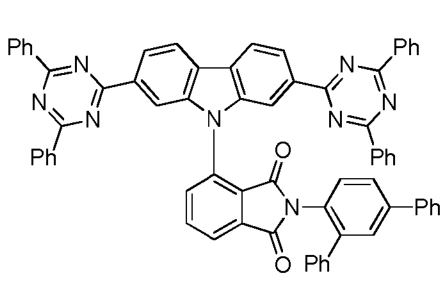

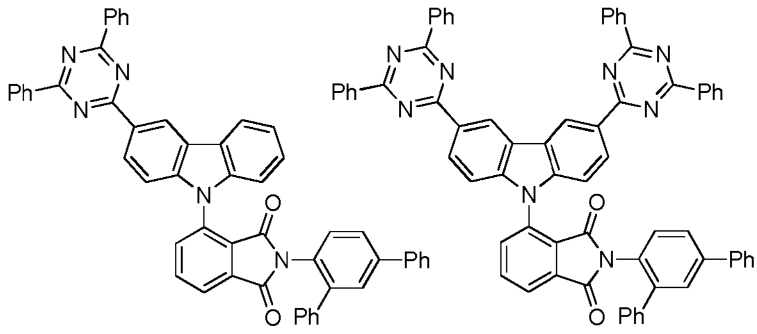

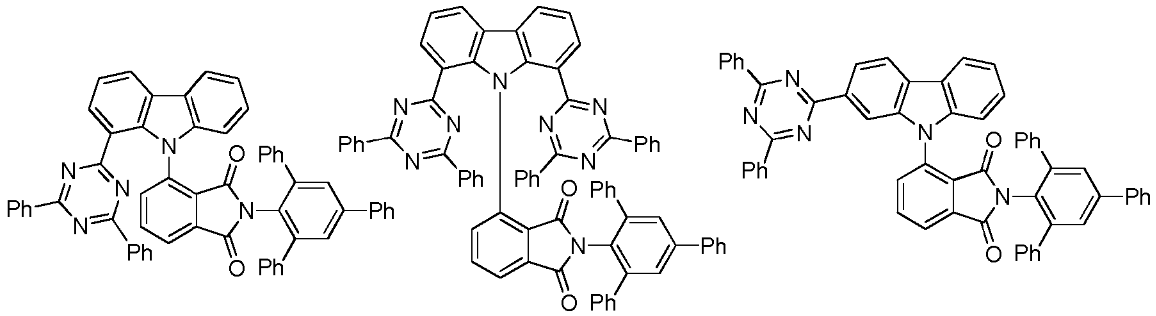

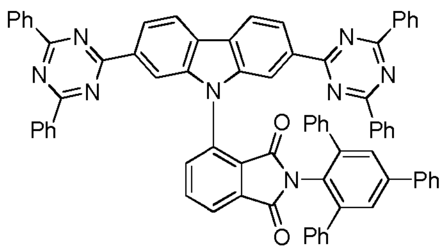

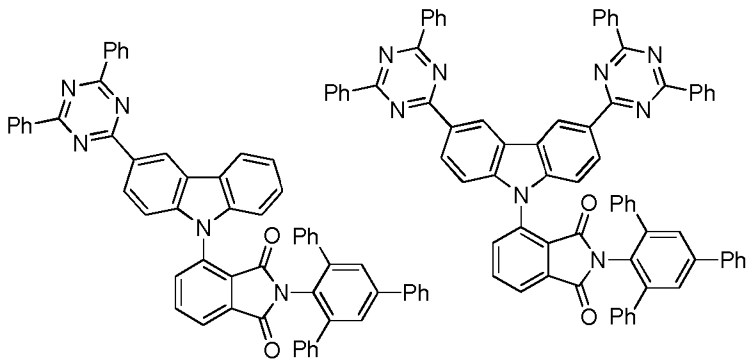

## Figuren

Es zeigen:

Figur 1 Emissionsspektrum von **1** (10 % in PMMA).
Figur 2 Emissionsspektrum von **2** (10 % in PMMA).
Figur 3 Emissionsspektrum von **3** (10 % in PMMA).
Figur 4 Emissionsspektrum von **4** (10 % in PMMA).
Figur 5 Emissionsspektrum von **5** (10 % in PMMA).

## Patentansprüche

**1.** Organisches Molekül, aufweisend

- eine erste chemische Einheit aufweisend eine oder bestehend aus einer Struktur der Formel A1

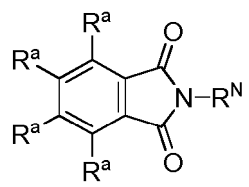

**Formel A1**

- mindestens eine zweite chemische Einheit aufweisend eine oder bestehend aus einer Struktur der Formel D1

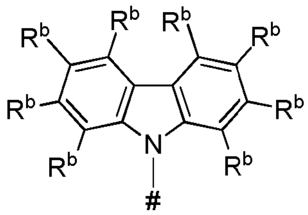

**Formel D1**

mit

# = Anknüpfungspunkt der zweiten chemischen Einheit an die erste chemische Einheit;
$R^N$ weist eine Struktur der Formel N1 auf oder besteht daraus

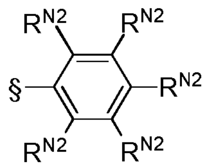

**Formel N1**

mit

§ = Anknüpfungspunkt der chemischen Einheit gemäß Formel N1 an die erste chemische Einheit;
$R^{N2}$ ist bei jedem Auftreten gleich oder verschieden H, Phenyl oder Naphtyl;
$R^a$ ist bei jedem Auftreten gleich oder verschieden ausgewählt aus der Gruppe bestehend aus einem Anknüpfungspunkt der ersten chemischen Einheit an die zweite chemische Einheit und H;
$R^b$ ist bei jedem Auftreten unabhängig voneinander ausgewählt aus der Gruppe bestehend aus H, Deuterium, $CF_3$, $C(=O)R^1$, CN, unsubstituiertes oder mit einem oder mehreren $R^2$ substituiertes Carbazolyl, $Si(R^4)_3$, einer unsubstituierten oder mit einem oder mehreren $R^2$ substituierten Alkylgruppe, einer unsubstituierten oder mit einem oder mehreren $R^2$ substituierten Arylgruppe und einer unsubstituierten oder mit einem oder mehreren $R^2$ substituierten Heteroarylgruppe;
$R^1$ ist bei jedem Auftreten unabhängig voneinander eine unsubstituierte oder mit einem oder mehreren $R^3$ substituierte Arylgruppe;
$R^2$ ist bei jedem Auftreten unabhängig voneinander ausgewählt aus der Gruppe bestehend aus $CF_3$, $C(=O)R^1$, CN, einer unsubstituierten oder mit einem oder mehreren $R^3$ substituierten Alkylgruppe, einer unsubstituierten oder mit einem oder mehreren $R^3$ substituierten Arylgruppe und einer unsubstituierten oder mit einem oder mehreren $R^3$ substituierten Heteroarylgruppe;
$R^3$ ist bei jedem Auftreten unabhängig voneinander ausgewählt aus der Gruppe bestehend aus einer unsubstituierten Alkylgruppe, einer unsubstituierten Arylgruppe und einer unsubstituierten Heteroarylgruppe;
$R^4$ ist bei jedem Auftreten unabhängig voneinander ausgewählt aus der Gruppe bestehend aus, einer unsubstituierten oder mit einem oder mehreren $R^3$ substituierten Arylgruppe und einer unsubstituierten oder mit einem oder mehreren $R^3$ substituierten Heteroarylgruppe;

dabei bilden optional zwei oder mehrere der Substituenten $R^1$, $R^2$ und $R^3$ miteinander ein monocyclisches, polycyclisches, aliphatisches, aromatisches und/oder benzoanelliertes Ringsystem;
wobei mindestens ein und maximal drei $R^{N2}$ Phenyl oder Naphtyl ist.

**2.** Organisches Molekül nach Anspruch 1, aufweisend eine Struktur der Formel A2

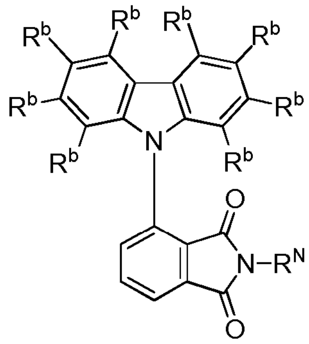

**Formel A2**

wobei die in Anspruch 1 angegebenen Definitionen gelten.

**3.** Organisches Molekül nach Anspruch 1 bis 2, aufweisend eine Struktur der Formel A3

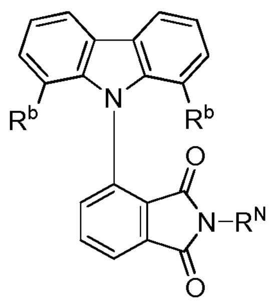

**Formel A3**

wobei die in Anspruch 1 angegebenen Definitionen gelten.

**4.** Organisches Molekül nach Anspruch 1 bis 2, aufweisend eine Struktur der Formel A4

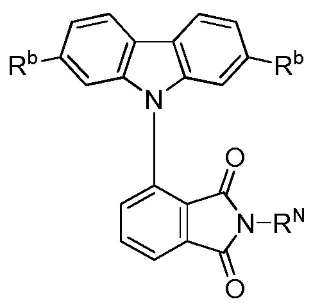

**Formel A4**

wobei die in Anspruch 1 angegebenen Definitionen gelten.

**5.** Organisches Molekül nach Anspruch 1 bis 2, aufweisend eine Struktur der Formel A5

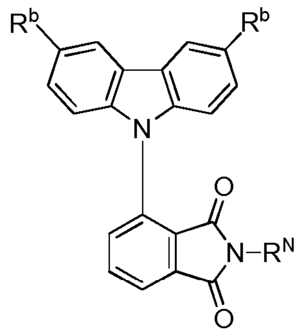

**Formel A5**

wobei die in Anspruch 1 angegebenen Definitionen gelten.

6. Organisches Molekül nach Anspruch 1, wobei $R^N$ bei jedem Auftreten gleich oder verschieden ausgewählt ist aus einer der Strukturen O1 bis O10:

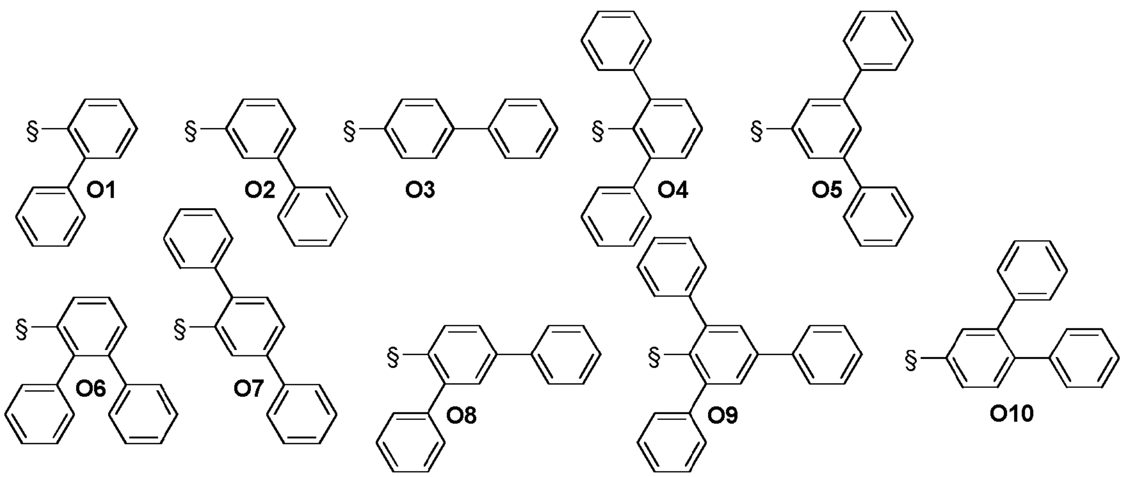

O1 O2 O3 O4 O5 O6 O7 O8 O9 O10

7. Organisches Molekül nach Anspruch 1, wobei $R^N$ bei jedem Auftreten gleich oder verschieden ausgewählt ist aus einer der Strukturen P1 bis P3:

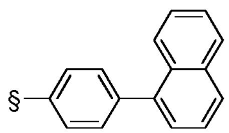

P1

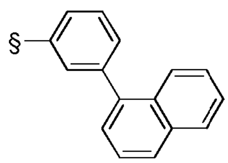

P2

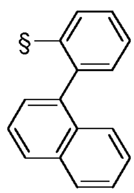

P3

8. Organisches Molekül nach Anspruch 1 bis 7, wobei $R^b$ bei jedem Auftreten gleich oder verschieden ausgewählt ist aus der Gruppe bestehend aus H, Deuterium, Methyl, i-Propyl, t-Butyl, $CF_3$, CN, einer unsubstituierten oder mit einem oder mehreren $R^6$ substituierten Phenylgruppe, einer unsubstituierten oder mit einem oder mehreren $R^6$ substituierten Pyridingruppe, einer unsubstituierten oder mit einem oder mehreren $R^6$ substituierten Pyrimidingruppe, einer unsubstituierten oder mit einem oder mehreren $R^6$ substituierten Pyrazingruppe und einer unsubstituierten oder mit einem oder mehreren $R^6$ substituierten Triazingruppe,
wobei $R^6$ bei jedem Auftreten gleich oder verschieden ausgewählt ist aus der Gruppe bestehend aus Deuterium, Methyl, i-Propyl, t-Butyl, $CF_3$, CN und Phenyl.

9. Verwendung eines organischen Moleküls nach Anspruch 1 bis 8 als Emitter und/oder Host.

**10.** Zusammensetzung aufweisend oder bestehend aus:

(a) mindestens einem organischen Molekül nach einem der Ansprüche 1 bis 8, insbesondere als Emitter und/oder Host, und
(b) einer oder mehrerer von dem Molekül nach einem der Ansprüche 1 bis 8 verschiedenen Emitter- und/oder Hostmaterialien und
(c) optional einem oder mehreren Farbstoffen und/ oder einem oder mehreren Lösungsmitteln.

**11.** Optoelektronisches Bauelement, aufweisend ein organisches Molekül nach Anspruch 1 bis 8 oder eine Zusammensetzung nach Anspruch 10.

**12.** Optoelektronisches Bauelement nach Anspruch 11, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die das organisches Molekül nach Anspruch 1 bis 8 oder eine Zusammensetzung nach Anspruch 10 aufweist.

**13.** Verfahren zur Herstellung eines optoelektronischen Bauelements, wobei ein organisches Molekül nach Anspruch 1 bis 8 verwendet wird.

**14.** Verfahren nach Anspruch 13, wobei das organische Molekül durch ein Verdampfungsverfahren oder aus einer Lösung aufgebracht wird.

**Claims**

**1.** Organic molecule comprising

- a first chemical moiety comprising or consisting of a structure of formula A1

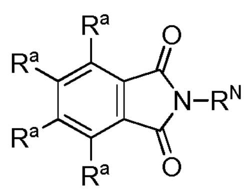

**Formula A1**

- at least one second chemical moiety comprising or consisting of a structure of formula D1

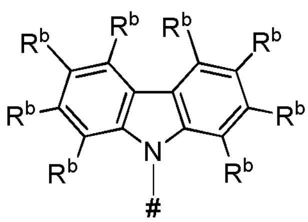

**Formula D1**

with

# = attachment point of the second chemical moiety to the first chemical moiety;
$R^N$ comprising or consisting of a structure of formula N1

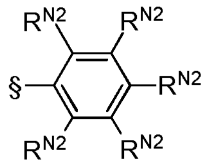

**Formula N1**

with

§ = attachment point of the chemical moiety according to formula N1 to the first chemical moiety;
$R^{N2}$ is at each occurrence the same or different and is H, phenyl or naphthyl;
$R^a$ is at each occurrence the same or different and is selected from the group consisting of an attachment point of the first chemical moiety to the second chemical moiety and H;
$R^b$ is at each occurrence independently of each other selected from the group consisting of H, deuterium, $CF_3$, $C(=O)R^1$, CN, carbazolyl which may be substituted by one or more $R^2$, $Si(R^4)_3$, an alkyl group which may be substituted by one or more $R^2$, an aryl group which may be substituted by one or more $R^2$ and a heteroaryl group which may be substituted by one or more $R^2$;
$R^1$ is at each occurrence independently of each other an aryl group which may be substituted by one or more $R^3$;
$R^2$ is at each occurrence independently of each other selected from the group consisting of $CF_3$, $C(=O)R^1$, CN, an alkyl group which may be substituted by one or more $R^3$, an aryl group which may be substituted by one or more $R^3$ and a heteroaryl group which may be substituted by one or more $R^3$;
$R^3$ is at each occurrence independently of each other selected from the group consisting of an unsubstituted alkyl group, an unsubstituted aryl group and an unsubstituted heteroaryl group;
$R^4$ at each occurrence independently of each other selected from the group consisting of an aryl group which may be substituted by one or more $R^3$ and a heteroaryl group which may be substituted by one or more $R^3$;
wherein, optionally, two or more of the substituents $R^1$, $R^2$ and $R^3$ form with each other a monocyclic, polycyclic, aliphatic, aromatic and/or benzo-fused ring system; wherein at least one and at most three $R^{N2}$ is phenyl or naphthyl.

2. Organic molecule according to claim 1, comprising a structure of formula A2

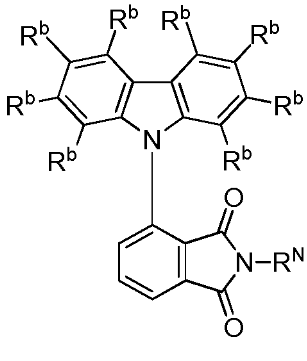

**Formula A2**

wherein the definitions of claim 1 apply.

3. Organic molecule according to claim 1 or 2, comprising a structure of formula A3

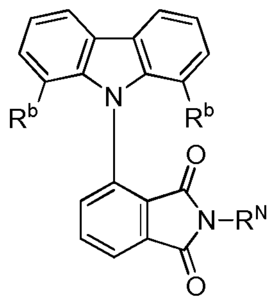

**Formula A3**

wherein the definitions of claim 1 apply.

4. Organic molecule according to claim 1 or 2, comprising a structure of formula A4

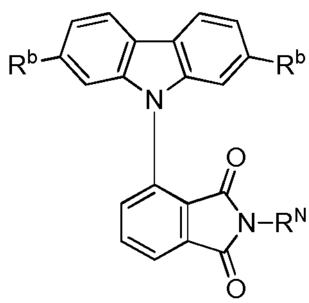

**Formula A4**

wherein the definitions of claim 1 apply.

5. Organic molecule according to claim 1 or 2, comprising a structure of formula A5

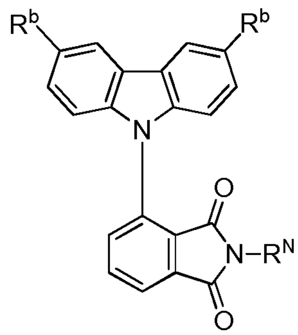

**Formula A5**

wherein the definitions of claim 1 apply.

6. Organic molecule according to claim 1, wherein $R^N$ is at each occurrence the same or different and is selected from any one of the structures 01 to 010:

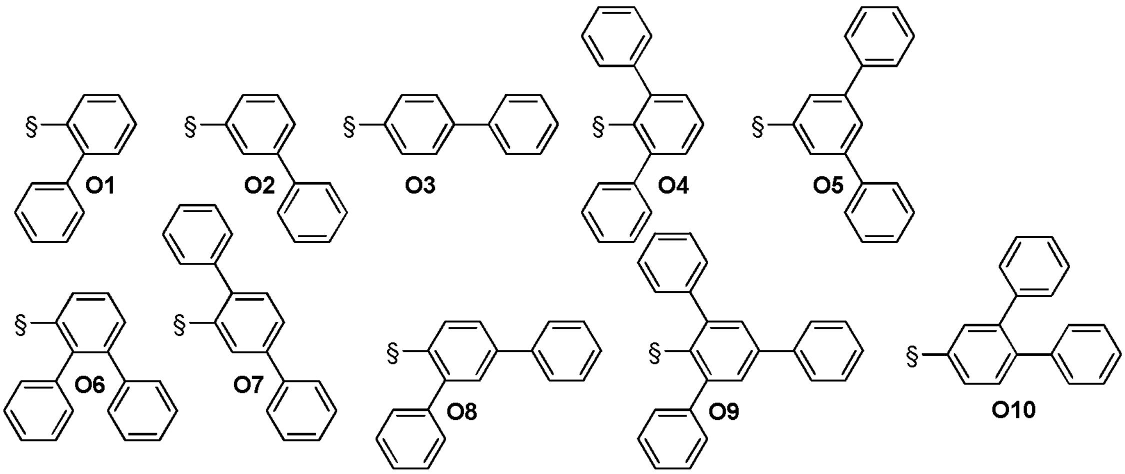

O1 O2 O3 O4 O5 O6 O7 O8 O9 O10

7. Organic molecule according to claim 1, wherein $R^N$ is at each occurrence the same or different and is selected from any one of the structures P1 to P3:

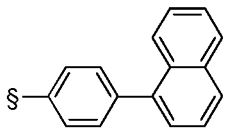
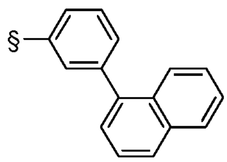
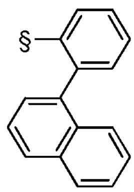

P1 P2 P3

8. Organic molecule according to claims 1 to 7, wherein $R^b$ is at each occurrence the same or different and is selected from the group consisting of H, deuterium, methyl, i-propyl, t-butyl, $CF_3$, CN, a phenyl group which may be substituted by one or more $R^6$, a pyridine group which may be substituted by one or more $R^6$, a pyrimidine group which may be substituted by one or more $R^6$, a pyrazine group which may be substituted by one or more $R^6$ and a triazine group which may be substituted by one or more $R^6$, wherein $R^6$ is at each occurrence the same or different and is selected from the group consisting of deuterium, methyl, i-propyl, t-butyl, $CF_3$, CN and a phenyl.

9. Use of an organic molecule according to claims 1 to 8 as an emitter and/or a host.

10. Composition, comprising or consisting of:

(a) at least one organic molecule according to any one of claims 1 to 8, in particular as an emitter and/or a host, and
(b) one or more emitter materials and/or host materials, which differ from the molecule according to any one of claims 1 to 8, and
(c) optionally, one or more dyes and/or one or more solvents.

11. Optoelectronic component, comprising an organic molecule according to claims 1 to 8 or a composition according to claim 10.

12. Optoelectronic component according to claim 11, comprising

- a substrate,
- an anode, and
- a cathode, wherein the anode or the cathode are disposed on the substrate, and
- at least one light-emitting layer, which is arranged between the anode and the cathode and which comprises the organic molecule according to claims 1 to 8 or a composition according to claim 10.

13. Method for producing an optoelectronic component, wherein an organic molecule according to claims 1 to 8 is used.

**14.** Method according to claim 13, wherein the organic molecule is applied by a vacuum evaporation method or from a solution.

## Revendications

**1.** Molécule organique, comprenant

- un premier motif chimique comprenant ou constitué d'une structure de formule A1

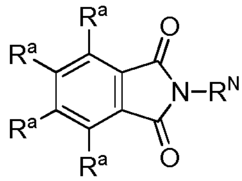

**formule A1**

- au moins un deuxième motif chimique comprenant ou constitué d'une structure de formule D1

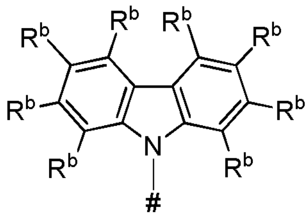

**formule D1**

avec

# = point de rattachement du deuxième motif chimique au premier motif chimique ;
$R^N$ comprenant une structure de formule N1 ou en étant constitué

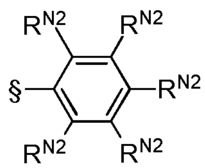

**formule N1**

avec

§ = point de rattachement du motif chimique selon la formule N1 au premier motif chimique ;
$R^{N2}$, identique ou différent en chaque occurrence, étant H, phényle ou naphtyle ;
$R^a$, identique ou différent en chaque occurrence, étant choisi dans le groupe constitué par un point de rattachement du premier motif chimique au deuxième motif chimique et H ; $R^b$ étant choisi en chaque occurrence indépendamment l'un de l'autre, dans le groupe constitué par H, deutérium, $CF_3$, $C(=O)R^1$, CN, carbazolyle non substitué ou substitué par un ou plusieurs $R^2$, $Si(R^4)_3$, un groupe alkyle non substitué ou substitué par un ou plusieurs $R^2$, un groupe aryle non substitué ou substitué par un ou plusieurs $R^2$ et un groupe hétéroaryle non substitué ou substitué par un ou plusieurs $R^2$ ;
$R^1$ étant indépendamment l'un de l'autre en chaque occurrence un groupe aryle non substitué ou substitué par un ou plusieurs $R^3$ ;
$R^2$ étant choisi en chaque occurrence indépendamment l'un de l'autre, dans le groupe constitué par $CF_3$, $C(=O)R^1$, CN, un groupe alkyle non substitué ou substitué par un ou plusieurs $R^3$, un groupe

aryle non substitué ou substitué par un ou plusieurs $R^3$ et un groupe hétéroaryle non substitué ou substitué par un ou plusieurs $R^3$ ;
$R^3$ étant choisi en chaque occurrence indépendamment l'un de l'autre, dans le groupe constitué par un groupe alkyle non substitué, un groupe aryle non substitué et un groupe hétéroaryle non substitué ;
$R^4$ étant choisi en chaque occurrence indépendamment l'un de l'autre, dans le groupe constitué par un groupe aryle non substitué ou substitué par un ou plusieurs $R^3$ et un groupe hétéroaryle non substitué ou substitué par un ou plusieurs $R^3$ ;
à cet égard, deux des substituants $R^1$, $R^2$ et $R^3$ ou plus formant éventuellement ensemble un système cyclique monocyclique, polycyclique, aliphatique, aromatique et/ou benzoannelé ;
au moins un et au maximum trois $R^{N2}$ étant phényle ou naphtyle.

**2.** Molécule organique selon la revendication 1 comprenant une structure de formule A2

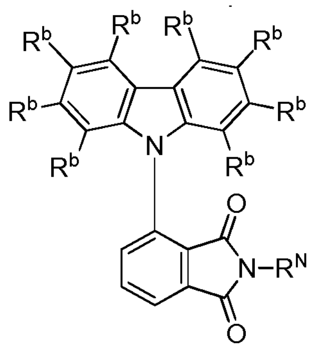

**formule A2**

les définitions mentionnées dans la revendication 1 s'appliquant.

**3.** Molécule organique selon les revendications 1 et 2, comprenant une structure de formule A3

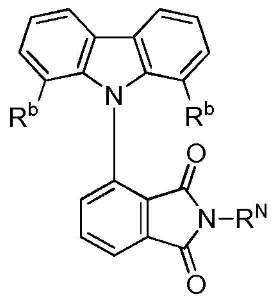

**formule A3**

les définitions mentionnées dans la revendication 1 s'appliquant.

**4.** Molécule organique selon les revendications 1 et 2, comprenant une structure de formule A4

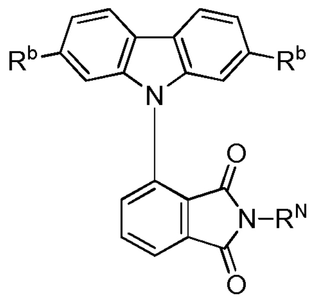

**formule A4**

les définitions mentionnées dans la revendication 1 s'appliquant.

5. Molécule organique selon les revendications 1 et 2, comprenant une structure de formule A5

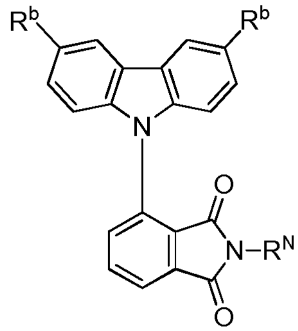

**formule A5**

les définitions mentionnées dans la revendication 1 s'appliquant.

6. Molécule organique selon la revendication 1, $R^N$, identique ou différent en chaque occurrence, étant choisi parmi l'une des structures 01 à O10 :

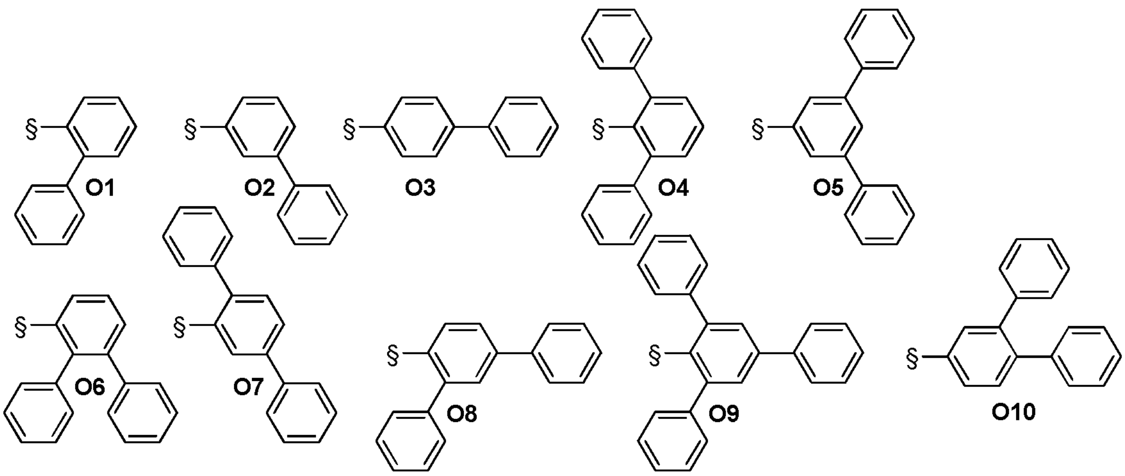

O1 O2 O3 O4 O5 O6 O7 O8 O9 O10

.

7. Molécule organique selon la revendication 1, $R^N$, identique ou différent en chaque occurrence, étant choisi parmi l'une des structures P1 à P3 :

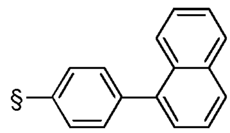

P1

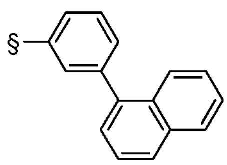

P2

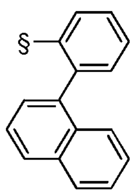

P3 .

8. Molécule organique selon les revendications 1 à 7, $R^b$, identique ou différent en chaque occurrence, étant choisi dans le groupe constitué par H, deutérium, méthyle, i-propyle, t-butyle, $CF_3$, CN, un groupe phényle non substitué ou substitué par un ou plusieurs $R^6$, un groupe pyridine non substitué ou substitué par un ou plusieurs $R^6$, un groupe pyrimidine non substitué ou substitué par un ou plusieurs $R^6$, un groupe pyrazine non substitué ou substitué par un ou plusieurs $R^6$ et un groupe triazine non substitué ou substitué par un ou plusieurs $R^6$,

$R^6$, identique ou différent en chaque occurrence, étant choisi dans le groupe constitué par deutérium, méthyle, i-propyle, t-butyle, $CF_3$, CN et phényle.

**9.** Utilisation d'une molécule organique selon les revendications 1 à 8 en tant qu'émetteur et/ou hôte.

**10.** Composition comprenant ou constituée de :

(a) au moins une molécule organique selon l'une quelconque des revendications 1 à 8, en particulier en tant qu'émetteur et/ou hôte, et
(b) un ou plusieurs matériaux émetteurs et/ou hôtes différents de la molécule selon l'une quelconque des revendications 1 à 8 et
(c) éventuellement un ou plusieurs colorants et/ou un ou plusieurs solvants.

**11.** Composant optoélectronique, comprenant une molécule organique selon les revendications 1 à 8 ou une composition selon la revendication 10.

**12.** Composant optoélectronique selon la revendication 11, présentant

- un substrat,
- une anode et
- une cathode, l'anode ou la cathode étant appliquée sur le substrat, et
- au moins une couche luminescente, qui est disposée entre l'anode et la cathode et qui présente la molécule organique selon les revendications 1 à 8 ou une composition selon la revendication 10.

**13.** Procédé pour la préparation d'un composant optoélectronique, une molécule organique selon les revendications 1 à 8 étant utilisée.

**14.** Procédé selon la revendication 13, la molécule organique étant appliquée par un procédé d'évaporation ou à partir d'une solution.

Figur 1

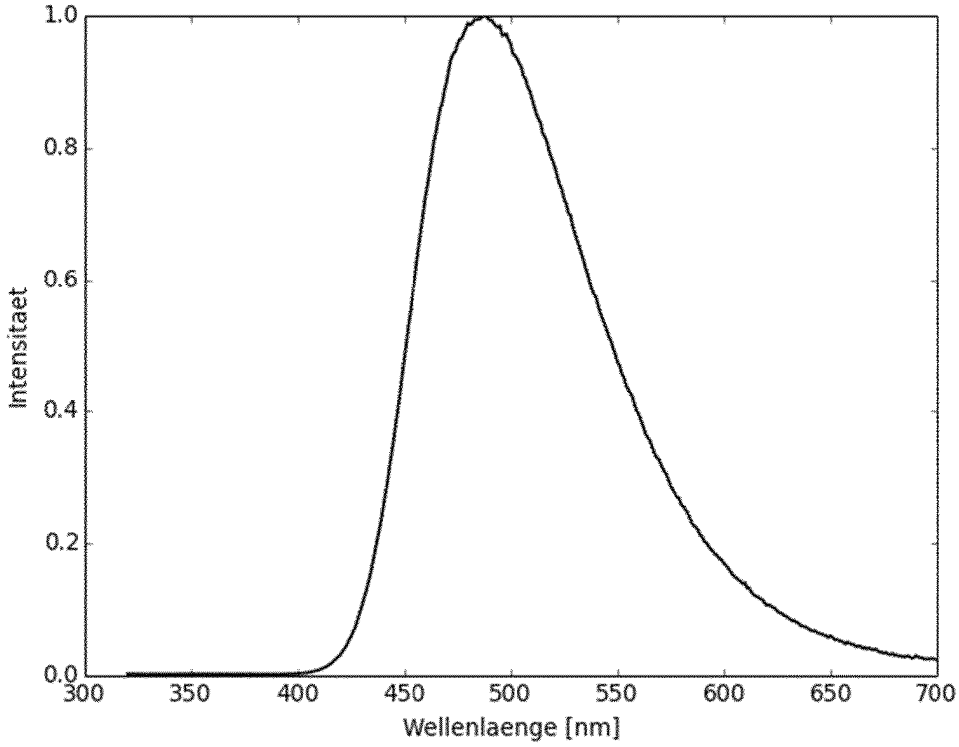


Figur 2

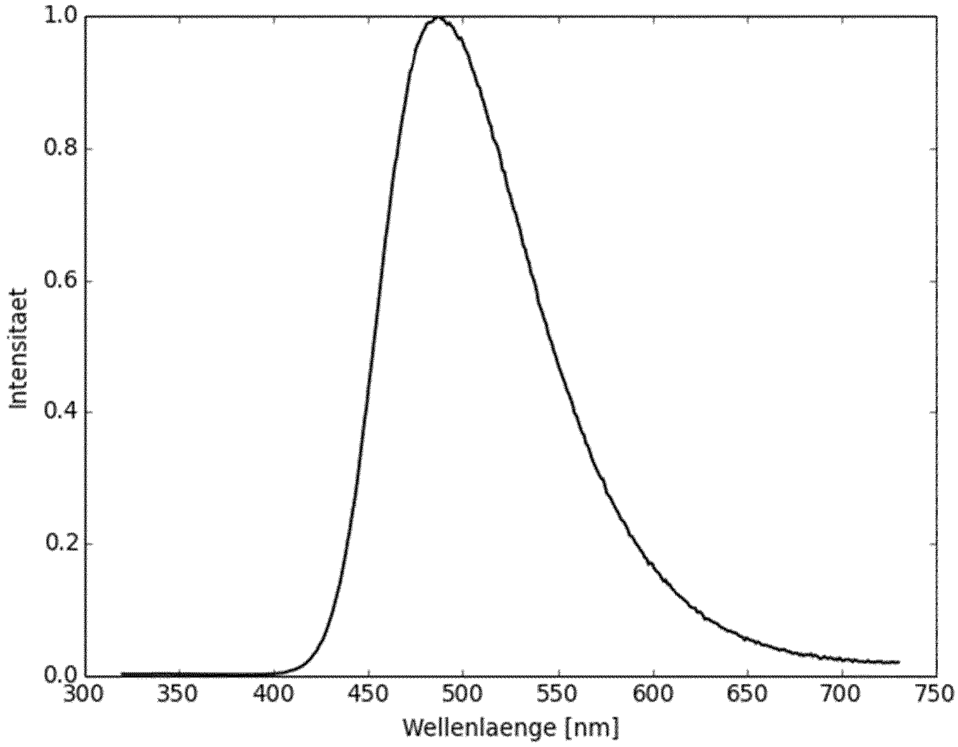

Figur 3

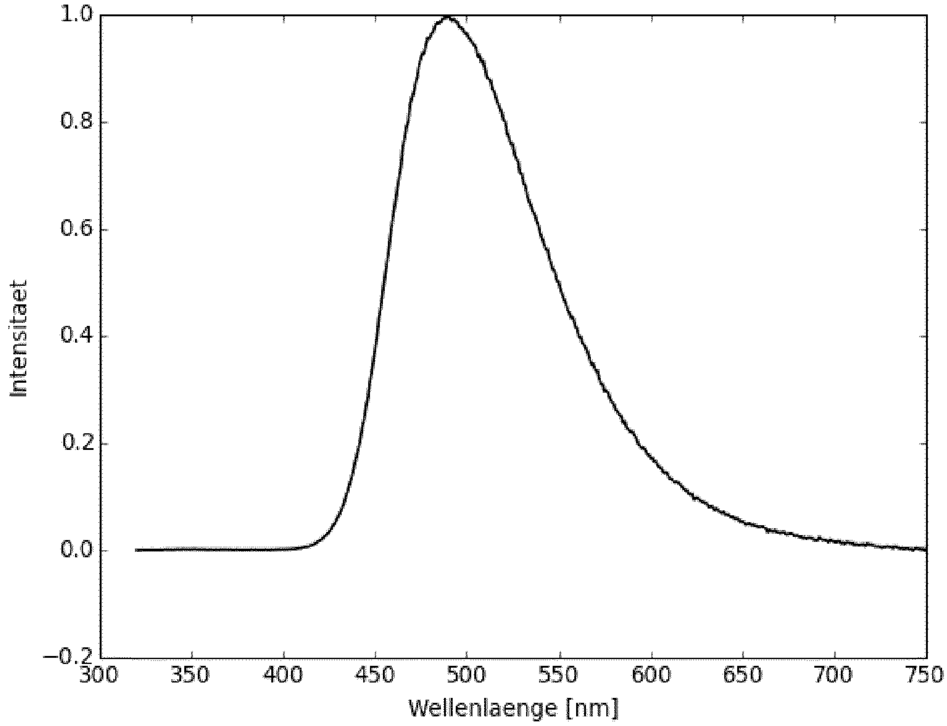


Figur 4

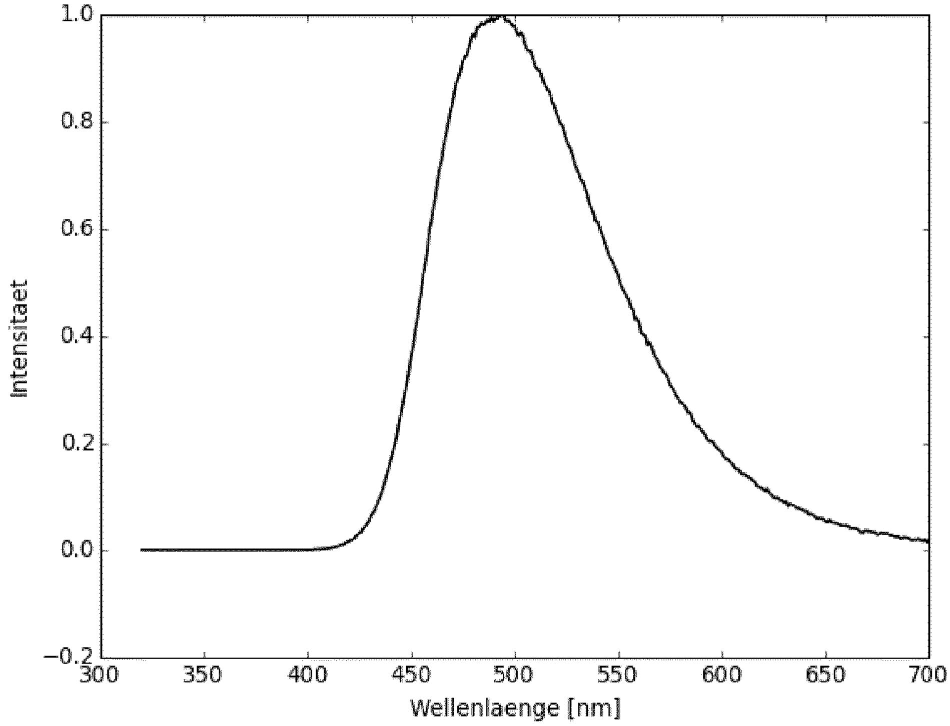

Figur 5

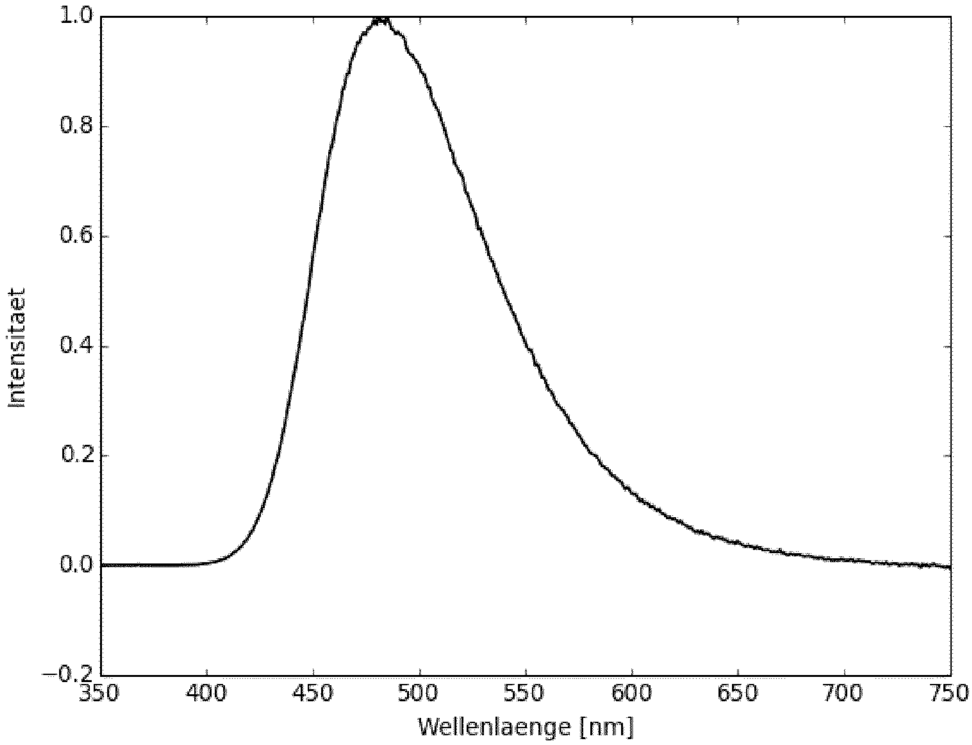
1.0
0.8
0.6
0.4
0.2
0.0
−0.2
Intensitaet
350
400
450
500
550
600
650
700
750
Wellenlaenge [nm]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010149748 A1, H. Yersin, U. Monkowius, T. Fischer, T. Hofbeck
- WO 2013161437 A1
- WO 2016042070 A


### In der Beschreibung aufgeführte Nicht-Patentliteratur


- **Q. ZHANG et al.** *J. Am. Chem. Soc.,* 2012, vol. 134, 14706
- **M.E. THOMPSON et al.** *Chem. Mater.,* 2004, vol. 16, 4743
- **BECKE, A. D.** *Phys. Rev. A,* 1988, vol. 38, 3098-3100
- **PERDEW, J. P.** *Phys. Rev. B,* 1986, vol. 33, 8822-8827
- **SIERKA, M. ; HOGEKAMP, A. ; AHLRICHS, R. J.** *Chem. Phys.,* 2003, vol. 118, 9136-9148
- **BECKE, A.D.** *J.Chem.Phys.,* 1993, vol. 98, 5648-5652
- **C; YANG, W ; PARR, R.G.** *Phys. Rev. B,* 1988, vol. 37, 785-789
- **LEE, C ; YANG, W ; PARR, R.G.** *Phys. Rev. B,* 1988, vol. 37, 785-789
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J.** *J.Phys.Chem.,* 1994, vol. 98, 11623-11627
- **WEIGEND, F. ; AHLRICHS, R.** *Phys. Chem. Chem. Phys.,* 2005, vol. 7, 3297-3305
- **RAPPOPORT, D. ; FURCHE, F.** *J. Chem. Phys.,* 2010, vol. 133, 134105-1, 134105-11